(19) Europäisches Patentamt
European Patent Office
Office européen des brevets

(11) Veröffentlichungsnummer : **0 093 922**
**B1**

(12) # EUROPÄISCHE PATENTSCHRIFT

(45) Veröffentlichungstag der Patentschrift : **30.12.86**

(51) Int. Cl.⁴ : **C 07 D265/18**, C 07 D413/12, C 07 D417/12, A 61 K 31/535

(21) Anmeldenummer : **83103980.5**

(22) Anmeldetag : **22.04.83**

(54) **Benzoxazin-2-one, deren Herstellung und diese Verbindungen enthaltende Arzneimittel.**

(30) Priorität : **06.05.82 DE 3217012**

(43) Veröffentlichungstag der Anmeldung : **16.11.83 Patentblatt 83/46**

(45) Bekanntmachung des Hinweises auf die Patenterteilung : **30.12.86 Patentblatt 86/52**

(84) Benannte Vertragsstaaten : **AT BE CH DE FR IT LI LU NL SE**

(56) Entgegenhaltungen :
**EP-A- 0 003 771**
**EP-A- 0 071 150**
**DE-A- 2 700 193**
**A. BURGER, Medicinal Chemistry, Part I, third Edition, 1951, Seite 77**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
**D-7950 Biberach (Riss) (DE)**

(72) Erfinder : **Narr, Berthold, Dr., Dipl.-Chem.**
**Obere Au 5**
**D-7950 Biberach 1 (DE)**
Erfinder : **Nickl, Josef, Dr., Dipl.-Chem.**
**Silcherstrasse 8**
**D-7950 Biberach 1 (DE)**
Erfinder : **Müller, Erich, Dr., Dipl.-Chem.**
**Talfeldstrasse 34**
**D-7950 Biberach (DE)**
Erfinder : **Haarmann, Walter, Dr.**
**Schlierholzweg 27**
**D-7950 Biberach 1 (DE)**
Erfinder : **Weisenberger, J. M., Dr., Dipl.-Chem.**
**Haydnweg 5**
**D-7950 Biberach 1 (DE)**
Erfinder : **Roch, Josef, Dr. Dipl.-Chem.**
**Stecherweg 19**
**D-7950 Biberach 1 (DE)**

**EP 0 093 922 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

In der EP-A-0.003.771 werden Carbostyril- und Oxindolderivate beschrieben, welche neben einer positiv inotropen Wirkung insbesondere antithrombotische Eigenschaften aufweisen.

Es wurde nun gefunden, daß die neuen Benzoxazin-2-one der allgemeinen Formel

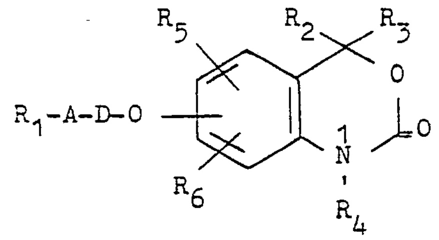

(I)

in der

A ein Schwefelatom, eine SO-, $SO_2$-, R—N=S- oder R—N=SO-Gruppe, wobei R ein Wasserstoftatom, eine geradkettige Alkanoylgruppe mit 1 bis 9 Kohlenstoffatomen, eine Pivaloylgruppe, eine gegebenenfalls durch ein Fluor-, Chlor- oder Bromatom, eine Methyl-, Äthyl-, Isopropyl-, tert.Butyl- oder Acetoxygruppe substituierte Benzoylgruppe, eine durch zwei Fluoratome, zwei Chloratome, zwei oder drei Methylgruppen substituierte Benzoylgruppe, eine gegebenenfalls durch eine Methylgruppe, ein Fluor-, Chlor- oder Bromatom substituierte Phenylsulfonylgruppe, eine Naphthoyl-, Thenoyl-, Pyridinoyl-, Hydroxysulfonyl-, Methansulfonyl-, Äthanolsulfonyl-, Pentamethyl-phenylsulfonyl-, Methoxycarbonyl-, Äthoxycarbonyl-, n-Propoxy-carbonyl-, Isopropoxycarbonyl-, Benzyloxycarbonyl-, Aminocarbonyl-, Methylaminocarbonyl- oder Dimethylaminocarbonylgruppe bedeutet,

D eine geradkettige oder verzweigte Alkylengruppe mit 2 bis 6 Kohlenstoffatomen,

$R_1$ eine gegebenenfalls durch eine Phenylgruppe substituierte Alkylgruppe mit 1 bis 3 Kohlenstoffatomen oder eine Phenylgruppe, wobei die vorstehend erwähnten Phenylkerne jeweils durch eine Alkylgruppe mit 1 bis 4 Kohlenstoffatomen, ein Halogenatom, eine Alkoxygruppe mit 1 bis 3 Kohlenstoffatomen, eine Hydroxy-, Cyclohexyl- oder Phenylgruppe, eine Aminogruppe oder eine Alkanoylaminogruppe mit 1 bis 3 Kohlenstoffatomen substituiert sein können, eine Alkylgruppe mit 4 bis 8 Kohlenstoffatomen, eine Cycloalkylgruppe mit 3 bis 7 Kohlenstoffatomen, eine durch Alkylgruppen mit 1 bis 4 Kohlenstoffatomen, Alkoxygruppen mit 1 bis 3 Kohlenstoffatomen und/oder Halogenatome di- oder trisubstituierte Phenyl- oder mono- oder disubstituierte Hydroxyphenyl- oder Aminophenylgruppe, wobei die Substituenten des Phenylkerns jeweils gleich oder verschieden sein können, eine gegebenenfalls durch eine oder zwei Methylgruppen substituierte Pyridyl- oder Pyrimidinylgruppe, eine Pyridyl-N-oxid-, Äthyl-pyridyl-, Äthyl-pyrimidinyl-, Propyl-pyrimidinyl-, Diäthyl-pyrimidinyl-, Pyrazinyl-, Pyridazinyl-, Pyrazolyl-, Imidazolyl-, Triazolyl-, Indolyl-, Indazolyl-, Chinolyl-, Isochinolyl-, Chinazolinyl-, Benzimidazolyl- oder Benzthiazolylgruppe,

$R_2$ und $R_3$, die gleich oder verschieden sein können, Wasserstoffatome, Phenylgruppen, Alkylgruppen mit 1 bis 6 Kohlenstoffatome oder Cycloalkylgruppen mit 3 bis 7 Kohlenstoffatomen,

$R_4$ ein Wasserstoffatom oder eine Alkylgruppe mit 1 bis 3 Kohlenstoffatomen,

$R_5$ ein Wasserstoff- oder Halogenatom, eine Nitro- oder Alkylgruppe mit 1 bis 3 Kohlenstoffatomen und

$R_6$ ein Wasserstoff- oder Halogenatom oder eine Alkylgruppe mit 1 bis 3 Kohlenstoffatomen bedeuten, wertvolle pharmakologische Eigenschaften aufweisen, insbesondere überlegene antithrombotische Wirkungen.

Gegenstand der vorliegenden Erfindung sind somit die neuen Benzoxazin-2-one der obigen allgemeinen Formel I, diese Verbindungen enthaltende Arzneimittel und Verfahren zu ihrer Herstellung.

Unter den bei der Definition des Restes $R_1$ erwähnten Halogenatomen ist insbesondere ein Fluor-, Chlor- oder Bromatom zu verstehen.

Für die bei der Definition der Reste A, D und $R_1$ bis $R_6$ eingangs erwähnten Bedeutungen kommt beispielsweise

für A die Bedeutung des Schwefelatoms, der Sulfoxid-, Sulfonyl-, Sulfimino-, N-Formyl-sulfimino-, N-Acetyl-sulfimino-, N-Propionyl-sulfimino-, N-Pivaloyl-sulfimino-, N-Pentanoyl-sulfimino-, N-Hexanoylsulfimino-, N-Heptanoyl-sulfimino-, N-Octanoyl-sulfimino-, N-Nonanoyl-sulfimino-, N-Methoxyacetyl-sulfimino-, N-Methoxypropionyl-sulfimino-, N-Benzoyl-sulfimino-, N-Fluorbenzoyl-sulfimino-, N-Chlorbenzoylsulfimino-, N-Brombenzoyl-sulfimino-, N-Methylbenzoyl-sulfimino-, N-Äthyl-benzoyl-sulfimino-, N-Isopropylbenzoyl-sulfimino-, N-tert.Butylbenzoyl-sulfimino-, N-Difluorbenzoyl-sulfimino-, N-Dichlorbenzoylsulfimino-, N-Dimethylbenzoyl-sulfimino-, N-Trimethylbenzoyl-sulfimino-, N-Naphthoyl-sulfimino-, N-Pyridinoyl-sulfimino-, N-Thenoyl-sulfimino-, N-Acetoxybenzoyl-sulfimino-, N-Hydroxysulfonyl-sulfimino-, N-Methansulfonyl-sulfimino-, N-Äthansulfonyl-sulfimino-, N-Phenylsulfonyl-sulfimino-, N-Methyl-phenylsulfonyl-sulfimino-, N-Fluorphenylsulfonyl-sulfimino-, N-Chlorphenylsulfonyl-sulfimino-, N-Bromphenylsulfonyl-sulfimino-, N-Pentamethylphenylsulfonyl-sulfimino-, N-Naphthylsulfonyl-sulfimino-, N-Methoxycarbonyl-sulfimino-, N-Äthoxycarbonyl-sulfimino-, N-Propoxycarbonyl-sulfimino-, N-Isopropoxycarbonyl-sulfimino-, N-Benzyloxycarbonyl-sulfimino-, N-Aminocarbonyl-sulfimino-, N-Methylaminocarbonylsulfimino-, N-Dimethylaminocarbonyl-sulfimino-, Sulfoximino-, N-Formyl-sulfoximino-, N-Acetyl-sulfoximino, N-Propionyl-sulfoximino-, N-Pivaloyl-sulfoximino-, N-Pentanoyl-sulfoximino-, N-Hexanoyl-sulfoximino-, N-Heptanoyl-sulfoximino-, N-Nonanoyl-sulfoximino-, N-Methoxyacetyl-sulfoximino-, N-Methoxypropyionyl-sulfoximino-, N-Benzoyl-sulfoximino-, N-Fluorbenzoyl-sulfoximino-, N-Chlorbenzoyl-sulfoximino-, N-Brombenzoyl-sulfoximino-, N-Methylbenzoyl-sulfoximino-, N-Äthylbenzoyl-sulfoximino-, N-Isopropylbenzoyl-sulfoximino-, N-tert.Butylbenzoyl-sulfoximino-, N-Difluorbenzoyl-sulfoximino-, N-Dichlorbenzoyl-sulfoximino-, N-Dimethylbenzoyl-sulfoximino-, N-Trimethylbenzoyl-sulfoximino-, N-Naphthoyl-sulfoximino-, N-Pyridinoyl-sulfoximino-, N-Thenoyl-sulfoximino-, N-Acetoxybenzoyl-sulfoximino-, N-Hydroxysulfoximino-, N-Methansulfonyl-sulfoximino-, N-Äthan-sulfonyl-sulfoximino-, N-Phenylsulfonylsulfoximino-, N-Methylphenylsulfonyl-sulfoximino-, N-Fluorphenylsulfonyl-sulfoximino-, N-Chlorphenylsulfonyl-sulfoximino-, N-Bromphenyl-sulfonyl-sulfoximino-, N-Pentamethylphenyl-sulfoximino-, N-Naphthylsulfonyl-sulfoximino-, N-Methoxy-N-Methoxycarbonyl-sulfoximino-, N-Äthoxycarbonyl-sulfoximino-, N-Propoxycarbonyl-sulfoximino-, N-Isopropoxycarbonyl-sulfoximino-, N-Benzyloxycarbonyl-sulfoximino-, N-Aminocarbonyl-sulfoximino-, N-Methylaminocarbonyl-sulfoximino- oder N-Dimethylaminocarbonyl-sulfoximinogruppe,

für D die der Äthylen-, n-Propylen-, n-Butylen-, n-Pentylen-, n-Hexylen-, 1-Methyl-äthylen-, 2-Methyl-äthylen-, 1-Methyl-n-propylen-, 2-Methyl-n-propylen-, 3-Methyl-n-propylen-, 1-Methyl-n-butylen-, 2-Methyl-n-butylen-, 3-Methyl-n-butylen-, 4-Methyl-n-butylen-, 1-Methyl-n-pentylen-, 2-Methyl-n-pentylen-, 3-Methyl-n-pentylen-, 4-Methyl-n-pentylen-, 5-Methyl-n-pentylen-, 1,1-Dimethyl-äthylen-, 1,2-Dimethyl-äthylen-, 2,2-Dimethyl-äthylen-, 1,1-Dimethyl-n-propylen-, 2,2-Dimethyl-n-propylen-, 3,3-Dimethyl-n-propylen-, 1,2-Dimethyl-n-propylen-, 1,3-Dimethyl-n-propylen-, 1,1-Dimethyl-n-butylen-, 2,2-Dimethyl-n-butylen-, 3,3-Dimethyl-n-butylen-, 4,4-Dimethyl-n-butylen-, 1,2-Dimethyl-n-butylen-, 1,3-Dimethyl-n-butylen-, 1,4-Dimethyl-n-butylen-, 2,3-Dimethyl-n-butylen-, 1-Äthyl-äthylen-, 2-Äthyl-äthylen-, 1-Äthyl-n-propylen-, 2-Äthyl-n-propylen-, 3-Äthyl-n-propylen-, 1-Äthyl-n-butylen-, 2-Äthyl-n-butylen-, 3-Äthyl-n-butylen-, 4-Äthyl-n-butylen-, 1-Methyl-2-äthyl-äthylen-, 1-Methyl-2-äthyl-n-propylen-, 1-Methyl-3-äthyl-n-propylen-, 1-Methyl-2-propyl-äthylen-, 1-Propyl-äthylen-, 1-Butyl-äthylen- oder 1-Propyl-n-propylengruppe,

für $R_1$ die der Methyl-, Äthyl-, n-Propyl-, Isopropyl-, n-Butyl-, 1-Methyl-propyl-, 2-Methyl-propyl-, tert.-Butyl-, n-Pentyl-, 1-Methyl-n-butyl-, 2-Methyl-n-butyl-, 3-Methyl-n-butyl-, 1-Äthyl-n-propyl-, tert.Pentyl-, n-Hexyl-, n-Heptyl-, n-Octyl-, Benzyl-, 1-Phenyläthyl-, 2-Phenyläthyl-, 1-Phenylpropyl-, 3-Phenylpropyl-, Fluorbenzyl-, Chlorbenzyl-, Brombenzyl-, Methylbenzyl-, Isopropylbenzyl-, Methoxybenzyl-, Äthoxybenzyl-, Cyclopropyl-, Cyclobutyl-, Cyclopentyl-, Cyclohexyl-, Cycloheptyl-, Phenyl-, Fluorphenyl-, Chlorphenyl-, Bromphenyl-, Methylphenyl-, Isopropylphenyl-, tert.Butylphenyl-, Hydroxyphenyl-, Methoxyphenyl-, Äthoxyphenyl-, n-Propoxyphenyl-, Formylaminophenyl-, Acetylaminophenyl-, Propionylaminophenyl-, Cyclohexylphenyl-, Biphenylyl-, Difluorphenyl-, Dichlorphenyl-, Dibromphenyl-, Dimethoxyphenyl-, Methoxy-chlorphenyl-, Methoxy-bromphenyl-, Dimethylphenyl-, Methyl-äthylphenyl-, Diäthylphenyl-, Methyl-tert.butylphenyl-, Methyl-chlorphenyl-, Methyl-bromphenyl-, tert.Butyl-bromphenyl-, Trimethylphenyl-, Dichlor-aminophenyl-, Dibrom-aminophenyl-, Dimethyl-aminophenyl-, Dichlor-hydroxyphenyl-, Dibrom-hydroxyphenyl-, Dimethyl-hydroxyphenyl-, Di-tert.butyl-hydroxyphenyl-, Trimethoxyphenyl-, Pyridyl-, Pyridyl-N-oxid-, Methyl-pyridyl-, Äthyl-pyridyl-, Dimethyl-pyridyl-, Pyrimidinyl-, Methyl-pyrimidinyl-, Äthyl-pyrimidinyl-, Propyl-pyrimidinyl-, Dimethyl-pyrimidinyl-, Diäthyl-pyrimidinyl-, Pyrazinyl-, Pyridazinyl-, Pyrazolyl-, Imidazolyl-, Triazolyl-, Indolyl-, Indazolyl-, Chinolyl-, Isochinolyl-, Chinazolinyl-, Benzimidazolyl- oder Benzthiazolylgruppe,

für $R_2$ und $R_3$, die gleich oder verschieden sein können, jeweils die des Wasserstoffatoms, der Methyl-, Äthyl-, n-Propyl-, Isopropyl-, n-Butyl-, 1-Methyl-n-propyl-, 2-Methyl-n-propyl-, tert.Butyl-, n-Pentyl-, 1-Methyl-n-butyl-, 2-Methyl-n-butyl-, 3-Methyl-n-butyl-, 1-Äthyl-n-propyl-, tert.Pentyl-, n-Hexyl-, Phenyl-, Cyclopropyl-, Cyclobutyl-, Cyclopentyl-, Cyclohexyl- oder Cycloheptylgruppe,

für $R_4$ die des Wasserstoffatoms, der Methyl-, Äthyl-, n-Propyl- oder Isopropylgruppe,

für $R_5$ die des Wasserstoff-, Fluor-, Chlor-, Brom- oder Jodatoms, der Nitro-, Methyl-, Äthyl-, n-Propyl- oder Isopropylgruppe und

für $R_6$ die des Wasserstoff-, Fluor-, Chlor- oder Bromatoms, der Methyl-, Äthyl-, n-Propyl- oder Isopropylgruppe in Betracht.

Bevorzugte Verbindungen der obigen allgemeinen Formel I sind diejenigen, in denen

A ein Schwefelatom, eine SO-, $SO_2$-, R—N=S- oder R—N=SO-Gruppe, wobei R ein Wasserstoffatom, einen gegebenenfalls durch eine Methylgruppe substituierten Benzoyl- oder Phenylsulfonylrest, eine Acetyl- oder Propionylgruppe darstellt,

D eine geradkettige Alkylengruppe mit 2 bis 6 Kohlenstoffatomen,

$R_1$ eine Alkylgruppe mit 1 bis 8 Kohlenstoffatomen, eine Cyclohexyl-, Benzyl-, Pyridyl-, Pyridyl-N-oxid-, 2-Benzthiazolyl- oder 1,2,4-Triazol-3-ylgruppe, eine gegebenenfalls durch eine oder zwei Methylgruppen substituierte 2-Pyrimidinylgruppe, eine gegebenenfalls durch eine Alkylgruppe mit 1 bis 4 Kohlenstoffatomen, eine Hydroxy-, Methoxy-, Cyclohexyl-, Phenyl- oder Acetylaminogruppe, ein Fluor-, Chlor- oder Bromatom substituierte Phenylgruppe, eine durch Chlor- oder Bromatome, Methyl- oder Methoxygruppen disubstituierte Phenylgruppe, wobei die Substituenten des Phenylkerns gleich oder verschieden sein können, oder eine durch zwei Alkylgruppen mit jeweils 1 bis 4 Kohlenstoffatomen, zwei Chlor- oder Bromatome substituierte Aminophenyl- oder Hydroxyphenylgruppe,

$R_2$ und $R_3$, die gleich oder verschieden sein können, Wasserstoffatome, Alkylgruppen mit 1 bis 6 Kohlenstoffatomen, Phenyl- oder Cyclohexylgruppen,

$R_4$ ein Wasserstoffatom oder eine Methylgruppe,

$R_5$ ein Wasserstoff-, Fluor-, Chlor- oder Bromatom, eine Nitro-, Methyl- oder Äthylgruppe und

$R_6$ ein Wasserstoff-, Chlor- oder Bromatom, eine Methyl- oder Äthylgruppe bedeuten, insbesondere jedoch die Verbindungen, in denen der Rest —O—D—A—$R_1$ in 6-Stellung steht.

Besonders bevorzugte Verbindungen sind die Verbindungen der allgemeinen Formel

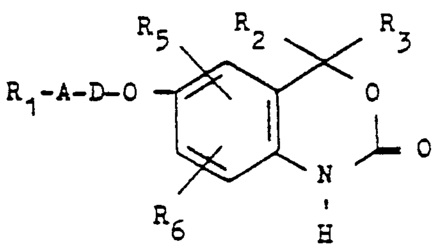

(Ia)

in der

A die SO-, $SO_2$-, H—N=SO-, $CH_3CO$—N=SO- und $CH_3$—$C_6H_4$—$SO_2$—N=SO-Gruppe,

D die n-Butylengruppe,

$R_1$ eine gegebenenfalls durch ein Fluor-, Chlor- oder Bromatom, eine Methyl-, Hydroxy-, Methoxy-, Cyclohexyl-, Phenyl- oder Acetaminogruppe substituierte Phenylgruppe, eine durch Chlor- oder Bromatome, Methyl- oder Methoxygruppen disubstituierte Phenylgruppe, wobei die Substituenten des Phenylkerns gleich oder verschieden sein können, eine 4-Amino-3,5-dibrom-phenyl-, eine 3,5-Di-tert.butyl-4-hydroxy-phenyl- oder Pyridylgruppe,

$R_2$ und $R_3$, die gleich oder verschieden sein können, je ein Wasserstoffatom oder eine Methylgruppe,

$R_5$ ein Wasserstoff-, Chlor- oder Bromatom, eine Nitro- oder eine Methylgruppe und

$R_6$ ein Wasserstoff-, Chlor- oder Bromatom oder eine Methylgruppe bedeuten.

Erfindungsgemäß erhält man die neuen Verbindungen nach folgenden Verfahren :

a) Umsetzung einer Hydroxyverbindung der allgemeinen Formel

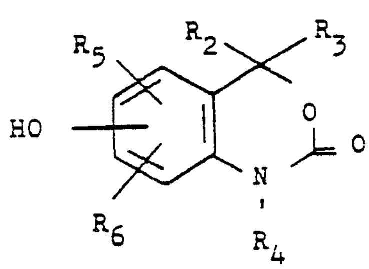

(II)

in der $R_2$ bis $R_6$ wie eingangs definiert sind, oder deren Salze mit anorganischen oder tertiären organischen Basen mit einer Verbindung der allgemeinen Formel

$$Z—D—A—R_1, \qquad (III)$$

in der

A, D und $R_1$ wie eingangs definiert sind und

Z eine nukleophil austauschbare Gruppe wie ein Halogenatom oder einen Sulfonsäureesterrest, z. B. ein Chlor-, Brom- oder Jodatom, eine p-Toluolsulfonyloxy- oder Methansulfonyloxygruppe, darstellen.

Die Umsetzung wird zweckmäßigerweise in einem geeigneten Lösungsmittel oder Lösungsmittelgemisch wie Methanol, Äthanol, Dioxan, Tetrahydrofuran, Chloroform oder Toluol, vorzugsweise jedoch in einem wasserfreien aprotischen Lösungsmittel wie Aceton, Dimethylformamid oder Dimethylsulfoxid, gegebenenfalls in Gegenwart einer Alkalibase oder eines Alkoholats wie Natriumcarbonat, Kaliumcarbonat, Natriumhydroxid oder Natriumäthylat bei Temperaturen zwischen 0 °C und der Siedetemperatur des verwendeten Lösungsmittels, z. B. bei Temperaturen zwischen 0 und 100 °C, vorzugsweise jedoch bei Temperaturen zwischen 10 und 80 °C, durchgeführt. Die Umsetzung kann jedoch auch ohne Lösungsmittel durchgeführt werden.

b) Zur Herstellung von Verbindungen der allgemeinen Formel I, in der A eine SO-, $SO_2$- oder R—N=SO-Gruppe darstellt :

Oxidation einer Verbindung der allgemeinen Formel

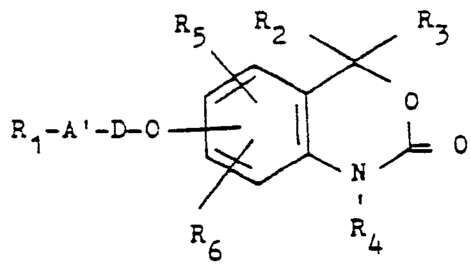

(IV)

in der

$R_1$ bis $R_6$ und D wie eingangs definiert sind und

A' ein Schwefelatom, eine SO- oder R—N=S-Gruppe darstellt, wobei R wie eingangs definiert ist.

Die Oxidation wird vorzugsweise in einem Lösungsmittel oder Lösungsmittelgemisch, z. B. in Wasser, Wasser/Pyridin, Äthanol, Methanol, Aceton, Eisessig, Ameisensäure, verdünnter Schwefelsäure oder Trifluoressigsäure, je nach dem verwendeten Oxidationsmittel zweckmäßigerweise bei Temperaturen zwischen —80 und 100 °C durchgeführt.

Zur Herstellung von Verbindungen der allgemeinen Formel I, in der A die SO- oder R—N=SO-Gruppe darstellt, wird die Oxidation zweckmäßigerweise mit einem Äquivalent des verwendeten Oxidationsmittels durchgeführt, z. B. mit Wasserstoffperoxid in Eisessig oder Ameisensäure bei 0 bis 20 °C oder in Aceton bei 0 bis 60 °C, mit einer Persäure wie Perameisensäure oder m-Chlor-perbenzoesäure in Eisessig oder Trifluoressigsäure bei 0 bis 20 °C, mit Natriummetaperjodat in wäßrigem Methanol oder Äthanol bei 15 bis 25 °C, mit N-Brom-succinimid in Äthanol bei 10 bis 50 °C, mit tert.Butyl-hypochlorit in Methanol bei —80 bis —30 °C, mit Jodbenzoldichlorid in wäßrigem Pyridin bei 0 bis 20 °C, mit Chromsäure in Eisessig oder in Aceton bei 0 bis 20 °C und mit Sulfurylchlorid in Methylenchlorid bei —70 °C, der hierbei erhaltene Thioäther-Chlor-Komplex wird zweckmäßigerweise mit wäßrigem Äthanol hydrolysiert.

Zur Herstellung von Verbindungen der allgemeinen Formel I, in der A die $SO_2$-Gruppe darstellt, wird die Oxidation zweckmäßigerweise mit einem bzw. mit zwei oder mehr Äquivalenten des verwendeten Oxidationsmittels durchgeführt, z. B. mit Wasserstoffperoxid in Eisessig oder in Ameisensäure bei 20 bis 100 °C oder in Aceton bei 0 bis 60 °C, mit einer Persäure wie Perameisensäure oder m-Chlorperbenzoesäure in Eisessig, Trifluoressigsäure oder Chloroform bei Temperaturen zwischen 0 und 50 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C. Bedeutet somit in einer Verbindung der obigen allgemeinen Formel IV A das Schwefelatom, so wird die Umsetzung vorzugsweise mit zwei oder mehr Äquivalenten des betreffenden Oxidationsmittels und ganz entsprechend mit mindestens einem Äquivalent durchgeführt, falls A die SO-Gruppe bedeutet.

c) Zur Herstellung von Verbindungen der allgemeinen Formel I, in der A ein Schwefelatom oder eine $SO_2$-Gruppe darstellt :

Umsetzung einer Verbindung der allgemeinen Formel

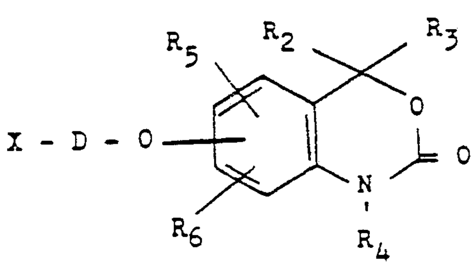

(V)

in der

D und $R_2$ bis $R_6$ wie eingangs definiert sind und

X eine nukleophil austauschbare Gruppe wie ein Halogenatom oder einen Sulfonsäureesterrest, z. B. ein Chlor-, Brom- oder Jodatom, eine p-Toluolsulfonyloxy- oder Methansulfonyloxygruppe darstellt, mit einer Verbindung der allgemeinen Formel

$$Y{-}R_1, \qquad (VI)$$

in der

$R_1$ wie eingangs definiert ist und

Y eine $MeSO_2$-Gruppe, wobei Me ein Alkali- oder Erdalkali/$_2$-Metallatom wie das Natrium-, Kalium- oder Calcium/$_2$-Atom darstellt, oder die Mercaptogruppe bedeutet.

Die Umsetzung wird zweckmäßigerweise in einem geeigneten Lösungmittel oder Lösungsmittelgemisch wie Dioxan, Tetrahydrofuran, Chloroform oder Toluol, vorzugsweise jedoch in einem wasserfreien aprotischen Lösungsmittel wie Aceton, Dimethylformamid oder Dimethylsulfoxid, gegebenenfalls in Gegenwart einer Alkalibase wie Natriumcarbonat, Kaliumcarbonat oder Natriumhydroxid bei Temperaturen zwischen 0 °C und der Siedetemperatur des verwendeten Lösungsmittels, z. B. bei Temperaturen zwischen 0 und 100 °C, vorzugsweise jedoch bei Temperaturen zwischen 10 und 50 °C, durchgeführt. Die Umsetzung kann jedoch auch ohne Lösungsmittel durchgeführt werden.

d) Zur Herstellung von Verbindungen der allgemeinen Formel I, in der A die H—N=SO-Gruppe darstellt:

Umsetzung eines Sulfoxids der allgemeinen Formel

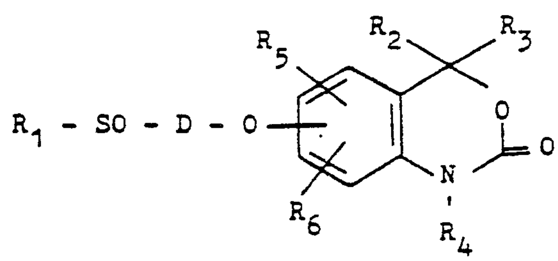

(VII)

in der D und $R_1$ bis $R_6$ wie eingangs definiert sind, mit gegebenenfalls im Reaktionsgemisch gebildeter Stickstoffwasserstoffsäure.

Die Umsetzung wird zweckmäßigerweise in einem Lösungsmittel oder Lösungsmittelgemisch wie Methylenchlorid, Dimethylformamid oder Tetrahydrofuran bei Temperaturen zwischen 0 und 40 °C, vorzugsweise bei Temperaturen zwischen 10 und 35 °C, durchgeführt. Besonders vorteilhaft wird die Umsetzung mit einem Alkaliazid, z. B. Natriumazid, und Polyphosphorsäure als Lösungsmittel durchgeführt.

e) Zur Herstellung von Verbindungen der allgemeinen der Formel I, in der A die H—N=SO-Gruppe darstellt:

Umsetzung eines Sulfoxids der allgemeinen Formel

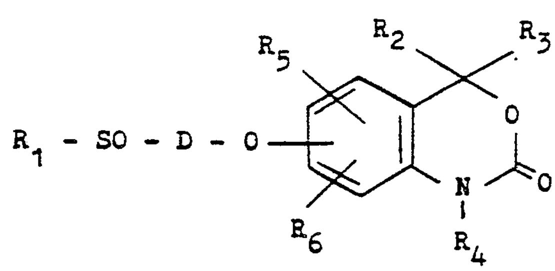

(VII)

in der D und $R_1$ bis $R_6$ wie eingangs definiert sind, mit einer Verbindung der allgemeinen Formel

$$H_2N{-}O{-}X{-}R_7, \qquad (VIII)$$

in der

X eine Carbonyl- oder Sulfonylgruppe und

$R_7$ eine in o-Stellung disubstituierte Arylgruppe wie eine 2,4,6-Trimethylphenyl- oder 2,4,6-Triisopropylphenylgruppe darstellen.

Die Umsetzung wird zweckmäßigerweise in einem Lösungsmittel oder Lösungsmittelgemisch wie Methlenchlorid, Chloroform, Dimethylformamid, Tetrahydrofuran oder Dioxan bei Temperaturen zwischen 0 und 50 °C, vorzugsweise jedoch bei Temperaturen zwischen 5 und 40 °C, und gegebenenfalls in Gegenwart einer katalytischen Menge einer Säure wie p-Toluolsulfonsäure durchgeführt. Besonders vorteilhaft wird die Umsetzung jedoch in der Weise durchgeführt, daß eine Verbindung der allgemeinen Formel VIII ohne ihre vorherige Isolierung eingesetzt bzw. im Reaktionsgemisch hergestellt wird.

f) Zur Herstellung einer Verbindung der allgemeinen Formel I, in der R kein Wasserstoffatom darstellt :

Acylierung einer Verbindung der allgemeinen Formel

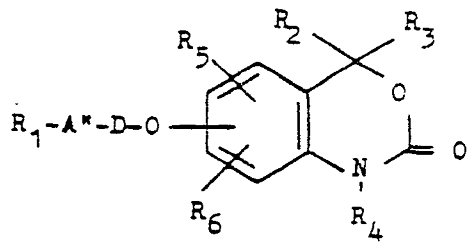

(IX)

in der

D und $R_1$ bis $R_6$ wie eingangs definiert sind und

$A''$ die H—N=S- oder H—N=SO-Gruppe darstellt.

Die Umsetzung wird zweckmäßigerweise in einem Lösungsmittel oder Lösungmittelgemisch wie Wasser, Methylenchlorid, Chloroform, Äther, Tetrahydrofuran, Dioxan oder Dimethylformamid mit einem entsprechenden Acylierungsmittel, z. B. mit einer Säure in Gegenwart eines die Säure aktivierenden oder wasserentziehenden Mittels wie Thionylchlorid, mit deren Anhydriden wie Essigsäureanhydrid, mit deren Estern wie p-Toluolsulfonsäureäthylester oder Kohlensäurediäthylester, mit deren Halogeniden wie Acetylchlorid, Chlorameisensäureäthylester oder p-Toluolsulfonsäurechlorid oder mit einem entsprechenden Isocyanat, wobei diese gegebenenfalls auch als Lösungsmittel dienen können, gegebenenfalls in Gegenwart einer anorganischen oder tertiären organischen Base, wie Natriumhydroxid, Kaliumcarbonat, Triäthylamin oder Pyridin, wobei die letzteren gleichzeitig auch als Lösungsmittel dienen können, bei Temperaturen zwischen —25 und 100 °C, vorzugsweise jedoch bei Temperaturen zwischen —10 und 80 °C, durchgeführt.

g) Zur Herstellung einer Verbindung der allgemeinen Formel I, in der A die R—N=S-Gruppe darstellt :

Umsetzung eines Thioäthers der allgemeinen Formel

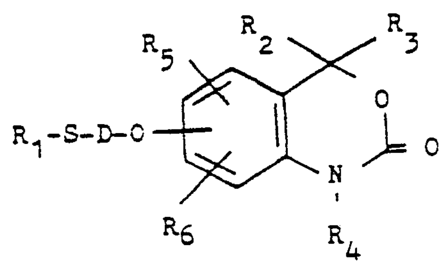

(X)

in der $R_1$ bis $R_6$ und D wie eingangs definiert sind, mit einem Halogenamid der allgemeinen Formel

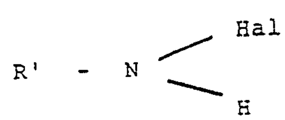

(XI)

in der

R′ mit Ausnahme des Wasserstoffatoms die für R eingangs erwähnten Bedeutungen besitzt und

Hal ein Chlor- oder Bromatom darstellt, oder mit dessen Alkalisalz und gegebenenfalls anschließende Hydrolyse.

Die Umsetzung wird vorzugsweise mit einem Alkalisalz einer Verbindung der allgemeinen Formel XI, z. B. dem Natriumsalz, gegebenenfalls in Gegenwart einer anorganischen Base wie einer Alkalibase in einem Lösungsmittel oder Lösungsmittelgemisch wie Methanol, Methanol/Wasser oder Äthanol, zweckmäßigerweise bei Temperaturen zwischen 0 und 80 °C, vorzugsweise jedoch bei Temperaturen zwischen 5 und 50 °C, durchgeführt.

Die gegebenenfalls anschließende Hydrolyse wird in Gegenwart einer Säure oder Base, vorzugsweise jedoch in Gegenwart einer Base wie Natriumhydroxid, in einem Lösungsmittel oder Lösungsmittelgemisch wie Wasser, Methanol, Methanol/Wasser oder Tetrahydrofuran/Wasser bei Temperaturen bis zur Siedetemperatur des verwendeten Lösungsmittels durchgeführt.

h) Zur Herstellung einer Verbindung der allgemeinen Formel I, in der A die H—N=S- oder H—N=SO-Gruppe darstellt:

Hydrolytische Abspaltung eines Acylrestes von einer Verbindung der allgemeinen Formel

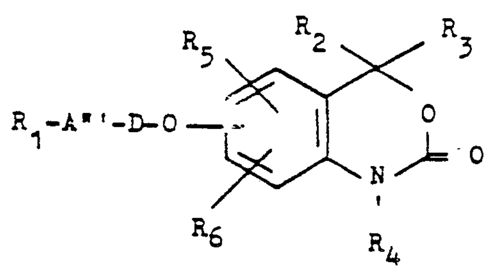

(XII)

in der

D und $R_1$ bis $R_6$ wie eingangs definiert sind und $A'''$ eine durch einen hydrolytisch abspaltbaren Acylrest substituierte H—N=S- oder H—N=SO-Gruppe darstellt.

Als Acylrest kommt beispielsweise der einer Carbonsäure oder eines Kohlensäurederivates wie die Acetyl-, Propionyl-, Butanoyl-, Benzoyl-, Pinanoyl-, Nicotinoyl-, Äthoxycarbonyl-, Aminocarbonyl- oder Dimethylaminocarbonylgruppe in Betracht.

Die Umsetzung wird in Gegenwart einer Säure oder Base, z. B. in Gegenwart von Salzsäure, Schwefelsäure, Natronlauge, Kalilauge oder Kaliumcarbonat, in einem Lösungsmittel oder Lösungsmittelgemisch wie Wasser, Methanol, Wasser/Methanol, Wasser/Äthanol oder Wasser/Tetrahydrofuran bei Temperaturen bis zur Siedetemperatur des verwendeten Lösungsmittels, z. B. bei Temperaturen zwischen 50 und 90 °C, durchgeführt.

i) Zur Herstellung einer Verbindung der allgemeinen Formel I, in der R kein Wasserstoffatom darstellt:

Umsetzung einer Verbindung der allgemeinen Formel

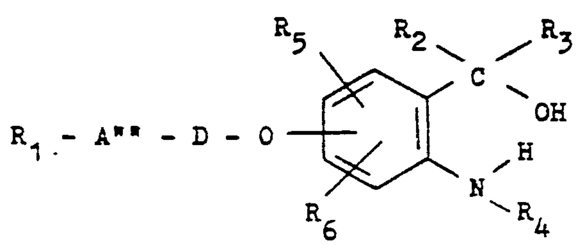

(XIII)

in der

D und $R_1$ bis $R_6$ wie eingangs definiert sind und $A''''$ ein Schwefelatom, eine SO-, $SO_2$-, R'—N=S- oder R'—N=SO-Gruppe darstellt, wobei R' mit Ausnahme eines Wasserstoffatoms die für R eingangs erwähnten Bedeutungen besitzt, mit einem Kohlensäurederivat der allgemeinen Formel

X—CO—X (XIV)

in der X, die gleich oder verschieden sein können, jeweils eine nukleophile Austrittsgruppe wie ein Chloratom, eine Methoxy-, Äthoxy-, Benzyloxyl- oder Imidazolylgruppe bedeuten.

Die Umsetzung wird zweckmäßigerweise in einem inerten Lösungsmittel wie Äther, Chloroform, Dioxan, Toluol oder Tetrahydrofuran/Toluol bei Temperaturen zwischen 0 und 80 °C, vorzugsweise jedoch bei Raumtemperatur, durchgeführt.

Erhält man erfindungsgemäß eine Verbindung der allgemeinen Formel I, in der $R_4$ ein Wasserstoffa-

tom darstellt, so kann diese mittels Alkylierung in eine entsprechende Verbindung der allgemeinen Formel I, in der $R_4$ eine Alkylgruppe mit 1 bis 3 Kohlenstoffatomen darstellt, übergeführt werden.

Die nachträgliche Alkylierung wird zweckmäßigerweise mit einem Alkylhalogenid wie Methyljodid oder Propylbromid oder einem Sulfonsäureester wie Dimethylsulfat vorzugsweise in Gegenwart einer Base wie Natriumhydroxid oder Pyridin, gegebenenfalls in Gegenwart eines Reaktionsbeschleunigers wie Tetrabutylammonium-hydrogensulfat, und vorzugsweise in einem Lösungsmittel wie Methanol, Äthanol, Wasser, Natronlauge, Tetrahydrofuran oder Dioxan bei Temperaturen zwischen 0 und 80 °C, vorzugsweise jedoch bei Raumtemperatur, durchgeführt.

Die als Ausgangsstoffe verwendeten Verbindungen der allgemeinen Formeln II bis XIV sind teilweise literaturbekannt bzw. man erhält diese nach üblichen Verfahren (siehe Beispiele A bis O).

So erhält man beispielsweise eine Hydroxyverbindung der allgemeinen Formel II durch Umsetzung einer entsprechenden Carbonylverbindung mit einer entsprechenden Grignard-Verbindung, anschließende Umsetzung mit Phosgen und Abspaltung des als Schutzrest für die Hydroxygruppe verwendeten Restes und eine Verbindung der allgemeinen Formel V durch anschließende Umsetzung eines so erhaltenen Hydroxy-4H-3,1-benzoxazin-2-ons mit einer entsprechenden Verbindung, z. B. mit einer entsprechenden Monohalogen- oder Dihalogenverbindung. Desweiteren erhält man eine Verbindung der allgemeinen Formel V auch durch Überführung der Hydroxygruppe einer entsprechenden Verbindung in eine nukleophile austauschbare Gruppe, z. B. mittels Thionylchlorid oder Methansulfonylchlorid.

Eine Verbindung der allgemeinen Formel III erhält man durch Umsetzung eines entsprechenden α,ω-disubstituierten Alkans mit einer entsprechenden Mercaptoverbindung und gegebenenfalls anschließender Oxidation.

Eine als Ausgangsstoff verwendete Verbindung der allgemeinen Formeln IV, VII oder XII erhält man durch Umsetzung einer entsprechenden Hydroxyverbindung der allgemeinen Formel II mit einer entsprechenden Verbindung. Eine so erhaltene Verbindung kann anschließend mittels Oxidation in eine Verbindung der allgemeinen Formeln IX oder XII übergeführt werden.

Eine als Ausgangsstoff verwendete Verbindung der allgemeinen Formel VIII erhält man beispielsweise durch Umsetzung eines entsprechenden O-Carbonyl- oder O-Sulfonylacethydroxamsäureesters mit Schwefelsäure und anschliessende Extraktion nach Zugabe einer Base.

Eine als Ausgangsstoff verwendete Verbindung der allgemeinen Formel X erhält man beispielsweise durch Umsetzung einer entsprechenden Hydroxy- oder Mercaptoverbindung mit einem entsprechenden Halogenid in Gegenwart einer Base.

Eine als Ausgangsstoff verwendete Verbindung der allgemeinen Formel XI erhält man durch Umsetzung eines entsprechenden Amids mit einem Hypohalogenit gegebenenfalls in Gegenwart einer Base.

Eine als Ausgangsstoff verwendete Verbindung der allgemeinen Formel XIII erhält man durch Umsetzung eines entsprechenden Anthranilsäureesters mit einer entsprechenden Grignard-Verbindung.

Ferner kann eine so hergestellte Ausgangsverbindung der allgemeinen Formel II kann anschließend mittels Chlorierung oder Bromierung, z. B. mittels Chlor, Sulfurylchlorid oder Brom, in die entsprechende Halogenverbindung bzw. mit Salpetersäure in die entsprechende Nitroverbindung übergeführt werden.

Die Verbindungen der allgemeinen Formel I, in der A ein Schwefelatom oder die R—N=S-Gruppe darstellt, stellen wertvolle Zwischenprodukte zur Herstellung der Verbindungen der allgemeinen Formel I dar, in der A dis SO-, $SO_2$- oder R—N=SO-Gruppe bedeutet.

Außerdem weisen die neuen Verbindungen der allgemeinen Formel I, wie bereits eingangs erwähnt, wertvolle pharmakologische Eigenschaften auf, insbesondere antithrombotische Wirkungen. Sie steigern die Synthese aggregationshemmender Prostaglandine in der Gefäßwand. Die Verbindungen der allgemeinen Formel I weisen auch eine Hemmwirkung auf die Tumormetastasierung auf. Diese beruht auf folgenden Eigenschaften der erfindungsgemäß hergestellten Verbindungen :

1. Sie sind Hemmer der Plättchen-Phosphodiesterase, die als Hemmer der Tumormetastasierung bekannt ist (H. Gastpar, Thrombosis Research *5*, 277-289 (1974) und K. V. Honn, Science *212*, 1270-1272 (1981)).

2. Die Verbindungen hemmen die primäre Hämostase, schon bei sehr niederiger Dosierung. Die Anhaftung von Thrombocyten am verletzten Gefäß und die Bildung eines reinen Plättchenthrombus wird vermieden, was zu einer starken Verlängerung der Blutungszeit führt. Das ist bei den Verbindungen der Formel I durch eine Einschränkung der Plättchenfunktion allein nicht mehr erklärbar, sondern durch eine zusätzlich erhöhte Freisetzung von plättchenwirksamen PG durch die Endothelzellen des Gefäßes. Eine Bestätigung hierfür ist das Unterbleiben der Blutungszeitverlängerung, wenn man die Prostacyclinsynthese der Endothelzelle durch vorher verabfolgte Cyclooxygenasehemmer unterbricht. Die Verbindungen stellen somit eine bisher noch unbekannte, optimale Kombination zweier Wirkprinzipien dar, nämlich die vermehrte Synthese thrombocythenwirksamer, c.AmP-steigender PG's durch die Gefäßwand und Hemmung des Abbaues des erhöhten cAmP durch Hemmung der PDE in den Thrombocyten.

Die so erfaßbare Steigerung der Prostagladin-($I_2$)-Aktivität bzw. der Synthese in der Gefäßwand ist nach HONN (K. V. Honn, Science *212*, 1270-1272 (1981)) ebenfalls eine Ursache für die Hemmung der Tumormetastasierung.

Beispielsweise wurden die Verbindungen

A = 6-[4-(4-Chlor-phenylsulfinyl)-butoxy]-4,4-dimethyl-4H-3,1-benzoxazin-2-on,
B = 6-[4-(4-Hydroxy-phenylsulfoximino)-butoxy]-4,4-dimethyl-4H-3,1-benzoxazin-2-on,
C = 6-[4-(4-Methoxy-phenylsulfoximino)-butoxy]-4,4-dimethyl-4H-3,1-benzoxazin-2-on,
D = 6-[4-(4-Methyl-phenylsulfinyl)-butoxy]-4,4-dimethyl-4H-3,1-benzoxazin-2-on,
E = 6-[4-(4-Cyclohexyl-phenylsulfinyl)-butoxy]-4,4-dimethyl-4H-3,1-benzoxazin-2-on,
F = 6-[4-(3,4-Dichlor-phenylsulfoximino)-butoxy]-4,4-dimethyl-4H-3,1-benzoxazin-2-on,
G = 6-[3-Methoxy-phenylsulfoximino)-butoxy]-4,4-dimethyl-4H-3,1-benzoxazin-2-on,
H = 6-[4-(4-Fluor-phenylsulfoximino)-butoxy]-4,4-dimethyl-4H-3,1-benzoxazin-2-on,
I = 6-[4-(4-Brom-phenylsulfoximino)-butoxy]-4,4-dimethyl-4H-3,1-benzoxazin-2-on,
K = 6(-4-Phenylsulfinyl-butoxy)-4H-3,1-benzoxazin-2-on,
L = 6-[4-(4-Brom-phenylsulfinyl)-butoxy]-4,4-dimethyl-4H-3,1-benzoxazin-2-on,
M = 6-[4-(4-Brom-3-methyl-phenylsulfoximino)-butoxy]-4,4-dimethyl-4H-3,1-benzoxazin-2-on,
N = 6-[4-(4-Methyl-phenylsulfoximino)-butoxy]-4,4-dimethyl-4H-3,1-benzoxazin-2-on,
O = 6-[4-(3,4-Dimethoxy-phenylsulfoximino)-butoxy]-4,4-dimethyl-4H-3,1-benzoxazin-2-on,
P = 6-[4-(4-Amino-3,5-dibrom-phenylsulfinyl)-butoxy]-4,4-dimethyl-4H-3,1-benzoxazin-2-on,
Q = 6-[4-(4-Biphenylyl-sulfoximino)-butoxy]-4,4-dimethyl-4H-3,1-benzoxazin-2-on,
R = 6-[4-(3,4-Dimethoxy-N-acetyl-phenylsulfoximino)-butoxy]-4,4-dimethyl-4H-3,1-benzoxazin-2-on,
S = 6-[4-(3,5-Di-tert. butyl-4-hydroxy-N-acetyl-phenylsulfoximino)-butoxy]-4,4-dimethyl-4H-3,1-benzoxazin-2-on,
T = 7-Brom-4,4-dimethyl-6-[4-(4-methyl-phenylsulfoximino)-butoxy]-4H-3,1-benzoxazin-2-on,
U = 7-Chlor-4,4-dimethyl-6-[4-(4-methyl-phenylsulfinyl)-butoxy]-4H-3,1-benzoxazin-2-on und
V = 7-Brom-6-[4-(3,4-dichlor-phenylsulfinyl)-butoxy]-4,4-dimethyl-4H-3,1-benzoxazin-2-on

auf ihre biologischen Eigenschaften wie folgt geprüft :

1. PDE-Hemmung

Prinzip

cAMP wird von Phosphodiesterase (PDE) aus verschiedenen Quellen, so auch aus Blutplättchen, zu AMP hydrolysiert. Diese Hydrolyse wird konzentrationsabhängig von PDE-Hemmern inhibiert.

Methode

Als Phosphodiesterase wird der 10 000 × g-Überstand von Humanplättchen verwendet, welche mit Wasser eingefroren und wieder aufgetaut wurden.

0,3 ml einer Mischung, die 0,1 Mol/l Trishydroxy-aminomethan (pH 7.4), 3 mMol/l Magnesiumchlorid, 1 mMol/l AMP 1 μMol/l $^3$H-cAMP (spez. Aktivität ca. 10 MBq/μMol), PDE sowie die zu untersuchende Substanz bzw. Wasser bei der Kontrolle enthält, werden 15 Minuten bei 37 °C inkubiert.

Die Inkubation wird durch Zugabe von 0,5 ml Zinksulfat (0,266 Mol/l) und 0,5 ml Bariumhydroxid (0,266 Mol/l) gestoppt, der Niederschlag abzentrifugiert und die im Überstand verbleibende Aktivität des nicht umgesetzten $^3$H-cAMP bestimmt. Aus dem Vergleich der Substanz-Ansätze gegenüber Kontroll-Ansätze wurde die Konzentration für eine 50 %ige Hemmwirkung ($IC_{50}$) der jeweiligen Substanz berechnet :

| Substanz | $IC_{50}$ [uMol/l] |
|---|---|
| A | 0,24 |
| B | 0,23 |
| C | 0,13 |
| D | 0,21 |
| E | 0,078 |
| F | 0,042 |
| G | 0,077 |
| H | 0,18 |
| I | 0,11 |

(Fortsetzung)

| Substanz | $IC_{50}$ [µMol/l] |
|---|---|
| K | 1,5 |
| L | 0,24 |
| M | 0,066 |
| N | 0,065 |
| O | 0,20 |
| P | 0,056 |
| Q | 0,0068 |
| R | 0,71 |
| S | 0,077 |
| T | 0,001 |
| U | 0,0034 |
| V | 0,0038 |

2. Antithrombotische Wirkung

Methodik

Die Thrombozytenaggregation wird nach der Methode von BORN und CROSS (J. Physiol. *170*, 397 (1964)) in plättchenreichem Plasma gesunder Versuchspersonen gemessen. Zur Gerinnungshemmung wird das Blut mit Natriumcitrat 3,14 % im Volumenverhältnis 1 : 10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der « optical density ».

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37 °C inkubiert.

Aus den erhaltenen Meßzahlen wird graphisch eine $EC_{50}$ berechnet, die sich auf eine 50 %ige Änderung der « optical density » im Sinne einer Aggregationshemmung bezieht.

Die nachfolgende Tabelle enthält die gefundenen Ergebnisse :

| Substanz | $EC_{50}$ [µMol/l] |
|---|---|
| A | 0,34 |
| B | 0,28 |
| C | 0,27 |
| D | 0,33 |
| E | 0,32 |
| F | 0,21 |
| G | 0,27 |
| H | 0,32 |
| I | 0,33 |
| K | 1,90 |

(Fortsetzung)

| Substanz | $EC_{50}$ [µMol/l] |
|---|---|
| L | 0,42 |
| M | 0,25 |
| N | 0,22 |
| O | 0,27 |
| P | 0,55 |
| Q | 0,25 |
| R | 2,30 |
| S | 2,20 |
| T | 0,069 |
| U | 0,028 |
| V | 0,044 |

3. Bestimmung der Verlängerung der Blutungszeit

Vorbemerkung

Der menschliche Organismus sowie der der Warmblütler besitzt einen sinnvollen Mechanismus, der ihn vor Blutverlusten im Falle von Verletzungen schützen soll. Dieses System besteht aus den Blutplättchen (Thrombozyten), welche mittels ihrer Klebeeigenschaften einen Gefäßdefekt rasch « verstopfen » sollen und so die primäre Hämostase herbeiführen. Neben diesem reinen cellulären Blutstillungsmechanismus besitzt der Körper ein Blutgerinnungssystem. Bei diesem System werden Plasmafaktoren (Eiweißkörper) in eine wirksame Form gebracht, welche schließlich das flüssige Plasmafibrinogen zu einem Fibringerinnsel werden lassen.

Das System der primären Hämostase, welches im wesentlichen von den Thrombozyten gestellt wird, und das Gerinnungssystem ergänzen sich in dem gemeinsamen Ziel, den Körper vor Blutverlusten wirkungsvoll zu schützen.

Bei manchen Krankheiten kann es auch bei einem intakten Gefäßsystem zum Ablaufen von Gerinnungsprozessen sowie zum Verklumpen von Thrombozyten kommen. Die Schwächung des Blutgerinnungssystems durch Cumarine oder Heparin ist bekannt und kann leicht mit Hilfe von bekannten Blutgerinnungstesten gemessen werden, welche unter Präparateeinwirkung eine Verlängerung anzeigen (Plasmarecalcif.-zeit, Quick-Bestimmung, Thrombinzeit, etc.).

Da im Falle einer Verletzung die erste rasche Blutstillung durch Thrombozyten geschieht, läßt sich beim Setzen einer standardisierten Verletzung die Funktion der Thrombozyten mit Hilfe der Mesung der Blutungszeit gut bestimmen. Die normale Blutungszeit beträgt beim Menschen etwa 1 bis 3 min., setzt aber leistungsfähige und in genügender Zahl vorhandene Thrombozyten voraus. Bei einer normalen Thrombozytenzahl weist also eine verlängerte Blutungszeit auf eine gestörte Funktion der Thrombozyten hin. Wir finden dies z. B. bei einigen angeborenen Thrombozytenfunktionsstörungen. Will man auf der anderen Seite die Neigung zu spontanem Zusammenballen der Thrombozyten mit der Folge von Gefäßverschlüssen im arteriellen System durch Medikamente verhindern, so muß folglich bei einer erfolgreichen thrombozytenwirksamen Therapie die Blutungszeit unter Substanzeinfluß verlängert werden. Bei einer thrombozytenwirksamen Substanz ist also eine Verlängerung der Blutungszeit und — da das plasmatische Gerinnungssystem ja nicht berührt wird — eine normale Blutgerinnungszeit zu erwarten.

Literatur : W. D. Keidel : Kurzgefaßtes Lehrbuch der Physiologie, Georg Thieme Verlag Stuttgart 1967, Seite 31 : Der Blutstillungsvorgang.

Zur Bestimmung der Blutungszeit wurden die zu untersuchenden Substanzen wachen Mäusen in einer Dosis von 10 mg/kg p. o. appliziert. Nach einer Stunde wurde von der Schwanzspitze jedes Tieres ca. 0,5 mm abgeschnitten und das austretende Blut in Abständen von 30 Sekunden bis zum Sistieren der Blutung vorsichtig mit einem Filterpapier abgetupft. Die Zahl der so erhaltenen Bluttropfen ergibt ein Maß für die Blutungszeit (5 Tiere pro Versuch). Die folgenden Zahlenangaben bedeuten Prozent-Verlängerung gegenüber Kontrollen ohne Substanzgabe :

| Substanz | Verlängerung der Blutungszeit in % nach 1 Stunde |
|---|---|
| A | > 248 |
| B | > 263 |
| C | > 241 |
| D | > 250 |
| E | 46 |
| F | > 275 |
| G | > 245 |
| H | > 211 |
| I | > 220 |
| K | 121 |
| L | > 266 |
| M | 100 |
| N | 63 |
| O | > 227 |
| P | 73 |
| Q | 128 |
| R | > 241 |
| S | > 226 |
| T | 60 |
| U | > 230 |
| V | 100 |

4. Akute Toxizität

Die akute Toxizität der zu untersuchenden Substanzen wurde orientierend an Gruppen von je 10 Mäusen nach oraler Gabe einer Einzeldosis bestimmt (Beobachtungszeit : 14 Tage) :

| Substanz | Orientierende akute Toxizität |
|---|---|
| A | 1.000 mg (0 von 10 Tieren gestorben) |
| B | 1.000 mg (0 von 10 Tieren gestorben) |
| C | 1.000 mg (0 von 10 Tieren gestorben) |
| D | 1.000 mg (0 von 10 Tieren gestorben) |
| E | 1.000 mg (0 von 10 Tieren gestorben) |
| R | 1.000 mg (0 von 10 Tieren gestorben) |
| S | 1.000 mg (0 von 10 Tieren gestorben) |
| T | 1.000 mg (0 von 10 Tieren gestorben) |
| U | 1.000 mg (0 von 10 Tieren gestorben) |
| V | 1.000 mg (0 von 10 Tieren gestorben) |

Aufgrund ihrer pharmakologischen Eigenschaften eignen sich die neuen Verbindungen zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe.

Die zur Erzielung einer entsprechenden Wirkung erforderliche Dosierung beträgt zweckmäßigerweise zwei- bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der allgemeinen Formel I, gegebenenfalls in Kombination mit anderen Wirksubstanzen, zusammen mit einem oder mehreren inerten üblichen Trägerstoffen und/-oder Verdünnungsmitteln, z. B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabeltten, Dragées, Kapseln, Pulver, Suspensionen oder Zäpfchen einarbeiten.

Die nachfolgenden Beispiele sollen die Erfindung näher erläutern :

Herstellung der Ausgangsprodukte

## Beispiel A

6-Methoxy-4,4-dimethyl-4H-3,1-benzoxazin-2-on

a) 2-Amino-5-methoxy-phenyl-dimethyl-carbinol

Unter Rühren wird eine Lösung von 261 ml (4,2 Mol) Methyljodid in 1 000 ml absolutem Äther zu einer Suspension von 102 g (4,2 Mol) Magnesium-Spänen in 300 ml absolutem Äther innerhalb von 2,5 Stunden zugetropft und noch eine halbe Stunde nachgerührt. Dann tropft man im Verlauf einer halben Stunde unter Kühlen — damit die Temperatur 20-25 °C nicht übersteigt — eine Lösung von 181,2 g (1 Mol) 5-Methoxy-anthranilsäure-methylester in 1,5 l absolutem Äther zu. Nach einstündigem Nachrühren wird auf Ammoniumchlorid-haltiges Eiswasser gegossen, die Ätherphase abgetrennt, mit Ammoniumchloridhaltigem Wasser gewaschen, mit Natriumsulfat getrocknet und der Äther abdestilliert.

b) 6-Methoxy-4,4-dimethyl-4H-3,1-benzoxazin-2-on

Der nach dem Abdestillieren des Äthers verbleibende Rückstand von 2-Amino-5-methoxy-phenyl-dimethyl-carbinol wird ohne weitere Reinigung in 1,5 l Chloroform gelöst, mit 276,4 g (2 Mol) Kaliumcarbonat versetzt und auf 40 °C erwärmt. Zu dieser Lösung tropft man im Verlauf von 2 Stunden 600 ml einer 20 %igen Lösung von Phosgen in Toluol (1,2 Mol) so schnell zu, daß die Temperatur der Reaktionsmischung zwischen 40 und 50 °C beträgt, dabei kann gelegentliches Aufschäumen erfolgen. Nach beendeter Zugabe läßt man über Nacht bei Zimmertemperatur stehen.

Danach versetzt man mit 1 l Wasser, trennt die Chloroform/Toluol-Phase ab und extrahiert die wässrige Phase mehrmals mit Chloroform, dem etwas Methanol beigegeben wird. Die vereinigten organischen Extrakte werden mit Wasser gewaschen, mit Natriumsulfat getrocknet und die Lösungsmittel abdestilliert. Der kristalline Rückstand wird aus Toluol/Essigester = 9 : 1 umkristallisiert.

Schmelzpunkt : 182-183 °C,

Ausbeute : 130 g (64 % der Theorie, bezogen auf eingesetzten 5-Methoxy-anthranilsäure-methylester).

In gleicher Weise werden folgende Verbindungen erhalten :

6-Methoxy-4,4-di-n-hexyl-4H-3,1-benzoxazin-2-on
aus 5-Methoxy-anthranilsäure-methylester, n-Hexyl-magnesiumbromid und Phosgen
Schmelzpunkt : 101-103 °C,
Ausbeute : 70,8 % der Theorie.
6-Methoxy-4,4-diphenyl-4H-3,1-benzoxazin-2-on
aus 5-Methoxy-anthranilsäure-methylester, Phenylmagnesiumbromid und Phosgen.
Schmelzpunkt : 237 °C,
Ausbeute : 69,5 % der Theorie.
6-Methoxy-4,4-dicyclohexyl-4H-3,1-benzoxazin-2-on
aus 5-Methoxy-anthranilsäure-methylester, Cyclohexylmagnesiumbromid und Phosgen.
Schmelzpunkt : 269-271 °C,
Ausbeute : 50,4 % der Theorie.
6-Methoxy-4-isopropyl-4H-3,1-benzoxazin-2-on
aus 5-Methoxy-2-amino-benzaldehyd (hergestellt aus 5-Methoxy-2-nitro-benzaldehyd und Pt/$H_2$ bei 30 bar und 35 °C), Isopropylmagnesiumjodid und Phosgen.
Schmelzpunkt : 124-125 °C,
Ausbeute : 37,1 % der Theorie.
6-Methoxy-4-äthyl-4H-3,1-benzoxazin-2-on
aus 5-Methoxy-2-amino-benzaldehyd, Äthylmagnesiumbromid und Phosgen.
Schmelzpunkt : 88-89 °C,
Ausbeute : 30,2 % der Theorie.
6-Methoxy-4-phenyl-4H-3,1-benzoxazin-2-on
aus 5-Methoxy-2-amino-benzaldehyd, Phenylmagnesiumbromid und Phosgen.

Schmelzpunkt : 157-158 °C,
Ausbeute : 36,3 % der Theorie.
6-Methoxy-4,4-diäthyl-4H-3,1-benzoxazin-2-on
aus 5-Methoxy-anthranilsäure-methylester, Äthylmagnesiumbromid und Phosgen.
Schmelzpunkt : 123-125 °C,
Ausbeute : 90,1 % der Theorie.
6-Methoxy-4-methyl-4H-3,1-benzoxazin-2-on
aus 5-Methoxy-2-amino-benzaldehyd, Methylmagnesiumjodid und Phosgen.
Schmelzpunkt : 120-121 °C,
Ausbeute : 20,2 % der Theorie.
5-Methoxy-4,4-dimethyl-4H-3,1-benzoxazin-2-on
aus 6-Methoxy-anthranilsäure-methylester, Methylmagnesiumjodid und Phosgen.
Schmelzpunkt : 139-141 °C,
Ausbeute : 22 % der Theorie.
7-Methoxy-4,4-dimethyl-4H-3,1-benzoxazin-2-on
aus 4-Methoxy-anthranilsäure-methylester, Methylmagnesiumjodid und Phosgen.
Schmelzpunkt : 119-121 °C,
Ausbeute : 62 % der Theorie.
8-Methoxy-4,4-dimethyl-4H-3,1-benzoxazin-2-on
aus 3-Methoxy-anthranilsäure-methylester, Methylmagnesiumjodid und Phosgen.
Schmelzpunkt : 95-96 °C,
Ausbeute : 42,1 % der Theorie.
6-Methoxy-4,4,7-trimethyl-4H-3,1-benzoxazin-2-on
aus 5-Methoxy-4-methyl-anthranilsäuremethylester, Methylmagnesiumjodid und Phosgen.
Schmelzpunkt : 134-135 °C,
Ausbeute : 45,5 % der Theorie.
6-Methoxy-4,4,8-trimethyl-4H-3,1-benzoxazin-2-on
aus 5-Methoxy-3-methyl-anthranilsäuremethylester, Methylmagnesiumjodid und Phosgen.
Schmelzpunkt : 218-219 °C,
Ausbeute : 65,8 % der Theorie.
6-Methoxy-5,7-dichlor-4,4-dimethyl-4H-3,1-benzoxazin-2-on
aus 5-Methoxy-4,6-dichlor-anthranilsäuremethylester, Methylmagnesiumjodid und Phosgen.
Schmelzpunkt : 203-204 °C,
Ausbeute : 44,0 % der Theorie.
6-Methoxy-5,7-dimethyl-4H-3,1-benzoxazin-2-on
aus 5-Methoxy-4,6-dimethyl-anthranilsäuremethylester, Methylmagnesiumjodid und Phosgen.
Schmelzpunkt : 152-153 °C,
Ausbeute : 38,7 % der Theorie.

## Beispiel B

6-Methoxy-4-H-3,1-benzoxazin-2-on

a) 2-Amino-5-methoxy-benzylalkohol

181,2 g (1 Mol) 5-Methoxy-2-nitro-benzaldehyd (hergestellt durch Methylierung von 2-Nitro-5-hydroxy-benzaldehyd mit Methyljodid/Kalium-tert.butylat in Dimethylsulfoxid) werden in 1,8 l Methanol gelöst, mit 50 g Raney-Nickel versetzt und bei Raumtemperatur und 5 bar hydriert. Nach 10 Stunden ist die Wasserstoffaufnahme beendet. Man saugt vom Katalysator ab und destilliert das Methanol ab.

b) 6-Methoxy-4H-3,1-benzoxazin-2-on

Der nach dem Abdestillieren des Methanols verbleibende Rückstand wird ohne weitere Reinigung in 1 l Chloroform gelöst und mit 175 g (1,25 Mol) Kaliumcarbonat versetzt. Zu dieser Suspension tropft man bei 50 °C unter Rühren 553 ml (1,05 Mol) einer 20 %igen Lösung von Phosgen in Toluol vorsichtig zu. Nach 2-stündigem Nachrühren bei Raumtemperatur gießt man auf Eiswasser, trennt die Chloroform/Toluol-Phase ab und extrahiert die wässerige Phase mehrmals mit Chloroform/Methanol = 5/l. Die vereinigten organischen Extrakte werden mit Wasser gewaschen, mit Natriumsulfat getrocknet und die Lösungsmittel abdestilliert. Der Rückstand wird durch Säulenchromatographie (Kieselgel, Chloroform/Aceton = 19 : 1) gereinigt. Die nach dem Abdestillieren des Elutionsmittels verbleibenden Kristalle schmelzen bei 154-156 °C.

Ausbeute : 110,5 g (61,7 % der Theorie).
Analog wird folgende Verbindung erhalten :
6-Methoxy-7-chlor-4H-3,1-benzoxazin-2-on
Schmelzpunkt : 208-210 °C,

Ausbeute : 38,7 % der Theorie.

Beispiel C

6-Hydroxy-4,4-dimethyl-4H-3,1-benzoxazin-2-on

237,5 g (1,146 Mol) 6-Methoxy-4,4-dimethyl-4H-3,1-benzoxazin-2-on werden in 2,4 l trockenem Äthylenchlorid gelöst und unter Rühren tropfenweise bei — 30 bis — 40 °C mit 125 ml (330,3 g = 1,3 Mol) Bortribromid versetzt. Nach beendeter Zugabe läßt man auf Raumtemperatur erwärmen und über Nacht stehen. Dann wird unter Kühlen und Rühren ein l 50 %iges Äthanol zugetropft, die Mischung auf ca. 500 ml eingeengt und mit 3 l Wasser verdünnt. Der ausfallende Niederschlag wird abgesaugt und getrocknet.

Schmelzpunkt : 202-204 °C (aus Essigester/Petroläther),
Ausbeute : 223,3 g (99,8 % der Theorie).

Analog werden folgende Verbindungen erhalten :

6-Hydroxy-4H-3,1-benzoxazin-2-on
Schmelzpunkt : 244-245 °C,
Ausbeute : 78,5 % der Theorie.
6-Hydroxy-4,4-di-n-hexyl-4H-3,1-benzoxazin-2-on
Schmelzpunkt : 144-146 °C,
Ausbeute : 92,4 % der Theorie.
6-Hydroxy-4,4-diphenyl-4H-3,1-benzoxazin-2-on
Schmelzpunkt : 239 °C (Zersetzung),
Ausbeute : 90,0 % der Theorie.
6-Hydroxy-4-isopropyl-4H-3,1-benzoxazin-2-on
Schmelzpunkt : 215-216 °C,
Ausbeute : 77,6 % der Theorie.
6-Hydroxy-4-äthyl-4H-3,1-benzoxazin-2-on
Schmelzpunkt : 216-218 °C,
Ausbeute : 68,5 % der Theorie.
6-Hydroxy-4,4-dicyclohexyl-4H-3,1-benzoxazin-2-on
Schmelzpunkt : > 280 °C,
Ausbeute : 97,8 % der Theorie.
6-Hydroxy-4,4-diäthyl-4H-3,1-benzoxazin-2-on
Schmelzpunkt : 194-195 °C,
Ausbeute : 95,5 % der Theorie.
6-Hydroxy-4-methyl-4H-3,1-benzoxazin-2-on
Schmelzpunkt : 226 °C (Zers.),
Ausbeute : 75,8 % der Theorie.
7-Hydroxy-4,4-dimethyl-4H-3,1-benzoxazin-2-on
Schmelzpunkt : 180-182 °C,
Ausbeute : 96,8 % der Theorie.
5-Hydroxy-4,4-dimethyl-4H-3,1-benzoxazin-2-on
Schmelzpunkt : 251 °C,
Ausbeute : 79 % der Theorie.
8-Hydroxy-4,4-dimethyl-4H-3,1-benzoxazin-2-on
Schmelzpunkt : 203-205 °C,
Ausbeute : 90 % der Theorie.
6-Hydroxy-4,4,8-trimethyl-4H-3,1-benzoxazin-2-on
Schmelzpunkt : 174 °C (Zers.),
Ausbeute : 94,3 % der Theorie.
6-Hydroxy-4,4,7-trimethyl-4H-3,1-benzoxazin-2-on
Schmelzpunkt : 150-152 °C,
Ausbeute : 85,8 % der Theorie.
8-Chlor-6-hydroxy-4,4-dimethyl-4H-3,1-benzoxazin-2-on
Schmelzpunkt : 196-198 °C,
Ausbeute : 52 % der Theorie.
7-Chlor-6-hydroxy-4,4-dimethyl-4H-3,1-benzoxazin-2-on
Schmelzpunkt : 218-219 °C,
Ausbeute : 97,1 % der Theorie.
7-Brom-6-hydroxy-4,4-dimethyl-4H-3,1-benzoxazin-2-on
Schmelzpunkt : 157-158 °C,
Ausbeute : 96 % der Theorie.
8-Brom-6-hydroxy-4,4-dimethyl-4H-3,1-benzoxazin-2-on

Schmelzpunkt : 212-214 °C,
Ausbeute : 46,7 % der Theorie.
7,8-Dibrom-6-hydroxy-4,4-dimethyl-4H-3,1-benzoxazin-2-on
Schmelzpunkt : 194-195 °C,
Ausbeute : 24 % der Theorie.
6-Hydroxy-7-chlor-4H-3,1-benzoxazin-2-on
Schmelzpunkt : 250 °C (Zers.)
Ausbeute : 96,2 % der Theorie.
6-Hydroxy-5,7-dichlor-4,4-dimethyl-4H-3,1-benzoxazin-2-on
Schmelzpunkt : 215-217 °C
Ausbeute : 88,0 % der Theorie.
6-Hydroxy-5,7-dimethyl-4H-3,1-benzoxazin-2-on
Schmelzpunkt : 210-211 °C,
Ausbeute : 70,5 % der Theorie.

## Beispiel D

6-(5-Brom-pentoxy)-4,4-dimethyl-4H-3,1-benzoxazin-2-on

Eine Lösung von 19,3 g (0,1 Mol) 6-Hydroxy-4,4-dimethyl-4H-3,1-benzoxazin-2-on in 200 ml Dimethylsulfoxid wird bei Raumtemperatur mit 41,5 g (0,3 Mol) Kaliumcarbonat und 92 g (0,4 Mol) 1,5-Dibrom-pentan versetzt, dabei steigt die Temperatur auf 45 °C an. Nach 2-stündigem Nachrühren wird mit Eiswasser versetzt und mit Chloroform extrahiert. Die Chloroform-Phase wäscht man mit Wasser, trocknet sie mit Natriumsulfat und destilliert das Chloroform ab. Den Rückstand kristallisiert man aus Essigester/Petroläther um.
Schmelzpunkt : 113-115 °C,
Ausbeute : 26,9 g (78,6 % der Theorie),
Analog werden folgende Verbindungen erhalten :

6-(3-Chlor-propoxy)-4-isopropyl-4H-3,1-benzoxazin-2-on
aus 6-Hydroxy-4-isopropyl-4H-3,1-benzoxazin-2-on und 3-Brompropylchlorid.
Schmelzpunkt : 104-105 °C,
Ausbeute : 68,0 % der Theorie.
6-(3-Chlor-propoxy)-4,4-dimethyl-4H-3,1-benzoxazin-2-on
aus 6-Hydroxy-4,4-dimethyl-4H-3,1-benzoxazin-2-on und 3-Brompropylchlorid.
Schmelzpunkt : 137-138 °C,
Ausbeute : 71,6 % der Theorie.
6-(6-Brom-hexyloxy)-4,4-dimethyl-4H-3,1-benzoxazin-2-on
aus 6-Hydroxy-4,4-dimethyl-4H-3,1-benzoxazin-2-on und 1,6-Dibromhexan.
Schmelzpunkt : 125-126 °C,
Ausbeute : 59,2 % der Theorie.
6-(2-Chlor-äthyl)-4,4-dimethyl-4H-3,1-benzoxazin-2-on
aus 6-Hydroxy-4,4-dimethyl-4H-3,1-benzoxazin-2-on und Benzolsulfonsäure-(2-chlor-äthyl)-ester.
Schmelzpunkt : 128-129 °C,
Ausbeute : 34,4 % der Theorie.

## Beispiel E

6-(4-Acetoxy-butoxy)-4H-3,1-benzoxazin-2-on

102 g (0,62 Mol) 6-Hydroxy-4H-3,1-benzoxazin-2-on werden in 1 l Dimethylsulfoxid gelöst und mit 227 g (1,64 Mol) Kaliumcarbonat und 166 g (0,73 Mol) 4-Acetoxy-butylbromid versetzt. Man rührt die Reaktionsmischung 6 Stunden lang bei 50 °C und versetzt dann mit Eiswasser. Der Niederschlag wird abgesaugt, mit Wasser gewaschen und getrocknet.
Schmelzpunkt : 119-121 °C,
Ausbeute : 142,8 g (82,7 % der Theorie).
Analog werden folgende Verbindungen erhalten :

6-(4-Acetoxy-butoxy)-4,4-dimethyl-4H-3,1-benzoxazin-2-on
Öl, RF-Wert : 0,8 (Kieselgelplatte ; Chloroform/Äthanol = 9 : 1)
Ausbeute : 100 % der Theorie.
6-(4-Acetoxy-butoxy)-4,4-di-n-hexyl-4H-3,1-benzoxazin-2-on
Öl, RF-Wert : 0,6 (Kieselgelplatte ; Chloroform/Aceton = 9 : 1),
Ausbeute : 100 % der Theorie.
6-(4-Acetoxy-butoxy)-4,4-diphenyl-4H-3,1-benzoxazin-2-on

Öl, RF-Wert : 0,55 (Kieselgelplatte ; Chloroform/Aceton = 9 : 1),
Ausbeute : 81 % der Theorie.
6-(4-Acetoxy-butoxy)-4,4-dicyclohexyl-4H-3,1-benzoxazin-2-on
Schmelzpunkt : 177-178 °C,
Ausbeute : 68,8 % der Theorie.
7-(4-Acetoxy-butoxy)-4,4-dimethyl-4H-3,1-benzoxazin-2-on
Öl, RF-Wert : 0,5 (Kieselgelplatte : Äthylenchlorid/Äthanol = 9 : 1),
Ausbeute : 100 % der Theorie.
8-(4-Acetoxy-butoxy)-4,4-dimethyl-4H-3,1-benzoxazin-2-on
Schmelzpunkt : 154-158 °C,
Ausbeute : 100 % der Theorie.
6-(4-Acetoxy-butoxy)-4,4-diäthyl-4H-3,1-benzoxazin-2-on
Öl, RF-Wert : 0,38 (Kieselgelplatte : Chloroform/Äthanol = 19 : 1)
Ausbeute : 95 % der Theorie.
6-(4-Acetoxy-butoxy)-4-methyl-4H-3,1-benzoxazin-2-on
Öl, RF-Wert : 0,5 (Kieselgelplatte : Chloroform/Äthanol = 9 : 1).
Ausbeute : 98 % der Theorie.
6-(4-Acetoxy-butoxy)-4,4-dimethyl-7-nitro-4H-3,1-benzoxazin-2-on
Öl, RF-Wert : 0,8 (Kieselgelplatte : Chloroform/Aceton = 9 : 1)
Ausbeute : 97 % der Theorie.
6-(4-Acetoxy-butoxy)-4,4,8-trimethyl-4H-3,1-benzoxazin-2-on
Öl, RF-Wert : 0,5 (Kieselgelplatte : Äthylenchlorid/Aceton = 9 : 1),
Ausbeute : 98 % der Theorie.

## Beispiel F

6-(4-Hydroxy-butoxy)-4H-3,1-benzoxazin-2-on

Zu einer Lösung von 150 g (0,537 Mol) 6-(4-Acetoxy-butoxy)-4H-3,1-benzoxazin-2-on in 500 ml Methanol wird unter Rühren bei 10 bis 15 °C eine Lösung von 24 g (0,6 Mol) Natriumhydroxid in 100 ml Wasser zugetropft. Nach einstündigem Nachrühren bei Raumtemperatur wird zunächst mit Eiswasser und dann mit 60 ml Eisessig versetzt. Das ausgefallene Produkt wird abgesaugt und aus Wasser umkristallisiert.

Schmelzpunkt : 133-134 °C,
Ausbeute : 61,5 g (48,4 % der Theorie).
Analog werden folgende Verbindungen erhalten :

6-(4-Hydroxy-butoxy)-4,4-dimethyl-4H-3,1-benzoxazin-2-on
Schmelzpunkt : 124 °C,
Ausbeute : 82,9 % der Theorie.
6-(4-Hydroxy-butoxy)-4,4-diphenyl-4H-3,1-benzoxazin-2-on
Schmelzpunkt : 188-189 °C,
Ausbeute : 79 % der Theorie.
6-(4-Hydroxy-butoxy)-4,4-di-n-hexyl-4H-3,1-benzoxazin-2-on
Schmelzpunkt : 105-107 °C,
Ausbeute : 56 % der Theorie.
6-(4-Hydroxy-butoxy)-4,4-dicyclohexyl-4H-3,1-benzoxazin-2-on
Schmelzpunkt : 233-234 °C,
Ausbeute : 77,6 % der Theorie.
7-(4-Hydroxy-butoxy)-4,4-dimethyl-4H-3,1-benzoxazin-2-on
Schmelzpunkt : 105-106 °C,
Ausbeute : 53,4 % der Theorie.
8-(4-Hydroxy-butoxy)-4,4-dimethyl-4H-3,1-benzoxazin-2-on
Schmelzpunkt : 150-151 °C,
Ausbeute : 73 % der Theorie.
6-(4-Hydroxy-butoxy)-4,4-diäthyl-4H-3,1-benzoxazin-2-on
Schmelzpunkt : 126-127 °C,
Ausbeute : 69,3 % der Theorie.
6-(4-Hydroxy-butoxy)-4-methyl-4H-3,1-benzoxazin-2-on
Öl, RF-Wert : 0,4 (Kieselgelplatte : Chloroform/Äthanol = 9 : 1)
Ausbeute : 61,6 % der Theorie.
6-(4-Hydroxy-butoxy)-4,4-dimethyl-7-nitro-4H-3,1-benzoxazin-2-on
Schmelzpunkt : 140-141 °C,
Ausbeute : 37,8 % der Theorie.
6-(4-Hydroxy-butoxy)-4,4,8-trimethyl-4H-3,1-benzoxazin-2-on

Schmelzpunkt : 128-129 °C,
Ausbeute : 64,2 % der Theorie.

Beispiel G

6-(4-Chlor-butoxy)-4H-3,1-benzoxazin-2-on

Zu 300 ml Thionylchlorid werden unter Rühren bei Raumtemperatur 60 g (0,253 Mol) 6-(4-Hydroxy-butoxy)-4H-3,1-benzoxazin-2-on und 5 ml Pyridin gegeben. Nach 2-stündigem Rückflußkochen wird das überschüssige Thionylchlorid am Rotationsverdampfer abdestilliert, der Rückstand in Chloroform aufgenommen und zusammen mit dem gleichen Volumen Eiswasser 30 Minuten gerührt. Danach trennt man die Chloroform-Phase ab, wäscht sie noch zweimal mit Wasser, trocknet mit Natriumsulfat und destilliert das Chloroform ab. Den Rückstand kristallisiert man aus Toluol/Diisopropyläther = 3 : 1 um.
Schmelzpunkt : 127-128 °C,
Ausbeute : 53 g (81,9 % der Theorie).
Analog werden folgende Verbindungen erhalten :

6-(4-Chlor-butoxy)-4,4-dimethyl-4H-3,1-benzoxazin-2-on
Schmelzpunkt : 138-139 °C,
Ausbeute : 75,7 % der Theorie.
6-(4-Chlor-butoxy)-4,4-di-n-hexyl-4H-3,1-benzoxazin-2-on
Öl, RF-Wert : 0,65 (Kieselgelplatte ; Chloroform/Aceton = 9 : 1),
Ausbeute : 100 % der Theorie.
6-(4-Chlor-butoxy)-4,4-diphenyl-4H-3,1-benzoxazin-2-on
Öl, RF-Wert : 0,38 (Kieselgelplatte ; Cyclohexan/Essigester = 9 : 1),
Ausbeute : 28,7 % der Theorie.
6-(4-Chlor-butoxy)-4,4-dicyclohexyl-4H-3,1-benzoxazin-2-on
Schmelzpunkt : 231-232 °C,
Ausbeute : 72,8 % der Theorie.
6-(4-Chlor-butoxy)-4,4-diäthyl-4H-3,1-benzoxazin-2-on
Schmelzpunkt : 90-92 °C,
Ausbeute : 99,4 % der Theorie.
6-(4-Chlor-butoxy)-4-methyl-4H-3,1-benzoxazin-2-on
Öl, RF-Wert : 0,8 (Kieselgelplatte : Chloroform/Äthanol = 9 : 1),
Ausbeute : 32,1 % der Theorie.
8-(4-Chlor-butoxy)-4,4-dimethyl-4H-3,1-benzoxazin-2-on
Schmelzpunkt : 155-156 °C,
Ausbeute : 93,8 % der Theorie.
6-(4-Chlor-butoxy)-4,4,8-trimethyl-4H-3,1-benzoxazin-2-on
Schmelzpunkt : 149-150 °C,
Ausbeute : 95,6 % der Theorie.

Beispiel H

7-(4-Methansulfonyloxy-butoxy)-4,4-dimethyl-4H-3,1-benzoxazin-2-on

5,3 g (0,02 Mol) 7-(4-Hydroxy-butoxy)-4,4-dimethyl-4H-3,1-benzoxazin-2-on werden in 20 ml Pyridin gelöst und unter Rühren bei 0-5 °C mit 4,6 g (0,04 Mol) Methansulfonsäurechlorid tropfenweise versetzt. Nach 10-minütigem Nachrühren wird mit Eiswasser/Salzsäure versetzt und die Mischung mit Chloroform extrahiert. Nach Waschen des Extraktes mit Wasser und Trocknen mit Natriumsulfat wird das Lösungsmittel im Vakuum abdestilliert. Den festen Rückstand verrührt man mit Diisopropyläther, saugt ab und trocknet.
Schmelzpunkt : 134-136 °C,
Ausbeute : 6,4 g (93,3 % der Theorie).
Analog wird folgende Verbindung hergestellt :

6-(4-Methansulfonyloxy-butoxy)-4,4-dimethyl-7-nitro-4H-3,1-benzoxazin-2-on
Schmelzpunkt : 149-151 °C,
Ausbeute : 76,6 % der Theorie.

Beispiel I

3-Methoxy-anthranilsäure-methylester

133 g (0,63 Mol) 3-Methoxy-2-nitro-benzoesäure-methylester werden in 1 000 ml Toluol gelöst und im

Autoklaven bei Raumtemperatur und 5 bar Wasserstoffdruck in Gegenwart von Platin im Verlauf von 2 Stunden hydriert. Man filtriert vom Katalysator ab, trocknet mit Natriumsulfat und destilliert das Toluol im Vakuum ab.

Öl, RF-Wert : 0,8 (Kieselgelplatte ; Chloroform/Aceton = 9 : 1),
Ausbeute : 110 g (96,4 % der Theorie).

Wegen relativer Instabilität wird die Substanz zweckmäßigerweise nicht aufbewahrt, sondern alsbald analog Beispiel A weiter umgesetzt.

Beispiel J

6-Methoxy-anthranilsäure-methylester-hydrochlorid

a) 2-Cyano-3-methoxy-anilin

110 g (0,617 Mol) 2-Methoxy-6-nitro-benzonitril werden in 1 l Methanol gelöst und in Gegenwart von 10 %iger Palladiumkohle bei Raumtemperatur und 5 bar Wasserstoffdruck innerhalb einer Stunde im Autoklaven hydriert. Man filtriert vom Katalysator ab, destilliert das Lösungsmittel ab und kristallisiert den Rückstand aus Äthanol/Diisopropyläther um.

Schmelzpunkt : 142-143 °C,
Ausbeute : 54,8 g (59,9 % der Theorie).

b) 6-Methoxy-anthranilsäure

54 g (0,364 Mol) des unter a) hergestellten Produktes werden mit 800 ml einer 25 %igen wässerigen Lösung von Kaliumhydroxid 16 Stunden lang zum Rückfluß erhitzt. Nach dem Abkühlen wird die Reaktionslösung durch Zugabe von Essigsäure auf $p_H = 6$ gebracht und mehrere Male mit je 1/2 l Essigester extrahiert. Die vereinigten Extrakte werden mit Natriumsulfat getrocknet und der Essigester abdestilliert. Der Rückstand wird säulenchromatographisch gereinigt (Kieselgel, Äthylenchlorid/Aceton = 19 : 1). Die einheitlichen Fraktionen werden zur Trockne eingeengt.

Schmelzpunkt : 76-78 °C,
Ausbeute : 37,5 g (61,7 % der Theorie).

c) 6-Methoxy-anthranilsäure-methylester-hydrochlorid

Die unter b) erhaltene Säure (37,5 g = 0,22 Mol) wird in 500 ml Methanol gelöst und in die Lösung 2 Stunden lang über konz. Schwefelsäure getrockneter Chlorwasserstoff eingeleitet. Dabei fällt ein dicker Kristallbrei an, der mit 500 ml Äther versetzt, abgesaugt und nochmals mit Äther gewaschen wird.

Schmelzpunkt : 209 °C (Zers.),
Ausbeute : 36,5 g (75,2 % der Theorie).

Beispiel K

4-Methoxy-anthranilsäure-methylester

a) 3-(Isonitroso-acetamido)-anisol

1 480 g (10,38 Mol) Natriumsulfat und 225 g (1,36 Mol) Chloralhydrat werden in 6 l Wasser gelöst und auf 50 °C erhitzt. Dazu gibt man unter Rühren eine Lösung von 154 g (1,25 Mol) m-Anisidin in 750 ml Wasser und 125 ml konz. Salzsäure. Nach weiteren 5 Minuten versetzt man mit einer Lösung von 275 g (3,96 Mol) Hydroxylammoniumhydrochlorid in 1,25 l Wasser und erhitzt auf 95 °C. Unter fortwährendem Rühren läßt man auf Zimmertemperatur abkühlen, saugt den Niederschlag ab, wäscht mit Wasser und trocknet.

Schmelzpunkt : 165-166 °C,
Ausbeute : 242,5 g (99,9 % der Theorie).

Analog werden folgende Verbindungen hergestellt :

4-(Isonitroso-acetamido)-3-methyl-anisol
Schmelzpunkt : 166-167 °C, Ausbeute : 94,1 % der Theorie.
4-(Isonitroso-acetamido)-2-methyl-anisol
Schmelzpunkt : 169-171 °C, Ausbeute : 86,2 % der Theorie.
2-Chlor-4-(isonitroso-acetamido)-anisol
Schmelzpunkt : 208 °C, Ausbeute : 91,6 % der Theorie.

b) 6-Methoxy-isatin

In 720 g Polyphosphorsäure rührt man bei 60 °C 140 g (0,72 Mol) 3-(Isonitroso-acetamido)-anisol ein. Dabei erwärmt sich das Gemisch auf ca. 100 °C. Bei dieser Temperatur rührt man noch 30 Minuten, kühlt die Reaktionsmischung dann ab und gießt sie unter Rühren in 4 l 30 °C warmes Wasser. Nach Stehen über Nacht wird der Niederschlag abgesaugt, mit Wasser gewaschen und mit Aceton/Chloroform = 1 : 1 ausgekocht. Die Lösung trocknet man mit Natriumsulfat und destilliert die Lösungsmittel ab. Der Rückstand wird mit Äthanol verrührt, abgesaugt und bei Raumtemperatur getrocknet.

Schmelzpunkt : 237-238 °C,
Ausbeute : 84,2 g (65,9 % der Theorie).
Analog werden folgende Verbindungen hergestellt :

5-Methoxy-7-methyl-isatin
Schmelzpunkt : 265-266 °C, Ausbeute : 36 % der Theorie.
5-Methoxy-6-methyl-isatin
Schmelzpunkt : 254-256 °C, Ausbeute : 87,2 % der Theorie.
6-Chlor-5-methoxy-isatin
Schmelzpunkt : 247 °C, Ausbeute : 33,2 % der Theorie.

c) 4-Methoxy-anthranilsäure-methylester

158 g (0,89 Mol) 6-Methoxy-isatin werden in 1300 ml Methanol suspendiert und mit einer Lösung von 54 g (1,34 Mol) Natriumäthylat in 500 ml Methanol versetzt. Dazu tropft man unter Rühren 108 ml (1,07 Mol) 30 %iges Wasserstoffperoxid und rührt noch 30 Minuten nach. Der Kolbeninhalt wird auf die Hälfte seines Volumens eingeengt, mit Wasser versetzt und mit Eisessig auf $p_H$ = 5-6 eingestellt. Nach dem Extrahieren mit Methylenchlorid wird über Kieselgur abgesaugt, mit Natriumsulfat getrocknet und das Lösungmittel abdestilliert. Den Rückstand kristallisiert man aus Petroläther/Cyclohexan = 2 : 1 um.

Schmelzpunkt : 80-82 °C,
Ausbeute : 109 g (67,7 % der Theorie).
Analog werden folgende Verbindungen hergestellt :

5-Methoxy-3-methyl-anthranilsäuremethylester
Schmelzpunkt : 81-82 °C,
Ausbeute : 59,6 % der Theorie.
5-Methoxy-4-methyl-anthranilsäuremethylester
Schmelzpunkt : 84-86 °C,
Ausbeute : 82,2 % der Theorie.

## Beispiel L

8-Chlor-6-methoxy-4,4-dimethyl-4H-3,1-benzoxazin-2-on und 7-Chlor-6-methoxy-4,4-dimethyl-4H-3,1-benzoxazin-2-on

Eine Lösung von 79 g (0,38 Mol) 6-Methoxy-4,4-dimethyl-4H-3,1-benzoxazin-2-on in 1 000 ml Chloroform wird mit 56,68 g (0,42 Mol) Sulfurylchlorid versetzt und 6 Stunden bei 10-20 °C gerührt. Nach Stehen über Nacht wird die Reaktionsmischung mit einer wässerigen Natriumkarbonat-Lösung versetzt, die organische Phase abgetrennt, mit Wasser gewaschen, mit Natriumsulfat getrocknet und das Chloroform abdestilliert. Der Rückstand wird säulenchromatographisch in die Isomeren aufgetrennt (Kieselgel, Äthylenchlorid/Aceton = 19 : 1). Die einheintlichen Fraktionen werden zur Trockene eingeengt und der Rückstand aus Essigester/Diisopropyläther umkristallisiert.

1) 8-Chlor-6-methoxy-4,4-dimethyl-4H-3,1-benzoxazin-2-on
Schmelzpunkt : 156-157 °C,
Ausbeute : 47,3 g (51,6 % der Theorie).
2) 7-Chlor-6-methoxy-4,4-dimethyl-4H-3,1-benzoxazin-2-on
Schmelzpunkt : 165-167 °C,
Ausbeute : 18,9 g (20,6 % der Theorie).

## Beispiel M

8-Brom-6-methoxy-4,4-dimethyl-4H-3,1-benzoxazin-2-on und 7-Brom-6-methoxy-4,4-dimethyl-4H-3,1-benzoxazin-2-on

82,7 g (0,4 Mol) 6-Methoxy-4,4-dimethyl-4H-3,1-benzoxazin-2-on werden in 800 ml Dioxan gelöst und bei Raumtemperatur tropfenweise mit 67,1 g (21,5 ml = 0,42 Mol) Brom versetzt. Nach 3-stündigem Rühren werden nochmals 67,1 g Brom und nach weiteren 3 Stunden ein drittes Mal 67,1 g Brom zugegeben. Das Rühren wird über Nacht fortgesetzt. Die Reaktionsmischung wird dann mit Eiswasser und Natriumbisulfit-Lösung versetzt und mit Essigester extrahiert. Den Extrakt wäscht man mit Wasser,

trocknet mit Natriumsulfat und destilliert den Essigester ab. Der Rückstand wird säulenchromatographisch in die Isomeren aufgetrennt (Kieselgel, Äthylenchlorid/Aceton = 60 : 1). Die reinen Fraktionen werden eingeengt und aus Essigester/Diisopropyläther umkristallisiert.

1) 8-Brom-6-methoxy-4,4-dimethyl-4H-3,1-benzoxazin-2-on

Schmelzpunkt : 128-130 °C (enthält noch wenig 7,8-Dibrom-6-methoxy-4,4-dimethyl-4H-3,1-benzoxazin-2-on)

Ausbeute : 45 g (39,3 % der Theorie).

2) 7-Brom-6-methoxy-4,4-dimethyl-4H-3,1-benzoxazin-2-on

Schmelzpunkt : 174-175 °C,

Ausbeute : 33,7 g (29,5 % der Theorie).

Beispiel N

6-Acetoxy-8-chlor-4H-3,1-benzoxazin-2-on

14,5 g (0,07 Mol) 6-Acetoxy-4H-3,1-benzoxazin-2-on (hergestellt aus 6-Hydroxy-4H-3,1-benzoxazin-2-on und Acetanhydrid, Schmelzpunkt : 173-175 °C) werden in 150 ml Eisessig gelöst, mit 10,8 g (6,5 ml = 0,08 Mol) Sulfurylchlorid versetzt und 36 Stunden bei Raumtemperatur gerührt. Nach Zusatz von 30 ml Äther wird auf 0 °C abgekühlt, der Niederschlag abgesaugt, mit Äther gewaschen und getrocknet.

Schmelzpunkt : 204-205 °C,

Ausbeute : 7,7 g (45,5 % der Theorie).

Beispiel O

8-Chlor-6-hydroxy-4H-3,1-benzoxazin-2-on

7,6 g (31,4 mMol) 6-Acetoxy-8-chlor-4H-3,1-benzoxazin-2-on werden in 50 ml Methanol suspendiert und unter Rühren mit 20 ml (40 mMol) 2n Natronlauge versetzt. Die sofort entstehende klare Lösung wird noch 10 Minuten bei Raumtemperatur gerührt mit Wasser versetzt und mit Essigsäure sauer gestellt. Der Niederschlag wird abgesaugt und aus Isopropanol umkristallisiert.

Schmelzpunkt : 250 °C (Zersetzung),

Ausbeute : 3,3 g (52,7 % der Theorie).

Herstellung der Endprodukte

Beispiel 1

6-[4-(4-Brom-phenylmercapto)-butoxy]-4,4-dimethyl-4H-3,1-benzoxazin-2-on

6,23 g (0,033 Mol) 4-Bromthiophenol und 8,28 g (0,06 Mol) Kaliumcarbonat werden in 100 ml über Molekularsieb getrocknetem Dimethylsulfoxid unter einer Stickstoffatmosphäre bei Raumtemperatur 15 Minuten lang gerührt und dann mit 8,51 g (0,03 Mol) 6-(4-Chlorbutoxy)-4,4-dimethyl-4H-3,1-benzoxazin-2-on versetzt. Nach 1-stündigem Rühren bei Raumtemperatur versetzt man portionsweise mit Wasser, bis ein dicker Kristallbrei entstanden ist. Man saugt ab, trocknet und kristallisiert aus Essigester/Diisopropyläther um.

Schmelzpunkt : 151-152 °C,

Ausbeute : 9,2 g (70,3 % der Theorie).

Beispiel 2

6-[4-(4-Brom-3-methyl-phenylsulfinyl)-butoxy]-4,4-dimethyl-4H-3,1-benzoxazin-2-on

9,2 g (0,02 Mol) 6-[4-(4-Brom-3-methyl-phenylmercapto)-butoxy]-4,4-dimethyl-4H-3,1-benzoxazin-2-on werden in 100 ml Eisessig gelöst und mit 2 ml (0,02 Mol) 30 %igem Wasserstoffperoxid versetzt. Man rührt 3 Stunden bei Raumtemperatur, gießt auf 400 ml Wasser und extrahiert 3 mal mit Essigester. Die organische Phase wird mit Wasser gewaschen, mit Natriumsulfat getrocknet und der Essigester abdestilliert. Der feste Rückstand wird aus Essigester/Diisopropyläther umkristallisiert.

Schmelzpunkt : 133-134 °C,

Ausbeute : 8,1 g (85,2 % der Theorie).

Beispiel 3

4,4-Dimethyl-6-[4-(2-pyridylsulfonyl)-butoxyl-4H-3,1-benzoxazin-2-on

3,58 g (0,01 Mol) 4,4-Dimethyl-6-[4-(2-pyridylmercapto)-butoxy]-4H-3,1-benzoxazin-2-on werden in

60 ml Ameisensäure gelöst und mit 2,5 ml (0,025 Mol) 30 %igem Wasserstoffperoxid versetzt. Nach 3-stündigem Rühren bei Raumtemperatur wird portionsweise mit Wasser verdünnt, mit Essigester extrahiert, die organische Phase mit Natriumsulfat getrocknet und der Essigester abdestilliert. Zur Reinigung wird an einer Kieselgelsäule (0,05-0,2 mm Teilchengröße) mit einem Gemisch von Chloroform/Äthanol (40 : 1) chromatographiert und die einheitlichen Fraktionen nach dem Abdestillieren des Elutionsgemisches aus Essigester umkristallisiert.

Schmelzpunkt : 142-143 °C,
Ausbeute : 2,1 g (53,8 % der Theorie).

Beispiel 4

6-[4-(3,4-Dichlor-phenylsulfinyl)-butoxy]-4,4-dimethyl-4H-3,1-benzoxazin-2-on

Eine Lösung von 1,9 g (0,01 Mol) 6-Hydroxy-4,4-dimethyl-4H-3,1-benzoxazin-2-on und 4,1 g (0,012 5 Mol) 4-(3,4-Dichlorphenyl-sulfinyl)-butylbromid (Schmelzpunkt : 63-64 °C) in 40 ml Dimethylsulfoxid wird mit 3,5 g (0,025 Mol) Kaliumcarbonat versetzt und 3 Stunden lang bei 40 °C gerührt. Nach dem Abkühlen wird mit reichlich Eiswasser versetzt und mit Chloroform extrahiert. Der Extrakt wird mit Wasser gewaschen, mit Natriumsulfat getrocknet und das Chloroform abdestilliert. Den Rückstand kristallisiert man aus Isopropanol/Diisopropyläther um.

Schmelzpunkt : 138 °C,
Ausbeute : 1,9 g (43 % der Theorie).

Beispiel 5

6-(4-Phenylmercapto-butoxy)-4H-3,1-benzoxazin-2-on

Zu einer äthanolischen Natriumäthylat-Lösung, hergestellt aus 150 ml Äthanol und 0,92 g (9,04 Mol) Natrium, werden 4,95 g (0,03 Mol) 6-Hydroxy-4H-3,1-benzoxazin-2-on gegeben und das Gemisch zum Rückfluß erhitzt. In die heiße Lösung werden 10 g (0,04 Mol) 4-Phenylmercapto-butylbromid (hergestellt aus Thiophenol und überschüssigem 1,4-Di-Brom-butan, $Kp_{0,03\ mb}$ : = 95-104 °C) eingetragen und weitere 2 Stunden zum Rückfluß erhitzt. Die abgekühlte Reaktionslösung wird auf Eiswasser gegossen und mit Essigester extrahiert. Der Extrakt wird mit Wasser gewaschen, mit Natriumsulfat getrocknet und der Essigester im Vakuum abdestilliert. Den Rückstand kristallisiert man aus Cyclohexan/Essigester um.

Schmelzpunkt : 96-97 °C,
Ausbeute : 51;7 % der Theorie.

Beispiel 6

6-[4-(3,4-Dichlor-phenylsulfoximino)-butoxy]-4,4-dimethyl-4H-3,1-benzoxazin-2-on

Eine Lösung von 32,65 g (0,074 Mol) 6-[4-(3,4-Dichlor-phenylsulfinyl)-butoxy]-4,4-dimethyl-4H-3,1-benzoxazin-2-on in 300 ml Dimethylformamid wird mit 66,6 g (0,35 Mol) p-Toluol-sulfonsäuremonohydrat und 42,9 g (0,15 Mol) O-Mesitylensulfonyl-acethydroxamsäure-äthylester versetzt und 30 Stunden bei Raumtemperatur gerührt. Nach Zugabe von Eiswasser wird mit Essigester extrahiert, die Essigesterphase mit Wasser gewaschen, mit Natriumsulfat getrocknet und der Essigester im Vakuum abdestilliert. Der Rückstand wird in 200 ml Methanol gelöst und die Lösung mit soviel 2 n Natronlauge versetzt, daß sie alkalisch reagiert. Durch Zugabe von Wasser wird das Reaktionsprodukt ausgefällt, mit Essigester extrahiert, der Extrakt mit Wasser gewaschen und mit Natriumsulfat getrocknet. Der nach dem Abdestillieren des Essigesters verbleibende Rückstand wird aus Essigester/Diisopropyläther umkristallisiert.

Schmelzpunkt : 166-167 °C,
Ausbeute : 28,3 g (83,8 % der Theorie).

Beispiel 7

6-[4-(2-Pyridylsulfinyl)-butoxy]-4,4-dimethyl-4H-3,1-benzoxazin-2-on

3,58 g (0,01 Mol) 6-[4-(2-Pyridylmercapto)-butoxy]-4,4-dimethyl-4H-3,1-benzoxazin-2-on werden in 100 ml Chloroform gelöst und unter Rühren mit 4,3 g (0,025 Mol) m-Chlor-perbenzoesäure versetzt. Nach 2-stündigem Rühren gibt man nochmals 2 g m-Chlorperbenzoesäure zu und läßt über Nacht stehen. Man schüttelt die Chloroformphase mit Wasser aus, trocknet die organische Phase mit Natriumsulfat und destilliert das Chloroform ab. Der uneinheitliche Rückstand wird durch Säulenchromatographie (Kieselgel ; Korngröße : 0,05-0,2 mm, Chloroform : Äthanol = 40 : 1) gereinigt. Die einheitlichen Fraktionen werden eingeengt und der Rückstand mit Essigester/Äther verrieben. Die so erhaltenen Kristalle werden abgesaugt und bei 50 °C getrocknet.

Schmelzpunkt : 90-91 °C (Zers.),
Ausbeute : 1,0 g (26,7 % der Theorie).

Beispiel 8

6-[4-(3,4-Dichlor-phenylsulfinyl)-butoxy]-4,4-dimethyl-4H-3,1-benzoxazin-2-on

2,14 g (0,005 Mol) 6-[4-(3,4-Dichlor-phenylmercapto)-butoxy]-4,4-dimethyl-4H-3,1-benzoxazin-2-on werden in 50 ml Methanol angelöst, mit einer Lösung von 1,3 g (0,06 Mol) Natriummetaperjodat in 10 ml Wasser versetzt und 2 Stunden lang bei Raumtemperatur gerührt. Danach wird noch 3 Stunden lang zum Rückfluß erhitzt, das Volumen der Reaktionsmischung auf etwa 3/4 eingeengt und mit Wasser verrührt. Das ausgeschiedene Öl wird durch Abdekantieren vom Lösungsmittel getrennt und in heißem Isopropanol/Diisopropyläther aufgenommen. Beim Abkühlen fallen Kristalle vom Schmelzpunkt 138-139 °C aus.
Ausbeute : 1,6 g (72,4 % der Theorie).

Beispiel 9

6-[4-(3,4-Dichlor-phenylsulfinyl)-butoxy]-4,4-dimethyl-4H-3,1-benzoxazin-2-on

Zu einer Lösung von 3,2 g (0,007 5 Mol) 6-[4-(3,4-Dichlorphenylmercapto)-butoxy]-4,4-dimethyl-4H-3,1-benzoxazin-2-on in 50 ml Methylenchlorid tropft man bei — 70 °C eine Lösung von 1 g (0,008 5 Mol) Sulfurylchlorid in 5 ml Methylenchlorid unter Rühren und setzt nach beendeter Zugabe das Rühren bei — 70 °C noch 15 Stunden lang fort. Nach Erwärmen auf Raumtemperatur gibt man unter starkem Rühren wässrige Sodalösung zu, trennt die organische Phase von der wässerigen ab, wäscht sie mit Wasser und trocknet mit Natriumsulfat. Den nach dem Abdestillieren des Methylenchlorids verbleibenden Rückstand kristallisiert man aus Isopropanol/Diisopropyläther um.
Schmelzpunkt : 138-139 °C,
Ausbeute : 2,1 g (63,3 % der Theorie).

Beispiel 10

6-[4-(3,4-Dichlor-phenylsulfinyl)-butoxy]-4,4-dimethyl-4H-3,1-benzoxazin-2-on

Zu einer Lösung von 2,13 g (0,005 Mol) 6-[4-(3,4-Dichlorphenylmercapto)-butoxy]-4,4-dimethyl-4H-3,1-benzoxazin-2-on in 50 ml Methanol gibt man unter Rühren nach und nach 1 g (0,005 6 Mol) N-Bromsuccinimid. Nach 24-stündiger Reaktionszeit werden 500 ml 80 °C heißes Wasser zugegeben und der Niederschlag nach 2-stündigem Rühren abgesaugt. Durch Umkristallisieren aus Isopropanol/Diisopropyläther erhält man Kristalle vom Schmelzpunkt 138-139 °C.
Ausbeute : 1,55 g (70,1 % der Theorie).

Beispiel 11

6-[4-(3,4-Dichlor-phenylsulfinyl)-butoxy]-1,4,4-trimethyl-4H-3,1-benzoxazin-2-on

Eine Lösung von 1,1 g (0,002 5 Mol) 6-[4-(3,4-Dichlor-phenylsulfinyl)-butoxy]-4,4-dimethyl-4H-3,1-benzoxazin-2-on und 1 ml Methyljodid in 30 ml Methylenchlorid werden mit 30 ml 2n Natronlauge (0,06 Mol) und einer Spatelspitze Tetrabutylammonium-hydrogensulfat versetzt und 18 Stunden bei Raumtemperatur gerührt. Die organische Phase wird abgetrennt, zweimal mit Wasser gewaschen, mit Natriumsulfat getrocknet und das Methylenchlorid abdestilliert. Der Rückstand wird durch Säulenchromatographie (Kieselgel, Chloroform/Aceton = 40 : 1) gereinigt. Die einheitlichen Fraktionen werden eingeengt, der Rückstand mit Diisopropyläther verrührt, der Kristallbrei abgesaugt und getrocknet.
Schmelzpunkt : 90-92 °C,
Ausbeute : 0,31 g (27,3 % der Theorie).

Beispiel 12

6-[4-(3,4-Dichlor-N-methansulfonyl-phenylsulfoximino)-butoxy]-4,4-dimethyl-4H-3,1-benzoxazin-2-on

2,3 g (0,005 Mol) 6-[4-(3,4-Dichlor-phenylsulfoximino)-butoxy]-4,4-dimethyl-4H-3,1-benzoxazin-2-on werden in 50 ml Chloroform gelöst und mit 0,5 ml Pyridin versetzt. Zu dieser Lösung gibt man 1,2 g (0,01 Mol) Methansulfonylchlorid und rührt 20 Stunden bei Raumtemperatur. Die Chloroformphase wird mehrmals mit Wasser gewaschen, mit Natriumsulfat getrocknet und das Chloroform abdestilliert. Den Rückstand reinigt man durch Säulenchromatographie (Kieselgel, Chloroform/Äthanol = 30 : 1). Die einheitlichen Fraktionen werden eingeengt und der Rückstand aus Butanol/Dimethylformamid umkristallisiert.

Schmelzpunkt : 198-200 °C,
Ausbeute : 2,4 g (89,6 % der Theorie).

## Beispiel 13

6-[4-(3,4-Dimethoxy-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 3,4-Dimethoxy-thiophenol.
Schmelzpunkt : 140-142 °C,
Ausbeute : 65,9 % der Theorie.

## Beispiel 14

6-[4-(4-Methoxy-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Methoxy-thiophenol.
Schmelzpunkt : 92-94 °C,
Ausbeute : 55,5 % der Theorie.

## Beispiel 15

6-[4-(4-Hydroxy-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Hydroxy-thiophenol.
Schmelzpunkt : 152-154 °C,
Ausbeute : 92,8 % der Theorie.

## Beispiel 16

6-[4-(4-Biphenylylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Phenyl-thiophenol.
Schmelzpunkt : 120-122 °C,
Ausbeute : 87,2 % der Theorie.

## Beispiel 17

6-[4-(3-Methoxy-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 3-Methoxy-thiophenol.
Schmelzpunkt : 115-116 °C,
Ausbeute : 79,6 % der Theorie.

## Beispiel 18

6-[4-(3-Methyl-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 3-Methyl-thiophenol.
Schmelzpunkt : 103-105 °C,
Ausbeute : 80,8 % der Theorie.

## Beispiel 19

6-[4-(3,5-Di-tert.butyl-4-hydroxy-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 3,5-Di-tert.butyl-4-hydroxythiophenol.
Schmelzpunkt : 86-87 °C,
Ausbeute : 76,2 % der Theorie.

Beispiel 20

6-[4-(4-Amino-3,5-dibrom-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Amino-3,5-dibrom-thiophenol.
Schmelzpunkt : 152-155 °C,
Ausbeute : 85,5 % der Theorie.

Beispiel 21

6-(4-n-Octylmercapto-butoxy)-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und n-Octylmercaptan.
Öl, RF-Wert : 0,8 (Kieselgelplatte ; Chloroform/Aceton = 9 : 1)
Ausbeute : 96,1 % der Theorie,
$C_{22}H_{35}NO_3S$ (393,59)
Ber. : C 67,14 H 8,96 N 3,56 S 8,15
Gef. : C 67,31 H 9,00 N 3,57 S 8,25

Beispiel 22

6-(4-Cyclohexylmercapto-butoxy)-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und Cyclohexylmercaptan.
Schmelzpunkt : 105-107 °C,
Ausbeute : 82,5 % der Theorie.

Beispiel 23

6-[4-(4-Methyl-phenylmercapto)-butoxy]-4,4-di-n-hexyl-4H-3,1-benzoxazin-2-on

Hergestellt analog Beispiel 1 aus 6-(4-Chlorbutoxy)-4,4-di-n-hexyl-4H-3,1-benzoxazin-2-on und 4-Methyl-thiophenol.
Schmelzpunkt : 76-78 °C,
Ausbeute : 78,1 % der Theorie.

Beispiel 24

6-[4-(4-Cyclohexyl-phenylmercapto)-butoxy]-4,4-di-n-hexyl-4H-3,1-benzoxazin-2-on

Hergestellt analog Beispiel 1 aus 6-(4-Chlorbutoxy)-4,4-di-n-hexyl-4H-3,1-benzoxazin-2-on (Öl, RF-Wert : 0,7 ; Kieselgelplatte ; Chloroform/Aceton = 19 : 1) und 4-Cyclohexyl-thiophenol.
Schmelzpunkt : 87-89 °C,
Ausbeute : 75,4 % der Theorie.

Beispiel 25

6-[4-(3,4-Dichlor-phenylmercapto)-butoxy]-4,4-di-n-hexyl-4H-3,1-benzoxazin-2-on

Hergestellt analog Beispiel 1 aus 6-(4-Chlorbutoxy)-4,4-di-n-hexyl-4H-3,1-benzoxazin-2-on und 3,4-Dichlor-thiophenol.
Schmelzpunkt : 63-64 °C,
Ausbeute : 67,9 % der Theorie.

Beispiel 26

6-[4-(4-Acetamido-phenylmercapto)-butoxy]-4,4-di-n-hexyl-4H-3,1-benzoxazin-2-on

Hergestellt analog Beispiel 1 aus 6-(4-Chlorbutoxy)-4,4-di-n-hexyl-4H-3,1-benzoxazin-2-on und 4-Acetamido-thiophenol.
Öl, RF-Wert : 0,5 (Kieselgelplatte : Chloroform/Äthanol = 9 : 1),

Ausbeute : 98,2 % der Theorie.

Beispiel 27

6-[4-(3,4-Dichlor-phenylmercapto)-butoxy]-4,4-dicyclohexyl-4H-3,1-benzoxazin-2-on

Hergestellt analog Beispiel 1 aus 6-(4-Chlorbutoxy)-4,4-dicyclohexyl-4H-3,1-benzoxazin-2-on und 3,4-Dichlor-thiophenol.
Schmelzpunkt : 181-182 °C,
Ausbeute : 77 % der Theorie.

Beispiel 28

6-[4-(3,4-Dichlor-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 3,4-Dichlor-thiophenol.
Schmelzpunkt : 155-156 °C,
Ausbeute : 72,8 % der Theorie.

Beispiel 29

6-[4-(4-Acetamido-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Acetamido-thiophenol.
Schmelzpunkt : 158 °C,
Ausbeute : 56,3 % der Theorie.

Beispiel 30

6-[4-(2-Pyridylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 2-Mercapto-pyridin.
Schmelzpunkt : 137-138 °C,
Ausbeute : 72,5 % der Theorie.

Beispiel 31

6-[4-(4-Cyclohexyl-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Cyclohexyl-thiophenol.
Schmelzpunkt : 109-110 °C,
Ausbeute : 47 % der Theorie.

Beispiel 32

6-[4-(4-Methyl-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Methyl-thiophenol.
Schmelzpunkt : 120-121 °C,
Ausbeute : 69,1 % der Theorie.

Beispiel 33

6-[4-(4-Brom-3-methyl-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Brom-3-methyl-thiophenol.
Schmelzpunkt : 129-130 °C,
Ausbeute : 57,0 % der Theorie.

Beispiel 34

6-[4-(4-Fluor-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Fluor-thiophenol.
Schmelzpunkt : 125-126 °C,
Ausbeute : 75,5 % der Theorie.

Beispiel 35

6-[4-(4-tert.Butyl-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-tert.Butyl-thiophenol.
Schmelzpunkt : 119-120 °C,
Ausbeute : 76,6 % der Theorie.

Beispiel 36

6-(4-Benzylmercapto-butoxy)-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und Benzylmercaptan.
Schmelzpunkt : 90-92 °C,
Ausbeute : 64,6 % der Theorie.

Beispiel 37

6-(4-Methylmercapto-butoxy)-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und Methylmercaptan.
Schmelzpunkt : 98-99 °C,
Ausbeute : 75,4 % der Theorie.

Beispiel 38

6-[4-(4-Methyl-phenylmercapto)-butoxy]-4,4-dicyclohexyl-4H-3,1-benzoxazin-2-on

Hergestellt analog Beispiel 1 aus 6-(4-Chlorbutoxy)-4,4-dicyclohexyl-4H-3,1-benzoxazin-2-on und 4-Methyl-thiophenol.
Schmelzpunkt : 149-150 °C,
Ausbeute : 81,4 % der Theorie.

Beispiel 39

6-[4-(4-Methyl-phenylmercapto)-butoxy]-4H-3,1-benzoxazin-2-on

Hergestellt analog Beispiel 1 aus 6-(4-Chlorbutoxy)-4H-3,1-benzoxazin-2-on und 4-Methylthio-phenol.
Schmelzpunkt : 125-126 °C,
Ausbeute : 74,7 % der Theorie.

Beispiel 40

6-[4-(4-Cyclohexyl-phenylmercapto)-butoxy]-4,4-dicyclohexyl-4H-3,1-benzoxazin-2-on

Hergestellt analog Beispiel 1 aus 6-(4-Chlorbutoxy)-4,4-dicyclohexyl-4H-3,1-benzoxazin-2-on und 4-Cyclohexyl-thiophenol.
Schmelzpunkt : 173-175 °C,
Ausbeute : 82,3 % der Theorie.

Beispiel 41

6-[4-Cyclohexyl-phenylmercapto)-butoxy]-4H-3,1-benzoxazin-2-on

Hergestellt analog Beispiel 1 aus 6-(4-Chlorbutoxy)-4H-3,1-benzoxazin-2-on und 4-Cyclohexyl-thiophenol.
Schmelzpunkt : 134-135 °C,
Ausbeute : 60,3 % der Theorie.

Beispiel 42

6-[4-(4-Acetamido-phenylmercapto)-butoxy]-4,4-dicyclohexyl-4H-3,1-benzoxazin-2-on

Hergestellt analog Beispiel 1 aus 6-(4-Chlorbutoxy)-4,4-dicyclohexyl-4H-3,1-benzoxazin-2-on und 4-Acetamido-thiophenol.
Schmelzpunkt : 115 °C (Zers.),
Ausbeute : 96,8 % der Theorie.

Beispiel 43

6-[4-(4-Acetamido-phenylmercapto)-butoxy]-4H-3,1-benzoxazin-2-on

Hergestellt analog Beispiel 1 aus 6-(4-Chlorbutoxy)-4H-3,1-benzoxazin-2-on und 4-Acetamido-thiophenol.
Schmelzpunkt : 163-164 °C,
Ausbeute : 71,6 % der Theorie.

Beispiel 44

6-[2-(3,4-Dichlor-phenylmercapto)-äthoxy]-4,4-dimethyl-4H-3,1-benzoxazin-2-on

Hergestellt analog Beispiel 1 aus 6-(2-Chlor-äthoxy)-4,4-dimethyl-4H-3,1-benzoxazin-2-on und 3,4-Dichlor-thiophenol.
Schmelzpunkt : 145-146 °C,
Ausbeute : 69,1 % der Theorie.

Beispiel 45

6-[3-(3,4-Dichlor-phenylmercapto)-propoxy]-4,4-dimethyl-4H-3,1-benzoxazin-2-on

Hergestellt analog Beispiel 1 aus 6-(3-Chlorpropoxy)-4,4-dimethyl-4H-3,1-benzoxazin-2-on und 3,4-Dichlor-thiophenol.
Schmelzpunkt : 115-116 °C,
Ausbeute : 88,9 % der Theorie.

Beispiel 46

6-[3-(4-Cyclohexyl-phenylmercapto)-propoxy]-4,4-dimethyl-4H-3,1-benzoxazin-2-on

Hergestellt analog Beispiel 1 aus 6-(3-Chlorpropoxy)-4,4-dimethyl-4H-3,1-benzoxazin-2-on und 4-Cyclohexyl-thiophenol.
Schmelzpunkt : 113-114 °C,
Ausbeute : 58,1 % der Theorie.

Beispiel 47

6-[4-(3,4-Dimethyl-phenylmercapto)-butoxy]-4H-3,1-benzoxazin-2-on

Hergestellt analog Beispiel 1 aus 6-(4-Chlorbutoxy)-4H-3,1-benzoxazin-2-on und 3,4-Dimethyl-thiophenol.
Schmelzpunkt : 124-125 °C,
Ausbeute : 73 % der Theorie.

Beispiel 48

6-[5-(4-Cyclohexyl-phenylmercapto)-pentoxy]-4,4-dimethyl-4H-3,1-benzoxazin-2-on

Hergestellt analog Beispiel 1 aus 6-(5-Brompentoxy)-4,4-dimethyl-4H-3,1-benzoxazin-2-on und 4-Cyclohexyl-thiophenol.

Schmelzpunkt : 108-110 °C,

Ausbeute : 62,6 % der Theorie.

Beispiel 49

6-[5-(3,4-Dichlor-phenylmercapto)-pentoxy]-4,4-dimethyl-4H-3,1-benzoxazin-2-on

Hergestellt analog Beispiel 1 aus 6-(5-Brompentoxy)-4,4-dimethyl-4H-3,1-benzoxazin-2-on und 3,4-Dichlor-thiophenol.

Schmelzpunkt : 110-112 °C,

Ausbeute : 81,3 % der Theorie.

Beispiel 50

6-[4-(4-tert.Butyl-phenylsulfinyl)-butoxy]-4,4-dimethyl-4H-3,1-benzoxazin-2-on

Hergestellt analog Beispiel 2 aus 6-[4-(4-tert.Butyl-phenylmercapto)-butoxy]-4,4-dimethyl-4H-3,1-benzoxazin-2-on und Wasserstoffperoxid.

Schmelzpunkt : 115 °C,

Ausbeute : 53,8 % der Theorie.

Beispiel 51

6-[4-(4-Acetamido-phenylsulfinyl)-butoxy]-4,4-dimethyl-4H-3,1-benzoxazin-2-on

Hergestellt analog Beispiel 2 aus 6-[4-(4-Acetamido-phenylmercapto)-butoxy]-4,4-dimethyl-4H-3,1-benzoxazin-2-on und Wasserstoffperoxid.

Schmelzpunkt : 180 °C,

Ausbeute : 55,9 % der Theorie.

Beispiel 52

6-(4-Phenylsulfinyl-butoxy)-4,4-dimethyl-4H-3,1-benzoxazin-2-on

Hergestellt analog Beispiel 2 aus 6-(4-Phenylmercapto-butoxy)-4,4-dimethyl-4H-3,1-benzoxazin-2-on und Wasserstoffperoxid.

Schmelzpunkt : 64-66 °C,

Ausbeute : 68,4 % der Theorie.

Beispiel 53

6-[4-(3,4-Dichlor-phenylsulfinyl)-butoxy]-4,4-dimethyl-4H-3,1-benzoxazin-2-on

Hergestellt analog Beispiel 2 aus 6-[4-(3,4-Dichlor-phenylmercapto)-butoxy]-4,4-dimethyl-4H-3,1-benzoxazin-2-on und Wasserstoffperoxid.

Schmelzpunkt : 139-140 °C,

Ausbeute : 83,8 % der Theorie.

Beispiel 54

6-[4-(4-Chlor-phenylsulfinyl)-butoxy]-4,4-dimethyl-4H-3,1-benzoxazin-2-on

Hergestellt analog Beispiel 2 aus 6-[4-(Chlor-phenylmercapto)-butoxy]-4,4-dimethyl-4H-3,1-benzoxazin-2-on und Wasserstoffperoxid.

Schmelzpunkt : 135-136 °C,

Ausbeute : 66,3 % der Theorie.

Beispiel 55

6-[4-(4-Cyclohexyl-phenylsulfinyl)-butoxy]-4,4-dimethyl-4H-3,1-benzoxazin-2-on

Hergestellt analog Beispiel 2 aus 6-[4-(4-Cyclohexyl-phenylmercapto)-butoxy]-4,4-dimethyl-4H-3,1-benzoxazin-2-on und Wasserstoffperoxid.

Schmelzpunkt : 147-148 °C,
Ausbeute : 74,3 % der Theorie.

Beispiel 56

6-[4-(4-Methyl-phenylsulfinyl)-butoxy]-4,4-dimethyl-4H-3,1-benzoxazin-2-on

Hergestellt analog Beispiel 2 aus 6-[4-(4-Methyl-phenylmercapto)-butoxy]-4,4-dimethyl-4H-3,1-benzoxazin-2-on und Wasserstoffperoxid.
Schmelzpunkt : 124-125 °C,
Ausbeute : 76,0 % der Theorie.

Beispiel 57

6-[4-(4-Fluor-phenylsulfinyl)-butoxy]-4,4-dimethyl-4H-3,1-benzoxazin-2-on

Hergestellt analog Beispiel 2 aus 6-[4-(4-Fluor-phenylmercapto)-butoxy]-4,4-dimethyl-4H-3,1-benzoxazin-2-on und Wasserstoffperoxid.
Schmelzpunkt : 118-120 °C,
Ausbeute : 79,2 % der Theorie.

Beispiel 58

6-[4-(3,4-Dimethoxy-phenylsulfinyl)-butoxy]-4,4-dimethyl-4H-3,1-benzoxazin-2-on

Hergestellt analog Beispiel 2 aus 6-[4-(3,4-Dimethoxy-phenylmercapto)-butoxy]-4,4-dimethyl-4H-3,1-benzoxazin-2-on und Wasserstoffperoxid.
Schmelzpunkt : 157-158 °C,
Ausbeute : 82,6 % der Theorie.

Beispiel 59

6-[4-(4-Methoxy-phenylsulfinyl)-butoxy]-4,4-dimethyl-4H-3,1-benzoxazin-2-on

Hergestellt analog Beispiel 2 aus 6-[4-(4-Methoxy-phenylmercapto)-butoxy]-4,4-dimethyl-4H-3,1-benzoxazin-2-on und Wasserstoffperoxid.
Schmelzpunkt : 130-133 °C,
Ausbeute : 82,9 % der Theorie.

Beispiel 60

6-[4-(4-Hydroxy-phenylsulfinyl)-butoxy]-4,4-dimethyl-4H-3,1-benzoxazin-2-on

Hergestellt analog Beispiel 2 aus 6-[4-(4-Hydroxy-phenylmercapto)-butoxy]-4,4-dimethyl-4H-3,1-benzoxazin-2-on und Wasserstoffperoxid.
Schmelzpunkt : 157-160 °C,
Ausbeute : 44,9 % der Theorie.

Beispiel 61

6-[4-(4-Biphenylylsulfinyl)-butoxy]-4,4-dimethyl-4H-3,1-benzoxazin-2-on

Hergestellt analog Beispiel 2 aus 6-[4-(4-Biphenylylmercapto)-butoxy]-4,4-dimethyl-4H-3,1-benzoxazin-2-on und Wasserstoffperoxid.
Schmelzpunkt : 155-157 °C,
Ausbeute : 68,0 % der Theorie.

Beispiel 62

6-[4-(3-Methoxy-phenylsulfinyl)-butoxy]-4,4-dimethyl-4H-3,1-benzoxazin-2-on

Hergestellt analog Beispiel 2 aus 6-[4-(3-Methoxy-phenylmercapto)-butoxy]-4,4-dimethyl-4H-3,1-benzoxazin-2-on und Wasserstoffperoxid.
Öl, RF-Wert : 0,55 (Kieselgelplatte ; Chloroform/Äthanol = 9 : 1),
Ausbeute : 88,4 % der Theorie,

$C_{21}H_{25}NO_5S$ (403,5)
Ber.: C 62,51 H 6,24 N 3,47 S 7,95
Gef.: C 62,31 H 6,17 N 3,33 S 7,84

Beispiel 63

6-[4-(3-Methyl-phenylsulfinyl)-butoxy]-4,4-dimethyl-4H-3,1-benzoxazin-2-on

Hergestellt analog Beispiel 2 aus 6-[4-(3-Methyl-phenylmercapto)-butoxy]-4,4-dimethyl-4H-3,1-benzoxazin-2-on und Wasserstoffperoxid.
Öl, RF-Wert: 0,3 (Kieselgelplatte; Chloroform/Aceton = 9 : 1),
Ausbeute: 87,1 % der Theorie,
$C_{21}H_{25}NO_4S$ (387,5)
Ber.: C 65,09 H 6,50 N 3,61 S 8,27
Gef.: C 64,98 H 6,65 N 3,53 S 8,30

Beispiel 64

6-[4-(4-Amino-3,5-dibrom-phenylsulfinyl)-butoxy]-4,4-dimethyl-4H-3,1-benzoxazin-2-on

Hergestellt analog Beispiel 2 aus 6-[4-(4-Amino-3,5-dibromphenylmercapto)-butoxy]-4,4-dimethyl-4H-3,1-benzoxazin-2-on und Wasserstoffperoxid.
Schmelzpunkt: 169-172 °C,
Ausbeute: 35,2 % der Theorie.

Beispiel 65

6-[4-(3,5-Di-tert.butyl-4-hydroxy-phenylsulfinyl)-butoxy]-4,4-dimethyl-4H-3,1-benzoxazin-2-on

Hergestellt analog Beispiel 2 aus 6-[4-(3,5-Di-tert.butyl-4-hydroxy-phenylmercapto)-butoxy]-4,4-dimethyl-4H-3,1-benzoxazin-2-on und Wasserstoffperoxid.
Schmelzpunkt: 177-179 °C,
Ausbeute: 74,6 % der Theorie.

Beispiel 66

6-[4-(4-Brom-phenylsulfinyl)-butoxy]-4,4-dimethyl-4H-3,1-benzoxazin-2-on

Hergestellt analog Beispiel 2 aus 6-[4-(4-Brom-phenylmercapto)-butoxy]-4,4-dimethyl-4H-3,1-benzoxazin-2-on und Wasserstoffperoxid.
Schmelzpunkt: 137-138 °C,
Ausbeute: 79,8 % der Theorie.

Beispiel 67

6-[4-(4-Methyl-phenylsulfinyl)-butoxy]-4H-3,1-benzoxazin-2-on

Hergestellt analog Beispiel 2 aus 6-[4-(4-Methyl-phenylmercapto)-butoxy]-4H-3,1-benzoxazin-2-on und Wasserstoffperoxid.
Schmelzpunkt: 143-144 °C,
Ausbeute: 82,2 % der Theorie.

Beispiel 68

6-[4-(4-Cyclohexyl-phenylsulfinyl)-butoxy]-4H-3,1-benzoxazin-2-on

Hergestellt analog Beispiel 2 aus 6-[4-(4-Cyclohexyl-phenylmercapto)-butoxy]-4H-3,1-benzoxazin-2-on und Wasserstoffperoxid.
Schmelzpunkt: 118-119 °C,
Ausbeute: 81,5 % der Theorie.

Beispiel 69

6-[4-(4-Acetamido-phenylsulfinyl)-butoxy]-4H-3,1-benzoxazin-2-on

Hergestellt analog Beispiel 2 aus 6-[4-(4-Acetamido-phenylmercapto)-butoxy]-4H-3,1-benzoxazin-2-on und Wasserstoffperoxid.
Schmelzpunkt : 183-184 °C,
Ausbeute : 84,0 % der Theorie.

Beispiel 70

6-[4-(3,4-Dimethyl-phenylsulfinyl)-butoxy]-4H-3,1-benzoxazin-2-on

Hergestellt analog Beispiel 2 aus 6-[4-(3,4-Dimethyl-phenylmercapto)-butoxy]-4H-3,1-benzoxazin-2-on und Wasserstoffperoxid.
Schmelzpunkt : 119-120 °C,
Ausbeute : 57,0 % der Theorie.

Beispiel 71

6-(4-Phenylsulfinyl-butoxy)-4H-3,1-benzoxazin-2-on

Hergestellt analog Beispiel 2 aus 6-(4-Phenylmercapto-butoxy)-4H-3,1-benzoxazin-2-on und Wasserstoffperoxid.
Schmelzpunkt : 115-116 °C,
Ausbeute : 66,7 % der Theorie.

Beispiel 72

6-[4-(3,4-Dichlor-phenylsulfinyl)-butoxy]-4H-3,1-benzoxazin-2-on

Hergestellt analog Beispiel 2 aus 6-[4-(3,4-Dichlor-phenylmercapto)-butoxy]-4H-3,1-benzoxazin-2-on und Wasserstoffperoxid.
Schmelzpunkt : 169-170 °C,
Ausbeute : 91,8 % der Theorie.

Beispiel 73

6-[4-(4-Chlor-phenylsulfinyl)-butoxy]-4H-3,1-benzoxazin-2-on

Hergestellt analog Beispiel 2 aus 6-[4-(4-Chlor-phenylmercapto)-butoxy]-4H-3,1-benzoxazin-2-on und Wasserstoffperoxid.
Schmelzpunkt : 169-171 °C,
Ausbeute : 86,0 % der Theorie.

Beispiel 74

6-(4-Benzylsulfinyl-butoxy)-4,4-dimethyl-4H-3,1-benzoxazin-2-on

Hergestellt analog Beispiel 2 aus 6-(4-Benzylmercapto-butoxy)-4,4-dimethyl-4H-3,1-benzoxazin-2-on und Wasserstoffperoxid.
Schmelzpunkt : 122 °C,
Ausbeute : 71,0 % der Theorie.

Beispiel 75

6-(4-Methylsulfinyl-butoxy)-4,4-dimethyl-4H-3,1-benzoxazin-2-on

Hergestellt analog Beispiel 2 aus 6-(4-Methylmercapto-butoxy)-4,4-dimethyl-4H-3,1-benzoxazin-2-on und Wasserstoffperoxid.
Schmelzpunkt : 124-126 °C,
Ausbeute : 68,1 % der Theorie.

Beispiel 76

6-(4-Cyclohexylsulfinyl-butoxy)-4,4-dimethyl-4H-3,1-benzoxazin-2-on

Hergestellt analog Beispiel 2 aus 6-(4-Cyclohexylmercapto-butoxy)-4,4-dimethyl-4H-3,1-benzoxazin-2-on und Wasserstoffperoxid.

Schmelzpunkt : 115 °C,
Ausbeute : 76,9 % der Theorie.

Beispiel 77

6-[4-(4-Acetamido-phenylsulfinyl)-butoxy]-4,4-dicyclohexyl-4H-3,1-benzoxazin-2-on

Hergestellt analog Beispiel 2 aus 6-[4-(4-Acetamido-phenylmercapto)-butoxy]-4,4-dicyclohexyl-4H-3,1-benzoxazin-2-on und Wasserstoffperoxid.
Schmelzpunkt : 206-207 °C,
Ausbeute : 53,5 % der Theorie.

Beispiel 78

6-[4-(4-Methyl-phenylsulfinyl)-butoxy]-4,4-dicyclohexyl-4H-3,1-benzoxazin-2-on

Hergestellt analog Beispiel 2 aus 6-[4-(4-Methyl-phenylmercapto)-butoxy]-4,4-dicyclohexyl-4H-3,1-benzoxazin-2-on und Wasserstoffperoxid.
Schmelzpunkt : 200-201 °C,
Ausbeute : 62,0 % der Theorie.

Beispiel 79

6-[4-(4-Cyclohexyl-phenylsulfinyl)-butoxy]-4,4-dicyclohexyl-4H-3,1-benzoxazin-2-on

Hergestellt analog Beispiel 2 aus 6-[4-(4-Cyclohexyl-phenylmercapto)-butoxy]-4,4-dicyclohexyl-4H-3,1-benzoxazin-2-on und Wasserstoffperoxid.
Schmelzpunkt : ab 146 °C (Zers.),
Ausbeute : 89,3 % der Theorie.

Beispiel 80

6-[4-(3,4-Dichlor-phenylsulfinyl)-butoxy]-4,4-dicyclohexyl-4H-3,1-benzoxazin-2-on

Hergestellt analog Beispiel 2 aus 6-[4-(3,4-Dichlor-phenylmercapto)-butoxy]-4,4-dicyclohexyl-4H-3,1-benzoxazin-2-on und Wasserstoffperoxid.
Schmelzpunkt : 186-187 °C,
Ausbeute : 49,2 % der Theorie.

Beispiel 81

6-[4-(4-Methyl-phenylsulfinyl)-butoxy]-4,4-di-n-hexyl-4H-3,1-benzoxazin-2-on

Hergestellt analog Beispiel 2 aus 6-[4-(4-Methyl-phenylmercapto)-butoxy]-4,4-di-n-hexyl-4H-3,1-benzoxazin-2-on und Wasserstoffperoxid.
Schmelzpunkt : 78-79 °C,
Ausbeute : 87,3 % der Theorie.

Beispiel 82

6-[4-(4-Cyclohexyl-phenylsulfinyl)-butoxy]-4,4-di-n-hexyl-4H-3,1-benzoxazin-2-on

Hergestellt analog Beispiel 2 aus 6-[4-(4-Cyclohexyl-phenylmercapto)-butoxy]-4,4-di-n-hexyl-4H-3,1-benzoxazin-2-on und Wasserstoffperoxid.
Schmelzpunkt : 72-75 °C,
Ausbeute : 71,4 der Theorie.

Beispiel 83

6-[4-(3,4-Dichlor-phenylsulfinyl)-butoxy]-4,4-di-n-hexyl-4H-3,1-benzoxazin-2-on

Hergestellt analog Beispiel 2 aus 6-[4-(3,4-Dichlor-phenylmercapto)-butoxy]-4,4-di-n-hexyl-4H-3,1-benzoxazin-2-on und Wasserstoffperoxid.
Schmelzpunkt : 83-85 °C,
Ausbeute : 71,7 % der Theorie.

Beispiel 84

6-[4-(4-Acetamido-phenylsulfinyl)-butoxy]-4,4-di-n-hexyl-4H-3,1-benzoxazin-2-on

Hergestellt analog Beispiel 2 aus 6-[4-(4-Acetamido-phenylmercapto)-butoxy]-4,4-di-n-hexyl-4H-3,1-benzoxazin-2-on und Wasserstoffperoxid.
Öl, RF-Wert : 0,48 (Kieselgelplatte : Chloroform/Äthanol = 9 : 1),
Ausbeute : 43,8 % der Theorie,
$C_{32}H_{46}N_2O_5S$ (570,79)
Ber. : C 67,34 H 8,12 N 4,91 S 5,62
Gef. : C 67,52 H 7,94 N 4,95 S 5,78

Beispiel 85

6-[2-(3,4-Dichlor-phenylsulfinyl)-äthoxy]-4,4-dimethyl-4H-3,1-benzoxazin-2-on

Hergestellt analog Beispiel 2 aus 6-[2-(3,4-Dichlor-phenylmercapto)-äthoxy]-4,4-dimethyl-4H-3,1-benzoxazin-2-on und Wasserstoffperoxid.
Schmelzpunkt : 200-201 °C,
Ausbeute : 63,3 % der Theorie.

Beispiel 86

6-[3-(3,4-Dichlor-phenylsulfinyl)-propoxy]-4,4-dimethyl-4H-3,1-benzoxazin-2-on

Hergestellt analog Beispiel 2 aus 6-[3-(3,4-Dichlor-phenylmercapto)-propoxy]-4,4-dimethyl-4H-3,1-benzoxazin-2-on und Wasserstoffperoxid.
Schmelzpunkt : 157-158 °C,
Ausbeute : 76,3 % der Theorie.

Beispiel 87

6-[3-(4-Cyclohexyl-phenylsulfinyl)-propoxy]-4,4-dimethyl-4H-3,1-benzoxazin-2-on

Hergestellt analog Beispiel 2 aus 6-[3-(4-Cyclohexyl-phenyl-mercapto)-propoxy]-4,4-dimethyl-4H-3,1-benzoxazin-2-on und Wasserstoffperoxid.
Öl, RF-Wert : 0,45 (Kieselgelplatte : Chloroform/Äthanol = 9 : 1),
Ausbeute : 96,3 % der Theorie,
$C_{25}H_{31}NO_4S$ (441,59)
Ber. : C 68,00 H 7,08 N 3,17 S 7,26
Gef. : C 67,75 H 7,01 N 3,17 S 7,13

Beispiel 88

6-[5-(4-Cyclohexyl-phenylsulfinyl)-pentoxy]-4,4-dimethyl-4H-3,1-benzoxazin-2-on

Hergestellt analog Beispiel 2 aus 6-[5-(4-Cyclohexyl-phenylmercapto)-pentoxy]-4,4-dimethyl-4H-3,1-benzoxazin-2-on und Wasserstoffperoxid.
Öl, RF-Wert : 0,45 (Kieselgelplatte : Chloroform/Äthanol = 9 : 1),
Ausbeute : 63,9 % der Theorie,
$C_{27}H_{35}NO_4S$ (469,65)
Ber. : C 69,05 H 7,51 N 2,98 S 6,83
Gef. : C 70,26 H 7,74 N 2,91 S 6,87

Beispiel 89

6-[5-(3,4-Dichlor-phenylsulfinyl)-pentoxy]-4,4-dimethyl-4H-3,1-benzoxazin-2-on

Hergestellt analog Beispiel 2 aus 6-[5-(3,4-Dichlor-phenylmercapto)-pentoxy]-4,4-dimethyl-4H-3,1-benzoxazin-2-on und Wasserstoffperoxid.
Schmelzpunkt : 119-120 °C,
Ausbeute : 83,8 % der Theorie.

Beispiel 90

6-[4-(3,4-Dimethoxy-phenylsulfoximino)-butoxy]-4,4-dimethyl-4H-3,1-benzoxazin-2-on

Hergestellt analog Beispiel 6 aus 6-[4-(3,4-Dimethoxy-phenylsulfinyl)-butoxy]-4,4-dimethyl-4H-3,1-benzoxazin-2-on und O-Mesitylensulfonyl-acethydroxamsäure-äthylester.
Schmelzpunkt : 162-163 °C,
Ausbeute : 51,3 % der Theorie.

Beispiel 91

6-[4-(4-Methoxy-phenylsulfoximino)-butoxy]-4,4-dimethyl-4H-3,1-benzoxazin-2-on

Hergestellt analog Beispiel 6 aus 6-[4-(4-Methoxy-phenylsulfinyl)-butoxy]-4,4-dimethyl-4H-3,1-benzoxazin-2-on und O-Mesitylensulfonyl-acethydroxamsäure-äthylester.
Schmelzpunkt : 134-140 °C,
Ausbeute : 47,8 % der Theorie.

Beispiel 92

6-[4-(4-Hydroxy-phenylsulfoximino)-butoxy]-4,4-dimethyl-4H-3,1-benzoxazin-2-on

Hergestellt analog Beispiel 6 aus 6-[4-(4-Hydroxy-phenylsulfinyl)-butoxy]-4,4-dimethyl-4H-3,1-benzoxazin-2-on und O-Mesitylensulfonyl-acethydroxamsäure-äthylester.
Schmelzpunkt : 182-184 °C,
Ausbeute : 28,4 % der Theorie.

Beispiel 93

6-[4-(3-Methoxy-phenylsulfoximino)-butoxy]-4,4-dimethyl-4H-3,1-benzoxazin-2-on

Hergestellt analog Beispiel 6 aus 6-[4-(3-Methoxy-phenylsulfinyl)-bytoxy]-4,4-dimethyl-4H-3,1-benzoxazin-2-on und O-Mesitylensulfonyl-acethydroxamsäure-äthylester.
Schmelzpunkt : 102-105 °C,
Ausbeute : 31,9 % der Theorie.

Beispiel 94

6-[4-(4-Biphenylylsulfoximino)-butoxy]-4,4-dimethyl-4H-3,1-benzoxazin-2-on

Hergestellt analog Beispiel 6 aus 6-[4-(4-Biphenylylsulfinyl)-butoxy]-4,4-dimethyl-4H-3,1-benzoxazin-2-on und O-Mesitylensulfonyl-acethydroxamsäure-äthylester.
Schmelzpunkt : 184-186 °C,
Ausbeute : 68,5 % der Theorie.

Beispiel 95

6-[4-(4-Amino-3,5-dibrom-phenylsulfoximino)-butoxy]-4,4-dimethyl-4H-3,1-benzoxazin-2-on

Hergestellt analog Beispiel 6 aus 6-[4-(4-Amino-3,5-dibromphenylsulfinyl)-butoxy]-4,4-dimethyl-4H-3,1-benzoxazin-2-on und O-Mesitylensulfonyl-acethydroxamsäure-äthylester.
Schmelzpunkt : 206-208 °C,
Ausbeute : 73,8 % der Theorie.

Beispiel 96

6-[4-(3,5-Di-tert.butyl-4-hydroxy-phenylsulfoximino)-butoxy]-4,4-dimethyl-4H-3,1-benzoxazin-2-on

Hergestellt analog Beispiel 6 aus 6-[4-(3,5-Di-tert.butyl-4-hydroxy-phenylsulfinyl)-butoxy]-4,4-dimethyl-4H-3,1-benzoxazin-2-on und O-Mesitylensulfonyl-acethydroxamsäure-äthylester.
Schmelzpunkt : 176-178 °C,
Ausbeute : 65,8 % der Theorie.

Beispiel 97

6-[4-(3-Methyl-phenylsulfoximino)-butoxy]-4,4-dimethyl-4H-3,1-benzoxazin-2-on

Hergestellt analog Beispiel 6 aus 6-[4-(3-Methyl-phenylsulfinyl)-butoxy]-4,4-dimethyl-4H-3,1-benzoxazin-2-on und O-Mesitylensulfonyl-acethydroxamsäure-äthylester.
Schmelzpunkt : 118-120 °C,
Ausbeute : 33 % der Theorie.

Beispiel 98

6-[4-(4-Fluor-phenylsulfoximino)-butoxy]-4,4-dimethyl-4H-3,1-benzoxazin-2-on

Hergestellt analog Beispiel 6 aus 6-[4-(4-Fluor-phenylsulfinyl)-butoxy]-4,4-dimethyl-4H-3,1-benzoxazin-2-on und O-Mesitylensulfonyl-acethydroxamsäure-äthylester.
Schmelzpunkt : 136-138 °C,
Ausbeute : 30,8 % der Theorie.

Beispiel 99

6-[4-(4-Brom-phenylsulfoximino)-butoxy]-4,4-dimethyl-4H-3,1-benzoxazin-2-on

Hergestellt analog Beispiel 6 aus 6-[4-(4-Brom-phenylsulfinyl)-butoxy]-4,4-dimethyl-4H-3,1-benzoxazin-2-on und O-Mesitylensulfonyl-acethydroxamsäure-äthylester.
Schmelzpunkt : 155-157 °C,
Ausbeute : 50 % der Theorie.

Beispiel 100

6-[4-(4-Brom-3-methyl-phenylsulfoximino)-butoxy]-4,4-dimethyl-4H-3,1-benzoxazin-2-on

Hergestellt analog Beispiel 6 aus 6-[4-(4-Brom-3-methyl-phenyl-sulfinyl)-butoxy]-4,4-dimethyl-4H-3,1-benzoxazin-2-on und O-Mesitylensulfonyl-acethydroxamsäure-äthylester.
Schmelzpunkt : 153-154 °C,
Ausbeute : 63,6 % der Theorie.

Beispiel 101

6-[4-(4-Cyclohexyl-phenylsulfoximino)-butoxy]-4,4-dimethyl-4H-3,1-benzoxazin-2-on

Hergestellt analog Beispiel 6 aus 6-[4-(4-Cyclohexyl-phenylsulfinyl)-butoxy]-4,4-dimethyl-4H-3,1-benzoxazin-2-on und O-Mesitylensulfonyl-acethydroxamsäure-äthylester.
Schmelzpunkt : 168-169 °C,
Ausbeute : 52,8 % der Theorie.

Beispiel 102

6-[4-(4-Methyl-phenylsulfoximino)-butoxy]-4,4-dimethyl-4H-3,1-benzoxazin-2-on

Hergestellt analog Beispiel 6 aus 6-[4-(4-Methyl-phenylsulfinyl)-butoxy]-4,4-dimethyl-4H-3,1-benzoxazin-2-on und O-Mesitylensulfonyl-acethydroxamsäure-äthylester.
Schmelzpunkt : 147-148 °C,
Ausbeute : 59,2 % der Theorie.

Beispiel 103

6-(4-Phenylsulfoximino-butoxy)-4,4-dimethyl-4H-3,1-benzoxazin-2-on

Hergestellt analog Beispiel 6 aus 6-(4-Phenylsulfinyl-butoxy)-4,4-dimethyl-4H-3,1-benzoxazin-2-on und O-Mesitylensulfonyl-acethydroxamsäure-äthylester.
Schmelzpunkt : 152 °C,
Ausbeute : 71,7 % der Theorie.

Beispiel 104

6-[4-(4-Chlor-phenylsulfoximino)-butoxy]-4,4-dimethyl-4H-3,1-benzoxazin-2-on

Hergestellt analog Beispiel 6 aus 6-[4-(4-Chlor-phenylsulfinyl)-butoxy]-4,4-dimethyl-4H-3,1-benzoxa-

zin-2-on und O-Mesitylensulfonyl-acethydroxamsäure-äthylester.
Schmelzpunkt : 132 °C,
Ausbeute : 61,6 % der Theorie.

Beispiel 105

6-[4-(4-tert.Butyl-phenylsulfoximino)-butoxy]-4,4-dimethyl-4H-3,1-benzoxazin-2-on

Hergestellt analog Beispiel 6 aus 6-[4-(4-tert.Butyl-phenylsulfinyl)-butoxy]-4,4-dimethyl-4H-3,1-benzoxazin-2-on und O-Mesitylensulfonyl-acethydroxamsäure-äthylester.
Schmelzpunkt : 187 °C,
Ausbeute : 72 % der Theorie.

Beispiel 106

6-[4-(4-Acetamido-phenylsulfoximino)-butoxy]-4,4-dimethyl-4H-3,1-benzoxazin-2-on

Hergestellt analog Beispiel 6 aus 6-[4-(4-Acetamido-phenylsulfinyl)-butoxy]-4,4-dimethyl-4H-3,1-benzoxazin-2-on und O-Mesitylensulfonyl-acethydroxamsäure-äthylester.
Schmelzpunkt : 185 °C,
Ausbeute : 44 % der Theorie.

Beispiel 107

6-[4-(4-Methyl-phenylsulfoximino)-butoxy]-4,4-di-n-hexyl-4H-3,1-benzoxazin-2-on

Hergestellt analog Beispiel 6 aus 6-[4-(4-Methyl-phenylsulfinyl)-butoxy]-4,4-di-n-hexyl-4H-3,1-benzoxazin-2-on und O-Mesitylensulfonyl-acethydroxamsäure-äthylester.
Schmelzpunkt : 89-91 °C,
Ausbeute : 87,5 % der Theorie.

Beispiel 108

6-[4-(4-Cyclohexyl-phenylsulfoximino)-butoxy]-4,4-di-n-hexyl-4H-3,1-benzoxazin-2-on

Hergestellt analog Beispiel 6 aus 6-[4-(4-Cyclohexyl-phenylsulfinyl)-butoxy]-4,4-di-n-hexyl-4H-3,1-benzoxazin-2-on und O-Mesitylensulfonyl-acethydroxamsäure-äthylester.
Schmelzpunkt : 78-80 °C,
Ausbeute : 48,3 % der Theorie.

Beispiel 109

6-[4-(3,4-Dichlor-phenylsulfoximino)-butoxy]-4,4-di-n-hexyl-4H-3,1-benzoxazin-2-on

Hergestellt analog Beispiel 6 aus 6-[4-(3,4-Dichlor-phenylsulfinyl)-butoxy]-4,4-di-n-hexyl-4H-3,1-benzoxazin-2-on und O-Mesitylensulfonyl-acethydroxamsäure-äthylester.
Schmelzpunkt : 106-108 °C,
Ausbeute : 66,9 % der Theorie.

Beispiel 110

6-[4-(4-Acetamido-phenylsulfoximino)-butoxy]-4,4-di-n-hexyl-4H-3,1-benzoxazin-2-on

Hergestellt analog Beispiel 6 aus 6-[4-(4-Acetamido-phenylsulfinyl)-butoxy]-4,4-di-n-hexyl-4H-3,1-benzoxazin-2-on und O-Mesitylensulfonyl-acethydroxamsäure-äthylester.
Schmelzpunkt : 146-148 °C,
Ausbeute : 78 % der Theorie.

Beispiel 111

6-[4-(4-Acetamido-phenylsulfoximino)-butoxy]-4,4-dicyclohexyl-4H-3,1-benzoxazin-2-on

Hergestellt analog Beispiel 6 aus 6-[4-(4-Acetamido-phenylsulfinyl)-butoxy]-4,4-dicyclohexyl-4H-3,1-benzoxazin-2-on und O-Mesitylensulfonyl-acethydroxamsäure-äthylester.
Schmelzpunkt : 149-150 °C.

Ausbeute : 54 % der Theorie.

Beispiel 112

6-[4-(4-Methyl-phenylsulfoximino)-butoxy]-4,4-dicyclohexyl-4H-3,1-benzoxazin-2-on

Hergestellt analog Beispiel 6 aus 6-[4-(4-Methyl-phenylsulfinyl)-butoxy]-4,4-dicyclohexyl-4H-3,1-benzoxazin-2-on und O-Mesitylensulfonyl-acethydroxamsäure-äthylester.
Schmelzpunkt : 176-177 °C,
Ausbeute : 58,6 % der Theorie.

Beispiel 113

6-[4-(4-Cyclohexyl-phenylsulfoximino)-butoxy]-4,4-dicyclohexyl-4H-3,1-benzoxazin-2-on

Hergestellt analog Beispiel 6 aus 6-[4-(4-Cyclohexyl-phenylsulfinyl)-butoxy]-4,4-dicyclohexyl-4H-3,1-benzoxazin-2-on und O-Mesitylensulfonyl-acethydroxamsäure-äthylester.
Schmelzpunkt : 219-220 °C,
Ausbeute : 73,2 % der Theorie.

Beispiel 114

6-[4-(4-Methyl-phenylsulfoximino)-butoxy]-4H-3,1-benzoxazin-2-on

Hergestellt analog Beispiel 6 aus 6-[4-(4-Methyl-phenylsulfinyl)-butoxy]-4H-3,1-benzoxazin-2-on und O-Mesitylensulfonyl-acethydroxamsäure-äthylester.
Schmelzpunkt : 153-154 °C,
Ausbeute : 60 % der Theorie.

Beispiel 115

6-[4-(4-Cyclohexyl-phenylsulfoximino)-butoxy]-4H-3,1-benzoxazin-2-on

Hergestellt analog Beispiel 6 aus 6-[4-(4-Cyclohexyl-phenylsulfinyl)-butoxy]-4H-3,1-benzoxazin-2-on und O-Mesitylensulfonyl-acethydroxamsäure-äthylester.
Schmelzpunkt : 187-188 °C,
Ausbeute : 65 % der Theorie.

Beispiel 116

6-[4-(4-Acetamido-phenylsulfoximino)-butoxy]-4H-3,1-benzoxazin-2-on

Hergestellt analog Beispiel 6 aus 6-[4-(4-Acetamido-phenylsulfinyl)-butoxy]-4H-3,1-benzoxazin-2-on und O-Mesitylensulfonyl-acethydroxamsäure-äthylester.
Schmelzpunkt : 135-137 °C,
Ausbeute : 64,7 % der Theorie.

Beispiel 117

6-[5-(3,4-Dichlor-phenylsulfoximino)-pentoxy]-4,4-dimethyl-4H-3,1-benzoxazin-2-on

Hergestellt analog Beispiel 6 aus 6-[5-(3,4-Dichlor-phenylsulfinyl)-pentoxy]-4,4-dimethyl-4H-3,1-benzoxazin-2-on und O-Mesitylensulfonyl-acethydroxamsäure-äthylester.
Schmelzpunkt : 169-170 °C,
Ausbeute : 85,7 % der Theorie.

Beispiel 118

6-[5-(4-Cyclohexyl-phenylsulfoximino)-pentoxy]-4,4-dimethyl-4H-3,1-benzoxazin-2-on

Hergestellt analog Beispiel 6 aus 6-[5-(4-Cyclohexyl-phenylsulfinyl)-pentoxy]-4,4-dimethyl-4H-3,1-benzoxazin-2-on und O-Mesitylensulfonyl-acethydroxamsäure-äthylester.
Schmelzpunkt : 110-111 °C,
Ausbeute : 67,1 % der Theorie.

Beispiel 119

6-[3-(3,4-Dichlor-phenylsulfoximino)-propoxy]-4,4-dimethyl-4H-3,1-benzoxazin-2-on

Hergestellt analog Beispiel 6 aus 6-[3-(3,4-Dichlor-phenylsulfinyl)-propoxy]-4,4-dimethyl-4H-3,1-benzoxazin-2-on und O-Mesitylensulfonyl-acethydroxamsäure-äthylester.
Schmelzpunkt : 135-136 °C,
Ausbeute : 67,8 % der Theorie.

Beispiel 120

6-[3-(4-Cyclohexyl-phenylsulfoximino)-propoxy]-4,4-dimethyl-4H-3,1-benzoxazin-2-on

Hergestellt analog Beispiel 6 aus 6-[3-(4-Cyclohexyl-phenylsulfinyl)-propoxy]-4,4-dimethyl-4H-3,1-benzoxazin-2-on und O-Mesitylensulfonyl-acethydroxamsäure-äthylester.
Schmelzpunkt : 175-176 °C,
Ausbeute : 50,3 % der Theorie.

Beispiel 121

6-[2-(3,4-Dichlor-phenylsulfoximino)-äthoxy]-4,4-dimethyl-4H-3,1-benzoxazin-2-on

Hergestellt analog Beispiel 6 aus 6-[2-(3,4-Dichlor-phenylsulfinyl)-äthoxy]-4,4-dimethyl-4H-3,1-benzoxazin-2-on und O-Mesitylensulfonyl-acethydroxamsäure-äthylester.
Schmelzpunkt : 139-140 °C,
Ausbeute : 64 % der Theorie.

Beispiel 122

6-(4-Cyclohexylsulfoximino-butoxy)-4,4-dimethyl-4H-3,1-benzoxazin-2-on

Hergestellt analog Beispiel 6 aus 6-(4-Cyclohexylsulfinyl-butoxy)-4,4-dimethyl-4H-3,1-benzoxazin-2-on und O-Mesitylensulfonyl-acethydroxamsäure-äthylester.
Schmelzpunkt : 108-110 °C,
Ausbeute : 28 % der Theorie.

Beispiel 123

6-(4-Benzylsulfoximino-butoxy)-4,4-dimethyl-4H-3,1-benzoxazin-2-on

Hergestellt analog Beispiel 6 aus 6-(4-Benzylsulfinyl-butoxy)-4,4-dimethyl-4H-3,1-benzoxazin-2-on und O-Mesitylensulfonyl-acethydroxamsäure-äthylester.
Schmelzpunkt : 132-138 °C,
Ausbeute : 75 % der Theorie.

Beispiel 124

6-(4-n-Octylsulfoximino-butoxy)-4,4-dimethyl-4H-3,1-benzoxazin-2-on

Hergestellt analog Beispiel 6 aus 6-(4-n-Octylsulfinyl-butoxy)-4,4-dimethyl-4H-3,1-benzoxazin-2-on und O-Mesitylensulfonyl-acethydroxamsäure-äthylester.
Schmelzpunkt : 82 °C,
Ausbeute : 73,2 % der Theorie.

Beispiel 125

6-[4-(4-Chlor-phenylmercapto)-butoxy]-4H-3,1-benzoxazin-2-on

Hergestellt analog Beispiel 5 aus 6-Hydroxy-4H-3,1-benzoxazin-2-on und 4-(4-Chlor-phenylmercapto)-butylchlorid hergestellt aus 4-(4-Chlor-phenylmercapto)-butanol und Thionylchlorid.
Öl, RF-Wert : 0,25 (Kieselgel : Petroläther/Cyclohexan = 1 : 1).
Schmelzpunkt : 143-144 °C,
Ausbeute : 28 % der Theorie.

Beispiel 126

6-[4-(3,4-Dichlor-phenylmercapto)-butoxy]-4H-3,1-benzoxazin-2-on

Hergestellt analog Beispiel 5 aus 6-Hydroxy-4H-3,1-benzoxazin-2-on und 4-(3,4-Dichlor-phenylmercapto)-butylbromid (hergestellt aus 3,4-Dichlorthiophenol und 1,4-Dibrom-butan, $Kp_{0,1\ mb}$ : 153-160 °C).
Schmelzpunkt : 128-129 °C,
Ausbeute : 57 % der Theorie.

Beispiel 127

6-(4-Phenylmercapto-butoxy)-4,4-dimethyl-4H-3,1-benzoxazin-2-on

Hergestellt analog Beispiel 5 aus 6-Hydroxy-4,4-dimethyl-4H-3,1-benzoxazin-2-on und 4-Phenylmercapto-butylbromid ($Kp_{0,03\ mb}$ : 95-104 °C).
Schmelzpunkt : 108-109 °C,
Ausbeute : 52,5 % der Theorie.

Beispiel 128

6-[4-(3,4-Dichlor-phenylmercapto)-butoxy]-4,4-dimethyl-4H-3,1-benzoxazin-2-on

Hergestellt analog Beispiel 5 aus 6-Hydroxy-4,4-dimethyl-4H-3,1-benzoxazin-2-on und 4-(3,4-Dichlor-phenylmercapto)-butylbromid.
Schmelzpunkt : 155-156 °C,
Ausbeute : 53 % der Theorie.

Beispiel 129

6-[4-(4-Chlor-phenylmercapto)-butoxy]-4,4-dimethyl-4H-3,1-benzoxazin-2-on

Hergestellt analog Beispiel 5 aus 6-Hydroxy-4,4-dimethyl-4H-3,1-benzoxazin-2-on und 4-(4-Chlor-phenylmercapto)-butylchlorid.
Schmelzpunkt : 150-151 °C,
Ausbeute : 55,6 % der Theorie.

Beispiel 130

6-[4-(3,4-Dimethyl-phenylsulfoximino)-butoxy]-4H-3,1-benzoxazin-2-on

Hergestellt analog Beispiel 6 aus 6-[4-(3,4-Dimethyl-phenyl-sulfinyl)-butoxy]-4H-3,1-benzoxazin-2-on und O-Mesitylensulfonyl-acethydroxamsäure-äthylester.
Schmelzpunkt : 169-170 °C,
Ausbeute : 82,5 % der Theorie.

Beispiel 131

6-[4-(3,4-Dimethyl-phenylsulfoximino)-butoxy]-4,4-dimethyl-4H-3,1-benzoxazin-2-on

Hergestellt analog Beispiel 6 aus 6-[4-(3,4-Dimethyl-phenyl-sulfinyl)-butoxy]-4,4-dimethyl-4H-3,1-benzoxazin-2-on und O-Mesitylensulfonyl-acethydroxamsäure-äthylester.
Schmelzpunkt : 162-163 °C,
Ausbeute : 70,4 % der Theorie.

Beispiel 132

6-[4-(3,4-Dimethyl-phenylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 3,4-Dimethyl-thiophenol.
Schmelzpunkt : 122-123 °C,
Ausbeute : 75,3 % der Theorie.

Beispiel 133

6-[6-(3,4-Dichlor-phenylmercapto)-hexyloxy]-4,4-dimethyl-4H-3,1-benzoxazin-2-on

Hergestellt analog Beispiel 1 aus 6-(6-Bromhexyloxy)-4,4-dimethyl-4H-3,1-benzoxazin-2-on und 3,4-Dichlor-thiophenol.
Schmelzpunkt : 102-103 °C,
Ausbeute : 77,4 % der Theorie.

Beispiel 134

6-[6-(3,4-Dimethoxy-phenylmercapto)-hexyloxy]-4,4-dimethyl-4H-3,1-benzoxazin-2-on

Hergestellt analog Beispiel 1 aus 6-(6-Bromhexyloxy)-4,4-dimethyl-4H-3,1-benzoxazin-2-on und 3,4-Dimethoxy-thiophenol.
Schmelzpunkt : 153-154 °C,
Ausbeute : 89,4 % der Theorie.

Beispiel 135

6-(4-Phenylsulfonyl-butoxy)-4,4-dimethyl-4H-3,1-benzoxazin-2-on

Hergestellt analog Beispiel 4 aus 6-Hydroxy-4,4-dimethyl-4H-3,1-benzoxazin-2-on und 4-Phenylsulfonyl-butylbromid (Schmelzpunkt : 57-58 °C).
Schmelzpunkt : 155-156 °C,
Ausbeute : 53,7 % der Theorie.

Beispiel 136

6-[6-(3,4-Dichlor-phenylsulfoximino)-hexyloxy]-4,4-dimethyl-4H-3,1-benzoxazin-2-on

Hergestellt analog Beispiel 6 aus 6-[6-(3,4-Dichlor-phenylsulfinyl)-hexyloxy]-4,4-dimethyl-4H-3,1-benzoxazin-2-on und O-Mesitylensulfonyl-acethydroxamsäure-äthylester.
Schmelzpunkt : 135-136 °C,
Ausbeute : 84,5 % der Theorie.

Beispiel 137

6-[4-(3,4-Dichlor-phenylmercapto)-butoxy]-4,4-dimethyl-4H-3,1-benzoxazin-2-on

Hergestellt analog Beispiel 4 aus 6-Hydroxy-4,4-dimethyl-4H-3,1-benzoxazin-2-on und 4-(3,4-Dichlor-phenylmercapto)-butyl-bromid ($Kp_{0,1\ mb}$ : 153-160 °C).
Schmelzpunkt : 152-153 °C,
Ausbeute : 63,4 % der Theorie.

Beispiel 138

6-[4-(3,4-Dichlor-phenylsulfinyl)-butoxy]-4-äthyl-4H-3,1-benzoxazin-2-on

Hergestellt analog Beispiel 4 aus 4-Äthyl-6-hydroxy-4H-3,1-benzoxazin-2-on und 4-(3,4-Dichlor-phenylsulfinyl)-butyl-bromid.
Schmelzpunkt : 83-84 °C,
Ausbeute : 61,9 % der Theorie.

Beispiel 139

6-[4-(3,4-Dichlor-phenylsulfinyl)-butoxy]-4-isopropyl-4H-3,1-benzoxazin-2-on

Hergestellt analog Beispiel 4 aus 6-Hydroxy-4-isopropyl-4H-3,1-benzoxazin-2-on und 4-(3,4-Dichlor-phenyl-sulfinyl)-butylbromid.
Schmelzpunkt : 73-75 °C,
Ausbeute : 48,2 % der Theorie.

Beispiel 140

6-[4-(3,4-Dichlor-phenylsulfoximino)-butoxy]-4-äthyl-4H-3,1-benzoxazin-2-on

Hergestellt analog Beispiel 6 aus 6-[4-(3,4-Dichlor-phenyl-sulfinyl)-butoxy]-4-äthyl-4H-3,1-benzoxa-

zin-2-on und O-Mesitylensulfonyl-acethydroxamsäure-äthylester.
Schmelzpunkt : 132-133 °C,
Ausbeute : 60 % der Theorie.

Beispiel 141

6-[4-(3,4-Dichlor-N-p-toluolsulfonyl-phenylsulfoximino)-butoxy]-4,4-dimethyl-4H-3,1-benzoxazin-2-on

Hergestellt analog Beispiel 12 aus 6-[4-(3,4-Dichlor-phenyl-sulfoximino)-butoxy]-4,4-dimethyl-4H-3,1-benzoxazin-2-on und p-Toluolsulfonsäurechlorid.
Schmelzpunkt : 174-175 °C,
Ausbeute : 78,4 % der Theorie.

Beispiel 142

6-[4-(3,4-Dichlor-N-benzoyl-phenylsulfoximino)-butoxy]-4,4-dimethyl-4H-3,1-benzoxazin-2-on

Hergestellt analog Beispiel 12 aus 6-[4-(3,4-Dichlor-phenyl-sulfoximino)-butoxy]-4,4-dimethyl-4H-3,1-benzoxazin-2-on und Benzoylchlorid.
Schmelzpunkt : 189-190 °C,
Ausbeute : 67,6 % der Theorie.

Beispiel 143

6-[4-(3,4-Dimethoxy-N-acetyl-phenylsulfoximino)-butoxy]-4,4-dimethyl-4H-3,1-benzoxazin-2-on

Eine Lösung von 1 g (0,002 2 Mol) 6-[4-(3,4-Dimethoxy-phenyl-sulfoximino)-butoxy]-4,4-dimethyl-4H-3,1-benzoxazin-2-on in 10 ml Eisessig wird mit 1,5 ml Acetanhydrid versetzt und 3 Stunden lang bei Raumtemperatur gerührt. Nach Versetzen mit Eiswasser wird mit Chloroform extrahiert, die Chloroformphase mit Wasser gewaschen, mit Natriumsulfat getrocknet, das Chloroform abdestilliert und der Rückstand aus Essigester umkristallisiert.
Schmelzpunkt : 155-157 °C,
Ausbeute : 0,95 g (88 % der Theorie).

Beispiel 144

6-[4-(4-Phenyl-N-acetyl-phenylsulfoximino)-butoxy]-4,4-dimethyl-4H-3,1-benzoxazin-2-on

Hergestellt analog Beispiel 143 aus 6-[4-(4-Phenyl-phenylsulfoximino)-butoxy]-4,4-dimethyl-4H-3,1-benzoxazin-2-on und Acetanhydrid.
Schmelzpunkt : 106-108 °C,
Ausbeute : 94,7 % der Theorie.

Beispiel 145

6-[4-(3,5-Di-tert.butyl-4-hydroxy-N-acetyl-phenylsulfoximino)-butoxy]-4,4-dimethyl-4H-3,1-benzoxazin-2-on

Hergestellt analog Beispiel 143 aus 6-[4-(3,5-Di-tert.butyl-4-hydroxy-phenylsulfoximino)-butoxy]-4,4-dimethyl-4H-3,1-benzoxazin-2-on und Acetanhydrid.
Schmelzpunkt : 206-207 °C,
Ausbeute : 93,1 % der Theorie.

Beispiel 146

6-[4-(4-Amino-3,5-dibrom-N-acetyl-phenylsulfoximino)-butoxy]-4,4-dimethyl-4H-3,1-benzoxazin-2-on

Hergestellt analog Beispiel 143 aus 6-[4-(4-Amino-3,5-dibrom-phenylsulfoximino)-butoxy]-4,4-dimethyl-4H-3,1-benzoxazin-2-on und Acetanhydrid.
Schmelzpunkt : 198-200 °C,
Ausbeute : 86,2 % der Theorie.

Beispiel 147

6-[4-(4-tert.Butyl-N-acetyl-phenylsulfoximino)-butoxy]-4,4-dimethyl-4H-3,1-benzoxazin-2-on

Hergestellt analog Beispiel 143 aus 6-[4-(4-tert.Butyl-phenyl-sulfoximino)-butoxy]-4,4-dimethyl-4H-3,1-benzoxazin-2-on und Acetanhydrid.
Schmelzpunkt : 235-237 °C,
Ausbeute : 92,9 % der Theorie.

Beispiel 148

6-[4-(4-Methyl-N-acetyl-phenylsulfoximino)-butoxy]-4,4-dimethyl-4H-3,1-benzoxazin-2-on

Hergestellt analog Beispiel 143 aus 6-[4-(4-Methyl-phenylsulfoximino)-butoxy]-4,4-dimethyl-4H-3,1-benzoxazin-2-on und Acetanhydrid.
Schmelzpunkt : 109-110 °C,
Ausbeute : 83,6 % der Theorie.

Beispiel 149

6-[4-(4-Brom-3-methyl-N-acetyl-phenylsulfoximino)-butoxy]-4,4-dimethyl-4H-3,1-benzoxazin-2-on

Hergestellt analog Beispiel 143 aus 6-[4-(4-Brom-3-methylphenylsulfoximino)-butoxy]-4,4-dimethyl-4H-3,1-benzoxazin-2-on und Acetanhydrid.
Schmelzpunkt : 152-154 °C,
Ausbeute : 85,5 % der Theorie.

Beispiel 150

6-[4-(3,4-Dichlor-N-acetyl-phenylsulfoximino)-butoxy]-4,4-dimethyl-4H-3,1-benzoxazin-2-on

Hergestellt analog Beispiel 143 aus 6-[4-(3,4-Dichlor-phenylsulfoximino)-butoxy]-4,4-dimethyl-4H-3,1-benzoxazin-2-on und Acetanhydrid.
Schmelzpunkt : 156-158 °C,
Ausbeute : 91,3 % der Theorie.

Beispiel 151

6-[4-(N-Acetyl-phenylsulfoximino)-butoxy]-4,4-dimethyl-4H-3,1-benzoxazin-2-on

Hergestellt analog Beispiel 143 aus 6-(4-Phenylsulfoximino-butoxy)-4,4-dimethyl-4H-3,1-benzoxazin-2-on und Acetanhydrid.
Schmelzpunkt : 112-114 °C,
Ausbeute : 89,2 % der Theorie.

Beispiel 152

6-[4-(4-Chlor-N-acetyl-phenylsulfoximino)-butoxy]-4,4-dimethyl-4H-3,1-benzoxazin-2-on

Hergestellt analog Beispiel 143 aus 6-[4-(4-Chlor-phenylsulfoximino)-butoxy]-4,4-dimethyl-4H-3,1-benzoxazin-2-on und Acetanhydrid.
Schmelzpunkt : 120-122 °C,
Ausbeute : 86,9 % der Theorie.

Beispiel 153

6-[4-(4-Methyl-N-acetyl-phenylsulfoximino)-butoxy]-4,4-di-n-hexyl-4H-3,1-benzoxazin-2-on

Hergestellt analog Beispiel 143 aus 6-[4-(4-Methyl-phenylsulfoximino)-butoxy]-4,4-di-n-hexyl-4H-3,1-benzoxazin-2-on und Acetanhydrid.
Öl, RF-Wert : 0,4 (Kieselgelplatte : Chloroform/Aceton = 9 : 1),
Ausbeute : 98,9 % der Theorie.
$C_{33}H_{48}N_2O_5S$ (584,82)
Ber. : C 67,78 H 8,27 S 5,48
Gef. : C 67,89 H 8,31 S 5,60

Beispiel 154

6-[4-(4-Cyclohexyl-N-acetyl-phenylsulfoximino)-butoxy]-4,4-di-n-hexyl-4H-3,1-benzoxazin-2-on

Hergestellt analog Beispiel 143 aus 6-[4-(4-Cyclohexyl-phenylsulfoximino)-butoxy]-4,4-di-n-hexyl-4H-3,1-benzoxazin-2-on und Acetanhydrid.
Schmelzpunkt : 134-136 °C,
Ausbeute : 81,9 % der Theorie.

Beispiel 155

6-[4-(3,4-Dichlor-N-acetyl-phenylsulfoximino)-butoxy]-4,4-di-n-hexyl-4H-3,1-benzoxazin-2-on

Hergestellt analog Beispiel 143 aus 6-[4-(3,4-Dichlor-phenylsulfoximino)-butoxy]-4,4-di-n-hexyl-4H-3,1-benzoxazin-2-on und Acetanhydrid.
Öl, RF-Wert : 0,54 (Kieselgelplatte ; Chloroform/Aceton = 9 : 1),
Ausbeute : 68,8 % der Theorie.
$C_{32}H_{44}Cl_2N_2O_5S$ (639,70)
Ber. : C 60,08 H 6,93 Cl 11,08 S 5,01
Gef. : C 59,87 H 7,13 Cl 11,20 S 4,95

Beispiel 156

6-[4-(4-Acetamido-N-acetyl-phenylsulfoximino)-butoxy]-4,4-di-n-hexyl-4H-3,1-benzoxazin-2-on

Hergestellt analog Beispiel 143 aus 6-[4-(4-Acetamido-phenylsulfoximino)-butoxy]-4,4-di-n-hexyl-4H-3,1-benzoxazin-2-on und Acetanhydrid.
Schmelzpunkt : 122-124 °C,
Ausbeute : 83,6 % der Theorie.

Beispiel 157

6-[4-(4-Acetamido-N-acetyl-phenylsulfoximino)-butoxy]-4,4-dicyclohexyl-4H-3,1-benzoxazin-2-on

Hergestellt analog Beispiel 143 aus 6-[4-(4-Acetamido-phenylsulfoximino)-butoxy]-4,4-dicyclohexyl-4H-3,1-benzoxazin-2-on und Acetanhydrid.
Schmelzpunkt : 146 °C (Zers.),
Ausbeute : 78,3 % der Theorie.

Beispiel 158

6-[4-(4-Methyl-N-acetyl-phenylsulfoximino)-butoxy]-4,4-dicyclohexyl-4H-3,1-benzoxazin-2-on

Hergestellt analog Beispiel 143 aus 6-[4-(4-Methyl-phenylsulfoximino)-butoxy]-4,4-dicyclohexyl-4H-3,1-benzoxazin-2-on und Acetanhydrid.
Schmelzpunkt : 148-149 °C,
Ausbeute : 78,9 % der Theorie.

Beispiel 159

6-[4-(4-Acetamido-N-acetyl-phenylsulfoximino)-butoxy]-4H-3,1-benzoxazin-2-on

Hergestellt analog Beispiel 143 aus 6-[4-(4-Acetamido-phenylsulfoximino)-butoxy]-4H-3,1-benzoxazin-2-on und Acetanhydrid.
Schmelzpunkt : 200-201 °C,
Ausbeute : 73,7 % der Theorie.

Beispiel 160

6-[4-(4-Methyl-N-acetyl-phenylsulfoximino)-butoxy]-4H-3,1-benzoxazin-2-on

Hergestellt analog Beispiel 143 aus 6-[4-(4-Methyl-phenylsulfoximino)-butoxy]-4H-3,1-benzoxazin-2-on und Acetanhydrid.
Öl, RF-Wert : 0,62 (Kieselgelplatte ; Chloroform/Essigester = 9 : 1),
Ausbeute : 45 % der Theorie.

Beispiel 161

6-[4-(4-Cyclohexyl-N-acetyl-phenylsulfoximino)-butoxy]-4H-3,1-benzoxazin-2-on

Hergestellt analog Beispiel 143 aus 6-[4-(4-Cyclohexyl-phenylsulfoximino)-butoxy]-4H-3,1-benzoxazin-2-on und Acetanhydrid.
Schmelzpunkt : 124-125 °C,
Ausbeute : 86,8 % der Theorie.

Beispiel 162

6-[4-(4-Cyclohexyl-N-acetyl-phenylsulfoximino)-butoxy]-4,4-dicyclohexyl-4H-3,1-benzoxazin-2-on

Hergestellt analog Beispiel 143 aus 6-[4-(4-Cyclohexyl-phenyl-sulfoximino)-butoxy]-4,4-dicyclohexyl-4H-3,1-benzoxazin-2-on und Acetanhydrid.
Schmelzpunkt : 130 °C,
Ausbeute : 70,2 % der Theorie.

Beispiel 163

6-[5-(3,4-Dichlor-N-acetyl-phenylsulfoximino)-pentoxy]-4,4-dimethyl-4H-3,1-benzoxazin-2-on

Hergestellt analog Beispiel 143 aus 6-[5-(3,4-Dichlor-phenylsulfoximino)-pentoxy]-4,4-dimethyl-4H-3,1-benzoxazin-2-on und Acetanhydrid.
Schmelzpunkt : 126-128 °C,
Ausbeute : 50,6 % der Theorie.

Beispiel 164

6-[5-(4-Cyclohexyl-N-acetyl-phenylsulfoximino)-pentoxy]-4,4-dimethyl-4H-3,1-benzoxazin-2-on

Hergestellt analog Beispiel 143 aus 6-[5-(4-Cyclohexyl-phenyl-sulfoximino)-pentoxy]-4,4-dimethyl-4H-3,1-benzoxazin-2-on und Acetanhydrid.
Schmelzpunkt : 95-96 °C,
Ausbeute : 83,5 % der Theorie.

Beispiel 165

6-[3-(4-Cyclohexyl-N-acetyl-phenylsulfoximino)-propoxy]-4,4-dimethyl-4H-3,1-benzoxazin-2-on

Hergestellt analog Beispiel 143 aus 6-[3-(4-Cyclohexyl-phenyl-sulfoximino)-propoxy]-4,4-dimethyl-4H-3,1-benzoxazin-2-on und Acetanhydrid.
Schmelzpunkt : 114-115 °C,
Ausbeute : 94,2 % der Theorie.

Beispiel 166

6-[3-(3,4-Dichlor-N-acetyl-phenylsulfoximino)-propoxy]-4,4-dimethyl-4H-3,1-benzoxazin-2-on

Hergestellt analog Beispiel 143 aus 6-[3-(3,4-Dichlor-phenylsulfoximino)-propoxy]-4,4-dimethyl-4H-3,1-benzoxazin-2-on und Acetanhydrid.
Schmelzpunkt : 198-200 °C,
Ausbeute : 81,2 % der Theorie.

Beispiel 167

6-[2-(3,4-Dichlor-N-acetyl-phenylsulfoximino)-äthoxy]-4,4-dimethyl-4H-3,1-benzoxazin-2-on

Hergestellt analog Beispiel 143 aus 6-[2-(3,4-Dichlor-phenylsulfoximino)-äthoxy]-4,4-dimethyl-4H-3,1-benzoxazin-2-on und Acetanhydrid.
Schmelzpunkt : 154-155 °C,
Ausbeute : 81,2 % der Theorie.

Beispiel 168

6-[4-(3,4-Dimethyl-phenylsulfinyl)-butoxy]-4,4-dimethyl-4H-3,1-benzoxazin-2-on

Hergestellt analog Beispiel 2 aus 6-[4-(3,4-Dimethyl-phenylmercapto)-butoxy]-4,4-dimethyl-4H-3,1-benzoxazin-2-on und Wasserstoffperoxid.
Schmelzpunkt : 131-132 °C,
Ausbeute : 83,3 % der Theorie.

Beispiel 169

6-[6-(3,4-Dichlor-phenylsulfinyl)-hexyloxy]-4,4-dimethyl-4H-3,1-benzoxazin-2-on

Hergestellt analog Beispiel 2 aus 6-[6-(3,4-Dichlor-phenylmercapto)-hexyloxy]-4,4-dimethyl-4H-3,1-benzoxazin-2-on und Wasserstoffperoxid.
Schmelzpunkt : 156-158 °C,
Ausbeute : 71,5 % der Theorie.

Beispiel 170

6-[6-(3,4-Dimethoxy-phenylsulfinyl)-hexyloxy]-4,4-dimethyl-4H-3,1-benzoxazin-2-on

Hergestellt analog Beispiel 2 aus 6-[6-(3,4-Dimethoxy-phenylmercapto)-hexyloxy]-4,4-dimethyl-4H-3,1-benzoxazin-2-on und Wasserstoffperoxid.
Schmelzpunkt : 131-132 °C,
Ausbeute : 61,0 % der Theorie.

Beispiel 171

6-[4-(3,4-Dimethyl-N-acetyl-phenylsulfoximino)-butoxy]-4H-3,1-benzoxazin-2-on

Hergestellt analog Beispiel 143 aus 6-[4-(3,4-Dimethyl-phenyl-sulfoximino)-butoxy]-4H-3,1-benzoxazin-2-on und Acetanhydrid.
Harz, RF-Wert : 0,7 (Kieselgelplatte ; Chloroform/Äthanol = 9 : 1),
Ausbeute : 90,2 % der Theorie.
$C_{22}H_{26}N_2O_5S$ (430,53)
Ber. : C 61,38 H 6,09 N 6,51 S 7,45
Gef. : C 61,79 H 6,32 N 6,24 S 7,38

Beispiel 172

6-[4-(3,4-Dimethyl-N-acetyl-phenylsulfoximino)-butoxy]-4,4-dimethyl-4H-3,1-benzoxazin-2-on

Hergestellt analog Beispiel 143 aus 6-[4-(3,4-Dimethyl-phenyl-sulfoximino)-butoxy]-4,4-dimethyl-4H-3,1-benzoxazin-2-on und Acetanhydrid.
Schmelzpunkt : 146 °C,
Ausbeute : 86,5 % der Theorie.

Beispiel 173

6-[6-(3,4-Dimethoxy-phenylsulfoximino)-hexyloxy]-4,4-dimethyl-4H-3,1-benzoxazin-2-on

Hergestellt analog Beispiel 6 aus 6-[6-(3,4-Dimethoxy-phenylsulfinyl)-hexyloxy]-4,4-dimethyl-4H-3,1-benzoxazin-2-on und O-Mesitylensulfonyl-acethydroxamsäure-äthylester.
Schmelzpunkt : 108-109 °C,
Ausbeute : 83,9 % der Theorie.

Beispiel 174

6-[3-(3,4-Dichlor-phenylmercapto)-propoxy]-4-isopropyl-4H-3,1-benzoxazin-2-on

Hergestellt analog Beispiel 1 aus 6-(3-Chlorpropoxy)-4-isopropyl-4H-3,1-benzoxazin-2-on und 3,4-Dichlor-thiophenol.
Schmelzpunkt : 109-111 °C,
Ausbeute : 75,5 % der Theorie.

Beispiel 175

4-Isopropyl-6-[3-(3,4-dimethoxy-phenylmercapto)-propoxy]-4H-3,1-benzoxazin-2-on

Hergestellt analog Beispiel 1 aus 6-(3-Chlorpropoxy)-4-isopropyl-4H-3,1-benzoxazin-2-on und 3,4-Dimethoxy-thiophenol.
Schmelzpunkt : 102-103 °C,
Ausbeute : 84,2 % der Theorie.

Beispiel 176

6-[4-(3,4-Dichlor-N-acetyl-phenylsulfoximino)-butoxy]-4-äthyl-4H-3,1-benzoxazin-2-on

Hergestellt analog Beispiel 143 aus 6-[4-(3,4-Dichlor-phenylsulfoximino)-butoxy]-4-äthyl-4H-3,1-benzoxazin-2-on und Acetanhydrid.
Schmelzpunkt : 156-158 °C,
Ausbeute : 87,3 % der Theorie.

Beispiel 177

6-[3-(3,4-Dichlor-phenylsulfinyl)-propoxy]-4-isopropyl-4H-3,1-benzoxazin-2-on

Hergestellt analog Beispiel 2 aus 6-[3-(3,4-Dichlor-phenylmercapto)-propoxy]-4-isopropyl-4H-3,1-benzoxazin-2-on und Wasserstoffperoxid.
Schmelzpunkt : 58-60 °C,
Ausbeute : 83,3 % der Theorie.

Beispiel 178

6-[3-(3,4-Dimethoxy-phenylsulfinyl)-propoxy]-4-isopropyl-4H-3,1-benzoxazin-2-on

Hergestellt analog Beispiel 2 aus 6-[3-(3,4-Dimethoxy-phenylmercapto)-propoxy]-4-isopropyl-4H-3,1-benzoxazin-2-on und Wasserstoffperoxid.
Schmelzpunkt : 58-60 °C,
Ausbeute : 91,1 % der Theorie.

Beispiel 179

6-[6-(3,4-Dichlor-N-acetyl-phenylsulfoximino)-hexyloxy]-4,4-dimethyl-4H-3,1-benzoxazin-2-on

Hergestellt analog Beispiel 143 aus 6-[6-(3,4-Dichlor-phenylsulfoximino)-hexyloxy]-4,4-dimethyl-4H-3,1-benzoxazin-2-on und Acetanhydrid.
Öl, RF-Wert : 0,64 (Kieselgelplatte : Chloroform/Äthanol =9 : 1),
Ausbeute : 83,1 % der Theorie,
$C_{24}H_{28}Cl_2N_2O_5S$ (527,48)
Ber. : C 54,65 H 5,35 S 6,08
Gef. : C 54,30 H 5,34 S 6,06

Beispiel 180

6-[4-(3,4-Dichlor-phenylsulfoximino)-butoxy]-4-isopropyl-4H-3,1-benzoxazin-2-on

Hergestellt analog Beispiel 6 aus 6-[4-(3,4-Dichlor-phenylsulfinyl)-butoxy]-4-isopropyl-4H-3,1-benzoxazin-2-on und O-Mesitylensulfonyl-acethydroxamsäure-äthylester.
Schmelzpunkt : 123-125 °C,
Ausbeute : 47,7 % der Theorie.

Beispiel 181

6-[6-(3,4-Dimethoxy-N-acetyl-phenylsulfoximino)-hexyloxy]-4,4-dimethyl-4H-3,1-benzoxazin-2-on

Hergestellt analog Beispiel 143 aus 6-[6-(3,4-Dimethoxy-phenylsulfoximino)-hexyloxy]-4,4-dimethyl-4H-3,1-benzoxazin-2-on und Acetanhydrid.
Öl, RF-Wert : 0,65 (Kieselgelplatte : Chloroform/Äthanol = 9 : 1).
Ausbeute : 83,5 % der Theorie,
$C_{26}H_{34}N_2O_7S$ (518,63)
Ber. : C 60,21 H 6,61 S 6,18
Gef. : C 59,90 H 6,59 S 6,13

Beispiel 182

6-[4-(3,4-Dichlor-phenylsulfoximino)-butoxy]-4,4-dimethyl-4H-3,1-benzoxazin-2-on

3,3 g (0,007 5 Mol) 6-[4-(3,4-Dichlor-phenylsulfinyl)-butoxy]-4,4-dimethyl-4H-3,1-benzoxazin-2-on werden bei 45 °C in 50 ml Polyphosphorsäure eingerührt. Nachdem praktisch alles gelöst ist, gibt man innerhalb 30 Minuten 0,65 g (0,01 Mol) Natriumazid in kleinen Portionen hinzu. Es ist eine schwache Entwicklung von Stickstoffgas zu bemerken. Man rührt die beigefarbene, cremig-schaumige Masse 3 Stunden bei 45-50 °C und versetzt mit 150 g Eis. Die entstehende trübe Lösung stellt man mit konzentriertem Ammoniak auf pH 8 und extrahiert das ausgefallene harzige Produkt mit Chloroform. Der ölige Eindampfrückstand wird aus Essigester/Diisopropyläther umkristallisiert. Man erhält weiße Kristalle.

Schmelzpunkt : 166-167 °C,
Ausbeute : 2,0 g (58,6 % der Theorie).

Beispiel 183

6-[4-(3,4-Dichlor-phenylsulfimino)-butoxy]-4,4-dimethyl-4H-3,1-benzoxazin-2-on

Hergestellt analog Beispiel 6 aus 6-[4-(3,4-Dichlor-phenylmercapto)-butoxy]-4,4-dimethyl-4H-3,1-benzoxazin-2-on und O-Mesitylensulfonyl-acethydroxamsäure-äthylester.

Schmelzpunkt : 165-166 °C,
Ausbeute : 10 % der Theorie.

Beispiel 184

6-[3-(3,4-Dimethoxy-phenylsulfoximino)-propoxy]-4-isopropyl-4H-3,1-benzoxazin-2-on

Hergestellt analog Beispiel 6 aus 6-[3-(3,4-Dimethoxy-phenylsulfinyl)-propoxy]-4-isopropyl-4H-3,1-benzoxazin-2-on und O-Mesitylensulfonyl-acethydroxamsäure-äthylester.

Schmelzpunkt : 123-125 °C,
Ausbeute : 77,3 % der Theorie.

Beispiel 185

6-[4-(3,4-Dichlor-N-p-toluolsulfonyl-phenylsulfimino)-butoxy]-4,4-dimethyl-4H-3,1-benzoxazin-2-on

4,26 g (0,01 Mol) 6-[4-(3,4-Dichlor-phenylmercapto)-butoxy]-4,4-dimethyl-4H-3,1-benzoxazin-2-on werden in 50 ml Methanol suspendiert und mit soviel Chloroform versetzt, daß eine klare Lösung entsteht (ca. 80 ml). Dazu gibt man unter Rühren eine Lösung von 3,38 g (0,012 Mol) N-Chlor-p-toluolsulfonsäureamid-Natriumsalz in 30 ml Methanol und läßt 4 Stunden bei Raumtemperatur stehen. Zu der gelben Reaktionslösung gibt man ca. 1 ml Eisessig und destilliert die Lösungsmittel am Rotationsverdampfer ab (Badtemperatur : < 20 °C). Der hellbraune zähe Rückstand wird über eine Kieselgelsäule (Elutionsmittel : Essigester/Cyclohexan 4 : 1) gereinigt. Die einheitlichen Fraktionen werden am Rotationsverdampfer zur Trockne eingeengt und der Rückstand einmal aus Essigester/Isopropanol umkristallisiert.

Schmelzpunkt : 162-163 °C,
Ausbeute : 2,0 g (33,6 % der Theorie).

Beispiel 186

6-[4-(3,4-Dichlor-N-p-toluolsulfonyl-phenylsulfoximino)-butoxy]-4,4-dimethyl-4H-3,1-benzoxazin-2-on

0,6 g (0,001 Mol) 6-[4-(3,4-Dichlor-N-p-toluolsulfonyl-phenylsulfimino)-butoxy]-4,4-dimethyl-4H-3,1-benzoxazin-2-on werden in 10 ml Methanol suspendiert, mit 1 ml 2n Natronlauge sowie 0,2 ml (0,002 Mol) 30 %igem Wasserstoffperoxid versetzt und 8 Stunden am Rückfluß gekocht. Es entsteht eine klare Lösung, die nach Beendigung der Reaktion zur Trockne eingeengt wird. Den Rückstand reinigt man über eine Kieselgelsäule (Chloroform). Die einheitlichen Fraktionen werden eingeengt und einmal aus Äthanol umkristallisiert.

Schmelzpunkt : 174-175 °C,
Ausbeute : 0,27 g (44,1 % der Theorie).

Beispiel 187

6-[3-(3,4-Dichlor-phenylsulfoximino)-propoxy]-4-isopropyl-4H-3,1-benzoxazin-2-on

Hergestellt analog Beispiel 6 aus 6-[3-(3,4-Dichlor-phenylsulfinyl)-propoxy]-4-isopropyl-4H-3,1-benzoxazin-2-on und O-Mesitylensulfonyl-acethydroxamsäure-äthylester.
Harz, RF-Wert : 0,45 (Kieselgelplatte : Chloroform/Äthanol = 19 : 1),
Ausbeute : 43,7 % der Theorie,
$C_{20}H_{22}Cl_2N_2O_4S$ (457,38)
Ber. : C 52,52 H 4,85 S 7,01
Gef. : C 52,49 H 5,12 S 6,63

Beispiel 188

6-[3-(3,4-Dimethoxy-N-acetyl-phenylsulfoximino)-propoxy]-4-isopropyl-4H-3,1-benzoxazin-2-on

Hergestellt analog Beispiel 143 aus 6-[3-(3,4-Dimethoxy-phenyl-sulfoximino)-propoxy]-4-isopropyl-4H-3,1-benzoxazin-2-on und Acetanhydrid.
Schmelzpunkt : 73-75 °C,
Ausbeute : 81,4 % der Theorie.

Beispiel 189

6-[4-(4-Cyclohexyl-phenylmercapto)-butoxy]-4,4-diäthyl-4H-3,1-benzoxazin-2-on

Hergestellt analog Beispiel 1 aus 6-(4-Chlorbutoxy)-4,4-diäthyl-4H-3,1-benzoxazin-2-on und 4-Cyclohexyl-thiophenol.
Schmelzpunkt : 85-88 °C,
Ausbeute : 69,1 % der Theorie.

Beispiel 190

6-[4-(3,4-Dichlor-phenylmercapto)-butoxy]-4,4-diäthyl-4H-3,1-benzoxazin-2-on

Hergestellt analog Beispiel 1 aus 6-(4-Chlorbutoxy)-4,4-diäthyl-4H-3,1-benzoxazin-2-on und 3,4-Dichlor-thiophenol.
Schmelzpunkt : 143-145 °C,
Ausbeute : 58,7 % der Theorie.

Beispiel 191

6-(4-Phenylmercapto-butoxy)-4,4-diäthyl-4H-3,1-benzoxazin-2-on

Hergestellt analog Beispiel 1 aus 6-(4-Chlorbutoxy)-4,4-diäthyl-4H-3,1-benzoxazin-2-on und Thiophenol.
Schmelzpunkt : 104-106 °C,
Ausbeute : 69,8 % der Theorie.

Beispiel 192

6-[4-(3,4-Dimethyl-phenylmercapto)-butoxy]-4,4-diäthyl-4H-3,1-benzoxazin-2-on

Hergestellt analog Beispiel 1 aus 6-(4-Chlorbutoxy)-4,4-diäthyl-4H-3,1-benzoxazin-2-on und 3,4-Dimethyl-thiophenol.
Schmelzpunkt : 124-125 °C,
Ausbeute : 83,4 % der Theorie.

Beispiel 193

6-[4-(3,4-Dimethoxy-phenylmercapto)-butoxy]-4,4-diäthyl-4H-3,1-benzoxazin-2-on

Hergestellt analog Beispiel 1 aus 6-(4-Chlorbutoxy)-4,4-diäthyl-4H-3,1-benzoxazin-2-on und 3,4-Dimethoxy-thiophenol.
Schmelzpunkt : 110-111 °C,
Ausbeute : 72,6 % der Theorie.

Beispiel 194

6-[4-(4-Acetamido-phenylmercapto)-butoxy]-4,4-diäthyl-4H-3,1-benzoxazin-2-on

Hergestellt analog Beispiel 1 aus 6-(4-Chlorbutoxy)-4,4-diäthyl-4H-3,1-benzoxazin-2-on und 4-Acetamido-thiophenol.
Schmelzpunkt : 90-92 °C,
Ausbeute : 62,9 % der Theorie.

Beispiel 195

6-[4-(3,4-Dichlor-phenylmercapto)-butoxy]-4-methyl-4H-3,1-benzoxazin-2-on

Hergestellt analog Beispiel 1 aus 6-(4-Chlorbutoxy)-4-methyl-4H-3,1-benzoxazin-2-on und 3,4-Dichlor-thiophenol.
Schmelzpunkt : 117-119 °C,
Ausbeute : 50,5 % der Theorie.

Beispiel 196

6-[4-(4-Methyl-phenylmercapto)-butoxy]-4-methyl-4H-3,1-benzoxazin-2-on

Hergestellt analog Beispiel 1 aus 6-(4-Chlorbutoxy)-4-methyl-4H-3,1-benzoxazin-2-on und 4-Methylthiophenol.
Schmelzpunkt : 111-113 °C,
Ausbeute : 54,4 % der Theorie.

Beispiel 197

6-[4-(4-Acetamido-phenylmercapto)-butoxy]-4-methyl-4H-3,1-benzoxazin-2-on

Hergestellt analog Beispiel 1 aus 6-(4-Chlorbutoxy)-4-methyl-4H-3,1-benzoxazin-2-on und 4-Acetamido-thiophenol.
Schmelzpunkt : 122-124 °C,
Ausbeute : 44,2 % der Theorie.

Beispiel 198

6-[4-(2-Benzthiazolylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 2-Mercapto-benzthiazol.
Schmelzpunkt : 183-184 °C,
Ausbeute : 56,3 % der Theorie.

Beispiel 199

6-[4-(4,6-Dimethyl-2-pyrimidinylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4,6-Dimethyl-2-mercaptopyrimidin.
Schmelzpunkt : 125-127 °C,
Ausbeute : 65,9 % der Theorie.

Beispiel 200

6-[4-(1-Oxido-2-pyridylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 2-Mercapto-pyridin-1-oxid.
Schmelzpunkt : 154-156 °C,
Ausbeute : 14,6 % der Theorie.

Beispiel 201

6-[4-(1,2,4-Triazol-3-yl-mercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 3-Mercapto-1,2,4-triazol.
Schmelzpunkt : 181-183 °C,
Ausbeute : 42,2 % der Theorie.

Beispiel 202

6-[4-(2-Pyrimidinylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 2-Mercapto-pyrimidin.
Schmelzpunkt : 142-144 °C,
Ausbeute : 53,9 % der Theorie.

Beispiel 203

6-[4-(4-Pyridylmercapto)-butoxy]-4,4-dimethyl-4H-3,1-benzoxazin-2-on

Hergestellt analog Beispiel 1 aus 6-(4-Chlorbutoxy)-4,4-dimethyl-4H-3,1-benzoxazin-2-on und 4-Mercapto-pyridin.
Schmelzpunkt : 153-155 °C,
Ausbeute : 64,2 % der Theorie.

Beispiel 204

8-[4-(4-Cyclohexyl-phenylmercapto)-butoxy]-4,4-dimethyl-4H-3,1-benzoxazin-2-on

Hergestellt analog Beispiel 1 aus 8-(4-Chlorbutoxy)-4,4-dimethyl-4H-3,1-benzoxazin-2-on und 4-Cyclohexyl-thiophenol.
Schmelzpunkt : 109-110 °C,
Ausbeute : 70,5 % der Theorie.

Beispiel 205

8-[4-(3,4-Dichlor-phenylmercapto)-butoxy]-4,4-dimethyl-4H-3,1-benzoxazin-2-on

Hergestellt analog Beispiel 1 aus 8-(4-Chlorbutoxy)-4,4-dimethyl-4H-3,1-benzoxazin-2-on und 3,4-Dichlor-thiophenol.
Schmelzpunkt : 137-138 °C,
Ausbeute : 74,5 % der Theorie.

Beispiel 206

8-(4-Phenylmercapto-butoxy)-4,4-dimethyl-4H-3,1-benzoxazin-2-on

Hergestellt analog Beispiel 1 aus 8-(4-Chlorbutoxy)-4,4-dimethyl-4H-3,1-benzoxazin-2-on und Thiophenol.
Schmelzpunkt : 98-100 °C,
Ausbeute : 87,9 % der Theorie.

Beispiel 207

8-[4-(3,4-Dimethyl-phenylmercapto)-butoxy]-4,4-dimethyl-4H-3,1-benzoxazin-2-on

Hergestellt analog Beispiel 1 aus 8-(4-Chlorbutoxy)-4,4-dimethyl-4H-3,1-benzoxazin-2-on und 3,4-Dimethyl-thiophenol.
Schmelzpunkt : 137-139 °C,
Ausbeute : 90,7 % der Theorie.

Beispiel 208

8-[4-(3,4-Dimethoxy-phenylmercapto)-butoxy]-4,4-dimethyl-4H-3,1-benzoxazin-2-on

Hergestellt analog Beispiel 1 aus 8-(4-Chlorbutoxy)-4,4-dimethyl-4H-3,1-benzoxazin-2-on und 3,4-Dimethoxy-thiophenol.
Schmelzpunkt : 116-117 °C,

Ausbeute : 93,8 % der Theorie.

Beispiel 209

8-[4-(4-Acetamido-phenylmercapto)-butoxy]-4,4-dimethyl-4H-3,1-benzoxazin-2-on

Hergestellt analog Beispiel 1 aus 8-(4-Chlorbutoxy)-4,4-dimethyl-4H-3,1-benzoxazin-2-on und 4-Acetamido-thiophenol.
Schmelzpunkt : 166-167 °C,
Ausbeute : 63,6 % der Theorie.

Beispiel 210

6-[4-(3,4-Dimethylphenylmercapto)-butoxy]-7-nitro-4,4-dimethyl-4H-3,1-benzoxazin-2-on

9,1 g (0,023 4 Mol) 6-(4-Methansulfonyloxy-butoxy)-7-nitro-4,4-dimethyl-4H-3,1-benzoxazin-2-on und 3,5 g (0,025 Mol) 3,4-Dimethyl-thiophenol werden in 80 ml Dimethylformamid gelöst. Unter Rühren werden 6,9 g (0,05 Mol) Kaliumcarbonat und schließlich 4 ml Wasser zugegeben. Die Reaktionsmischung erwärmt sich kurz und wird nach Abklingen der Wärmetönnung noch 2 Stunden bei Raumtemperatur gerührt. Nach Zugabe von Eiswasser extrahiert man mit Chloroform, wäscht die organische Phase mit Wasser, trocknet mit Natriumsulfat und destilliert das Lösungsmittel im Vakuum ab. Der Rückstand wird über eine Kieselgelsäule (Elutionsmittel : Chloroform/Äthanol = 40 : 1) gereinigt.
Öl, RF-Wert : 0,4 (Kieselgelplatte : Chloroform/Äthanol = 9 : 1),
Ausbeute : 7,6 g (75,4 % der Theorie).
$C_{22}H_{26}N_2O_5S$ (430,52)

| | | | | |
|---|---|---|---|---|
| Ber. : | C 61,38 | H 6,09 | N 6,51 | S 7,45 |
| Gef. : | C 61,10 | H 6,07 | N 6,24 | S 7,28 |

Beispiel 211

6-[4-(4-Acetamido-phenylmercapto)-butoxy]-7-nitro-4,4-dimethyl-4H-3,1-benzoxazin-2-on

Hergestellt analog Beispiel 210 aus 6-(4-Methansulfonyloxy-butoxy)-7-nitro-4,4-dimethyl-4H-3,1-benzoxazin-2-on und 4-Acetamido-thiophenol.
Schmelzpunkt : 203-205 °C,
Ausbeute : 69,2 % der Theorie.

Beispiel 212

6-[4-(4-Chlor-phenylmercapto)-butoxy]-7-nitro-4,4-dimethyl-4H-3,1-benzoxazin-2-on

Hergestellt analog Beispiel 210 aus 6-(4-Methansulfonyloxy-butoxy)-7-nitro-4,4-dimethyl-4H-3,1-benzoxazin-2-on und 4-Chlor-thiophenol.
Schmelzpunkt : 155-156 °C,
Ausbeute : 69,5 % der Theorie.

Beispiel 213

6-[4-(2-Pyridylmercapto)-butoxy]-7-nitro-4,4-dimethyl-4H-3,1-benzoxazin-2-on

Hergestellt analog Beispiel 210 aus 6-(4-Methansulfonyloxy-butoxy)-7-nitro-4,4-dimethyl-4H-3,1-benzoxazin-2-on und 2-Mercapto-pyridin.
Schmelzpunkt : 98-100 °C,
Ausbeute : 59,3 % der Theorie.

Beispiel 214

6-[4-(4-Methyl-phenylmercapto)-butoxy]-7-nitro-4,4-dimethyl-4H-3,1-benzoxazin-2-on

Hergestellt analog Beispiel 210 aus 6-(4-Methansulfonyloxy-butoxy)-7-nitro-4,4-dimethyl-4H-3,1-benzoxazin-2-on und 4-Methyl-thiophenol.
Schmelzpunkt : 128-129 °C,
Ausbeute : 77,7 % der Theorie.

Beispiel 215

6-[4-(3,4-Dimethoxy-phenylmercapto)-butoxy]-7-nitro-4,4-dimethyl-4H-3,1-benzoxazin-2-on

Hergestellt analog Beispiel 210 aus 6-(4-Methansulfonyloxy-butoxy)-7-nitro-4,4-dimethyl-4H-3,1-benzoxazin-2-on und 3,4-Dimethoxy-thiophenol.
Schmelzpunkt : 115-117 °C,
Ausbeute : 82,5 % der Theorie.

Beispiel 216

7-[4-(3,4-Dimethyl-phenylmercapto)-butoxy]-4,4-dimethyl-4H-3,1-benzoxazin-2-on

Hergestellt analog Beispiel 210 aus 7-(4-Methansulfonyloxy-butoxy)-4,4-dimethyl-4H-3,1-benzoxazin-2-on und 3,4-Dimethylthiophenol.
Schmelzpunkt : 120-122 °C,
Ausbeute : 84,5 % der Theorie.

Beispiel 217

7-[4-(4-Acetamido-phenylmercapto)-butoxy]-4,4-dimethyl-4H-3,1-benzoxazin-2-on

Hergestellt analog Beispiel 210 aus 7-(4-Methansulfonyloxy-butoxy)-4,4-dimethyl-4H-3,1-benzoxazin-2-on und 4-Acetamidothiophenol.
Schmelzpunkt : 162-164 °C,
Ausbeute : 97,7 % der Theorie.

Beispiel 218

7-[4-(2-Pyridylmercapto)-butoxy]-4,4-dimethyl-4H-3,1-benzoxazin-2-on

Hergestellt analog Beispiel 210 aus 7-(4-Methansulfonyloxy-butoxy)-4,4-dimethyl-4H-3,1-benzoxazin-2-on und 2-Mercaptopyridin.
Schmelzpunkt : 125-127 °C,
Ausbeute : 75,4 % der Theorie.

Beispiel 219

7-[4-(4-Methyl-phenyl-mercapto)-butoxy]-4,4-dimethyl-4H-3,1-benzoxazin-2-on

Hergestellt analog Beispiel 210 aus 7-(4-Methansulfonyloxy-butoxy)-4,4-dimethyl-4H-3,1-benzoxazin-2-on und 4-Methylthiophenol.
Schmelzpunkt : 120-122 °C,
Ausbeute : 80,7 % der Theorie.

Beispiel 220

7-[4-(4-Chlor-phenylmercapto)-butoxy]-4,4-dimethyl-4H-3,1-benzoxazin-2-on

Hergestellt analog Beispiel 210 aus 7-(4-Methansulfonyloxy-butoxy)-4,4-dimethyl-4H-3,1-benzoxazin-2-on und 4-Chlorthiophenol.
Schmelzpunkt : 117-119 °C,
Ausbeute : 86,7 % der Theorie.

Beispiel 221

7-[4-(3,4-Dichlor-phenylmercapto)-butoxy]-4,4-dimethyl-4H-3,1-benzoxazin-2-on

Hergestellt analog Beispiel 210 aus 7-(4-Methansulfonyloxy-butoxy)-4,4-dimethyl-4H-3,1-benzoxazin-2-on und 3,4-Dichlor-thiophenol.
Schmelzpunkt : 104-106 °C,
Ausbeute : 79,7 % der Theorie.

Beispiel 222

7-(4-Phenylmercapto-butoxy)-4,4-dimethyl-4H-3,1-benzoxazin-2-on

Hergestellt analog Beispiel 210 aus 7-(4-Methansulfonyloxy-butoxy)-4,4-dimethyl-4H-3,1-benzoxazin-2-on und Thiophenol.
Schmelzpunkt : 123-125 °C,
Ausbeute : 89,5 % der Theorie.

Beispiel 223

7-[4-(3,4-Dimethoxy-phenylmercapto)-butoxy]-4,4-dimethyl-4H-3,1-benzoxazin-2-on

Hergestellt analog Beispiel 210 aus 7-(4-(Methansulfonyloxy-butoxy)-4,4-dimethyl-4H-3,1-benzoxazin-2-on und 3,4-Dimethoxy-thiophenol.
Öl, RF-Wert : 0,6 (Kieselgelplatte : Äthylenchlorid/Äthanol = 9 : 1),
Ausbeute : 80,7 % der Theorie,
$C_{22}H_{27}NO_5S$ (417,53)
Ber. : C 63,29 H 6,52 N 3,35
Gef. : C 63,00 H 6,54 N 3,38

Beispiel 224

7-[4-(3,4-Dichlor-phenylsulfinyl)-butoxy]-4,4-dimethyl-4H-3,1-benzoxazin-2-on

Hergestellt analog Beispiel 2 aus 7-[4-(3,4-Dichlor-phenylmercapto)-butoxy]-4,4-dimethyl-4H-3,1-benzoxazin-2-on und Wasserstoffperoxid.
Schmelzpunkt : 82-84 °C,
Ausbeute : 92,1 % der Theorie.

Beispiel 225

7-(4-Phenylsulfinyl-butoxy)-4,4-dimethyl-4H-3,1-benzoxazin-2-on

Hergestellt analog Beispiel 2 aus 7-(4-Phenylmercapto-butoxy)-4,4-dimethyl-4H-3,1-benzoxazin-2-on und Wasserstoffperoxid.
Schmelzpunkt : 117-119 °C,
Ausbeute : 96,7 % der Theorie.

Beispiel 226

7-[4-(3,4-Dimethyl-phenylsulfinyl)-butoxy]-4,4-dimethyl-4H-3,1-benzoxazin-2-on-hydrat

Hergestellt analog Beispiel 2 aus 7-[4-(3,4-Dimethyl-phenylmercapto)-butoxy]-4,4-dimethyl-4H-3,1-benzoxazin-2-on und Wasserstoffperoxid.
Öl, RF-Wert : 0,6 (Kieselgelplatte : Äthylenchlorid/Äthanol = 9 : 1),
Ausbeute : 95,5 % der Theorie,
$C_{22}H_{29}NO_5S$ (419,54)
Ber. : C 62,98 H 6,97 N 3,34 S 7,64
Gef. : C 63,24 H 6,85 N 3,40 S 7,64

Beispiel 227

7-[4-(4-Acetamido-phenylsulfinyl)-butoxy]-4,4-dimethyl-4H-3,1-benzoxazin-2-on

Hergestellt analog Beispiel 2 aus 7-[4-(4-Acetamido-phenylmercapto)-butoxy]-4,4-dimethyl-4H-3,1-benzoxazin-2-on und Wasserstoffperoxid.
Schmelzpunkt : 145-147 °C,
Ausbeute : 77,8 % der Theorie.

Beispiel 228

7-[4-(2-Pyridylsulfinyl)-butoxy]-4,4-dimethyl-4H-3,1-benzoxazin-2-on

Hergestellt analog Beispiel 2 aus 7-[4-(2-Pyridylmercapto)-butoxy]-4,4-dimethyl-4H-3,1-benzoxazin-2-on und Wasserstoffperoxid.
Schmelzpunkt : 153-155 °C,
Ausbeute : 64,9 % der Theorie.

Beispiel 229

7-[4-(4-Methyl-phenylsulfinyl)-butoxy]-4,4-dimethyl-4H-3,1-benzoxazin-2-on

Hergestellt analog Beispiel 2 aus 7-[4-(4-Methyl-phenylmercapto)-butoxy]-4,4-dimethyl-4H-3,1-benzoxazin-2-on und Wasserstoffperoxid.
Schmelzpunkt : 128-130 °C,
Ausbeute : 94,4 % der Theorie.

Beispiel 230

7-[4-(4-Chlor-phenylsulfinyl)-butoxy]-4,4-dimethyl-4H-3,1-benzoxazin-2-on

Hergestellt analog Beispiel 2 aus 7-[4-(4-Chlor-phenylmercapto)-butoxy]-4,4-dimethyl-4H-3,1-benzoxazin-2-on und Wasserstoffperoxid.
Schmelzpunkt : 140-142 °C,
Ausbeute : 91,6 % der Theorie.

Beispiel 231

7-[4-(3,4-Dimethoxy-phenylsulfinyl)-butoxy]-4,4-dimethyl-4H-3,1-benzoxazin-2-on

Hergestellt analog Beispiel 2 aus 7-[4-(3,4-Dimethoxy-phenylmercapto)-butoxy]-4,4-dimethyl-4H-3,1-benzoxazin-2-on und Wasserstoffperoxid.
Harz, RF-Wert : 0,4 (Kieselgelplatte : Äthylenchlorid/Äthanol = 9 : 1),
Ausbeute : 40,8 % der Theorie.
$C_{22}H_{27}NO_6S$ (433,53)

| | | | | |
|---|---|---|---|---|
| Ber. : | C 60,95 | H 6,28 | N 3,23 | S 7,40 |
| Gef. : | C 60,70 | H 6,25 | N 3,03 | S 7,53 |

Beispiel 232

8-[4-(4-Cyclohexyl-phenylsulfinyl)-butoxy]-4,4-dimethyl-4H-3,1-benzoxazin-2-on

Hergestellt analog Beispiel 2 aus 8-[4-(4-Cyclohexyl-phenylmercapto)-butoxy]-4,4-dimethyl-4H-3,1-benzoxazin-2-on und Wasserstoffperoxid.
Schmelzpunkt : 144-145 °C,
Ausbeute : 71,9 % der Theorie.

Beispiel 233

8-[4-(3,4-Dichlor-phenylsulfinyl)-butoxy]-4,4-dimethyl-4H-3,1-benzoxazin-2-on

Hergestellt analog Beispiel 2 aus 8-[4-(3,4-Dichlorphenylmercapto)-butoxy]-4,4-dimethyl-4H-3,1-benzoxazin-2-on und Wasserstoffperoxid.
Schmelzpunkt : 113-114 °C,
Ausbeute : 81,0 % der Theorie.

Beispiel 234

8-(4-Phenylsulfinyl-butoxy)-4,4-dimethyl-4H-3,1-benzoxazin-2-on

Hergestellt analog Beispiel 2 aus 8-(4-Phenylmercapto-butoxy)-4,4-dimethyl-4H-3,1-benzoxazin-2-on und Wasserstoffperoxid.
Schmelzpunkt : 162-163 °C,
Ausbeute : 91,6 % der Theorie.

Beispiel 235

8-[4-(4-Acetamido-phenylsulfinyl)-butoxy]-4,4-dimethyl-4H-3,1-benzoxazin-2-on

Hergestellt analog Beispiel 2 aus 8-[4-(4-Acetamido-phenylmercapto)-butoxy]-4,4-dimethyl-4H-3,1-benzoxazin-2-on und Wasserstoffperoxid.
Schmelzpunkt : 166-167 °C,

Ausbeute : 63,2 % der Theorie.

Beispiel 236

8-[4-(3,4-Dimethyl-phenylsulfinyl)-butoxy]-4,4-dimethyl-4H-3,1-benzoxazin-2-on

Hergestellt analog Beispiel 2 aus 8-[4-(3,4-Dimethyl-phenylmercapto)-butoxy]-4,4-dimethyl-4H-3,1-benzoxazin-2-on und Wasserstoffperoxid.
Schmelzpunkt : 111-112 °C,
Ausbeute : 63,9 % der Theorie.

Beispiel 237

8-[4-(3,4-Dimethoxy-phenylsulfinyl)-butoxy]-4,4-dimethyl-4H-3,1-benzoxazin-2-on

Hergestellt analog Beispiel 2 aus 8-[4-(3,4-Dimethoxy-phenylmercapto)-butoxy]-4,4-dimethyl-4H-3,1-benzoxazin-2-on und Wasserstoffperoxid.
Schmelzpunkt : 102-103 °C,
Ausbeute : 90,2 % der Theorie.

Beispiel 238

6-[4-(4-Cyclohexyl-phenylsulfinyl)-butoxy]-4,4-diäthyl-4H-3,1-benzoxazin-2-on

Hergestellt analog Beispiel 2 aus 6-[4-(4-Cyclohexyl-phenylmercapto)-butoxy]-4,4-diäthyl-4H-3,1-benzoxazin-2-on und Wasserstoffperoxid.
Schmelzpunkt : 168-170 °C,
Ausbeute : 82,7 % der Theorie.

Beispiel 239

6-[4-(3,4-Dichlor-phenylsulfinyl)-butoxy]-4,4-diäthyl-4H-3,1-benzoxazin-2-on

Hergestellt analog Beispiel 2 aus 6-[4-(3,4-Dichlor-phenylmercapto)-butoxy]-4,4-diäthyl-4H-3,1-benzoxazin-2-on und Wasserstoffperoxid.
Schmelzpunkt : 91-93 °C,
Ausbeute : 70,9 % der Theorie.

Beispiel 240

6-(4-Phenylsulfinyl-butoxy)-4,4-diäthyl-4H-3,1-benzoxazin-2-on

Hergestellt analog Beispiel 2 aus 6-(4-Phenylmercapto-butoxy)-4,4-diäthyl-4H-3,1-benzoxazin-2-on und Wasserstoffperoxid.
Harz, RF-Wert : 0,6 (Kieselgelplatte : Chloroform/Äthanol = 9 : 1),
Ausbeute : 94,3 % der Theorie.
$C_{22}H_{27}NO_4S$ (401,53)
Ber. : C 65,81 H 6,78 N 3,49 S 7,98
Gef. : C 65,55 H 6,75 N 3,40 S 7,71

Beispiel 241

6-[4-(3,4-Dimethyl-phenylsulfinyl)-butoxy]-4,4-diäthyl-4H-3,1-benzoxazin-2-on

Hergestellt analog Beispiel 2 aus 6-[4-(3,4-Dimethyl-phenylmercapto)-butoxy]-4,4-diäthyl-4H-3,1-benzoxazin-2-on und Wasserstoffperoxid.
Schmelzpunkt : 137-138 °C,
Ausbeute : 83,1 % der Theorie.

Beispiel 242

6-[4-(3,4-Dimethoxy-phenylsulfinyl)-butoxy]-4,4-diäthyl-4H-3,1-benzoxazin-2-on

Hergestellt analog Beispiel 2 aus 6-[4-(3,4-Dimethoxy-phenylmercapto)-butoxy]-4,4-diäthyl-4H-3,1-benzoxazin-2-on und Wasserstoffperoxid.

Schmelzpunkt : 161-163 °C,
Ausbeute : 87,7 % der Theorie.

Beispiel 243

6-[4-(4-Acetamido-phenylsulfinyl)-butoxy]-4,4-diäthyl-4H-3,1-benzoxazin-2-on

Hergestellt analog Beispiel 2 aus 6-[4-(4-Acetamido-phenylmercapto)-butoxy]-4,4-diäthyl-4H-3,1-benzoxazin-2-on und Wasserstoffperoxid.
Schmelzpunkt : 69-70 °C,
Ausbeute : 92,6 % der Theorie.

Beispiel 244

6-[4-(4-Pyridylsulfinyl)-butoxy]-4,4-dimethyl-4H-3,1-benzoxazin-2-on

Hergestellt analog Beispiel 2 aus 6-[4-(4-Pyridylmercapto)-butoxy]-4,4-dimethyl-4H-3,1-benzoxazin-2-on und Wasserstoffperoxid.
Schmelzpunkt : 141-143 °C,
Ausbeute : 36,9 % der Theorie.

Beispiel 245

6-[4-(4,6-Dimethyl-2-pyrimidinylsulfinyl)-butoxy]-4,4-dimethyl-4H-3,1-benzoxazin-2-on

Hergestellt analog Beispiel 2 aus 6-[4-(4,6-Dimethyl-2-pyrimidinylmercapto)-butoxy]-4,4-dimethyl-4H-3,1-benzoxazin-2-on und Wasserstoffperoxid.
Harz, RF-Wert : 0,4 (Kieselgelplatte : Äthylenchlorid/Äthanol = 9 : 1),
Ausbeute : 15,0 % der Theorie.
$C_{20}H_{25}N_3O_4S$ (403,50)
Ber. : C 59,53 H 6,25 S 7,95
Gef. : C 59,30 H 5,99 S 7,88

Beispiel 246

6-[4-(3,4-Dichlor-phenylsulfinyl)-butoxy]-4-methyl-4H-3,1-benzoxazin-2-on

Hergestellt analog Beispiel 2 aus 6-[4-(3,4-Dichlor-phenylmercapto)-butoxy]-4-methyl-4H-3,1-benzoxazin-2-on und Wasserstoffperoxid.
Schmelzpunkt : 138-139 °C,
Ausbeute : 86,0 % der Theorie.

Beispiel 247

6-[4-(4-Methyl-phenylsulfinyl)-butoxy]-4-methyl-4H-3,1-benzoxazin-2-on

Hergestellt analog Beispiel 2 aus 6-[4-(4-Methyl-phenylmercapto)-butoxy]-4-methyl-4H-3,1-benzoxazin-2-on und Wasserstoffperoxid.
Schmelzpunkt : 120-121 °C,
Ausbeute : 58,4 % der Theorie.

Beispiel 248

6-[4-(4-Acetamido-phenylsulfinyl)-butoxy]-4-methyl-4H-3,1-benzoxazin-2-on

Hergestellt analog Beispiel 2 aus 6-[4-(4-Acetamido-phenylmercapto)-butoxy]-4-methyl-4H-3,1-benzoxazin-2-on und Wasserstoffperoxid.
Schmelzpunkt : 124-126 °C,
Ausbeute : 60,4 % der Theorie.

Beispiel 249

6-[4-(2-Benzthiazolylsulfonyl)-butoxy]-4,4-dimethyl-4H-3,1-benzoxazin-2-on

Hergestellt analog Beispiel 3 aus 6-[4-(2-Benzthiazolylmercapto)-butoxy]-4,4-dimethyl-4H-3,1-benzo-

xazin-2-on und Wasserstoffperoxid/Eisessig.
Schmelzpunkt : 177-179 °C,
Ausbeute : 55,9 % der Theorie.

Beispiel 250

6-[4-(1,2,4-Triazolyl-3-sulfonyl)-butoxy]-4,4-dimethyl-4H-3,1-benzoxazin-2-on

Hergestellt analog Beispiel 3 aus 6-[4-(1,2,4-Triazolyl-3-mercapto)-butoxy]-4,4-dimethyl-4H-3,1-benzoxazin-2-on und Wasserstoffperoxid/Eisessig.
Schmelzpunkt : 197-199 °C,
Ausbeute : 83,0 % der Theorie.

Beispiel 251

6-[4-(2-Pyrimidinylsulfonyl)-butoxy]-4,4-dimethyl-4H-3,1-benzoxazin-2-on

Hergestellt analog Beispiel 3 aus 6-[4-(2-Pyrimidinylmercapto)-butoxy]-4,4-dimethyl-4H-3,1-benzoxazin-2-on und Wasserstoffperoxid/Eisessig.
Schmelzpunkt : 184-186 °C,
Ausbeute : 69,0 % der Theorie.

Beispiel 252

5-[4-(3,4-Dichlor-phenylsulfinyl)-butoxy]-4,4-dimethyl-4H-3,1-benzoxazin-2-on

Hergestellt analog Beispiel 4 aus 5-Hydroxy-4,4-dimethyl-4H-3,1-benzoxazin-2-on und 4-(3,4-Dichlor-phenylsulfinyl)-butylbromid.
Schmelzpunkt : 89-90 °C,
Ausbeute : 73,2 % der Theorie.

Beispiel 253

5-[4-(3,4-Dichlor-phenylsulfoximino)-butoxy]-4,4-dimethyl-4H-3,1-benzoxazin-2-on

Hergestellt analog Beispiel 6 aus 5-[4-(3,4-Dichlor-phenylsulfinyl)-butoxy]-4,4-dimethyl-4H-3,1-benzoxazin-2-on und O-Mesitylensulfonyl-acethydroxamsäure-äthylester.
Schmelzpunkt : 127-128 °C,
Ausbeute : 50 % der Theorie.

Beispiel 254

7-[4-(3,4-Dichlor-phenylsulfoximino)-butoxy]-4,4-dimethyl-4H-3,1-benzoxazin-2-on

Hergestellt analog Beispiel 6 aus 7-[4-(3,4-Dichlor-phenylsulfinyl)-butoxy]4,4-dimethyl-4H-3,1-benzoxazin-2-on und O-Mesitylensulfonyl-acethydroxamsäure-äthylester.
Harz, RF-Wert : 0,4 (Kieselgelplatte : Äthylenchlorid/Äthanol = 9 : 1),
Ausbeute : 39,2 % der Theorie.
$C_{20}H_{22}Cl_2N_2O_4S$ (457,39)

| | | | | | |
|---|---|---|---|---|---|
| Ber. : | C 52,52 | H 4,85 | Cl 15,50 | N 6,12 | S 7,01 |
| Gef. : | C 52,34 | H 4,80 | Cl 15,50 | N 6,16 | S 7,01 |

Beispiel 255

7-[4-(3,4-Dimethyl-phenylsulfoximino)-butoxy]-4,4-dimethyl-4H-3,1-benzoxazin-2-on

Hergestellt analog Beispiel 6 aus 7-[4-(3,4-Dimethyl-phenylsulfinyl)-butoxy]-4,4-dimethyl-4H-3,1-benzoxazin-2-on und O-Mesitylensulfonyl-acethydroxamsäure-äthylester.
Schmelzpunkt : 161-163 °C,
Ausbeute : 77,2 % der Theorie.

Beispiel 256

7-[4-(4-Acetamido-phenylsulfoximino)-butoxy]-4,4-dimethyl-4H-3,1-benzoxazin-2-on

Hergestellt analog Beispiel 6 aus 7-[4-(4-Acetamido-phenylsulfinyl)-butoxy]-4,4-dimethyl-4H-3,1-benzoxazin-2-on und O-Mesitylensulfonyl-acethydroxamsäure-äthylester.
Schmelzpunkt : 197-198 °C,
Ausbeute : 29,2 % der Theorie.

Beispiel 257

7-[4-(4-Methyl-phenylsulfoximino)-butoxy]-4,4-dimethyl-4H-3,1-benzoxazin-2-on

Hergestellt analog Beispiel 6 aus 7-[4-(4-Methyl-phenylsulfinyl)-butoxy]-4,4-dimethyl-4H-3,1-benzoxazin-2-on und O-Mesitylensulfonyl-acethydroxamsäure-äthylester.
Schmelzpunkt : 122-124 °C,
Ausbeute : 54,6 % der Theorie.

Beispiel 258

7-[4-(4-Chlor-phenylsulfoximino)-butoxy]-4,4-dimethyl-4H-3,1-benzoxazin-2-on

Hergestellt analog Beispiel 6 aus 7-[4-(4-Chlor-phenylsulfinyl)-butoxy]-4,4-dimethyl-4H-3,1-benzoxazin-2-on und O-Mesitylensulfonyl-acethydroxamsäure-äthylester.
Schmelzpunkt : 103-105 °C,
Ausbeute : 53,8 % der Theorie.

Beispiel 259

7-(4-Phenylsulfoximino-butoxy)-4,4-dimethyl-4H-3,1-benzoxazin-2-on

Hergestellt analog Beispiel 6 aus 7-(4-Phenylsulfinyl-butoxy)-4,4-dimethyl-4H-3,1-benzoxazin-2-on und O-Mesitylensulfonyl-acethydroxamsäure-äthylester.
Schmelzpunkt : 130-132 °C,
Ausbeute : 75,1 % der Theorie.

Beispiel 260

7-[4-(3,4-Dimethoxy-phenylsulfoximino)-butoxy]-4,4-dimethyl-4H-3,1-benzoxazin-2-on

Hergestellt analog Beispiel 6 aus 7-[4-(3,4-Dimethoxy-phenylsulfinyl)-butoxy]-4,4-dimethyl-4H-3,1-benzoxazin-2-on und O-Mesitylensulfonyl-acethydroxamsäure-äthylester.
Schmelzpunkt : 110-112 °C,
Ausbeute : 81,2 % der Theorie.

Beispiel 261

8-[4-(4-Cyclohexyl-phenylsulfoximino)-butoxy]-4,4-dimethyl-4H-3,1-benzoxazin-2-on

Hergestellt analog Beispiel 6 aus 8-[4-(4-Cyclohexyl-phenylsulfinyl)-butoxy]-4,4-dimethyl-4H-3,1-benzoxazin-2-on und O-Mesitylensulfonyl-acethydroxamsäure-äthylester.
Schmelzpunkt : 115-116 °C,
Ausbeute : 47,9 % der Theorie.

Beispiel 262

8-[4-(3,4-Dimethyl-phenylsulfoximino)-butoxy]-4,4-dimethyl-4H-3,1-benzoxazin-2-on

Hergestellt analog Beispiel 6 aus 8-[4-(3,4-Dimethylphenylsulfinyl)-butoxy]-4,4-dimethyl-4H-3,1-benzoxazin-2-on und O-Mesitylensulfonyl-acethydroxamsäure-äthylester.
Schmelzpunkt : 130-131 °C,
Ausbeute : 89,6 % der Theorie.

Beispiel 263

8-[4-(3,4-Dichlor-phenylsulfoximino)-butoxy]-4,4-dimethyl-4H-3,1-benzoxazin-2-on

Hergestellt analog Beispiel 6 aus 8-[4-(3,4-Dichlor-phenylsulfinyl)-butoxy]-4,4-dimethyl-4H-3,1-benzoxazin-2-on und O-Mesitylensulfonyl-acethydroxamsäure-äthylester.

Schmelzpunkt : 144-145 °C,
Ausbeute : 59,6 % der Theorie.

Beispiel 264

8-(4-Phenylsulfoximino-butoxy)-4,4-dimethyl-4H-3,1-benzoxazin-2-on

Hergestellt analog Beispiel 6 aus 8-(4-Phenylsulfinyl-butoxy)-4,4-dimethyl-4H-3,1-benzoxazin-2-on und O-Mesitylensulfonyl-acethydroxamsäure-äthylester.
Schmelzpunkt : 103-104 °C,
Ausbeute : 60 % der Theorie.

Beispiel 265

8-[4-(3,4-Dimethoxy-phenylsulfoximino)-butoxy]-4,4-dimethyl-4H-3,1-benzoxazin-2-on

Hergestellt analog Beispiel 6 aus 8-[4-(3,4-Dimethoxy-phenylsulfinyl)-butoxy]-4,4-dimethyl-4H-3,1-benzoxazin-2-on und O-Mesitylensulfonyl-acethydroxamsäure-äthylester.
Schmelzpunkt : 120-121 °C,
Ausbeute : 74,3 % der Theorie.

Beispiel 266

8-[4-(4-Acetamido-phenylsulfoximino)-butoxy]-4,4-dimethyl-4H-3,1-benzoxazin-2-on

Hergestellt analog Beispiel 6 aus 8-[4-(4-Acetamido-phenylsulfinyl)-butoxy]-4,4-dimethyl-4H-3,1-benzoxazin-2-on und O-Mesitylensulfonyl-acethydroxamsäure-äthylester.
Schmelzpunkt : 166-167 °C,
Ausbeute : 56,6 % der Theorie.

Beispiel 267

6-[4-(3,4-Dimethyl-phenylsulfoximino)-butoxy]-4,4-diäthyl-4H-3,1-benzoxazin-2-on

Hergestellt analog Beispiel 6 aus 6-[4-(3,4-Dimethyl-phenylsulfinyl)-butoxy]-4,4-diäthyl-4H-3,1-benzoxazin-2-on und O-Mesitylensulfonyl-acethydroxamsäure-äthylester.
Schmelzpunkt : 104-105 °C,
Ausbeute : 52,7 % der Theorie.

Beispiel 268

6-[4-(3,4-Dimethoxy-phenylsulfoximino)-butoxy]-4,4-diäthyl-4H-3,1-benzoxazin-2-on

Hergestellt analog Beispiel 6 aus 6-[4-(3,4-Dimethoxy-phenylsulfinyl)-butoxy]-4,4-diäthyl-4H-3,1-benzoxazin-2-on und O-Mesitylensulfonyl-acethydroxamsäure-äthylester.
Schmelzpunkt : 93-95 °C,
Ausbeute : 36,9 % der Theorie.

Beispiel 269

6-[4-(4-Cyclohexyl-phenylsulfoximino)-butoxy]-4,4-diäthyl-4H-3,1-benzoxazin-2-on

Hergestellt analog Beispiel 6 aus 6-[4-(4-Cyclohexyl-phenylsulfinyl)-butoxy]-4,4-diäthyl-4H-3,1-benzoxazin-2-on und O-Mesitylensulfonyl-acethydroxamsäure-äthylester.
Schmelzpunkt : 158-160 °C,
Ausbeute : 36,6 % der Theorie.

Beispiel 270

6-[4-(4-Acetamido-phenylsulfoximino)-butoxy]-4,4-diäthyl-4H-3,1-benzoxazin-2-on

Hergestellt analog Beispiel 6 aus 6-[4-(4-Acetamido-phenylsulfinyl)-butoxy]-4,4-diäthyl-4H-3,1-benzoxazin-2-on und O-Mesitylensulfonyl-acethydroxamsäure-äthylester.
Schmelzpunkt : 144-146 °C,
Ausbeute : 64,0 % der Theorie.

Beispiel 271

6-[4-(3,4-Dichlor-phenylsulfoximino)-butoxy]-4,4-diäthyl-4H-3,1-benzoxazin-2-on

Hergestellt analog Beispiel 6 aus 6-[4-(3,4-Dichlor-phenylsulfinyl)-butoxy]-4,4-diäthyl-4H-3,1-benzoxazin-2-on und O-Mesitylensulfonyl-acethydroxamsäure-äthylester.
Schmelzpunkt : 136-138 °C,
Ausbeute : 68,7 % der Theorie.

Beispiel 272

6-(4-Phenylsulfoximino-butoxy)-4,4-diäthyl-4H-3,1-benzoxazin-2-on

Hergestellt analog Beispiel 6 aus 6-(4-Phenylsulfinyl-butoxy)-4,4-diäthyl-4H-3,1-benzoxazin-2-on und O-Mesitylensulfonyl-acethydroxamsäure-äthylester.
Schmelzpunkt : 134-135 °C,
Ausbeute : 69,6 % der Theorie.

Beispiel 273

6-[4-(3,4-Dichlor-phenylsulfoximino)-butoxy]-4-methyl-4H-3,1-benzoxazin-2-on

Hergestellt analog Beispiel 6 aus 6-[4-(3,4-Dichlor-phenylsulfinyl)-butoxy]-4-methyl-4H-3,1-benzoxazin-2-on und O-Mesitylensulfonyl-acethydroxamsäure-äthylester.
Schmelzpunkt : 179-180 °C,
Ausbeute : 67,6 % der Theorie.

Beispiel 274

6-[4-(4-Methyl-phenylsulfoximino)-butoxy]-4-methyl-4H-3,1-benzoxazin-2-on

Hergestellt analog Beispiel 6 aus 6-[4-(4-Methyl-phenylsulfinyl)-butoxy]-4-methyl-4H-3,1-benzoxazin-2-on und O-Mesitylensulfonyl-acethydroxamsäure-äthylester.
Schmelzpunkt : 121-123 °C,
Ausbeute : 45,5 % der Theorie.

Beispiel 275

6-[4-(4-Acetamido-phenylsulfoximino)-butoxy]-4-methyl-4H-3,1-benzoxazin-2-on

Hergestellt analog Beispiel 6 aus 6-[4-(4-Acetamido-phenylsulfinyl)-butoxy]-4-methyl-4H-3,1-benzoxazin-2-on und O-Mesitylensulfonyl-acethydroxamsäure-äthylester.
Schmelzpunkt : 123-125 °C,
Ausbeute : 45,2 % der Theorie.

Beispiel 276

7-[4-(3,4-Dimethyl-N-acetyl-phenylsulfoximino)-butoxy]-4,4-dimethyl-4H-3,1-benzoxazin-2-on

Hergestellt analog Beispiel 143 aus 7-[4-(3,4-Dimethyl-phenylsulfoximino)-butoxy]-4,4-dimethyl-4H-3,1-benzoxazin-2-on und Acetanhydrid.
Schmelzpunkt : 151-153 °C,
Ausbeute : 70,0 % der Theorie.

Beispiel 277

7-[4-(4-Acetamido-N-acetyl-phenylsulfoximino)-butoxy]-4,4-dimethyl-4H-3,1-benzoxazin-2-on

Hergestellt analog Beispiel 143 aus 7-[4-(4-Acetamido-phenylsulfoximino)-butoxy]-4,4-dimethyl-4H-3,1-benzoxazin-2-on und Acetanhydrid.
Schmelzpunkt : 136-138 °C,
Ausbeute : 38,8 % der Theorie.

Beispiel 278

7-[4-(4-Methyl-N-acetyl-phenylsulfoximino)-butoxy]-4,4-dimethyl-4H-3,1-benzoxazin-2-on

Hergestellt analog Beispiel 143 aus 7-[4-(4-Methyl-phenylsulfoximino)-butoxy]-4,4-dimethyl-4H-3,1-benzoxazin-2-on und Acetanhydrid.
Schmelzpunkt : 142-144 °C,
Ausbeute : 50,7 % der Theorie.

Beispiel 279

7-[4-(4-Chlor-N-acetyl-phenylsulfoximino)-butoxy]-4,4-dimethyl-4H-3,1-benzoxazin-2-on

Hergestellt analog Beispiel 143 aus 7-[4-(4-Chlor-phenylsulfoximino)-butoxy]-4,4-dimethyl-4H-3,1-benzoxazin-2-on und Acetanhydrid.
Schmelzpunkt : 127-129 °C,
Ausbeute : 85,0 % der Theorie.

Beispiel 280

7-[4-(3,4-Dichlor-N-acetyl-phenylsulfoximino)-butoxy]-4,4-dimethyl-4H-3,1-benzoxazin-2-on

Hergestellt analog Beispiel 143 aus 7-[4-(3,4-Dichlor-phenylsulfoximino)-butoxy]-4,4-dimethyl-4H-3,1-benzoxazin-2-on und Acetanhydrid.
Schmelzpunkt : 130-132 °C,
Ausbeute : 95,6 % der Theorie.

Beispiel 281

7-[4-(N-Acetyl-phenylsulfoximino)-butoxy]-4,4-dimethyl-4H-3,1-benzoxazin-2-on

Hergestellt analog Beispiel 143 aus 7-(4-Phenylsulfoximino-butoxy)-4,4-dimethyl-4H-3,1-benzoxazin-2-on und Acetanhydrid.
Schmelzpunkt : 123-125 °C,
Ausbeute : 84,3 % der Theorie.

Beispiel 282

7-[4-(3,4-Dimethoxy-N-acetyl-phenylsulfoximino)-butoxy]-4,4-dimethyl-4H-3,1-benzoxazin-2-on

Hergestellt analog Beispiel 143 aus 7-[4-(3,4-Dimethoxy-phenylsulfoximino)-butoxy]-4,4-dimethyl-4H-3,1-benzoxazin-2-on und Acetanhydrid.
Schmelzpunkt : 202-204 °C,
Ausbeute : 82,5 % der Theorie.

Beispiel 283

8-[4-(4-Cyclohexyl-N-acetyl-phenylsulfoximino)-butoxy]-4,4-dimethyl-4H-3,1-benzoxazin-2-on

Hergestellt analog Beispiel 143 aus 8-[4-(4-Cyclohexyl-phenylsulfoximino)-butoxy]-4,4-dimethyl-4H-3,1-benzoxazin-2-on und Acetanhydrid.
Schmelzpunkt : 167-168 °C,
Ausbeute : 92,9 % der Theorie.

Beispiel 284

8-[4-(3,4-Dichlor-N-acetyl-phenylsulfoximino)-butoxy]-4,4-dimethyl-4H-3,1-benzoxazin-2-on

Hergestellt analog Beispiel 143 aus 8-[4-(3,4-Dichlor-phenylsulfoximino)-butoxy]-4,4-dimethyl-4H-3,1-benzoxazin-2-on und Acetanhydrid.
Schmelzpunkt : 170-171 °C,
Ausbeute : 100 % der Theorie.

Beispiel 285

8-[4-(3,4-Dimethyl-N-acetyl-phenylsulfoximino)-butoxy]-4,4-dimethyl-4H-3,1-benzoxazin-2-on

Hergestellt analog Beispiel 143 aus 8-[4-(3,4-Dimethyl-phenylsulfoximino)-butoxy]-4,4-dimethyl-4H-3,1-benzoxazin-2-on und Acetanhydrid.
Schmelzpunkt : 145-146 °C,
Ausbeute : 93,9 % der Theorie.

Beispiel 286

8-[4-(N-Acetyl-phenylsulfoximino)-butoxy]-4,4-dimethyl-4H-3,1-benzoxazin-2-on

Hergestellt analog Beispiel 143 aus 8-(4-Phenylsulfoximino-butoxy)-4,4-dimethyl-4H-3,1-benzoxazin-2-on und Acetanhydrid.
Schmelzpunkt : 113-114 °C,
Ausbeute : 75,6 % der Theorie.

Beispiel 287

8-[4-(3,4-Dimethoxy-N-acetyl-phenylsulfoximino)-butoxy]-4,4-dimethyl-4H-3,1-benzoxazin-2-on

Hergestellt analog Beispiel 143 aus 8-[4-(3,4-Dimethoxy-phenylsulfoximino)-butoxy]-4,4-dimethyl-4H-3,1-benzoxazin-2-on und Acetanhydrid.
Schmelzpunkt : 137-138 °C,
Ausbeute : 90,2 % der Theorie.

Beispiel 288

8-[4-(4-Acetamido-N-acetyl-phenylsulfoximino)-butoxy]-4,4-dimethyl-4H-3,1-benzoxazin-2-on

Hergestellt analog Beispiel 143 aus 8-[4-(4-Acetamido-phenylsulfoximino)-butoxy]-4,4-dimethyl-4H-3,1-benzoxazin-2-on und Acetanhydrid.
Schmelzpunkt : 183-184 °C,
Ausbeute : 88,6 % der Theorie.

Beispiel 289

6-[4-(4-Cyclohexyl-N-acetyl-phenylsulfoximino)-butoxy]-4,4-diäthyl-4H-3,1-benzoxazin-2-on

Hergestellt analog Beispiel 143 aus 6-[4-(4-Cyclohexyl-phenylsulfoximino)-butoxy]-4,4-diäthyl-4H-3,1-benzoxazin-2-on und Acetanhydrid.
Schmelzpunkt : 176-178 °C,
Ausbeute : 87,3 % der Theorie.

Beispiel 290

6-[4-(3,4-Dimethyl-N-acetyl-phenylsulfoximino)-butoxy]-4,4-diäthyl-4H-3,1-benzoxazin-2-on

Hergestellt analog Beispiel 143 aus 6-[4-(3,4-Dimethyl-phenylsulfoximino)-butoxy]-4,4-diäthyl-4H-3,1-benzoxazin-2-on und Acetanhydrid.
Harz, RF-Wert : 0,52 (Kieselgelplatte, Chloroform/Äthanol = 9 : 1),
Ausbeute : 87,7 % der Theorie.
$C_{26}H_{34}N_2O_5S$ (486,64)
Ber. : C 64,17 H 7,04 N 5,76 S 6,59
Gef. : C 63,90 H 6,90 N 5,51 S 6,94

Beispiel 291

6-[4-(4-Acetamido-N-acetyl-phenylsulfoximino)-butoxy]-4,4-diäthyl-4H-3,1-benzoxazin-2-on

Hergestellt analog Beispiel 143 aus 6-[4-(4-Acetamidophenylsulfoximino)-butoxy]-4,4-diäthyl-4H-3,1-benzoxazin-2-on und Acetanhydrid.
Schmelzpunkt : 146-149 °C,
Ausbeute : 88,6 % der Theorie.

Beispiel 292

6-[4-(3,4-Dimethoxy-N-acetyl-phenylsulfoximino)-butoxy]-4,4-diäthyl-4H-3,1-benzoxazin-2-on

Hergestellt analog Beispiel 143 aus 6-[4-(3,4-Dimethoxy-phenylsulfoximino)-butoxy]-4,4-diäthyl-4H-3,1-benzoxazin-2-on und Acetanhydrid.
Harz, RF-Wert : 0,5 (Kieselgelplatte : Chloroform/Äthanol = 9 : 1),
Ausbeute : 87,2 % der Theorie.
$C_{26}H_{34}N_2O_7S$ (518,64)
Ber. : C 60,21 H 6,61 N 5,40 S 6,18
Gef. : C 59,95 H 6,58 N 5,19 S 6,31

## Beispiel 293

6-[4-(3,4-Dichlor-N-acetyl-phenylsulfoximino)-butoxy]-4,4-diäthyl-4H-3,1-benzoxazin-2-on

Hergestellt analog Beispiel 143 aus 6-[4-(3,4-Dichlor-phenylsulfoximino)-butoxy]-4,4-diäthyl-4H-3,1-benzoxazin-2-on und Acetanhydrid.
Schmelzpunkt : 164-166 °C,
Ausbeute : 86,6 % der Theorie.

## Beispiel 294

6-[4-(N-Acetyl-phenylsulfoximino)-butoxy]-4,4-diäthyl-4H-3,1-benzoxazin-2-on

Hergestellt analog Beispiel 143 aus 6-(4-Phenylsulfoximino-butoxy)-4,4-diäthyl-4H-3,1-benzoxazin-2-on und Acetanhydrid.
Schmelzpunkt : 120-122 °C,
Ausbeute : 89,4 % der Theorie.

## Beispiel 295

6-[4-(3,4-Dichlor-phenylsulfoximino)-butoxy]-4,4-dimethyl-4H-3,1-benzoxazin-2-on

Hergestellt analog Beispiel 4 aus 6-Hydroxy-4,4-dimethyl-4H-3,1-benzoxazin-2-on und 4-(3,4-Dichlor-phenylsulfoximino)-butylbromid-mesitylensulfonat (Schmelzpunkt : 170-173 °C).
Schmelzpunkt : 164-166 °C,
Ausbeute : 35,4 % der Theorie.

## Beispiel 296

6-[4-(3,4-Dichlor-N-acetyl-phenylsulfoximino)-butoxy]-4,4-dimethyl-4H-3,1-benzoxazin-2-on

Hergestellt analog Beispiel 4 aus 6-Hydroxy-4,4-dimethyl-4H-3,1-benzoxazin-2-on und 4-(3,4-Dichlor-N-acetyl-phenylsulfoximino)-butylbromid (Schmelzpunkt : 78-79 °C).
Schmelzpunkt : 156-158 °C,
Ausbeute : 66,5 % der Theorie.

## Beispiel 297

6-(4-Phenylsulfinyl-butoxy)-4H-3,1-benzoxazin-2-on

4,2 g (0,013 3 Mol) 2-Amino-5-(4-phenylsulfinyl-butoxy)-benzylalkohol (hergestellt aus 2-Nitro-5-(4-phenylsulfinyl-butoxy)-benzylalkohol durch katalytische Hydrierung) werden in 150 ml Chloroform gelöst und mit 6,9 g (0,05 Mol) Kaliumcarbonat versetzt. Zu dieser Mischung tropft man langsam unter Rühren 10 ml einer 20 %igen Lösung von Phosgen in Toluol. Nach 2-stündigem Nachrühren wird die Lösung mit Wasser gewaschen, mit Natriumsulfat getrocknet und die Lösungsmittel im Vakuum abdestilliert. Den Rückstand kristallisiert man aus Essigester/Cyclohexan um.
Schmelzpunkt : 115-116 °C,
Ausbeute : 2,1 g (45,8 % der Theorie).

## Beispiel 298

7-(4-Phenylsulfoximino-butoxy)-4,4-dimethyl-4H-3,1-benzoxazin-2-on

1,56 g (2,78 mMol) Kaliumhydroxid werden in 10 ml Methanol gelöst und unter Rühren bei Raumtemperatur mit 0,8 g (1,86 mMol) 7-[4-(N-Acetyl-phenylsulfoximino)-butoxy]-4,4-dimethyl-4H-3,1-benzoxazin-2-on versetzt. Die entstehende klare Lösung läßt man über Nacht stehen, versetzt dann mit

Wasser, saugt den Niederschlag ab, wäscht ihn mit Wasser und mit Diisopropyläther und trocknet bei 60 °C.

Schmelzpunkt : 130-132 °C,

Ausbeute : 0,65 g (90 % der Theorie).

Beispiel 299

7,8-Dibrom-6-[4-(4-methyl-phenylmercapto)-butoxy]-4,4-dimethyl-4H-3,1-benzoxazin-2-on

Hergestellt analog Beispiel 4 aus 7,8-Dibrom-6-hydroxy-4,4-dimethyl-4H-3,1-benzoxazin-2-on und 4-(4-Methyl-phenylmercapto)-butylchlorid.

Schmelzpunkt : 114-115 °C,

Ausbeute : 45,4 % der Theorie.

Beispiel 300

6-(4-Phenylsulfinyl-butoxy)-4,4,7-trimethyl-4H-3,1-benzoxazin-2-on

Hergestellt analog Beispiel 4 aus 6-Hydroxy-4,4,7-trimethyl-4H-3,1-benzoxazin-2-on und 4-Phenylsulfinyl-butylbromid.

Schmelzpunkt : 124-125 °C,

Ausbeute : 51,6 % der Theorie.

Beispiel 301

6-[4-(3,4-Dichlor-phenylsulfinyl)-butoxy]-4,4,7-trimethyl-4H-3,1-benzoxazin-2-on

Hergestellt analog Beispiel 4 aus 6-Hydroxy-4,4,7-trimethyl-4H-3,1-benzoxazin-2-on und 4-(3,4-Dichlor-phenylsulfinyl)-butylbromid.

Schmelzpunkt : 151-152 °C,

Ausbeute : 42,3 % der Theorie.

Beispiel 302

6-[4-(4-Methyl-phenylmercapto)-butoxy]-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und 4-Methyl-thiophenol.

Schmelzpunkt : 125-126 °C,

Ausbeute : 78,4 % der Theorie.

Beispiel 303

6-[4-(3,4-Dichlor-phenylmercapto)-butoxy]-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und 3,4-Dichlor-thiophenol.

Schmelzpunkt : 136-137 °C,

Ausbeute : 68,8 % der Theorie.

Beispiel 304

6-[4-(4-Acetamido-phenylmercapto)-butoxy]-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und 4-Acetamido-thiophenol.

Schmelzpunkt : 167-168 °C,

Ausbeute : 64,2 % der Theorie.

Beispiel 305

6-[4-(2-Pyridylmercapto)-butoxy]-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und 2-Mercapto-pyridin.

Schmelzpunkt : 144-145 °C,
Ausbeute : 64,0 % der Theorie.

Beispiel 306

6-[4-(4-Chlor-phenylmercapto)-butoxy]-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und 4-Chlor-thiophenol.
Schmelzpunkt : 141-142 °C,
Ausbeute : 63,5 % der Theorie.

Beispiel 307

6-(4-Phenylmercapto-butoxy)-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und Thiophenol.
Schmelzpunkt : 125-126 °C,
Ausbeute : 83,1 % der Theorie.

Beispiel 308

6-[4-(3,4-Dimethyl-phenylmercapto)-butoxy]-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und 3,4-Dimethyl-thiophenol.
Schmelzpunkt : 146-147 °C,
Ausbeute : 78,1 % der Theorie.

Beispiel 309

6-[4-(3,4-Dimethoxy-phenylmercapto)-butoxy]-4,4,8-trimethyl-4H-3,1-benzoxazin-2-on

Hergestellt analog Beispiel 1 aus 6-(4-Chlorbutoxy)-4,4,8-trimethyl-4H-3,1-benzoxazin-2-on und 3,4-Dimethoxy-thiophenol.
Schmelzpunkt : 130-131 °C,
Ausbeute : 73,3 % der Theorie.

Beispiel 310

6-[4-(4-Acetamido-phenylsulfinyl)-butoxy]-7-nitro-4,4-dimethyl-4H-3,1-benzoxazin-2-on

Hergestellt analog Beispiel 2 aus 6-[4-(4-Acetamido-phenylmercapto)-butoxy]-7-nitro-4,4-dimethyl-4H-3,1-benzoxazin-2-on und Wasserstoffperoxid.
Schmelzpunkt : 240-241 °C,
Ausbeute : 72,6 % der Theorie.

Beispiel 311

6-[4-(4-Chlor-phenylsulfinyl)-butoxy]-7-nitro-4,4-dimethyl-4H-3,1-benzoxazin-2-on

Hergestellt analog Beispiel 2 aus 6-[4-(4-Chlor-phenylmercapto)-butoxy]-7-nitro-4,4-dimethyl-4H-3,1-benzoxazin-2-on und Wasserstoffperoxid.
Schmelzpunkt : 159-160 °C,
Ausbeute : 83,4 % der Theorie.

Beispiel 312

6-[4-(2-Pyridylsulfinyl)-butoxy]-7-nitro-4,4-dimethyl-4H-3,1-benzoxazin-2-on

Hergestellt analog Beispiel 2 aus 6-[4-(2-Pyridylmercapto)-butoxy]-7-nitro-4,4-dimethyl-4H-3,1-benzoxazin-2-on und Wasserstoffperoxid.
Schmelzpunkt : 156-157 °C,
Ausbeute : 69,1 % der Theorie.

Beispiel 313

6-[4-(4-Methyl-phenylsulfinyl)-butoxy]-7-nitro-4,4-dimethyl-4H-3,1-benzoxazin-2-on

Hergestellt analog Beispiel 2 aus 6-[4-(4-Methyl-phenylmercapto)-butoxy]-7-nitro-4,4-dimethyl-4H-3,1-benzoxazin-2-on und Wasserstoffperoxid.
Schmelzpunkt : 176-177 °C,
Ausbeute : 76,1 % der Theorie.

Beispiel 314

6-[4-(3,4-Dimethoxy-phenylsulfinyl)-butoxy]-7-nitro-4,4-dimethyl-4H-3,1-benzoxazin-2-on

Hergestellt analog Beispiel 2 aus 6-[4-(3,4-Dimethoxy-phenylmercapto)-butoxy]-7-nitro-4,4-dimethyl-4H-3,1-benzoxazin-2-on und Wasserstoffperoxid.
Schmelzpunkt : 190-192 °C,
Ausbeute : 84,3 % der Theorie.

Beispiel 315

6-[4-(3,4-Dimethyl-phenylsulfinyl)-butoxy]-7-nitro-4,4-dimethyl-4H-3,1-benzoxazin-2-on

Hergestellt analog Beispiel 2 aus 6-[4-(3,4-Dimethyl-phenylmercapto)-butoxy]-7-nitro-4,4-dimethyl-4H-3,1-benzoxazin-2-on und Wasserstoffperoxid.
Schmelzpunkt : 146-147 °C,
Ausbeute : 68,2 % der Theorie.

Beispiel 316

8-Chlor-6-[4-(4-methyl-phenylsulfinyl-butoxy]-4,4-dimethyl-4H-3,1-benzoxazin-2-on

Hergestellt analog Beispiel 2 aus 8-Chlor-6-[4-(4-methyl-phenylmercapto)-butoxy]-4,4-dimethyl-4H-3,1-benzoxazin-2-on und Wasserstoffperoxid.
Schmelzpunkt : 113-114 °C,
Ausbeute : 90,3 % der Theorie.

Beispiel 317

7-Chlor-6-[4-(4-methyl-phenylsulfinyl)-butoxy]-4,4-dimethyl-4H-3,1-benzoxazin-2-on

Hergestellt analog Beispiel 2 aus 7-Chlor-6-[4-(4-methyl-phenylmercapto)-butoxy]-4,4-dimethyl-4H-3,1-benzoxazin-2-on und Wasserstoffperoxid.
Schmelzpunkt : 143-144 °C,
Ausbeute : 85,3 % der Theorie.

Beispiel 318

6-[4-(4-Chlor-phenylsulfinyl)-butoxy]-8-chlor-4,4-dimethyl-4H-3,1-benzoxazin-2-on

Hergestellt analog Beispiel 2 aus 8-Chlor-6-[4-(4-chlor-phenylmercapto)-butoxy]-4,4-dimethyl-4H-3,1-benzoxazin-2-on und Wasserstoffperoxid.
Schmelzpunkt : 83-85 °C,
Ausbeute : 78,1 % der Theorie.

Beispiel 319

6-[4-(4-Chlor-phenylsulfinyl)-butoxy]-7-chlor-4,4-dimethyl-4H-3,1-benzoxazin-2-on

Hergestellt analog Beispiel 2 aus 7-Chlor-6-[4-(4-chlor-phenylmercapto)-butoxy]-4,4-dimethyl-4H-3,1-benzoxazin-2-on und Wasserstoffperoxid.
Schmelzpunkt : 162-163 °C,
Ausbeute : 86 % der Theorie.

Beispiel 320

7-Brom-6-[4-(4-methyl-phenylsulfinyl)-butoxy]-4,4-dimethyl-4H-3,1-benzoxazin-2-on

Hergestellt analog Beispiel 2 aus 7-Brom-6-[4-(4-methylphenylmercapto)-butoxy]-4,4-dimethyl-4H-3,1-benzoxazin-2-on und Wasserstoffperoxid.
Schmelzpunkt : 143-145 °C,
Ausbeute : 78,5 % der Theorie.

Beispiel 321

8-Brom-6-[4-methyl-phenylsulfinyl)-butoxy]-4,4-dimethyl-4H-3,1-benzoxazin-2-on

Hergestellt analog Beispiel 2 aus 8-Brom-6-[4-(4-methylphenylmercapto)-butoxy]-4,4-dimethyl-4H-3,1-benzoxazin-2-on und Wasserstoffperoxid.
Schmelzpunkt : 123-124 °C,
Ausbeute : 86,4 % der Theorie.

Beispiel 322

6-[4-(4-Methyl-phenylsulfinyl)-butoxy]-4,4,7-trimethyl-4H-3,1-benzoxazin-2-on

Hergestellt analog Beispiel 2 aus 6-[4-(4-Methyl-phenylmercapto)-butoxy]-4,4,7-trimethyl-4H-3,1-benzoxazin-2-on und Wasserstoffperoxid.
Schmelzpunkt : 152-153 °C,
Ausbeute : 87,2 % der Theorie.

Beispiel 323

7,8-Dibrom-6-[4-(4-methyl-phenylsulfinyl)-butoxy]-4,4-dimethyl-4H-3,1-benzoxazin-2-on

Hergestellt analog Beispiel 1 aus 7,8-Dibrom-6-[4-(4-methyl-phenylmercapto)-butoxy]-4,4-dimethyl-4H-3,1-benzoxazin-2-on und Wasserstoffperoxid.
Schmelzpunkt : 138-139 °C,
Ausbeute : 70,0 % der Theorie.

Beispiel 324

6-[4-(3,4-Dichlor-phenylsulfinyl)-butoxy]-4,4,8-trimethyl-4H-3,1-benzoxazin-2-on

Hergestellt analog Beispiel 2 aus 6-[4-(3,4-Dichlor-phenylmercapto)-butoxy]-4,4,8-trimethyl-4H-3,1-benzoxazin-2-on und Wasserstoffperoxid.
Schmelzpunkt : 158 °C,
Ausbeute : 68 % der Theorie.

Beispiel 325

6-[4-(4-Methyl-phenylsulfinyl)-butoxy]-4,4,8-trimethyl-4H-3,1-benzoxazin-2-on

Hergestellt analog Beispiel 2 aus 6-[4-(4-Methyl-phenylmercapto)-butoxy]-4,4,8-trimethyl-4H-3,1-benzoxazin-2-on und Wasserstoffperoxid.
Schmelzpunkt : 137-138 °C,
Ausbeute : 40 % der Theorie.

Beispiel 326

6-[4-(4-Chlor-phenylsulfinyl)-butoxy]-4,4,8-trimethyl-4H-3,1-benzoxazin-2-on

Hergestellt analog Beispiel 2 aus 6-[4-(4-Chlor-phenylmercapto)-butoxy]-4,4,8-trimethyl-4H-3,1-benzoxazin-2-on und Wasserstoffperoxid.
Schmelzpunkt : 163-164 °C,
Ausbeute : 66,2 % der Theorie.

Beispiel 327

6-[4-(3,4-Dimethoxy-phenylsulfinyl)-butoxy]-4,4,8-trimethyl-4H-3,1-benzoxazin-2-on

Hergestellt analog Beispiel 2 aus 6-[4-(3,4-Dimethoxy-phenylmercapto)-butoxy]-4,4,8-trimethyl-4H-3,1-benzoxazin-2-on und Wasserstoffperoxid.
Schmelzpunkt : 142-143 °C,
Ausbeute : 70,5 % der Theorie.

Beispiel 328

6-[4-(3,4-Dimethyl-phenylsulfinyl)-butoxy]-4,4,8-trimethyl-4H-3,1-benzoxazin-2-on

Hergestellt analog Beispiel 2 aus 6-[4-(3,4-Dimethyl-phenylmercapto)-butoxy]-4,4,8-trimethyl-4H-3,1-benzoxazin-2-on und Wasserstoffperoxid.
Schmelzpunkt : 113-114 °C,
Ausbeute : 72,9 % der Theorie.

Beispiel 329

6-(4-Phenylsulfinyl-butoxy)-4,4,8-trimethyl-4H-3,1-benzoxazin-2-on

Hergestellt analog Beispiel 2 aus 6-(4-Phenylmercapto-butoxy)-4,4,8-trimethyl-4H-3,1-benzoxazin-2-on und Wasserstoffperoxid.
Schmelzpunkt : 143-144 °C,
Ausbeute : 77,6 % der Theorie.

Beispiel 330

6-[4-(4-Acetamido-phenylsulfinyl)-butoxy]-4,4,8-trimethyl-4H-3,1-benzoxazin-2-on

Hergestellt analog Beispiel 2 aus 6-[4-(4-Acetamido-phenylmercapto)-butoxy]-4,4,8-trimethyl-4H-3,1-benzoxazin-2-on und Wasserstoffperoxid.
Schmelzpunkt : 212-213 °C,
Ausbeute : 57,9 % der Theorie.

Beispiel 331

6-[4-(2-Pyridylsulfinyl)-butoxy]-4,4,8-trimethyl-4H-3,1-benzoxazin-2-on

Hergestellt analog Beispiel 2 aus 6-[4-(2-Pyridylmercapto)-butoxy]-4,4,8-trimethyl-4H-3,1-benzoxazin-2-on und Wasserstoffperoxid.
Schmelzpunkt : 163-164 °C,
Ausbeute : 65,8 % der Theorie.

Beispiel 332

6-[4-(3,4-Dimethoxy-phenylsulfoximino)-butoxy]-4,4,8-trimethyl-4H-3,1-benzoxazin-2-on

Hergestellt analog Beispiel 6 aus 6-[4-(3,4-Dimethoxy-phenylsulfinyl)-butoxy]-4,4,8-trimethyl-4H-3,1-benzoxazin-2-on und O-Mesitylensulfonyl-acethydroxamsäure-äthylester.
Schmelzpunkt : 175-176 °C,
Ausbeute : 58,7 % der Theorie.

Beispiel 333

6-[4-(3,4-Dimethyl-phenylsulfoximino)-butoxy]-4,4,8-trimethyl-4H-3,1-benzoxazin-2-on

Hergestellt analog Beispiel 6 aus 6-[4-(3,4-Dimethyl-phenylsulfinyl)-butoxy]-4,4,8-trimethyl-4H-3,1-benzoxazin-2-on und O-Mesitylensulfonyl-acethydroxamsäure-äthylester.
Schmelzpunkt : 174-175 °C,
Ausbeute : 61,8 % der Theorie.

Beispiel 334

6-(4-Phenylsulfoximino-butoxy)-4,4,8-trimethyl-4H-3,1-benzoxazin-2-on

Hergestellt analog Beispiel 6 aus 6-(4-Phenylsulfinyl-butoxy)-4,4,8-trimethyl-4H-3,1-benzoxazin-2-on

und O-Mesitylensulfonyl-acethydroxamsäure-äthylester.
Schmelzpunkt : 147-148 °C,
Ausbeute : 66,8 % der Theorie.

Beispiel 335

6-[4-(3,4-Dichlor-phenylsulfoximino)-butoxy]-4,4,8-trimethyl-4H-3,1-benzoxazin-2-on

Hergestellt analog Beispiel 6 aus 6-[4-(3,4-Dichlor-phenylsulfinyl)-butoxy]-4,4,8-trimethyl-4H-3,1-benzoxazin-2-on und O-Mesitylensulfonyl-acethydroxamsäure-äthylester.
Schmelzpunkt : 116-117 °C,
Ausbeute : 50,0 % der Theorie.

Beispiel 336

6-[4-(4-Chlor-phenylsulfoximino)-butoxy]-4,4,8-trimethyl-4H-3,1-benzoxazin-2-on

Hergestellt analog Beispiel 6 aus 6-[4-(4-Chlor-phenylsulfinyl)-butoxy]-4,4,8-trimethyl-4H-3,1-benzoxazin-2-on und O-Mesitylensulfonyl-acethydroxamsäure-äthylester.
Schmelzpunkt : 167-168 °C,
Ausbeute : 72,8 % der Theorie.

Beispiel 337

6-[4-(4-Methyl-phenylsulfoximino)-butoxy]-4,4,8-trimethyl-4H-3,1-benzoxazin-2-on

Hergestellt analog Beispiel 6 aus 6-[4-(4-Methyl-phenylsulfinyl)-butoxy]-4,4,8-trimethyl-4H-3,1-benzoxazin-2-on und O-Mesitylensulfonyl-acethydroxamsäure-äthylester.
Schmelzpunkt : 183-184 °C,
Ausbeute : 71,5 % der Theorie.

Beispiel 338

6-[4-(4-Acetamido-phenylsulfoximino)-butoxy]-4,4,8-trimethyl-4H-3,1-benzoxazin-2-on

Hergestellt analog Beispiel 6 aus 6-[4-(4-Acetamido-phenylsulfinyl)-butoxy]-4,4,8-trimethyl-4H-3,1-benzoxazin-2-on und O-Mesitylensulfonyl-acethydroxamsäure-äthylester.
Schmelzpunkt : 168-169 °C,
Ausbeute : 66,1 % der Theorie.

Beispiel 339

6-[4-(4-Acetamido-phenylsulfoximino)-butoxy]-4,4-dimethyl-7-nitro-4H-3,1-benzoxazin-2-on

Hergestellt analog Beispiel 6 aus 6-[4-(4-Acetamido-phenylsylfinyl)-butoxy]-4,4-dimethyl-7-nitro-4H-3,1-benzoxazin-2-on und O-Mesitylensulfonyl-acethydroxamsäure-äthylester.
Schmelzpunkt : 209-211 °C,
Ausbeute : 55,4 % der Theorie.

Beispiel 340

6-[4-(4-Chlor-phenylsulfoximino)-butoxy]-4,4-dimethyl-7-nitro-4H-3,1-benzoxazin-2-on

Hergestellt analog Beispiel 6 aus 6-[4-(4-Chlor-phenylsulfinyl)-butoxy]-4,4-dimethyl-7-nitro-4H-3,1-benzoxazin-2-on und O-Mesitylensulfonyl-acethydroxamsäure-äthylester.
Schmelzpunkt : 116-118 °C,
Ausbeute : 88,3 % der Theorie.

Beispiel 341

6-[4-(4-Methyl-phenylsulfoximino)-butoxy]-4,4-dimethyl-7-nitro-4H-3,1-benzoxazin-2-on

Hergestellt analog Beispiel 6 aus 6-[4-(4-Methyl-phenylsulfinyl)-butoxy]-4,4-dimethyl-7-nitro-4H-3,1-benzoxazin-2-on und O-Mesitylensulfonyl-acethydroxamsäure-äthylester.
Schmelzpunkt : 112-114 °C,

Ausbeute : 86,2 % der Theorie.

Beispiel 342

6-[4-(3,4-Dimethoxy-phenylsulfoximino)-butoxy]-4,4-dimethyl-7-nitro-4H-3,1-benzoxazin-2-on

Hergestellt analog Beispiel 6 aus 6-[4-(3,4-Dimethoxy-phenylsulfinyl)-butoxy]-4,4-dimethyl-7-nitro-4H-3,1-benzoxazin-2-on und O-Mesitylensulfonyl-acethydroxamsäure-äthylester.
Schmelzpunkt : 147-149 °C,
Ausbeute : 41,4 % der Theorie.

Beispiel 343

6-[4-(3,4-Dimethyl-phenylsulfoximino)-butoxy]-4,4-dimethyl-7-nitro-4H-3,1-benzoxazin-2-on

Hergestellt analog Beispiel 6 aus 6-[4-(3,4-Dimethyl-phenylsulfinyl)-butoxy]-4,4-dimethyl-7-nitro-4H-3,1-benzoxazin-2-on und O-Mesitylensulfonyl-acethydroxamsäure-äthylester.
Schmelzpunkt : 145-146 °C,
Ausbeute : 67 % der Theorie.

Beispiel 344

8-Chlor-6-[4-(3,4-dichlor-phenylsulfoximino)-butoxy]-4,4-dimethyl-4H-3,1-benzoxazin-2-on

Hergestellt analog Beispiel 6 aus 8-Chlor-6-[4-(3,4-dichlorphenylsulfinyl)-butoxy]-4,4-dimethyl-4H-3,1-benzoxazin-2-on und O-Mesitylensulfonyl-acethydroxamsäure-äthylester.
Schmelzpunkt : 135-136 °C,
Ausbeute : 74,3 % der Theorie.

Beispiel 345

7-Chlor-6-[4-(3,4-dichlor-phenylsulfoximino)-butoxy]-4,4-dimethyl-4H-3,1-benzoxazin-2-on

Hergestellt analog Beispiel 6 aus 7-Chlor-6-[4-(3,4-dichlorphenylsulfinyl)-butoxy]-4,4-dimethyl-4H-3,1-benzoxazin-2-on und O-Mesitylensulfonyl-acethydroxamsäure-äthylester.
Schmelzpunkt : ab 87 °C,
Ausbeute : 58,3 % der Theorie.

Beispiel 346

8-Chlor-6-[4-(4-methyl-phenylsulfoximino)-butoxy]-4,4-dimethyl-4H-3,1-benzoxazin-2-on

Hergestellt analog Beispiel 6 aus 8-Chlor-6-[4-(4-methyl-phenylsulfinyl)-butoxy]-4,4-dimethyl-4H-3,1-benzoxazin-2-on und O-Mesitylensulfonyl-acethydroxamsäure-äthylester.
Schmelzpunkt : 132-133 °C
Ausbeute : 55,1 % der Theorie.

Beispiel 347

7-Chlor-6-[4-(4-methyl-phenylsulfoximino)-butoxy]-4,4-dimethyl-4H-3,1-benzoxazin-2-on

Hergestellt analog Beispiel 6 aus 7-Chlor-6-[4-(4-methyl-phenylsulfinyl)-butoxy]-4,4-dimethyl-4H-3,1-benzoxazin-2-on und O-Mesitylensulfonyl-acethydroxamsäure-äthylester.
Schmelzpunkt : 115-116 °C,
Ausbeute : 55,1 % der Theorie.

Beispiel 348

8-Chlor-6-[4-(4-chlor-phenylsulfoximino)-butoxy]-4,4-dimethyl-4H-3,1-benzoxazin-2-on

Hergestellt analog Beispiel 6 aus 8-Chlor-6-[4-(4-chlor-phenylsulfinyl)-butoxy]-4,4-dimethyl-4H-3,1-benzoxazin-2-on und O-Mesitylensulfonyl-acethydroxamsäure-äthylester.
Schmelzpunkt : 162-163 °C,
Ausbeute : 57 % der Theorie.

Beispiel 349

7-Chlor-6-[4-(4-chlor-phenylsulfoximino)-butoxy]-4,4-dimethyl-4H-3,1-benzoxazin-2-on

Hergestellt analog Beispiel 6 aus 7-Chlor-6-[4-(4-chlor-phenylsulfinyl)-butoxy]-4,4-dimethyl-4H-3,1-benzoxazin-2-on und O-Mesitylensulfonyl-acethydroxamsäure-äthylester.
Schmelzpunkt : 132-133 °C,
Ausbeute : 65,8 % der Theorie.

Beispiel 350

6-[4-(3,4-Dichlor-phenylsulfoximino)-butoxy]-4,4,7-trimethyl-4H-3,1-benzoxazin-2-on

Hergestellt analog Beispiel 6 aus 6-[4-(3,4-Dichlor-phenylsulfinyl)-butoxy]-4,4,7-trimethyl-4H-3,1-benzoxazin-2-on und O-Mesitylensulfonyl-acethydroxamsäure-äthylester.
Schmelzpunkt : 135-137 °C,
Ausbeute : 63,6 % der Theorie.

Beispiel 351

6-(4-Phenylsulfoximino-butoxy)-4,4,7-trimethyl-4H-3,1-benzoxazin-2-on

Hergestellt analog Beispiel 6 aus 6-(4-Phenylsulfinyl-butoxy)-4,4,7-trimethyl-4H-3,1-benzoxazin-2-on und O-Mesitylensulfonyl-acethydroxamsäure-äthylester.
Schmelzpunkt : 123-124 °C,
Ausbeute : 77 % der Theorie.

Beispiel 352

6-[4-(4-Methyl-phenylsulfoximino)-butoxy]-4,4,7-trimethyl-4H-3,1-benzoxazin-2-on

Hergestellt analog Beispiel 6 aus 6-[4-(4-Methyl-phenylsulfinyl)-butoxy]-4,4,7-trimethyl-4H-3,1-benzoxazin-2-on und O-Mesitylensulfonyl-acethydroxamsäure-äthylester.
Schmelzpunkt : 153-154 °C,
Ausbeute : 57,6 % der Theorie.

Beispiel 353

6-[4-(4-Acetamido-N-acetyl-phenylsulfoximino)-butoxy]-7-nitro-4,4-dimethyl-4H-3,1-benzoxazin-2-on

Hergestellt analog Beispiel 143 aus 6-[4-(4-Acetamido-phenylsulfoximino)-butoxy]-7-nitro-4,4-dimethyl-4H-3,1-benzoxazin-2-on und Acetanhydrid.
Schmelzpunkt : 250-252 °C,
Ausbeute : 92 % der Theorie.

Beispiel 354

6-[4-(4-Chlor-N-acetyl-phenylsulfoximino)-butoxy]-7-nitro-4,4-dimethyl-4H-3,1-benzoxazin-2-on

Hergestellt analog Beispiel 143 aus 6-[4-(4-Chlor-phenylsulfoximino)-butoxy]-7-nitro-4,4-dimethyl-4H-3,1-benzoxazin-2-on und Acetanhydrid.
Schmelzpunkt : 211-213 °C,
Ausbeute : 82,4 % der Theorie.

Beispiel 355

6-[4-(4-Methyl-N-acetyl-phenylsulfoximino)-butoxy]-7-nitro-4,4-dimethyl-4H-3,1-benzoxazin-2-on

Hergestellt analog Beispiel 143 aus 6-[4-(4-Methyl-phenylsulfoximino)-butoxy-7-nitro-4,4-dimethyl-4H-3,1-benzoxazin-2-on und Acetanhydrid.
Schmelzpunkt : 214-216 °C,
Ausbeute : 85,8 % der Theorie.

Beispiel 356

6-[4-(3,4-Dimethoxy-N-acetyl-phenylsulfoximino)-butoxy]-7-nitro-4,4-dimethyl-4H-3,1-benzoxazin-2-on

Hergestellt analog Beispiel 143 aus 6-[4-(3,4-Dimethoxy-phenylsulfoximino)-butoxy]-7-nitro-4,4-dimethyl-4H-3,1-benzoxazin-2-on und Acetanhydrid.
Schmelzpunkt : 120-122 °C,
Ausbeute : 82,2 % der Theorie.

Beispiel 357

6-[4-(3,4-Dimethoxy-N-acetyl-phenylsulfoximino)-butoxy]-4,4,8-trimethyl-4H-3,1-benzoxazin-2-on

Hergestellt analog Beispiel 143 aus 6-[4-(3,4-Dimethoxy-phenylsulfoximino)-butoxy]-4,4,8-trimethyl-4H-3,1-benzoxazin-2-on und Acetanhydrid.
Schmelzpunkt : 123-124 °C,
Ausbeute : 76,5 % der Theorie.

Beispiel 358

6-[4-(3,4-Dimethyl-N-acetyl-phenylsulfoximino)-butoxy]-4,4,8-trimethyl-4H-3,1-benzoxazin-2-on

Hergestellt analog Beispiel 143 aus 6-[4-(3,4-Dimethyl-phenylsulfoximino)-butoxy]-4,4,8-trimethyl-4H-3,1-benzoxazin-2-on und Acetanhydrid.
Schmelzpunkt : 146-147 °C,
Ausbeute : 83,1 % der Theorie.

Beispiel 359

6-[4-(N-Acetyl-phenylsulfoximino)-butoxy]-4,4,8-trimethyl-4H-3,1-benzoxazin-2-on

Hergestellt analog Beispiel 143 aus 6-(4-Phenylsulfoximinobutoxy)-4,4,8-trimethyl-4H-3,1-benzoxazin-2-on und Acetanhydrid.
Schmelzpunkt : 99-100 °C,
Ausbeute : 71,0 % der Theorie.

Beispiel 360

6-[4-(3,4-Dichlor-N-acetyl-phenylsulfoximino)-butoxy]-4,4,8-trimethyl-4H-3,1-benzoxazin-2-on

Hergestellt analog Beispiel 143 aus 6-[4-(3,4-Dichlor-phenylsulfoximino)-butoxy]-4,4,8-trimethyl-4H-3,1-benzoxazin-2-on und Acetanhydrid.
Schmelzpunkt : 140-141 °C,
Ausbeute : 76,9 % der Theorie.

Beispiel 361

6-[4-(4-Chlor-N-acetyl-phenylsulfoximino)-butoxy]-4,4,8-trimethyl-4H-3,1-benzoxazin-2-on

Hergestellt analog Beispiel 143 aus 6-[4-(4-Chlor-phenylsulfoximino)-butoxy]-4,4,8-trimethyl-4H-3,1-benzoxazin-2-on und Acetanhydrid.
Schmelzpunkt : 178-179 °C,
Ausbeute : 79,9 % der Theorie.

Beispiel 362

8-Chlor-6-[4-(3,4-dichlor-phenylsulfinyl)-butoxy]-4,4-dimethyl-4H-3,1-benzoxazin-2-on

Hergestellt analog Beispiel 4 aus 8-Chlor-6-hydroxy-4,4-dimethyl-4H-3,1-benzoxazin-2-on und 4-(3,4-Dichlor-phenylsulfinyl)-butylbromid.
Schmelzpunkt : 121-122 °C,
Ausbeute : 62,0 % der Theorie.

Beispiel 363

7-Chlor-6-[4-(3,4-dichlor-phenylsulfinyl)-butoxy]-4,4-dimethyl-4H-3,1-benzoxazin-2-on

Hergestellt analog Beispiel 4 aus 7-Chlor-6-hydroxy-4,4-dimethyl-4H-3,1-benzoxazin-2-on und 4-(3,4-Dichlor-phenylsulfinyl)-butylbromid.
Schmelzpunkt ; 125-127 °C,
Ausbeute : 41,5 % der Theorie.

Beispiel 364

8-Chlor-6-[4-(4-methyl-phenylmercapto)-butoxy]-4,4-dimethyl-4H-3,1-benzoxazin-2-on

Hergestellt analog Beispiel 4 aus 8-Chlor-6-hydroxy-4,4-dimethyl-4H-3,1-benzoxazin-2-on und 4-(4-Methyl-phenylmercapto)-butylchlorid.
Schmelzpunkt : 111-112 °C,
Ausbeute : 56,0 % der Theorie.

Beispiel 365

8-Chlor-6-(4-phenylsulfinyl-butoxy)-4,4-dimethyl-4H-3,1-benzoxazin-2-on

Hergestellt analog Beispiel 4 aus 8-Chlor-6-hydroxy-4,4-dimethyl-4H-3,1-benzoxazin-2-on und 4-Phenylsulfinyl-butylbromid.
Schmelzpunkt : 154-155 °C,
Ausbeute : 39,3 % der Theorie.

Beispiel 366

7-Chlor-6-(4-phenylsulfinyl-butoxy)-4,4-dimethyl-4H-3,1-benzoxazin-2-on

Hergestellt analog Beispiel 4 aus 7-Chlor-6-hydroxy-4,4-dimethyl-4H-3,1-benzoxazin-2-on und 4-Phenylsulfinyl-butylbromid.
Schmelzpunkt : 124-125 °C,
Ausbeute : 31,5 % der Theorie.

Beispiel 367

7-Chlor-6-[4-(4-methyl-phenylmercapto)-butoxy]-4,4-dimethyl-4H-3,1-benzoxazin-2-on

Hergestellt analog Beispiel 4 aus 7-Chlor-6-hydroxy-4,4-dimethyl-4H-3,1-benzoxazin-2-on und 4-(4-Methyl-phenylmercapto)-butylchlorid.
Schmelzpunkt : 118-119 °C,
Ausbeute : 52,6 % der Theorie.

Beispiel 368

7-Brom-6-[4-(3,4-dichlor-phenylsulfinyl)-butoxy]-4,4-dimethyl-4H-3,1-benzoxazin-2-on

Hergestellt analog Beispiel 4 aus 7-Brom-6-hydroxy-4,4-dimethyl-4H-3,1-benzoxazin-2-on und 4-(3,4-Dichlor-phenylsulfinyl)-butylbromid.
Schmelzpunkt : 136-138 °C,
Ausbeute : 75,0 % der Theorie.

Beispiel 369

7-Brom-6-(4-phenylsulfinyl-butoxy)-4,4-dimethyl-4H-3,1-benzoxazin-2-on

Hergestellt analog Beispiel 4 aus 7-Brom-6-hydroxy-4,4-dimethyl-4H-3,1-benzoxazin-2-on und 4-Phenylsulfinyl-butylbromid.
Schmelzpunkt : 119-120 °C,
Ausbeute : 53,1 % der Theorie.

Beispiel 370

8-Chlor-6-[4-(4-chlor-phenylmercapto)-butoxy]-4,4-dimethyl-4H-3,1-benzoxazin-2-on

Hergestellt analog Beispiel 4 aus 8-Chlor-6-hydroxy-4,4-dimethyl-4H-3,1-benzoxazin-2-on und 4-(4-Chlor-phenylmercapto)-butylchlorid.

Schmelzpunkt : 138-139 °C,
Ausbeute : 56,7 % der Theorie.

Beispiel 371

8-Brom-6-[4-(3,4-dichlor-phenylsulfinyl)-butoxy]-4,4-dimethyl-4H-3,1-benzoxazin-2-on

Hergestellt analog Beispiel 4 aus 8-Brom-6-hydroxy-4,4-dimethyl-4H-3,1-benzoxazin-2-on und 4-(3,4-Dichlor-phenylsulfinyl)-butylbromid.
Schmelzpunkt : 74 °C,
Ausbeute : 82,5 % der Theorie.

Beispiel 372

8-Brom-6-(4-phenylsulfinyl-butoxy)-4,4-dimethyl-4H-3,1-benzoxazin-2-on

Hergestellt analog Beispiel 4 aus 8-Brom-6-hydroxy-4,4-dimethyl-4H-3,1-benzoxazin-2-on und 4-Phenylsulfinyl-butylbromid.
Schmelzpunkt : 129-130 °C,
Ausbeute : 44,1 % der Theorie.

Beispiel 373

7-Chlor-6-[4-(4-chlor-phenylmercapto)-bytoxy]-4,4-dimethyl-4H-3,1-benzoxazin-2-on

Hergestellt analog Beispiel 4 aus 7-Chlor-6-hydroxy-4,4-dimethyl-4H-3,1-benzoxazin-2-on und 4-(4-Chlor-phenylmercapto)-butylchlorid.
Schmelzpunkt : 125-126 °C,
Ausbeute : 45,2 % der Theorie.

Beispiel 374

6-[4-(4-Methyl-phenylmercapto)-butoxy]-4,4,7-trimethyl-4H-3,1-benzoxazin-2-on

Hergestellt analog Beispiel 4 aus 6-Hydroxy-4,4,7-trimethyl-4H-3,1-benzoxazin-2-on und 4-(4-Methyl-phenylmercapto)-butylchlorid.
Schmelzpunkt : 114-115 °C,
Ausbeute : 51,9 % der Theorie.

Beispiel 375

7-Brom-6-[4-(4-methyl-phenylmercapto)-butoxy]-4,4-dimethyl-4H-3,1-benzoxazin-2-on

Hergestellt analog Beispiel 4 aus 7-Brom-6-hydroxy-4,4-dimethyl-4H-3,1-benzoxazin-2-on und 4-(4-Methyl-phenylmercapto)-butylchlorid.
Schmelzpunkt : 122-123 °C,
Ausbeute : 43,3 % der Theorie.

Beispiel 376

8-Brom-6-[4-(4-methyl-phenylmercapto)-butoxy]-4,4-dimethyl-4H-3,1-benzoxazin-2-on

Hergestellt analog Beispiel 4 aus 8-Brom-6-hydroxy-4,4-dimethyl-4H-3,1-benzoxazin-2-on und 4-(4-Methyl-phenylmercapto)-butylchlorid.
Schmelzpunkt : 115-116 °C,
Ausbeute : 43,3 % der Theorie.

Beispiel 377

8-Chlor-6-[4-(4-methyl-phenylsulfinyl)-butoxy]-4H-3,1-benzoxazin-2-on

Hergestellt analog Beispiel 2 aus 8-Chlor-6-[4-(4-methyl-phenylmercapto)-butoxy]-4H-3,1-benzoxazin-2-on und Wasserstoffperoxid.
Schmelzpunkt : 114-115 °C,
Ausbeute : 72,7 % der Theorie.

Beispiel 378

6-[4-(4-Acetamido-phenylsulfinyl)-butoxy]-4,4,7-trimethyl-4H-3,1-benzoxazin-2-on

Hergestellt analog Beispiel 2 aus 6-[4-(Acetamido-phenylmercapto)-butoxy]-4,4,7-trimethyl-4H-3,1-benzoxazin-2-on und Wasserstoffperoxid.
Schmelzpunkt : 210-211 °C,
Ausbeute : 70,6 % der Theorie.

Beispiel 379

6-[4-(4-Methyl-phenylsulfinyl)-butoxy]-4,4,7-trimethyl-4H-3,1-benzoxazin-2-on

Hergestellt analog Beispiel 2 aus 6-[4-(4-Methyl-phenylmercapto)-butoxy]-4,4,7-trimethyl-4H-3,1-benzoxazin-2-on und Wasserstoffperoxid.
Schmelzpunkt : 152-153 °C,
Ausbeute : 87,2 % der Theorie.

Beispiel 380

8-Chlor-6-[4-(4-acetamido-phenylsulfinyl)-butoxy]-4H-3,1-benzoxazin-2-on

Hergestellt analog Beispiel 2 aus 8-Chlor-6-[4-(4-acetamido-phenylmercapto)-butoxy]-4H-3,1-benzoxazin-2-on und Wasserstoffperoxid.
Schmelzpunkt : 130-132 °C,
Ausbeute : 85,0 % der Theorie.

Beispiel 381

5,7-Dimethyl-6-[4-(4-methyl-phenylmercapto)-butoxy]-4H-3,1-benzoxazin-2-on

Hergestellt analog Beispiel 4 aus 5,7-Dimethyl-6-hydroxy-4H-3,1-benzoxazin-2-on und 4-(4-Methyl-phenylmercapto)-butylchlorid.
Schmelzpunkt : 107-109 °C,
Ausbeute : 28,3 % der Theorie.

Beispiel 382

6-[4-(4-Methyl-phenylsulfinyl)-butoxy]-5,7-dimethyl-4H-3,1-benzoxazin-2-on

Hergestellt analog Beispiel 2 aus 6-[4-(4-Methyl-phenylmercapto)-butoxy]-5,7-dimethyl-4H-3,1-benzoxazin-2-on und Wasserstoffperoxid.
Schmelzpunkt : 114-116 °C,
Ausbeute : 52,6 % der Theorie.

Beispiel 383

6-[4-(3,4-Dichlor-phenylsulfinyl)-butoxy]-5,7-dimethyl-4H-3,1-benzoxazin-2-on

Hergestellt analog Beispiel 2 aus 6-[4-(3,4-Dichlor-phenylmercapto)-butoxy]-5,7-dimethyl-4H-3,1-benzoxazin-2-on und Wasserstoffperoxid.
Schmelzpunkt : 126-128 °C,
Ausbeute : 39,8 % der Theorie.

Beispiel 384

7-Chlor-6-[4-(4-methyl-phenylsulfinyl)-butoxy]-4H-3,1-benzoxazin-2-on

Hergestellt analog Beispiel 2 aus 7-Chlor-6-[4-(4-methyl-phenylmercapto)-butoxy]-4H-3,1-benzoxazin-2-on und Wasserstoffperoxid.
Schmelzpunkt : 177-178 °C,
Ausbeute : 72,0 % der Theorie.

Beispiel 385

5-Chlor-6-[4-(4-methyl-phenylsulfinyl)-butoxy]-4H-3,1-benzoxazin-2-on

Hergestellt analog Beispiel 2 aus 5-Chlor-6-[4-(4-methyl-phenylmercapto)-butoxy]-4H-3,1-benzoxazin-2-on und Wasserstoffperoxid.
Schmelzpunkt : 183-185 °C,
Ausbeute : 20,6 % der Theorie.

Beispiel 386

5,7-Dichlor-6-[4-(4-methyl-phenylsulfinyl)-butoxy]-4,4-dimethyl-4H-3,1-benzoxazin-2-on

Hergestellt analog Beispiel 2 aus 5,7-Dichlor-6-[4-(4-methyl-phenylmercapto)-butoxy]-4,4-dimethyl-4H-3,1-benzoxazin-2-on und Wasserstoffperoxid.
Schmelzpunkt : 169-170 °C,
Ausbeute : 75,0 % der Theorie.

Beispiel 387

5,7-Dichlor-6-(4-phenylsulfinyl-butoxy)-4,4-dimethyl-4H-3,1-benzoxazin-2-on

Hergestellt analog Beispiel 2 aus 5,7-Dichlor-6-(4-phenylmercapto-butoxy)-4,4-dimethyl-4H-3,1-benzoxazin-2-on und Wasserstoffperoxid.
Schmelzpunkt : 186-187 °C,
Ausbeute : 74,0 % der Theorie.

Beispiel 388

6-[4-(4-Acetamido-phenylmercapto)-butoxy]-4,4,7-trimethyl-4H-3,1-benzoxazin-2-on

Hergestellt analog Beispiel 4 aus 6-Hydroxy-4,4,7-trimethyl-4H-3,1-benzoxazin-2-on und 4-(Acetamido-phenylmercapto)-butylmesylat.
Schmelzpunkt : 104-106 °C,
Ausbeute : 50,9 % der Theorie.

Beispiel 389

7-Chlor-6-[4-(4-methyl-phenylmercapto)-butoxy]-4H-3,1-benzoxazin-2-on

Hergestellt analog Beispiel 4 aus 7-Chlor-6-hydroxy-4H-3,1-benzoxazin-2-on und 4-(4-Methyl-phenylmercapto)-butylchlorid.
Schmelzpunkt : 145-147 °C,
Ausbeute : 44 % der Theorie.

Beispiel 390

7-Chlor-6-(4-phenylsulfinyl-butoxy)-4H-3,1-benzoxazin-2-on

Hergestellt analog Beispiel 4 aus 7-Chlor-6-hydroxy-4H-3,1-benzoxazin-2-on und 4-Phenylsulfinylbutylchlorid.
Schmelzpunkt : 177-179 °C,
Ausbeute : 18,1 % der Theorie.

Beispiel 391

5-Chlor-6-[4-(4-methyl-phenylmercapto)-butoxy]-4H-3,1-benzoxazin-2-on

Hergestellt analog Beispiel 4 aus 5-Chlor-6-hydroxy-4H-3,1-benzoxazin-2-on und 4-(4-Methyl-phenylmercapto)-butylchlorid.
Schmelzpunkt : 144-145 °C,
Ausbeute : 12,7 % der Theorie.

Beispiel 392

5,7-Dichlor-6-[4-(4-methyl-phenylmercapto)-butoxy]-4,4-dimethyl-4H-3,1-benzoxazin-2-on

Hergestellt analog Beispiel 4 aus 5,7-Dichlor-6-hydroxy-4,4-dimethyl-4H-3,1-benzoxazin-2-on und 4-(4-Methyl-phenylmercapto)-butylchlorid.
Schmelzpunkt : 127-128 °C,
Ausbeute : 50,0 % der Theorie.

Beispiel 393

6-[4-(4-Acetamido-phenylsulfoximino)-butoxy]-4,4,7-trimethyl-4H-3,1-benzoxazin-2-on

Hergestellt analog Beispiel 6 aus 6-[4-(4-Acetamido-phenyl-sulfinyl)-butoxy]-4,4,7-trimethyl-4H-3,1-benzoxazin-2-on und O-Mesitylensulfonyl-acethydroxamsäure-äthylester.
Schmelzpunkt : 159-161 °C,
Ausbeute : 53,3 % der Theorie.

Beispiel A

Dragées mit 4 mg 6-[4-(3,4-Dichlor-phenylsulfoximino)-butoxy]-4,4-dimethyl-4H-3,1-benzoxazin-2-on

Zusammensetzung
1 Dragéekern enthält :

| | | |
|---|---|---|
| Wirksubstanz | (1) | 4,0 mg |
| Milchzucker | (2) | 27,0 mg |
| Maisstärke | (3) | 14,5 mg |
| Polyvinylpyrrolidon | (4) | 4,0 mg |
| Magnesiumstearat | (5) | 0,5 mg |
| | | 50,0 mg |

Herstellung

Die Stoffe 1-3 werden mit einer wäßrigen Lösung von 4 gleichmäßig befeuchtet, durch 1 mm-Maschenweite gesiebt, getrocknet und erneut durch 1 mm-Maschenweite gesiebt. Nach Zumischen von 5 wird die Mischung zu Dragéekernen verpreßt.
Dragéekerne : 5 mm O, bikonvex, rund

Dragierung

Übliche Zuckerdragierung auf 70 mg Endgewicht.

Beispiel B

Tabletten mit 8 mg 6-[4-(3,4-Dichlor-phenylsulfoximino)-butoxy]-4,4-dimethyl-4H-3,1-benzoxazin-2-on

1 Tablette enthält :

| | |
|---|---|
| Wirksubstanz | 8,0 mg |
| Milchzucker | 23,0 mg |
| Maisstärke | 14,5 mg |
| Polyvinylpyrrolidon | 4,0 mg |
| Magnesiumstearat | 0,5 mg |
| | 50,0 mg |

Herstellung

Analog den Dragéekernen.

Tablettenbeschreibung

Gewicht : 50 mg
Durchmesser : 5 mm, biplan, beidseitige Facette

Beispiel C

Suppositorien zu 25 mg 6-[4-(3,4-Dichlor-phenylsulfoximino)-butoxy]-4,4-dimethyl-4H-3,1-benzoxazin-2-

on

1 Zäpfchen enthält :

| | |
|---|---|
| Wirksubstanz | 0,025 g |
| Hartfett (z. B. Witepsol H 19 und Witepsol W 45) | 1,695 g |
| | 1,700 g |

Herstellung

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Zäpfchengewicht : 1,7 g

Beispiel D

Suspension mit 8 mg 6-[4-(3,4-Dichlor-phenylsulfoximino)-butoxy-4,4-dimethyl-4H-3,1-benzoxazin-2-on

100 ml Suspension enthalten :

| | |
|---|---|
| Wirksubstanz | 0,16 g |
| Carboxymethylcellulose | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70 % | 20,0 g |
| Aroma | 0,3 g |
| Wasser dest. ad | 100,0 ml |

Herstellungsfahren

Dest. Wasser wird auf 70 °C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

Beispiel E

Tabletten mit 100 mg 6-[4-(3,4-Dimethyl-phenylsulfoximino)-butoxy]-4,4-dimethyl-4H-3,1-benzoxazin-2-on

Zusammensetzung

1 Tablette enthält :

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren

Wirkstoff, Milchzucker und Stärke werden gemischt und mit einer wäßrigen Lösung des Polyvinylpyrrolidons gleichmäßig befeuchtet. Nach Siebung der feuchten Massen (2,0 mm-Maschenweite) und Trocknen im Hordentrockenschrank bei 50 °C wird erneut gesiebt (1,5 mm-Maschenweite) und das Schmiermittel zugemischt. Die preßfertige Mischung wird zu Tabletten verarbeitet.

Tablettengewicht : 220 mg

Durchmesser : 10 mg, biplan mit beidseitiger Facette und einseitiger Teilkerbe.

Beispiel F

Hartgelatine-Kapseln mit 150 mg 6-[4-(3,4-Dimethyl-phenylsulfoximino)-butoxy]-4,4-dimethyl-4H-3,1-benzoxazin-2-on

1 Kapsel enthält :

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 320,0 mg |

Herstellung

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.

Kapselfüllung : ca. 320 mg

Kapselhülle : Hartgelatine-Kapsel Größe 1.

## Beispiel G

Suppositorien mit 150 mg 6-[4-(3,4-Dimethyl-phenylsulfoximino)-butoxy]-4,4-dimethyl-4H-3,1-benzoxazin-2-on

1 Zäpfchen enthält :

| | |
|---|---|
| Wirkstoff | 150,0 mg |
| Polyäthylenglykol 1 500 | 550,0 mg |
| Polyäthylenglykol 6 000 | 460,0 mg |
| Polyäthylensorbitanmonostearat | 840,0 mg |
| | 2 000,0 mg |

Herstellung

Nach dem Aufschmelzen der Suppositorienmassen wird der Wirkstoff darin homogen verteilt und die Schmelze in vorgekühlte Formen gegossen.

## Beispiel H

Suspensionen mit 50 mg 6-[4-(3,4-Dimethyl-phenylsulfoximino)-butoxy]-4,4-dimethyl-4H-3,1-benzoxazin-2-on

100 ml Suspension enthalten :

| | |
|---|---|
| Wirkstoff | 1,0 g |
| Carboxymethylcellulose-Na-Salz | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70 %ig | 20,0 g |
| Aroma | 0,3 g |
| Wasser dest. | ad 100 ml |

Herstellung

Dest. Wasser wird auf 70 °C erhitzt. Hierin wird unter Rühren p-Hydroxybenzoesäuremethylester und -propylester wobei Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

## Beispiel I

Tabletten mit 150 mg 6-[4-(3,4-Dimethyl-phenylsulfoximino)-butoxy]-4,4-dimethyl-4H-3,1-benzoxazin-2-on

Zusammensetzung

1 Tablette enthält :

| | |
|---|---|
| Wirksubstanz | 150,0 mg |
| Milchzucker pulv. | 89,0 mg |
| Maisstärke | 40,0 mg |
| Kolloidale Kieselsäure | 10,0 mg |
| Polyvinylpyrrolidon | 10,0 mg |
| Magnesiumstearat | 1,0 mg |
| | 300,0 mg |

Herstellung

Die mit Milchzucker, Maisstärke und Kieselsäure gemischte Wirksubstanz wird mit einer 20 %igen wäßrigen Polyvinylpyrrolidonlösung befeuchtet und durch ein Sieb mit 1,5 mm-Maschenweite geschlagen.

Das bei 45 °C getrocknete Granulat wird nochmals durch dasselbe Sieb gerieben und mit der angegebenen Menge Magnesiumstearat gemischt. Aus der Mischung werden Tabletten gepreßt.

Tablettengewicht : 300 mg

Stempel : 10 mm, flach

## Beispiel K

Dragées mit 75 mg 6-[4-(3,4-Dimethyl-phenylsulfoximino)-butoxy]-4,4-dimethyl-4H-3,1-benzoxazin-2-on

1 Dragéekern enthält :

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

Kerngewicht : 230 mg

Stempel : 9 mm, gewölbt

Die so hergestellten Dragéekerne werden mit einem Film überzogen, der im wesentlichen aus Hydroxypropylmethylcellulose besteht. Die fertigen Filmdragées werden mit Bienenwaschs geglänzt.

Dragéegewicht : 245 mg.

Selbstverständlich können alle übrigen Verbindungen der allgemeinen Formel I als Wirkstoffe in den vorstehenden galenischen Zubereitungen eingesetzt werden.

**Patentansprüche** (für die Vertragsstaaten : BE, CH, DE, FR, IT, LI, LU, NL, SE)

1. Benzoxazin-2-one der allgemeinen Formel

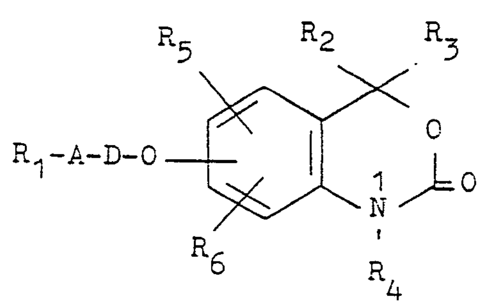

(I)

in der

A ein Schwefelatom, eine SO-, $SO_2$-, R—N=S- oder R—N=SO-Gruppe, wobei R ein Wasserstoffatom, eine geradkettige Alkanoylgruppe mit 1 bis 9 Kohlenstoffatomen, eine Pivaloylgruppe, eine gegebenenfalls durch ein Fluor-, Chlor- oder Bromatom, eine Methyl-, Äthyl-, Isopropyl-, tert.Butyl- oder Acetoxygruppe substituierte Benzoylgruppe, eine durch zwei Fluoratome, zwei Chloratome, zwei oder drei Methylgruppen substituierte Benzoylgruppe, eine gegebenenfalls durch eine Methylgrupe, ein Fluor-, Chlor- oder Bromatom substituierte Phenylsulfonylgruppe, eine Naphthoyl-, Thenoyl-, Pyridinoyl-, Hydroxysulfonyl-, Methansulfonyl-, Äthanolsulfonyl-, Pentamethyl-phenylsulfonyl-, Methoxycarbonyl-, Äthoxycarbonyl-, n-Propoxy-carbonyl-, Isopropoxycarbonyl-, Benzyloxycarbonyl-, Aminocarbonyl-, Methylaminocarbonyl- oder Dimethylaminocarbonylgruppe bedeutet,

D eine geradkettige oder verzweigte Alkylengruppe mit 2 bis 6 Kohlenstoffatomen,

$R_1$ eine gegebenenfalls durch eine Phenylgruppe substituierte Alkylgruppe mit 1 bis 3 Kohlenstoffatomen oder eine Phenylgruppe, wobei die vorstehend erwähnten Phenylkerne jeweils durch eine Alkylgruppe mit 1 bis 4 Kohlenstoffatomen, ein Halogenatom, eine Alkoxygruppe mit 1 bis 3 Kohlenstoffatomen, eine Hydroxy-, Cyclohexyl- oder Phenylgruppe, eine Aminogruppe oder eine Alkanoylaminogruppe mit 1 bis 3 Kohlenstoffatomen substituiert sein können, eine Alkylgruppe mit 4 bis 8 Kohlenstoffatomen, eine Cycloalkylgruppe mit 3 bis 7 Kohlenstoffatomen, eine durch Alkylgruppen mit 1 bis 4 Kohlenstoffatomen, Alkoxygruppen mit 1 bis 3 Kohlenstoffatomen und/oder Halogenatome di- oder trisubstituierte Phenyl- oder mono- oder disubstituierte Hydroxyphenyl- oder Aminophenylgruppe, wobei die Substituenten des Phenylkerns jeweils gleich oder verschieden sein können, eine gegebenenfalls durch eine oder zwei Methylgruppen substituierte Pyridyl- oder Pyrimidinylgruppe, eine Pyridyl-N-oxid-, Äthylpyridyl-, Äthyl-pyrimidinyl-, Propyl-pyrimidinyl-, Diäthyl-pyrimidinyl-, Pyrazinyl-, Pyridazinyl-, Pyrazolyl-, Imidazolyl-, Triazolyl-, Indolyl-, Indazolyl-, Chinolyl-, Isochinolyl-, Chinazolinyl-, Benzimidazolyl- oder Benzthiazolylgruppe,

$R_2$ und $R_3$, die gleich oder verschieden sein können, Wasserstoffatome, Phenylgruppen, Alkylgruppen mit 1 bis 6 Kohlenstoffatome oder Cycloalkylgruppen mit 3 bis 7 Kohlenstoffatomen,

$R_4$ ein Wasserstoffatom oder eine Alkylgruppe mit 1 bis 3 Kohlenstoffatomen,

$R_5$ ein Wasserstoff- oder Halogenatom, eine Nitro- oder Alkylgruppe mit 1 bis 3 Kohlenstoffatomen und

$R_6$ ein Wasserstoff- oder Halogenatom oder eine Alkylgruppe mit 1 bis 3 Kohlenstoffatomen bedeuten.

2. Benzoxazin-2-one der allgemeinen Formel I gemäß Anspruch 1, in der

A ein Schwefelatom, eine SO-, $SO_2$-, R—N=S- oder R—N=SO-Gruppe, wobei R ein Wasserstoffatom, einen gegebenenfalls durch eine Methylgruppe substituierten Benzoyl- oder Phenylsulfonylrest, eine Acetyl- oder Propionylgruppe darstellt,

D eine geradkettige Alkylengruppe mit 2 bis 6 Kohlenstoffatomen,

$R_1$ eine Alkylgruppe mit 1 bis 8 Kohlenstoffatomen, eine Cyclohexyl-, Benzyl-, Pyridyl-, Pyridyl-N-oxid-, 2-Benzthiazolyl- oder 1,2,4-Triazol-3-ylgruppe, eine gegebenenfalls durch eine oder zwei Methylgruppen substituierte 2-Pyrimidinylgruppe, eine gegebenenfalls durch eine Alkylgruppe mit 1 bis 4 Kohlenstoffatomen, eine Hydroxy-, Methoxy-, Cyclohexyl-, Phenyl- oder Acetylaminogruppe, ein Fluor-, Chlor- oder Bromatom substituierte Phenylgruppe, eine durch Chlor- oder Bromatome, Methyl- oder Methoxygruppen disubstituierte Phenylgruppe, wobei die Substituenten des Phenylkerns gleich oder verschieden sein können, oder eine durch zwei Alkylgruppen mit jeweils 1 bis 4 Kohlenstoffatomen, zwei Chlor- oder Bromatome substituierte Aminophenyl- oder Hydroxyphenylgruppe,

$R_2$ und $R_3$, die gleich oder verschieden sein können, Wasserstoffatome, Alkylgruppen mit 1 bis 6 Kohlenstoffatomen, Phenyl- oder Cyclohexylgruppen,

$R_4$ ein Wasserstoffatom oder eine Methylgruppe,

$R_5$ ein Wasserstoff-, Fluor-, Chlor- oder Bromatom, eine Nitro-, Methyl- oder Äthylgruppe und

$R_6$ ein Wasserstoff-, Chlor- oder Bromatom, eine Methyl- oder Äthylgruppe bedeuten.

3. Benzoxazin-2-one der allgemeinen Formel

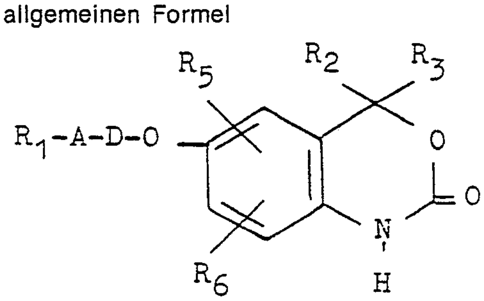

(Ia)

in der A, D, $R_1$ bis $R_3$, $R_5$ und $R_6$ wie im Anspruch 2 definiert sind.

4. Benzoxazin-2-one der allgemeinen Formel Ia gemäß Anspruch 3, in der

A die SO-, $SO_2$-, H—N=SO-, $CH_3CO$—N=SO- und $CH_3$—$C_6H_4$—$SO_2$—N=SO-Gruppe,

D die n-Butylengruppe,

$R_1$ eine gegebenenfalls durch ein Fluor-, Chlor- oder Bromatom, eine Methyl-, Hydroxy-, Methoxy-, Cyclohexyl-, Phenyl- oder Acetaminogruppe substituierte Phenylgruppe, eine durch Chlor- oder Bromatome, Methyl- oder Methoxygruppen disubstituierte Phenylgruppe, wobei die Substituenten des Phenylkerns gleich oder verschieden sein können, eine 4- Amino-3,5-dibrom-phenyl-, eine 3,5-Di-tert.butyl-4-hydroxy-phenyl- oder Pyridylgruppe,

$R_2$ und $R_3$, die gleich oder verschieden sein können, je ein Wasserstoffatom oder eine Methylgruppe,

$R_5$ ein Wasserstoff-, Chlor- oder Bromatom, eine Nitro- oder eine Methylgruppe und

$R_6$ ein Wasserstoff-, Chlor- oder Bromatom oder eine Methylgruppe bedeuten.

5. Benzoxazin-2-one der allgemeinen Formel Ia gemäß Anspruch 3, in der

A die —SO-, NH=SO- oder $CH_3CO$—N=SO-Gruppe,

D die n-Butylengruppe,

$R_1$ eine gegebenenfalls in 4-Stellung durch ein Fluor-, Chlor- oder Bromatom, eine Methyl-, Cyclohexyl-, Phenyl-, Hydroxy- oder Methoxygruppe substituierte Phenylgruppe, eine 3-Methoxy-phenyl-, 3,4-Dichlor-phenyl-, 3,4-Dimethoxy-phenyl-, 4-Brom-3-methyl-phenyl-, 4-Amino-3,5-dibrom-phenyl- oder 4-Hydroxy-3,5-di-tert.butyl-phenylgruppe,

$R_2$ und $R_3$ je ein Wasserstoffatom oder eine Methylgruppe,

$R_5$ ein Wasserstoffatom oder in 7-Stellung ein Chlor- oder Bromatom und

$R_6$ ein Wasserstoffatom bedeuten.

6. 6-[4-(4-Biphenylylsulfinyl)-butoxy]-4,4-dimethyl-4H-3,1-benzoxazin-2-on.

7. 6-[4-(4-Brom-phenylsulfinyl)-butoxy]-4,4-dimethyl-4H-3,1-benzoxazin-2-on.

8. Arzneimittel, enthaltend eine Verbindung gemäß den Ansprüchen 1 bis 7 neben einem oder mehreren inerten Trägerstoffen und/oder Verdünnungsmitteln.

9. Verfahren zur Herstellung eines Arzneimittels zur Behandlung oder zur Prophylaxe thromboembolischer Erkrankungen, zur Prophylaxe der Arteriosklerose und zur Metastasenprophylaxe gemäß Anspruch 8, dadurch gekennzeichnet, daß auf nichtclonischem Wege eine Verbindung gemäß den Ansprüchen 1 bis 7 in einen oder mehrere inerte Trägerstoffe eingearbeitet wird.

10. Verfahren zur Herstellung von neuen Benzoxazin-2-onen der allgemeinen Formel I gemäß den Ansprüchen 1 bis 7, dadurch gekennzeichnet, daß

a) eine Hydroxyverbindung der allgemeinen Formel

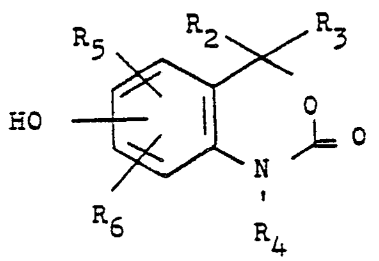

(II)

in der $R_2$ bis $R_6$ wie eingangs definiert sind, oder deren Salze mit einer anorganischen oder tertiären organischen Base mit einer Verbindung der allgemeinen Formel

$$Z—D—A—R_1 \quad (III)$$

in der

A, D und $R_1$ wie eingangs definiert sind und

Z eine nukleophil austauschbare Gruppe darstellen, umgesetzt wird oder

b) zur Herstellung einer Verbindung der allgemeinen Formel I, in der A eine —SO-, —$SO_2$- oder R—N=SO-Gruppe darstellt, eine Verbindung der allgemeinen Formel

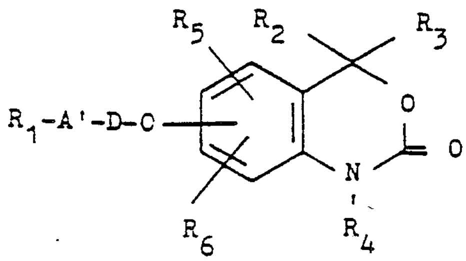

(IV)

in der

$R_1$ bis $R_6$ und D wie eingangs definiert sind und

A' ein Schwefelatom, eine —SO- oder R—N=S-Gruppe darstellt, wobei R wie eingangs definiert ist, oxidiert wird oder

c) zur Herstellung einer Verbindung der allgemeinen Formel I, in der A ein Schwefelatom oder eine —$SO_2$-Gruppe darstellt, eine Verbindung der allgemeinen Formel

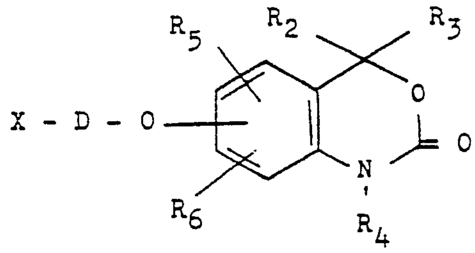

(V)

in der

$R_2$ bis $R_6$ und D wie eingangs definiert sind und

X eine nukleophile austauschbare Gruppe darstellt, mit einer Verbindung der allgemeinen Formel

$$Y—R_1 \qquad (VI)$$

in der

$R_1$ wie eingangs definiert ist und

Y eine $MeSO_2$-Gruppe, wobei Me ein Alkali- oder Erdalkali/$_2$-Metallatom darstellt, oder die Mercaptogruppe bedeutet, umgesetzt wird oder

d) zur Herstellung einer Verbindung der allgemeinen Formel I, in der A die H—N=SO-Gruppe darstellt, ein Sulfoxid der allgemeinen Formel

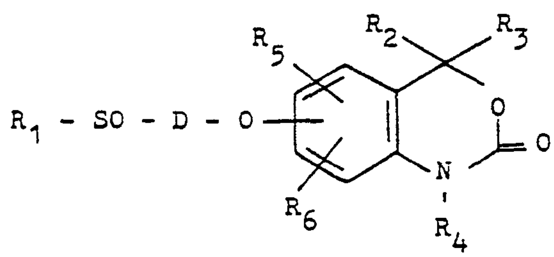

(VII)

in der $R_1$ bis $R_6$ und D wie eingangs definiert sind, mit gegebenenfalls im Reaktionsgemisch gebildeter Stickstoffwasserstoffsäure umgesetzt wird oder

e) zur Herstellung einer Verbindung der allgemeinen Formel I, in der A die H—N=SO-Gruppe darstellt, ein Sulfoxid der allgemeinen Formel

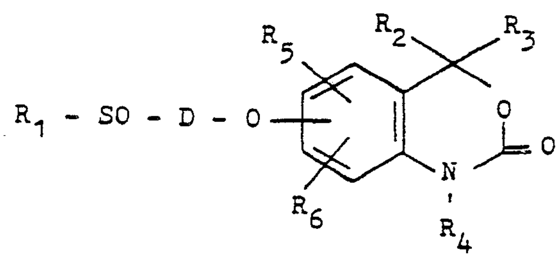

(VII)

in der $R_1$ bis $R_6$ und D wie eingangs definiert sind, mit einer Verbindung der allgemeinen Formel

$$H_2N—O—X—R_7 \qquad (VIII)$$

in der

X eine Carbonyl- oder Sulfonylgruppe und

$R_7$ eine in o-Stellung disubstituierte Arylgruppe darstellen, umgesetzt wird oder

f) zur Herstellung einer Verbindung der allgemeinen Formel I, in der R kein Wasserstoffatom darstellt, eine Verbindung der allgemeinen Formel

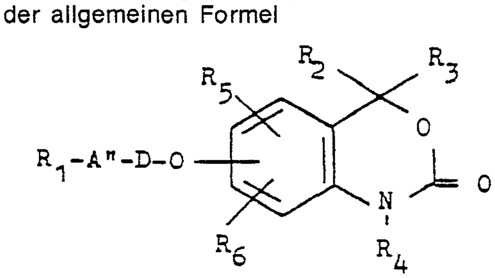

(IX)

in der

$R_1$ bis $R_6$ und D wie eingangs definiert sind und

A″ die H—N=S- oder H—N=SO-Gruppe darstellt, acyliert wird oder

g) zur Herstellung einer Verbindung der allgemeinen Formel I, in der A die R—N=S-Gruppe darstellt, ein Thioäther der allgemeinen Formel

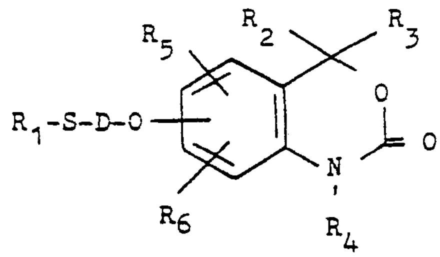

(X)

in der $R_1$ bis $R_6$ und D wie eingangs definiert sind, mit einem Halogenamid der allgemeinen Formel

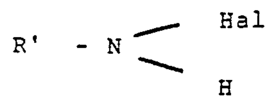

(XI)

in der

R′ mit Ausnahme des Wasserstoffatoms die für R eingangs erwähnten Bedeutungen besitzt und

Hal ein Chlor- oder Bromatom darstellt, oder mit dessen Alkalisalz umgesetzt wird und gegebenenfalls eine so erhaltene Verbindung anschließend hydrolysiert wird oder

h) zur Herstellung einer Verbindung der allgemeinen Formel I, in der A die H—N = SO-Gruppe darstellt, von einem Sulfoxid der allgemeinen Formel

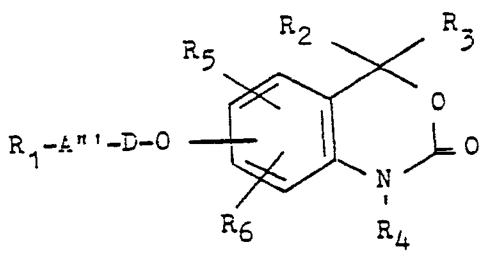

(XII)

in der

$R_1$ bis $R_6$ und D wie eingangs definiert sind und

A‴ eine durch einen hydrolytisch abspaltbaren Acylrest substituierte H—N=S- oder H—N=SO-Gruppe darstellt, ein Acylrest hydrolytisch abgespalten wird oder

i) zur Herstellung einer Verbindung der allgemeinen Formel I, in der R kein Wasserstoffatom darstellt, eine Verbindung der allgemeinen Formel

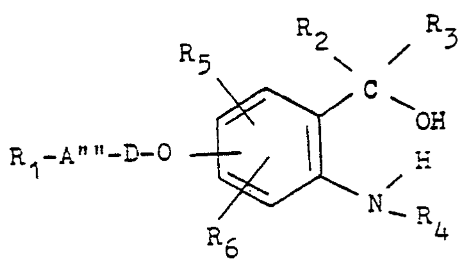

(XIII)

in der

D und $R_1$ bis $R_6$ wie eingangs definiert sind und

A″″ ein Schwefelatom, eine SO-, $SO_2$-, R′—N=S- oder R′—N=SO-Gruppe darstellt, wobei R′ mit Ausnahme eines Wasserstoffatoms die für R eingangs erwähnten Bedeutungen besitzt, mit einem Kohlensäurederivat der allgemeinen Formel

X—CO—X (XIV)

in der X, die gleich oder verschieden sein können, jeweils eine nukleophile Austrittsgruppe darstellen, umgesetzt wird und gewünschtenfalls anschließend eine erfindungsgemäß erhaltene Verbindung der allgemeinen Formel I, in der $R_4$ ein Wasserstoffatom darstellt, mittels Alkylierung in eine entsprechende Verbindung der allgemeinen Formel I, in der $R_4$ eine Alkylgruppe mit 1 bis 3 Kohlenstoffatomen darstellt, übergeführt wird.

**Patentansprüche** (für den Vertragsstaat AT)

1. Verfahren zur Herstellung von neuen Benzoxazin-2-onen der allgemeinen Formel

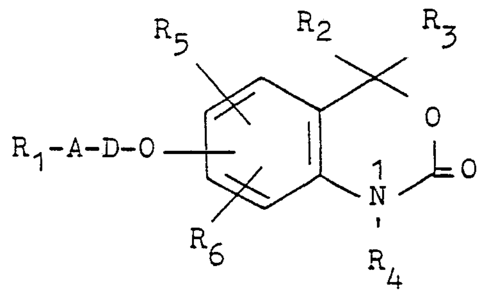

(I)

in der

A ein Schwefelatom, eine SO-, $SO_2$-, R—N=S- oder R—N=SO-Gruppe, wobei R ein Wasserstoffatom, eine geradkettige Alkanoylgruppe mit 1 bis 9 Kohlenstoffatomen, eine Pivaloylgruppe, eine gegebenenfalls durch ein Fluor-, Chlor- oder Bromatom, eine Methyl-, Äthyl-, Isopropyl-, tert.Butyl- oder Acetoxygruppe substituierte Benzoylgruppe, eine durch zwei Fluoratome, zwei Chloratome, zwei oder drei Methylgruppen substituierte Benzoylgruppe, eine gegebenenfalls durch eine Methylgruppe, ein Fluor-, Chlor- oder Bromatom substituierte Phenylsulfonylgruppe, eine Naphthoyl-, Thenoyl-, Pyridinoyl-, Hydroxysulfonyl-, Methansulfonyl-, Äthanolsulfonyl-, Pentamethyl-phenylsulfonyl-, Methoxycarbonyl-, Äthoxycarbonyl-, n-Propoxy-carbonyl-, Isopropoxycarbonyl-, Benzyloxycarbonyl-, Aminocarbonyl-, Methylaminocarbonyl- oder Dimethylaminocarbonylgruppe bedeutet,

D eine geradkettige oder verzweigte Alkylengruppe mit 2 bis 6 Kohlenstoffatomen,

$R_1$ eine gegebenenfalls durch eine Phenylgruppe substituierte Alkylgruppe mit 1 bis 3 Kohlenstoffatomen oder eine Phenylgruppe, wobei die vorstehend erwähnten Phenylkerne jeweils durch eine Alkylgruppe mit 1 bis 4 Kohlenstoffatomen, ein Halogenatom, eine Alkoxygruppe mit 1 bis 3 Kohlenstoffatomen, eine Hydroxy-, Cyclohexyl- oder Phenylgruppe, eine Aminogruppe oder eine Alkanoylaminogruppe mit 1 bis 3 Kohlenstoffatomen substituiert sein können, eine Alkylgruppe mit 4 bis 8 Kohlenstoffatomen, eine Cycloalkylgruppe mit 3 bis 7 Kohlenstoffatomen, eine durch Alkylgruppen mit 1 bis 4 Kohlenstoffatomen, Alkoxygruppen mit 1 bis 3 Kohlenstoffatomen und/oder Halogenatome di- oder trisubstituierte Phenyl- oder mono- oder disubstituierte Hydroxyphenyl- oder Aminophenylgruppe, wobei die Substituenten des Phenylkerns jeweils gleich oder verschieden sein können, eine gegebenenfalls durch eine oder zwei Methylgruppen substituierte Pyridyl- oder Pyrimidinylgruppe, eine Pyridyl-N-oxid-, Äthylpyridyl-, Äthyl-pyrimidinyl-, Propyl-pyrimidinyl-, Diäthyl-pyrimidinyl-, Pyrazinyl-, Pyridazinyl-, Pyrazolyl-, Imidazolyl-, Triazolyl-, Indolyl-, Indazolyl-, Chinolyl-, Isochinolyl-, Chinazolinyl-, Benzimidazolyl- oder Benzthiazolylgruppe,

$R_2$ und $R_3$, die gleich oder verschieden sein können, Wasserstoffatome, Phenylgruppen, Alkylgruppen mit 1 bis 6 Kohlenstoffatome oder Cycloalkylgruppen mit 3 bis 7 Kohlenstoffatomen,

$R_4$ ein Wasserstoffatom oder eine Alkylgruppe mit 1 bis 3 Kohlenstoffatomen,

$R_5$ ein Wasserstoff- oder Halogenatom, eine Nitro- oder Alkylgruppe mit 1 bis 3 Kohlenstoffatomen und

$R_6$ ein Wasserstoff- oder Halogenatom oder eine Alkylgruppe mit 1 bis 3 Kohlenstoffatomen bedeuten, dadurch gekennzeichnet, daß

a) eine Hydroxyverbindung der allgemeinen Formel

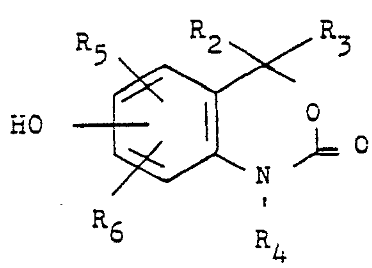

(II)

in der $R_2$ bis $R_6$ wie eingangs definiert sind, oder deren Salze mit einer anorganischen oder tertiären organischen Base mit einer Verbindung der allgemeinen Formel

$$Z—D—A—R_1 \qquad (III)$$

in der

A, D und $R_1$ wie eingangs definiert sind und

Z eine nukleophil austauschbare Gruppe darstellt, umgesetzt wird oder

b) zur Herstellung einer Verbindung der allgemeinen Formel I, in der A eine —SO-, —$SO_2$- oder R—N=SO-Gruppe darstellt, eine Verbindung der allgemeinen Formel

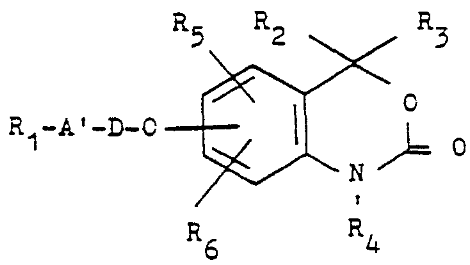

(IV)

in der

$R_1$ bis $R_6$ und D wie eingangs definiert sind und

A' ein Schwefelatom, eine —SO- oder R—N=S-Gruppe darstellt, wobei R wie eingangs definiert ist, oxidiert wird oder

c) zur Herstellung einer Verbindung der allgemeinen Formel I, in der A ein Schwefelatom oder eine —$SO_2$-Gruppe darstellt, eine Verbindung der allgemeinen Formel

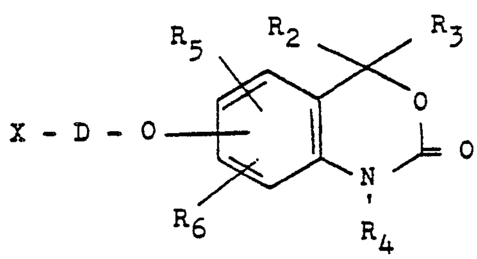

(V)

in der

$R_2$ bis $R_6$ und D wie eingangs definiert sind und X eine nukleophile austauschbare Gruppe darstellt, mit einer Verbindung der allgemeinen Formel

$$Y—R_1 \qquad (VI)$$

in der

$R_1$ wie eingangs definiert ist und

Y eine $MeSO_2$-Gruppe, wobei Me ein Alkali- oder Erdalkali/$_2$-Metallatom darstellt, oder die Mercaptogruppe bedeutet, umgesetzt wird oder

d) zur Herstellung einer Verbindung der allgemeinen Formel I, in der A die H—N=SO-Gruppe darstellt, ein Sulfoxid der allgemeinen Formel

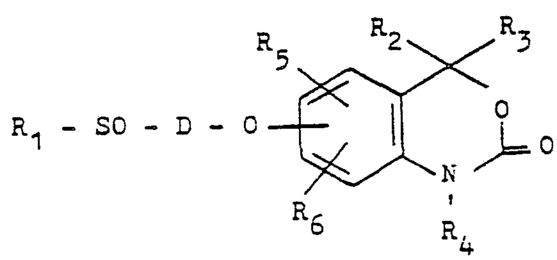

(VII)

in der $R_1$ bis $R_6$ und D wie eingangs definiert sind, mit gegebenenfalls im Reaktionsgemisch gebildeter Stickstoffwasserstoffsäure umgesetzt wird oder

e) zur Herstellung einer Verbindung der allgemeinen Formel I, in der A die H—N=SO-Gruppe

darstellt, ein Sulfoxid der allgemeinen Formel

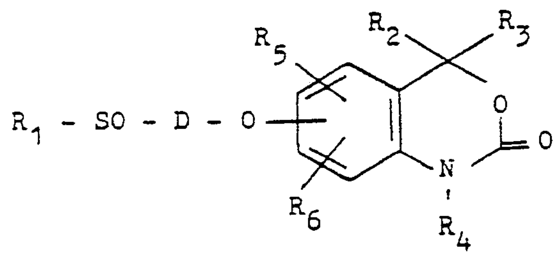

(VII)

in der $R_1$ bis $R_6$ und D wie eingangs definiert sind, mit einer Verbindung der allgemeinen Formel

$$H_2N—O—X—R_7 \quad \text{(VIII)}$$

in der
X eine Carbonyl- oder Sulfonylgruppe und
$R_7$ eine in o-Stellung disubstituierte Arylgruppe darstellen, umgesetzt wird oder
f) zur Herstellung einer Verbindung der allgemeinen Formel I, in der R kein Wasserstoffatom darstellt, eine Verbindung der allgemeinen Formel

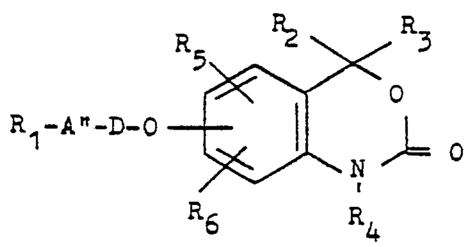

(IX)

in der
$R_1$ bis $R_6$ und D wie eingangs definiert sind und
A" die H—N=S- oder H—N=SO-Gruppe darstellt, acyliert wird oder
g) zur Herstellung einer Verbindung der allgemeinen Formel I, in der A die R—N=S-Gruppe darstellt, ein Thioäther der allgemeinen Formel

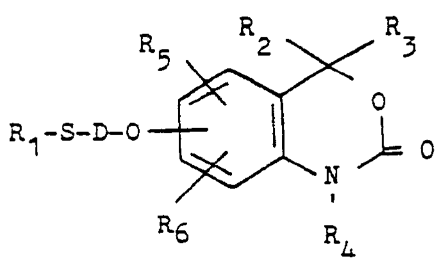

(X)

in der $R_1$ bis $R_6$ und D wie eingangs definiert sind, mit einem Halogenamid der allgemeinen Formel

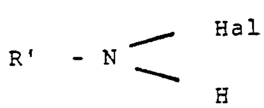

(XI)

in der
R' mit Ausnahme des Wasserstoffatoms die für R eingangs erwähnten Bedeutungen besitzt und
Hal ein Chlor- oder Bromatom darstellt, oder mit dessen Alkalisalz umgesetzt wird und gegebenenfalls eine so erhaltene Verbindung anschließend hydrolysiert wird oder
h) zur Herstellung einer Verbindung der allgemeinen Formel I, in der A die H—N=SO-Gruppe darstellt, von einem Sulfoxid der allgemeinen Formel

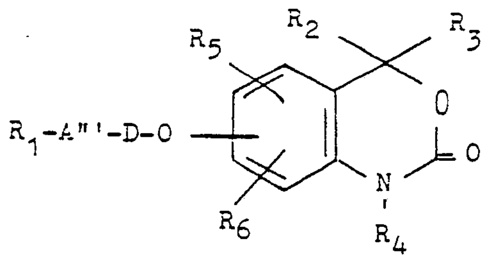

(XII)

in der

$R_1$ bis $R_6$ und D wie eingangs definiert sind und

A‴ eine durch einen hydrolytisch abspaltbaren Acylrest substituierte H—N=S- oder H—N=SO-Gruppe darstellt, ein Acylrest hydrolytisch abgespalten wird oder

i) zur Herstellung einer Verbindung der allgemeinen Formel I, in der R kein Wasserstoffatom darstellt, eine Verbindung der allgemeinen Formel

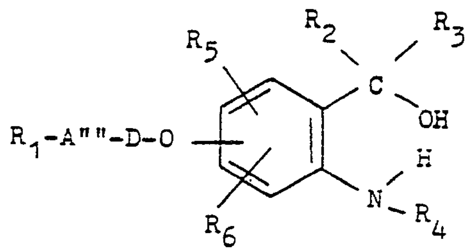

(XIII)

in der

D und $R_1$ bis $R_6$ wie eingangs definiert sind und

A⁗ ein Schwefelatom, eine SO-, $SO_2$-, R'—N=S- oder R'—N=SO-Gruppe darstellt, wobei R' mit Ausnahme eines Wasserstoffatoms die für R eingangs erwähnten Bedeutungen besitzt, mit einem Kohlensäurederivat der allgemeinen Formel

X—CO—X (XIV)

in der X, die gleich oder verschieden sein können, jeweils eine nukleophile Austrittsgruppe darstellen, umgesetzt wird und gewünschtenfalls anschließend eine erfindungsgemäß erhaltene Verbindung der allgemeinen Formel I, in der $R_4$ ein Wasserstoffatom darstellt, mittels Alkylierung in eine entsprechende Verbindung der allgemeinen Formel I, in der $R_4$ eine Alkylgruppe mit 1 bis 3 Kohlenstoffatomen darstellt, übergeführt wird.

2. Verfahren gemäß Anspruch 1 zur Herstellung von neuen Benzoxazin-2-onen der allgemeinen Formel

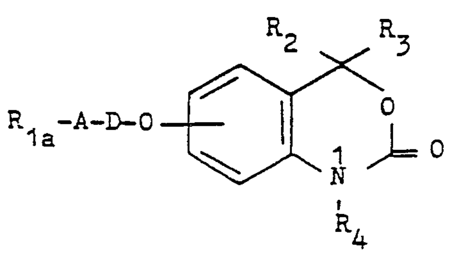

(Ia)

in der $R_2$ bis $R_4$, A und D wie im Anspruch 1 definiert sind und $R_{1a}$ eine gegebenenfalls durch eine Phenylgruppe substituierte Alkylgruppe mit 1 bis 3 Kohlenstoffatomen oder eine Phenylgruppe, wobei die vorstehend erwähnten Phenylkerne jeweils durch eine Alkylgruppe mit 1 bis 4 Kohlenstoffatomen, ein Halogenatom, eine Alkoxygruppe mit 1 bis 3 Kohlenstoffatomen, eine Hydroxy-, Cyclohexyl- oder Phenylgruppe oder eine Alkanoylaminogruppe mit 1 bis 3 Kohlenstoffatomen substituiert sein können, eine Alkylgruppe mit 4 bis 8 Kohlenstoffatomen, eine Cycloalkylgruppe mit 3 bis 7 Kohlenstoffatomen, eine durch Alkylgruppen mit 1 bis 4 Kohlenstoffatomen, Alkoxygruppen mit 1 bis 3 Kohlenstoffatomen und/oder Halogenatome di- oder trisubstituierte Phenyl- oder disubstituierte Hydroxy- oder Aminophenylgruppe, wobei die Substituenten des Phenylkerns jeweils gleich oder verschieden sein können, oder eine Pyridylgruppe bedeutet, dadurch gekennzeichnet, daß

a) zur Herstellung einer Verbindung der allgemeinen Formel Ia, in der A ein Schwefelatom, eine R—N=S-, SO- oder $SO_2$-Gruppe darstellt, eine Hydroxyverbindung der allgemeinen Formel

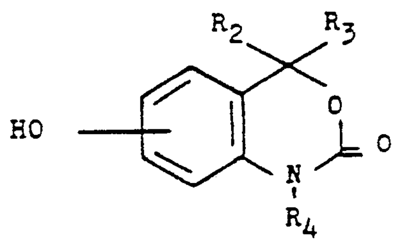

(IIa)

in der $R_2$ bis $R_4$ wie in Anspruch 1 definiert sind, oder deren Salze mit einer anorganischen oder tertiären organischen Base mit einer Verbindung der allgemeinen Formel

$$Z—D—A_a—R_{1a} \qquad \text{(IIIa)}$$

in der
D und Z wie im Anspruch 1 definiert sind,
$R_{1a}$ die eingangs erwähnten Bedeutungen besitzt und
$A_a$ ein Schwefelatom, eine R—N=S-, SO- oder $SO_2$-Gruppe, wobei R wie im Anspruch 1 definiert ist, darstellt, umgesetzt wird oder

b) zur Herstellung einer Verbindung der allgemeinen Formel Ia, in der A eine SO-, $SO_2$- oder R—N=SO-Gruppe darstellt, eine Verbindung der allgemeinen Formel

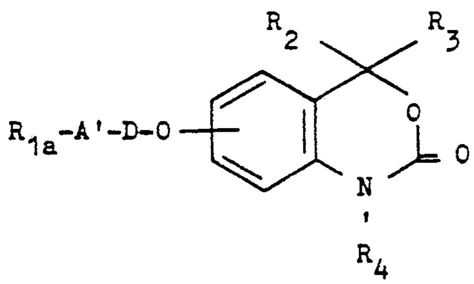

(IVa)

in der
$R_2$ bis $R_4$, A' und D wie im Anspruch 1 definiert sind und
$R_{1a}$ die eingangs erwähnten Bedeutungen besitzt, oxidiert wird oder

c) zur Herstellung eine Verbindung der allgemeinen Formel Ia, in der A ein Schwefelatom oder eine $SO_2$-Gruppe dargestellt, eine Verbindung der allgemeinen Formel

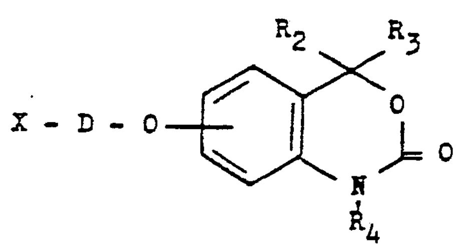

(Va)

in der $R_2$ bis $R_4$, D und X wie im Anspruch 1 definiert sind, mit einer Verbindung der allgemeinen Formel

$$Y—R_{1a} \qquad \text{(VIa)}$$

in der
Y wie im Anspruch 1 und
$R_{1a}$ wie eingangs definiert ist, umgesetzt wird oder

d) zur Herstellung einer Verbindung der allgemeinen Formel Ia, in der A die H—N=SO-Gruppe darstellt, ein Sulfoxid der allgemeinen Formel

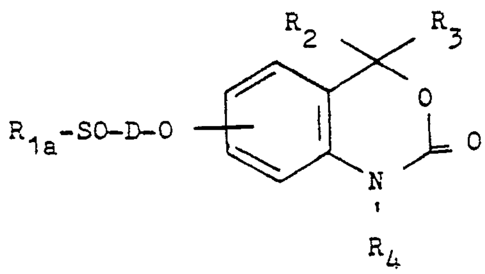

(VIIa)

in der

$R_2$ bis $R_4$ und D wie im Anspruch 1 definiert sind und

$R_{1a}$ die eingangs erwähnten Bedeutungen besitzt, mit gegebenenfalls im Reaktionsgemisch gebildeter Stickstoffwasserstoffsäure umgesetzt wird oder

e) zur Herstellung einer Verbindung der allgemeinen Formel Ia, in der A die H—N=SO-Gruppe darstellt, ein Sulfoxid der allgemeinen Formel

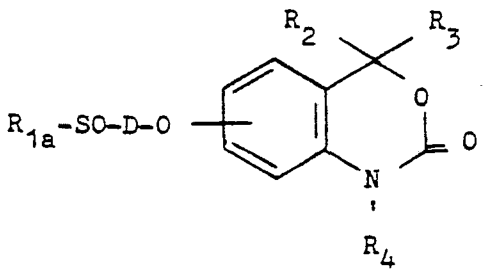

(VIIa)

in der

$R_2$ bis $R_4$ und D wie im Anspruch 1 definiert sind und

$R_{1a}$ die eingangs erwähnten Bedeutungen besitzt, mit einer Verbindung der allgemeinen Formel

$$H_2N—O—X—R_7 \quad (VIII)$$

in der $R_7$ und X wie im Anspruch 1 definiert sind, umgesetzt wird oder

f) zur Herstellung eine Verbindung der allgemeinen Formel Ia, in der R kein Wasserstoffatom darstellt, eine Verbindung der allgemeinen Formel

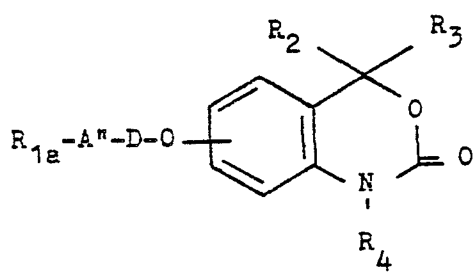

(IXa)

in der

$R_2$ bis $R_4$, A" und D wie im Anspruch 1 definiert sind und

$R_{1a}$ die eingangs erwähnten Bedeutungen besitzt acyliert wird oder

g) zur Herstellung einer Verbindung der allgemeinen Formel Ia, in der A die R—N=S-Gruppe darstellt, ein Thioäther der allgemeinen Formel

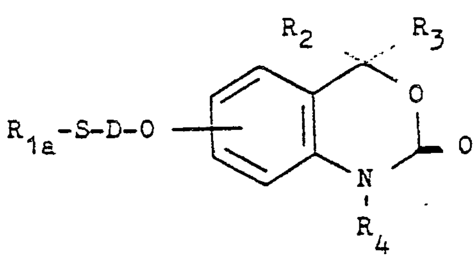

(Xa)

in der

$R_2$ bis $R_4$ und D wie im Anspruch 1 definiert sind und

$R_{1a}$ die eingangs erwähnten Bedeutungen besitzt, mit einem Halogenamid der allgemeinen Formel

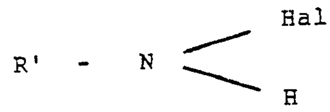

(XI)

in der R′ und Hal wie im Anspruch 1 definiert sind, oder mit dessen Alkalisalz umgesetzt wird und gegebenenfalls eine so erhaltene Verbindung anschließend hydrolysiert wird und gewünschtenfalls anschließend eine erfindungsgemäß erhaltene Verbindung der allgemeinen Formel I, in der $R_4$ ein Wasserstoffatom darstellt, mittels Alkylierung in eine entsprechende Verbindung der allgemeinen Formel I, in der $R_4$ eine Alkylgruppe mit 1 bis 3 Kohlenstoffatomen darstellt, übergeführt wird.

3. Verfahren nach den Ansprüchen 1 und 2, dadurch gekennzeichnet, daß die Umsetzung in einem Lösungsmittel durchgeführt wird.

4. Verfahren nach den Ansprüchen 1a, 2a und 3, dadurch gekennzeichnet, daß die Umsetzung in Gegenwart einer Alkalibase oder eines Alkoholates durchgeführt wird.

5. Verfahren nach den Ansprüchen 1b, 2b und 3, dadurch gekennzeichnet, daß zur Herstellung einer Verbindung der allgemeinen Formel I, in der A eine SO- oder R—N=SO-Gruppe darstellt, die Oxidation mit einem Äquivalent des eingesetzten Oxidationsmittels durchgeführt wird.

6. Verfahren nach den Ansprüchen 1b, 2b und 3, dadurch gekennzeichnet, daß zur Herstellung einer Verbindung der allgemeinen Formel I, in der A eine $SO_2$-Gruppe darstellt, die Oxidation ausgehend von einem Thioäther der allgemeinen Formel I mit zwei Äquivalenten bzw. ausgehend von einer Sulfinylverbindung der allgemeinen Formel I mit einem Äquivalent des eingesetzten Oxidationsmittels durchgeführt wird.

7. Verfahren nach den Ansprüchen 1c, 2c, 1g, 2g und 3, dadurch gekennzeichnet, daß die Umsetzung in Gegenwart einer Alkalibase durchgeführt wird.

8. Verfahren nach den Ansprüchen 1e, 2e, 3 und 12, dadurch gekennzeichnet, daß eine Verbindung der allgemeinen Formel VIII im Reaktionsgemisch hergestellt wird.

9. Verfahren nach den Ansprüchen 1f, 2f und 3, dadurch gekennzeichnet, daß die Umsetzung in Gegenwart einer anorganischen oder tertiären organischen Base durchgeführt wird.

10. Verfahren nach den Ansprüchen 1h und 3, dadurch gekennzeichnet, daß als Acylrest ein Acylrest einer Carbonsäure oder eines Kohlensäurederivates verwendet wird.

**Claims** (for the Contracting States : BE, CH, DE, FR, IT, LI, LU, NL, SE)

1. Benzoxazin-2-ones of general formula

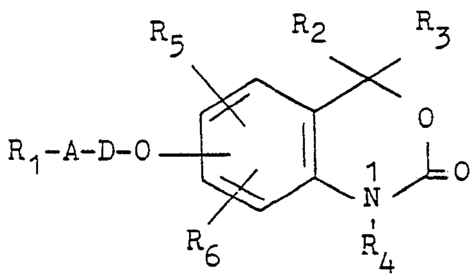

(I)

wherein

A represents a sulphur atom, an SO-, $SO_2$-, R—N=S- or R—N=SO-group, wherein R represents a hydrogen atom, a straight-chained alkanoyl group with 1 to 9 carbon atoms, a pivaloyl group, a benzoyl group optionally substituted by a fluorine, chlorine or bromine atom or by a methyl, ethyl, isopropyl, tert.butyl or acetoxy group, a benzoyl group substituted by two fluorine atoms, two chlorine atoms, two or three methyl groups, a phenylsulphonyl group optionally substituted by a methyl group or by a fluorine, chlorine or bromine atom, a naphthoyl, thenoyl, pyridinoyl, hydroxysulphonyl, methanesulphonyl, ethanolsulphonyl, pentamethyl-phenylsulphonyl, methoxycarbonyl, ethoxycarbonyl, n-propoxy-carbonyl, isopropoxycarbonyl, benzyloxycarbonyl, aminocarbonyl, methylaminocarbonyl or dimethylaminocarbonyl group,

D represents a straight-chained or branched alkylene group with 2 to 6 carbon atoms,

$R_1$ represents an alkyl group with 1 to 3 carbon atoms optionally substituted by a phenyl group, or a phenyl group, whilst the above-mentioned phenyl nuclei may each be substituted by an alkyl group with 1 to 4 carbon atoms, a halogen atom, an alkoxy group with 1 to 3 carbon atoms, a hydroxy, cyclohexyl or phenyl group, an amino group or an alkanoylamino group with 1 to 3 carbon atoms ; an alkyl group with 4 to 8 carbon atoms, a cycloalkyl group with 3 to 7 carbon atoms, a phenyl group di- or trisubstituted, or a hydroxyphenyl or aminophenyl group mono- or disubstituted, by alkyl groups with 1 to 4 carbon atoms, alkoxy groups with 1 to 3 carbon atoms and/or halogen atoms, whilst the substituents of the phenyl nucleus may be identical or different, a pyridyl or pyrimidinyl group optionally substituted by one or two methyl groups, or a pyridyl-N-oxide, ethyl-pyridyl, ethyl-pyrimidinyl, propyl-pyrimidinyl, diethyl-pyrimidinyl, pyrazinyl, pyridazinyl, pyrazolyl, imidazolyl, triazolyl, indolyl, indazolyl, quinolyl, isoquinolyl,

quinazolinyl, benzimidazolyl or benzthiazolyl group,

$R_2$ and $R_3$, which may be identical or different, represent hydrogen atoms, phenyl groups, alkyl groups with 1 to 6 carbon atoms or cycloalkyl groups with 3 to 7 carbon atoms,

$R_4$ represents a hydrogen atom or an alkyl group with 1 to 3 carbon atoms,

$R_5$ represents a hydrogen or halogen atom, a nitro or alkyl group with 1 to 3 carbon atoms and

$R_6$ represents a hydrogen or halogen atom or an alkyl group with 1 to 3 carbon atoms.

2. Benzoxazin-2-ones of general formula I as claimed in claim 1, wherein

A represents a sulphur atom, or an SO, $SO_2$, R—N=S or R—N=SO group wherein R represents a hydrogen atom, a benzoyl or phenylsulphonyl group (optionally substituted by a methyl group), or an acetyl or propionyl group,

D represents a straight-chained alkylene group with 2 to 6 carbon atoms,

$R_1$ represents an alkyl group with 1 to 8 carbon atoms, a cyclohexyl, benzyl, pyridyl, pyridyl-N-oxide, 2-benzothiazolyl or 1,2,4-triazol-3-yl group, a 2-pyrimidinyl group optionally substituted by one or two methyl groups, a phenyl group optionally substituted by an alkyl group with 1 to 4 carbon atoms or by a hydroxy, methoxy, cyclohexyl, phenyl or acetylamino group or by a fluorine, chlorine or bromine atom, a phenyl group disubstituted by chlorine or bromine atoms or by methyl or methoxy groups, wherein the substituents of the phenyl nucleus may be identical or different, or an aminophenyl or hydroxyphenyl group substituted by two alkyl groups each having 1 to 4 carbon atoms or by two chlorine or bromine atoms,

$R_2$ and $R_3$, which may be the same or different, represent hydrogen atoms, alkyl groups with 1 to 6 carbon atoms or phenyl or cyclohexyl groups,

$R_4$ represents a hydrogen atom or a methyl group,

$R_5$ represents a hydrogen, fluorine, chlorine or bromine atom or a nitro, methyl or ethyl group and

$R_6$ represents a hydrogen, chlorine or bromine atom or a methyl or ethyl group.

3. Benzoxazin-2-ones of general formula

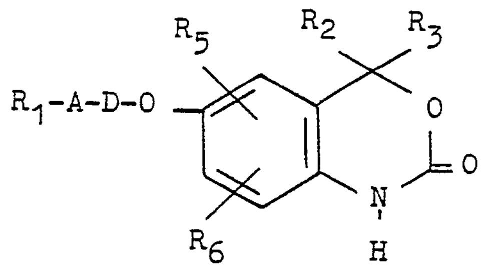

Ia)

wherein A, D, $R_1$ to $R_3$ and $R_5$ and $R_6$ are defined as in claim 2.

4. Benzoxazin-2-ones of general formula Ia as claimed in claim 3, wherein

A represents an SO, $SO_2$, H—N=SO, $CH_3CO$—N=SO or $CH_3$—$C_6H_4$—$SO_2$—N=SO group,

D represents an n-butylene group,

$R_1$ represents a phenyl group optionally substituted by a fluorine, chlorine or bromine atom, or by a methyl, hydroxy, methoxy, cyclohexyl, phenyl or acetamino group ; a phenyl group disubstituted by chlorine or bromine atoms or by methyl or methoxy groups, whilst the substituents of the phenyl nucleus may be the same or different, or a 4-amino-3,5-dibromophenyl, a 3,5-di-tert.butyl-4-hydroxyphenyl or pyridyl group,

$R_2$ and $R_3$ which may be the same or different each represent a hydrogen atom or a methyl group,

$R_5$ represents a hydrogen, chlorine or bromine atom or a nitro or methyl group, and

$R_6$ represents a hydrogen, chlorine or bromine atom or a methyl group.

5. Benzoxazin-2-ones of general formula Ia as claimed in claim 3, wherein

A represents an SO, HN=SO or $CH_3CO$—N=SO group,

D represents an n-butylene group,

$R_1$ represents a phenyl group optionally substituted in the 4-position by a fluorine, chlorine or bromine atom or by a methyl, cyclohexyl, phenyl, hydroxy or methoxy group, or a 3-methoxyphenyl, 3,4-dichlorophenyl, 3,4-dimethoxyphenyl, 4-bromo-3-methyl-phenyl, 4-amino-3,5-dibromo-phenyl or 4-hydroxy-3,5-di-tert.butylphenyl group,

$R_2$ and $R_3$ each represent a hydrogen atom or a methyl group,

$R_5$ represents a hydrogen atom or, in the 7-position, a chlorine or bromine atom and

$R_6$ represents a hydrogen atom.

6. 6-[4-(4-Biphenylsulfinyl)-butoxy]-4,4-dimethyl-4H-3,1-benzoxazin-2-one.

7. 6-[4-(4-Bromophenylsulfinyl)-butoxy]-4,4-dimethyl-4H-3,1-benzoxazin-2-one.

8. Pharmaceutical compositions containing a compound as claimed in claims 1 to 7 together with one or more inert carriers and/or diluents.

9. Process for the preparation of a pharmaceutical composition for the prophylaxis of thromboembolic disorders, for the prophylaxis of arteriosclerosis and for the prophylaxis of metastasis, as claimed in claim 8, characterised in that a compound as claimed in claims 1 to 7 is incorporated in one or more inert carriers by a non-clonic method.

10. Process for the preparation of new benzoxazin-2-ones of general formula I as claimed in claims 1 to 7, characterised in that

a) a hydroxy compound of general formula

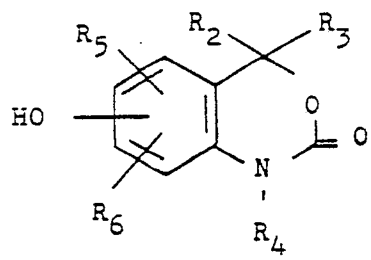

(II)

wherein $R_2$ to $R_6$ are as hereinbefore defined, or a salt thereof with an inorganic or tertiary organic base, is reacted with a compound of general formula

$$Z—D—A—R_1 \quad (III)$$

wherein

A, D and $R_1$ are as hereinbefore defined and

Z represents a nucleophilically exchangeable group, or

b) in order to prepare a compound of general formula I wherein A represents an SO, $SO_2$ or R—N=SO group, a compound of general formula

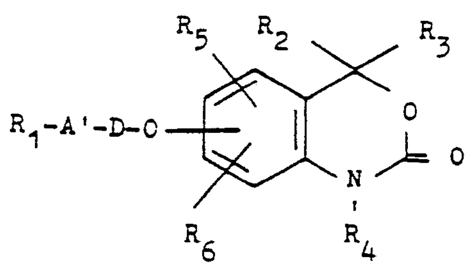

(IV)

wherein

$R_1$ to $R_6$ and D are as hereinbefore defined and

A′ represents a sulphur atom or an SO or R—N=S group wherein R is as hereinbefore defined, is oxidised, or

c) in order to prepare a compound of general formula I wherein A represents a sulphur atom or an $SO_2$ group, a compound of general formula

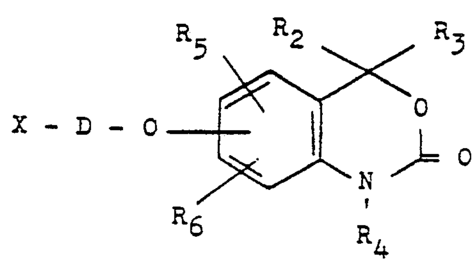

(V)

wherein

$R_2$ to $R_6$ and D are as hereinbefore defined and

X represents a nucleophilically exchangeable group, is reacted with a compound of general formula

$$Y—R_1 \quad (VI)$$

wherein

$R_1$ is as hereinbefore defined and

Y represents an $MeSO_2$ group wherein Me represents an alkali or alkaline earth/$_2$ metal atom, or a mercapto group, or

d) in order to prepare a compound of general formula I wherein A represents an H—N=SO group, a sulphoxide of general formula

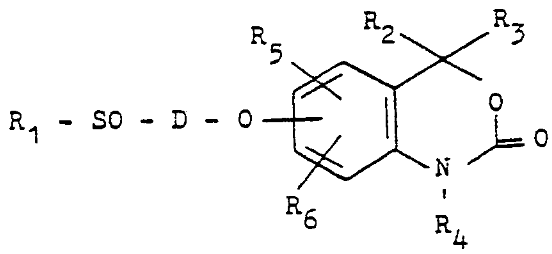

(VII)

wherein $R_1$ to $R_6$ and D are as hereinbefore defined, is reacted with hydrazoic acid optionally formed in the reaction mixture, or

e) in order to prepare a compound of general formula I wherein A represents an H—N=SO group, a sulphoxide of general formula

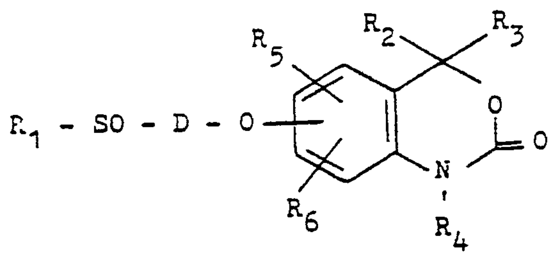

(VII)

wherein $R_1$ to $R_6$ and D are as hereinbefore defined, is reacted with a compound of general formula

$$H_2N—O—X—R_7 \qquad \text{(VIII)}$$

wherein

X represents a carbonyl or sulphonyl group and

$R_7$ represents an aryl group disubstituted in the o-positions, or

f) in order to prepare a compound of general formula I wherein R does not represent a hydrogen atom, a compound of general formula

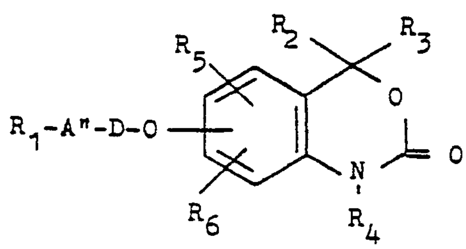

(IX)

wherein

$R_1$ to $R_6$ and D are as hereinbefore defined and

A″ represents an H—N=S or H—N=SO group, is acylated, or

g) in order to prepare a compound of general formula I wherein A represents an R—N=S group, a thioether of general formula

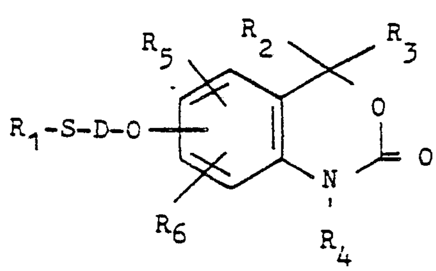

(X)

wherein $R_1$ to $R_6$ and D are as hereinbefore defined, is reacted with a haloamide of general formula

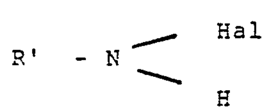

(XI)

wherein

R′ has the meanings given for R hereinbefore, with the exception of the hydrogen atom, and

Hal represents a chlorine or bromine atom, or with an alkali metal salt thereof, and optionally a compound thus obtained is subsequently hydrolysed, or

h) in order to prepare a compound of general formula I wherein A represents an H—N=SO group, an acyl group is split off hydrolytically from a sulphoxide of general formula

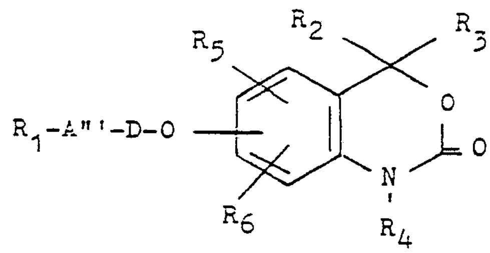

(XII)

wherein

$R_1$ to $R_6$ and D are as hereinbefore defined and

A‴ represents an H—N=S or H—N=SO group substituted by a hydrolytically removable acyl group, or

i) in order to prepare a compound of general I wherein R does not represent a hydrogen atom, a compound of general formula

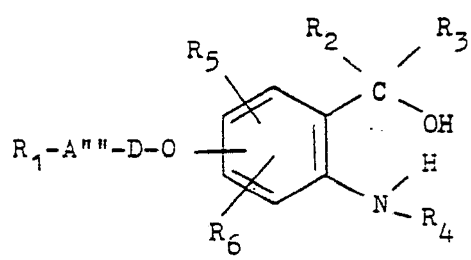

(XIII)

wherein

D and $R_1$ to $R_6$ are as hereinbefore defined and

A⁗ represents a sulphur atom or an SO, $SO_2$, R′—N=S or R′—N=SO group, wherein R′ has the meanings given for R hereinbefore, with the exception of a hydrogen atom, is reacted with a carbonic acid derivative of general formula

X—CO—X (XIV)

wherein X, which may represent identical or different groups, represents a nucleophilic leaving group in each case, and subsequently, if desired, a compound of general formula I obtained according to the invention wherein $R_4$ represents a hydrogen atom is converted by alkylation into a corresponding compound of general formula I wherein $R_4$ represents an alkyl group with 1 to 3 carbon atoms.

**Claims** (for the Contracting State AT)

1. Process for preparing new benzoxazin-2-ones of general formula

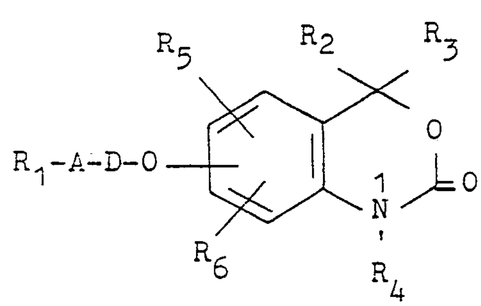

(I)

wherein

A represents a sulphur atom, an SO-, $SO_2$-, R—N=S- or R—N=SO-group, wherein R represents a hydrogen atom, a straight-chained alkanoyl group with 1 to 9 carbon atoms, a pivaloyl group, a benzoyl group optionally substituted by a fluorine, chlorine or bromine atom or by a methyl, ethyl, isopropyl, tert.butyl or acetoxy group, a benzoyl group substituted by two fluorine atoms, two chlorine atoms, two or three methyl groups, a phenylsulphonyl group optionally substituted by a methyl group or by a fluorine, chlorine or bromine atom, a naphthoyl, thenoyl, pyridinoyl, hydroxysulphonyl, methanesulphonyl, ethanolsulphonyl, pentamethyl-phenylsulphonyl, methoxycarbonyl, ethoxycarbonyl, n-propoxy-carbonyl, isopropoxycarbonyl, benzyloxycarbonyl, aminocarbonyl, methylaminocarbonyl or dimethylaminocarbonyl group,

D represents a straight-chained or branched alkylene group with 2 to 6 carbon atoms,

$R_1$ represents an alkyl group with 1 to 3 carbon atoms optionally substituted by a phenyl group, or a phenyl group, whilst the above-mentioned phenyl nuclei may each be substituted by an alkyl group with 1 to 4 carbon atoms, a halogen atom, an alkoxy group with 1 to 3 carbon atoms, a hydroxy, cyclohexyl or phenyl group, an amino group or an alkanoylamino group with 1 to 3 carbon atoms ; an alkyl group with 4 to 8 carbon atoms, a cycloalkyl group with 3 to 7 carbon atoms, a phenyl group di- or trisubstituted, or a hydroxyphenyl or aminophenyl group mono- or disubstituted, by alkyl groups with 1 to 4 carbon atoms, alkoxy groups with 1 to 3 carbon atoms and/or halogen atoms, whilst the substituents of the phenyl nucleus may be identical or different, a pyridyl or pyrimidinyl group optionally substituted by one or two methyl groups, or a pyridyl-N-oxide, ethyl-pyridyl, ethyl-pyrimidinyl- propyl-pyrimidinyl, diethyl-pyrimidinyl, pyrazinyl, pyridazinyl, pyrazolyl, imidazolyl, triazolyl, indolyl, indazolyl, quinolyl, isoquinolyl, quinazolinyl, benzimidazolyl or benzthiazolyl group,

$R_2$ and $R_3$, which may be identical or different, represent hydrogen atoms, phenyl groups, alkyl groups with 1 to 6 carbon atoms or cycloalkyl groups with 3 to 7 carbon atoms,

$R_4$ represents a hydrogen atom or an alkyl group with 1 to 3 carbon atoms,

$R_5$ represents a hydrogen or halogen atom, a nitro or alkyl group with 1 to 3 carbon atoms and

$R_6$ represents a hydrogen or halogen atom or an alkyl group with 1 to 3 carbon atoms,

characterised in that

a) a hydroxy compound of general formula

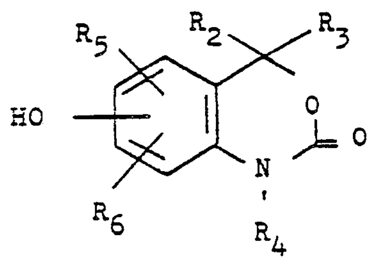

(II)

wherein $R_2$ to $R_6$ are as hereinbefore defined, or a salt thereof with an inorganic or tertiary organic base, is reacted with a compound of general formula

$$Z—D—A—R_1 \qquad (III)$$

wherein

A, D and $R_1$ are as hereinbefore defined and

Z represents a nucleophilically exchangeable group, or

b) in order to prepare a compound of general formula I wherein A represents an SO, $SO_2$ or R—N=SO group, a compound of general formula

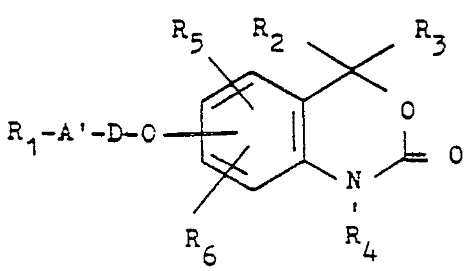

(IV)

wherein

$R_1$ to $R_6$ and D are as hereinbefore defined and

A' represents a sulphur atom or an SO or R—N=S group wherein R is as hereinbefore defined, is oxidised, or

c) in order to prepare a compound of general formula I wherein A represents a sulphur atom or an

$SO_2$ group, a compound of general formula

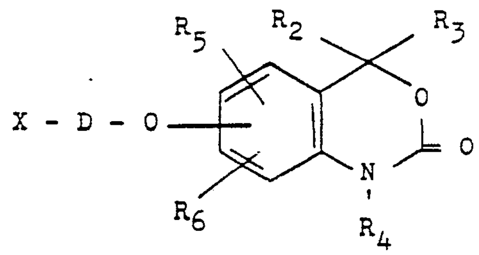

(V)

wherein

$R_2$ to $R_6$ and D are as hereinbefore defined and

X represents a nucleophilically exchangeable group, is reacted with a compound of general formula

$$Y—R_1 \quad (VI)$$

wherein

$R_1$ is as hereinbefore defined and

Y represents an $MeSO_2$ group wherein Me represents an alkali or alkaline earth/$_2$ metal atom, or a mercapto group, or

d) in order to prepare a compound of general formula I wherein A represents an H—N=SO group, a sulphoxide of general formula

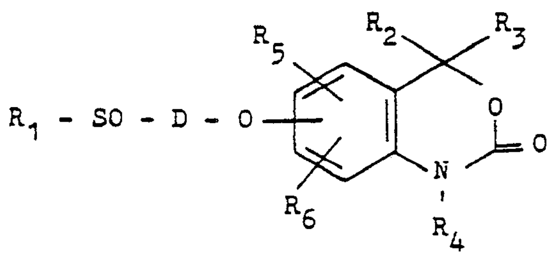

(VII)

wherein $R_1$ to $R_6$ and D are as hereinbefore defined, is reacted with hydrazoic acid optionally formed in the reaction mixture, or

e) in order to prepare a compound of general formula I wherein A represents an H—N=SO group, a sulphoxide of general formula

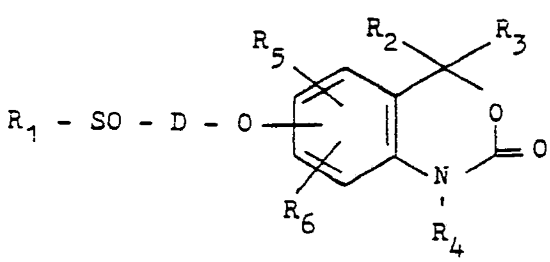

(VII)

wherein $R_1$ to $R_6$ and D are as hereinbefore defined, is reacted with a compound of general formula

$$H_2N—O—X—R_7 \quad (VIII)$$

wherein

X represents a carbonyl or sulphonyl group and

$R_7$ represents an aryl group disubstituted in the o-positions, or

f) in order to prepare a compound of general formula I wherein R does not represent a hydrogen atom, a compound of general formula

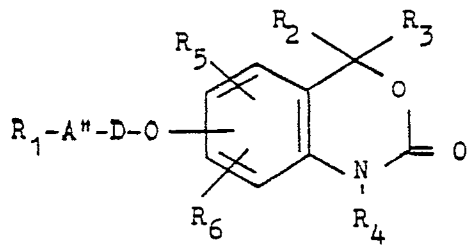

(IX)

wherein

$R_1$ to $R_6$ and D are as hereinbefore defined and

A″ represents an H—N=S or H—N=SO group, is acylated, or

g) in order to prepare a compound of general formula I wherein A represents an R—N=S group, a thioether of general formula

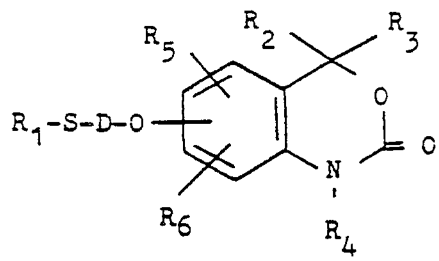

(X)

wherein $R_1$ to $R_6$ and D are as hereinbefore defined, is reacted with a haloamide of general formula

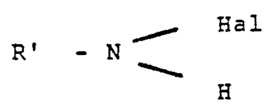

(XI)

wherein

R′ has the meanings given for R hereinbefore, with the exception of the hydrogen atom, and

Hal represents a chlorine or bromine atom, or with an alkali metal salt thereof, and optionally a compound thus obtained is subsequently hydrolysed, or

h) in order to prepare a compound of general formula I wherein A represents an H—N=SO group, an acyl group is split off hydrolytically from a sulphoxide of general formula

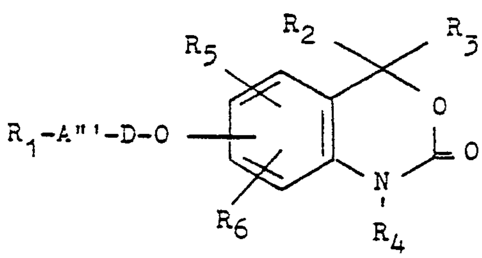

(XII)

wherein

$R_1$ to $R_6$ and D are as hereinbefore defined and

A‴ represents an H—N=S or H—N=SO group substituted by a hydrolytically removable acyl group, or

i) in order to prepare a compound of general I wherein R does not represent a hydrogen atom, a compound of general formula

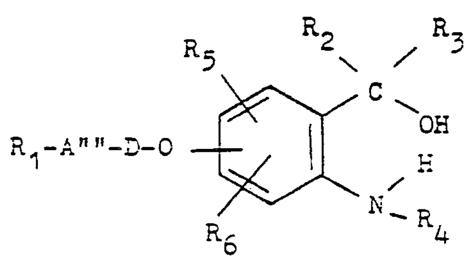

(XIII)

wherein

D and $R_1$ to $R_6$ are as hereinbefore defined and

A⁗ represents a sulphur atom or an SO, $SO_2$, R′—N=S or R′—N=SO group, wherein R′ has the meanings given for R hereinbefore, with the exception of a hydrogen atom, is reacted with a carbonic acid

derivative of general formula

$$X—CO—X \quad (XIV)$$

wherein X, which may represent identical or different groups, represents a nucleophilic leaving group in each case, and subsequently, if desired, a compound of general formula I obtained according to the invention wherein $R_4$ represents a hydrogen atom is converted by alkylation into a corresponding compound of general formula I wherein $R_4$ represents an alkyl group with 1 to 3 carbon atoms.

2. Process as claimed in claim 1 for preparing new benzoxazin-2-ones of general formula

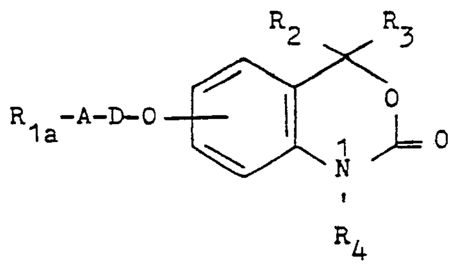

(Ia)

wherein $R_2$ to $R_4$, A and D are defined as in claim 1 and $R_{1a}$ represents an alkyl group with 1 to 3 carbon atoms optionally substituted by a phenyl group, or a phenyl group, whilst the above-mentioned phenyl nuclei may each be substituted by an alkyl group with 1 to 4 carbon atoms, a halogen atom, an alkoxy group with 1 to 3 carbon atoms, a hydroxy, cyclohexyl or phenyl group or an alkanoylamino group with 1 to 3 carbon atoms, an alkyl group with 4 to 8 carbon atoms, a cycloalkyl group with 3 to 7 carbon atoms, a phenyl group di- or trisubstituted or a hydroxy or aminophenyl group disubstituted by alkyl groups with 1 to 4 carbon atoms, alkoxy groups with 1 to 3 carbon atoms and/or halogen atoms, whilst the substituents of the phenyl nucleus may be identical of different, or a pyridyl group, characterised in that

a) in order to prepare a compound of general formula Ia wherein A represents a sulphur atom, an R—N=S-, SO- or $SO_2$-group, a hydroxy compound of general formula

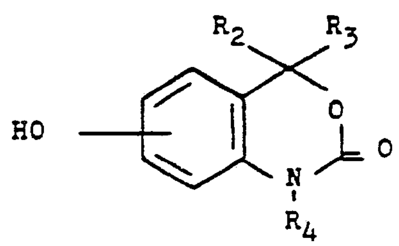

(IIa)

wherein $R_2$ to $R_4$ are defined as in claim 1, or the salts thereof with an inorganic or tertiary organic base, is reacted with a compound of general formula

$$Z—D—A_a—R_{1a} \quad (IIIa)$$

wherein

D and Z are defined as in claim 1,

$R_{1a}$ has the meanings given hereinbefore and

$A_a$ represents a sulphur atom, an R—N=S-, SO- or $SO_2$-group, wherein R is defined as in claim 1, or

b) in order to prepare a compound of general formula Ia wherein A represents an SO, $SO_2$ or R—N=SO group, a compound of general formula

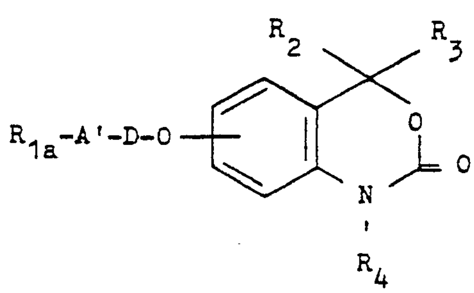

(IVa)

wherein

$R_2$ to $R_4$, A' and D are defined as in claim 1 and

$R_{1a}$ has the meanings given hereinbefore, is oxidised or

c) in order to prepare the compound of general formula Ia wherein A represents a sulphur atom or an $SO_2$ group, a compound of general formula

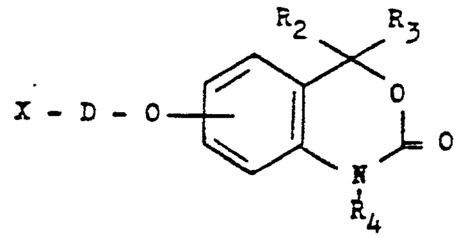

(Va)

wherein $R_2$ to $R_4$, D and X are defined as in claim 1, is reacted with a compound of general formula

$$Y—R_{1a} \qquad (VIa)$$

wherein

Y is defined as in claim 1 and

$R_{1a}$ is as hereinbefore defined, or

d) in order to prepare a compound of general formula Ia wherein A represents an H—N=SO group, a sulphoxide of general formula

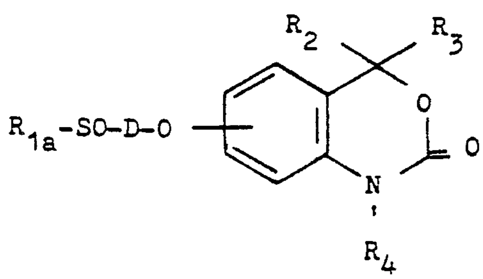

(VIIa)

wherein

$R_2$ to $R_4$ and D are defined as in claim 1 and

$R_{1a}$ is as hereinbefore defined, is reacted with hydrazoic acid optionally formed in the reaction mixture, or

e) in order to prepare a compound of general formula Ia wherein A represents an H—N=SO group, a sulphoxide of general formula

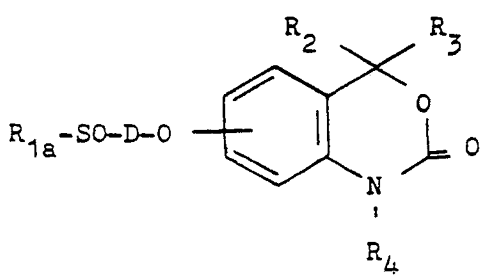

(VIIa)

wherein

$R_2$ to $R_4$ and D are defined as in claim 1 and

$R_{1a}$ has the meanings given hereinbefore, is reacted with a compound of general formula

$$H_2N—O—X—R_7 \qquad (VIII)$$

wherein $R_7$ and X are defined as in claim 1, or

f) in order to prepare a compound of general formula Ia wherein R does not represent a hydrogen atom, a compound of general formula

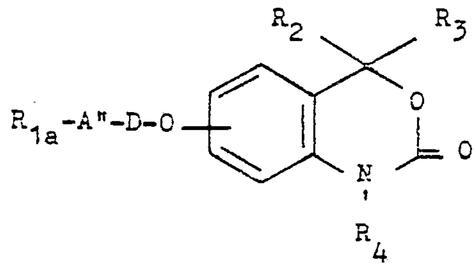

(IXa)

wherein

$R_2$ to $R_4$, A″ and D are defined as in claim 1 and

$R_{1a}$ has the meanings given hereinbefore, is acylated or

g) in order to prepare a compound of general formula Ia wherein A represents an R—N=S group, a thioether of general formula

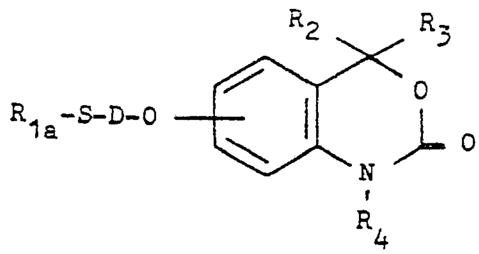

(Xa)

wherein

$R_2$ to $R_4$ and D are defined as in claim 1 and

$R_{1a}$ has the meanings given hereinbefore, is reacted with a haloamide of general formula

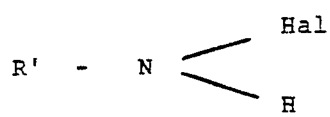

(XI)

wherein R′ and Hal are defined as in claim 1, or with an alkali metal salt thereof, and subsequently, if desired, the compound thus obtained is hydrolysed, and subsequently, if desired, a compound of general formula I obtained according to the invention wherein $R_4$ represents a hydrogen atom, is converted by alkylation into a corresponding compound of general formula I wherein $R_4$ represents an alkyl group with 1 to 3 carbon atoms.

3. Process as claimed in claims 1 and 2, characterised in that the reaction is carried out in a solvent.

4. Process as claimed in claims 1a, 2a and 3, characterised in that the reaction is carried out in the presence of an alkali metal base or an alkoxide.

5. Process as claimed in claims 1b, 2b and 3, characterised in that, in order to prepare a compound of general formula I wherein A represents an SO or R—N=SO group, the oxidation is carried out with one equivalent of the oxidising agent used.

6. Process as claimed in claims 1b, 2b and 3, characterised in that, in order to prepare a compound of general formula I wherein A represents an $SO_2$ group, the oxidation is carried out with two equivalents of the oxidising agent when starting from a thioether of general formula I and with one equivalent of the oxidising agent when starting from a sulphinyl compound of general formula I.

7. Process as claimed in claims 1c, 2c, 1g, 2g and 3, characterised in that the reaction is carried out in the presence of an alkali metal base.

8. Process as claimed in claims 1e, 2e, 3 and 12, characterised in that a compound of general formula VIII is prepared in the reaction mixture.

9. Process as claimed in claims 1f, 2f and 3, characterised in that the reaction is carried out in the presence of an inorganic or tertiary organic base.

10. Process as claimed in claims 1h and 3, characterised in that an acyl group of a carboxylic acid or of a carbonic acid derivative is used as the acyl group.

**Revendications** (pour les Etats contractants : BE, CH, DE, FR, IT, LI, LU, NL, SE)

1. Benzoxazine-2-ones de formule générale

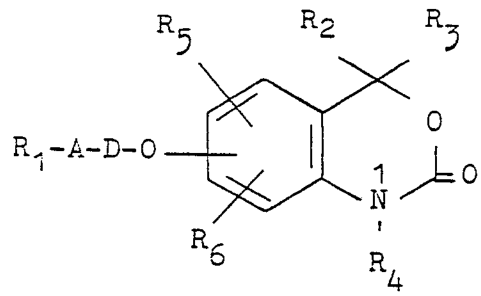

(I)

dans laquelle

A représente un atome de soufre, un groupe —SO—, —$SO_2$—, R—N=S— ou R—N=SO—, R représentant un atome d'hydrogène, un groupe alcanoyle rectiligne avec 1 à 9 atomes de carbone, un groupe pivaloyle, un groupe benzoyle éventuellement substitué par un atome de fluor, de chlore ou de brome, un groupe méthyle, éthyle, isopropyle, tert.butyle ou acétoxy, ou représentant un groupe benzoyle substitué par deux atomes de fluor, deux atomes de chlore, deux ou trois groupes méthyle, ou représentant un groupe phénylsulfonyle éventuellement substitué par un groupe méthyle, un atome de fluor, de chlore ou de brome, ou représentant un groupe naphtoyle, thénoyle, pyridinoyle, hydroxysulfonyle, méthanesulfonyle, éthanolsulfonyle, pentaméthyl-phénylsulfonyle, méthoxycarbonyle, éthoxycarbonyle, n-propoxycarbonyle, isopropoxycarbonyle, benzyloxycarbonyle, aminocarbonyle, méthylaminocarbonyle ou diméthylaminocarbonyle,

D représente un groupe alcoylène rectiligne ou ramifié avec 2 à 6 atomes de carbone,

$R_1$ représente un groupe alcoyle avec 1 à 3 atomes de carbone, éventuellement substitué par un groupe phényle ou représente un groupe phényle, les noyaux phényle mentionnés plus haut pouvant dans chaque cas être substitués par un groupe alcoyle avec 1 à 4 atomes de carbone, un atome d'halogène, un groupe alcoxy avec 1 à 3 atomes de carbone, un groupe hydroxy, cyclohexyle ou phényle, un groupe amino ou un groupe alcanoylamino avec 1 à 3 atomes de carbone, ou représente un groupe alcoyle avec 4 à 8 atomes de carbone, un groupe cycloalcoyle avec 3 à 7 atomes de carbone, un groupe phényle di- ou trisubstitué ou hydroxyphényle ou aminophényle mono- ou disubstitué par des groupes alcoyle avec 1 à 4 atomes de carbone, des groupes alcoxy avec 1 à 3 atomes de carbone et/ou des atomes d'halogène, les substituants du noyau phényle pouvant dans chaque cas être identiques ou différents, ou représente un groupe pyridyle ou pyrimidinyle éventuellement substitué par un ou deux groupes méthyle, ou représente un groupe pyridyl-N-oxyde, éthylpyridyle, éthylpyrimidinyle, propylpyrimidinyle, diéthylpyrimidinyle, pyrazinyle, pyridazinyle, pyrazolyle, imidazolyle, triazolyle, indolyle, indazolyle, quinoléyle, isoquinoléyle, quinazolinyle, benzimidazolyle ou benzthiazolyle,

$R_2$ et $R_3$ qui peuvent être identiques ou différents, représentent des atomes d'hydrogène, des groupes phényle, des groupes alcoyle avec 1 à 6 atomes de carbone ou des groupes cycloalcoyle avec 3 à 7 atomes de carbone,

$R_4$ représente un atome d'hydrogène ou un groupe alcoyle avec 1 à 3 atomes de carbone,

$R_5$ représente un atome d'hydrogène ou d'halogène, un groupe nitro ou alcoyle avec 1 à 3 atomes de carbone et

$R_6$ représente un atome d'hydrogène ou d'halogène ou un groupe alcoyle avec 1 à 3 atomes de carbone.

2. Benzoxazine-2-ones de formule générale I selon la revendication 1, dans laquelle

A représente un atome de soufre, un groupe —SO—, —$SO_2$—, R—N=S— ou R—N=SO—, R représentant un atome d'hydrogène, un radical benzoyle ou phénylsulfonyle éventuellement substitué par un groupe méthyle, ou représente un groupe acétyle ou propionyle,

D représente un groupe alcoylène rectiligne avec 2 à 6 atomes de carbone,

$R_1$ représente un groupe alcoyle avec 1 à 8 atomes de carbone, un groupe cyclohexyle, benzyle, pyridyle, pyridyl-N-oxyde, 2-benzthiazolyle ou 1,2,4-triazol-3-yle, un groupe 2-pyrimidinyle éventuellement substitué par un ou deux groupes méthyle, un groupe phényle éventuellement substitué par un groupe alcoyle avec 1 à 4 atomes de carbone, un groupe hydroxy, méthoxy, cyclohexyle, phényle ou acétylamino, un atome de fluor, de chlore ou de brome, ou représente un groupe phényle disubstitué par des atomes de chlore ou de brome, des groupes méthyle ou méthoxy, les substituants du noyau phényle pouvant être identiques ou différents, ou représente un groupe aminophényle ou hydroxyphényle substitué par deux groupes alcoyle avec chacun 1 à 4 atomes de carbone, deux atomes de chlore ou de brome,

$R_2$ et $R_3$ qui peuvent être identiques ou différents, représentent des atomes d'hydrogène, des groupes alcoyle avec 1 à 6 atomes de carbone, des groupes phényle ou cyclohexyle,

$R_4$ représente un atome d'hydrogène ou un groupe méthyle,

$R_5$ représente un atome d'hydrogène, de fluor, de chlore ou de brome, un groupe nitro, méthyle ou éthyle et

$R_6$ représente un atome d'hydrogène, de chlore ou de brome, un groupe méthyle ou éthyle.

3. Benzoxazine-2-ones de formule générale

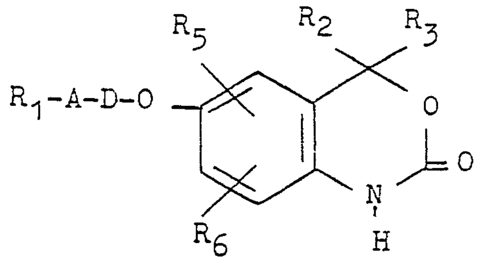

(Ia)

dans laquelle A, D, $R_1$ à $R_3$, $R_5$ et $R_6$ sont définis comme dans la revendication 2.

4. Benzoxazine-2-ones de formule générale Ia selon la revendication 3, dans laquelle

A représente le groupe —SO—, —$SO_2$—, H—N=SO—, $CH_3CO$—N=SO— et $CH_3$—$C_6H_4$—$SO_2$—N=SO—,

D représente le groupe n-butylène,

$R_1$ représente un groupe phényle éventuellement substitué par un atome de fluor, de chlore ou de brome, un groupe méthyle, hydroxy, méthoxy, cyclohexyle, phényle ou acétamino, ou représente un groupe phényle disubstitué par des atomes de chlore ou de brome, des groupes méthyle ou méthoxy, les substituants du noyau phényle pouvant être identiques ou différents, ou représente un groupe 4-amino-3,5-dibromophényle, un groupe 3,5-di-tert.butyl-4-hydroxy-phényle ou pyridyle,

$R_2$ et $R_3$ qui peuvent être identiques ou différents, représentent chacun un atome d'hydrogène ou un groupe méthyle,

$R_5$ représente un atome d'hydrogène, de chlore ou de brome, un groupe nitro ou un groupe méthyle et

$R_6$ représente un atome d'hydrogène, de chlore ou de brome ou un groupe méthyle.

5. Benzoxazine-2-ones de formule générale Ia selon la revendication 3, dans laquelle

A représente le groupe —SO—, HN=SO— ou $CH_3CO$—N=SO—,

D représente le groupe n-butylène,

$R_1$ représente un groupe phényle éventuellement substitué en position 4 par un atome de fluor, de chlore ou de brome, un groupe méthyle, cyclohexyle, phényle, hydroxy ou méthoxy, ou représente un groupe 3-méthoxyphényle, 3,4-dichlorophényle, 3,4-diméthoxyphényle, 4-bromo-3-méthylphényle, 4-amino-3,5-dibromophényle ou 4-hydroxy-3,5-di-tert.butylphényle,

$R_2$ et $R_3$ représentent chacun un atome d'hydrogène ou un groupe méthyle,

$R_5$ représente un atome d'hydrogène ou, en position 7, un atome de chlore ou de brome et

$R_6$ représente un atome d'hydrogène.

6. La 6-[4-(4-biphénylylsulfinyl)-butoxy]-4,4-diméthyl-4H-3,1-benzoxazine-2-one.

7. La 6-[4-(4-bromo-phénylsulfinyl)-butoxy]-4,4-diméthyl-4H-3,1-benzoxazine-2-one.

8. Médicament contenant un composé selon les revendications 1 à 7 conjointement à un ou plusieurs excipients et/ou diluants inertes.

9. Procédé pour la préparation d'un médicament pour le traitement ou pour la prophylaxie des maladies thrombo-emboliques, pour la prophylaxie de l'artérioschlérose et pour la prophylaxie des métastases selon la revendication 8, caractérisé en ce que l'on introduit, selon une voie non clonique, un composé selon les revendications 1 à 7 dans un ou plusieurs excipients inertes.

10. Procédé pour la préparation des nouvelles benzoxazine-2-ones de formule générale I selon les revendications 1 à 7, caractérisé en ce que

a) on fait réagir un composé hydroxy de formule générale

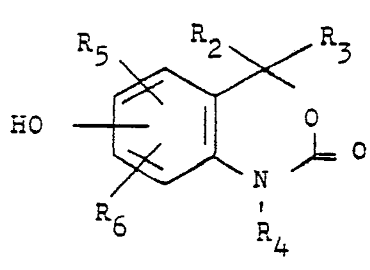

(II)

dans laquelle $R_2$ à $R_6$ sont définis comme au début, ou son sel avec une base minérale ou organique tertiaire, avec un composé de formule générale

$$Z—D—A—R_1 \qquad (III)$$

dans laquelle

A, D et $R_1$ sont définis comme au début et

Z représente un groupe échangeable de façon nucléophile ou

b) pour la préparation d'un composé de formule générale I dans laquelle A représente un groupe —SO—, —$SO_2$— ou R—N=SO—, on oxyde un composé de formule générale

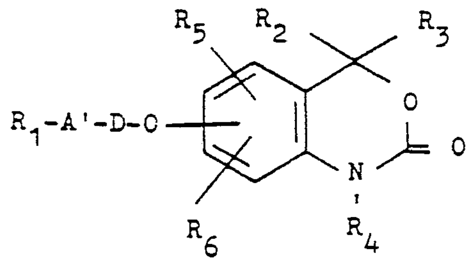

(IV)

dans laquelle

$R_1$ à $R_6$ et D sont définis comme au début et

A' représente un atome de soufre, un groupe —SO— ou R—N=S—, R étant défini comme au début, ou

c) pour la préparation d'un composé de formule générale I dans laquelle A représente un atome de soufre ou un groupe —$SO_2$—, on fait réagir un composé de formule générale

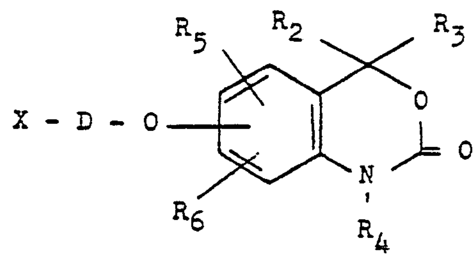

(V)

dans laquelle

$R_2$ à $R_6$ et D sont définis comme au début et

X représente un groupe échangeable nucléophile, avec un composé de formule générale

$$Y—R_1 \qquad (VI)$$

dans laquelle

$R_1$ est défini comme au début et

Y représente un groupe $MeSO_2$—, Me représentant un atome de métal alcalin ou alcalino-terreux/$_2$, ou représente le groupe mercapto ou

d) pour la préparation d'un composé de formule générale I dans laquelle A représente le groupe H—N=SO—, on fait réagir un sulfoxyde de formule générale

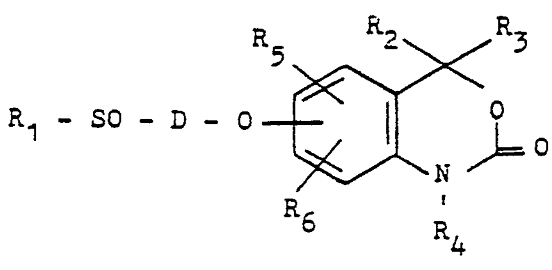

(VII)

dans laquelle $R_1$ à $R_6$ et D sont définis comme au début, avec de l'acide hydrazoïque éventuellement formé dans le mélange réactionnel ou

e) pour la préparation d'un composé de formule générale I dans laquelle A représente le groupe H—N=SO—, on fait réagir un sulfoxyde de formule générale

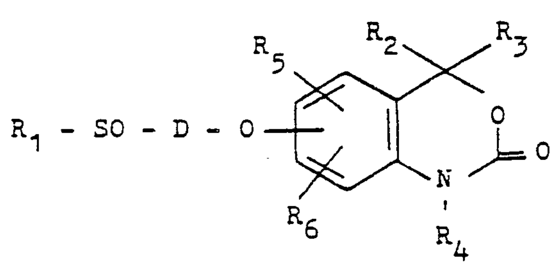

(VII)

dans laquelle $R_1$ à $R_6$ et D sont définis comme au début, avec un composé de formule générale

$$H_2N—O—X—R_7 \qquad (VIII)$$

dans laquelle

X représente un groupe carbonyle ou sulfonyle et

$R_7$ représente un groupe aryle disubstitué en position o ou

f) pour la préparation d'un composé de formule générale I dans laquelle R ne représente pas un atome d'hydrogène, on acyle un composé de formule générale

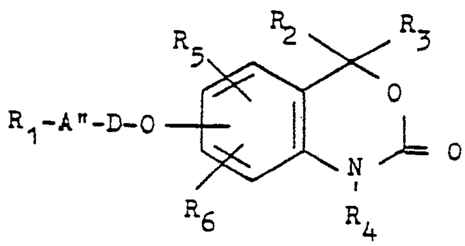

(IX)

dans laquelle

$R_1$ à $R_6$ et D sont définis comme au début et

A″ représente le groupe H—N=S— ou H—N=SO— ou

g) pour la préparation d'un composé de formule générale I dans laquelle A représente le groupe R—N=S—, on fait réagir un thioéther de formule générale

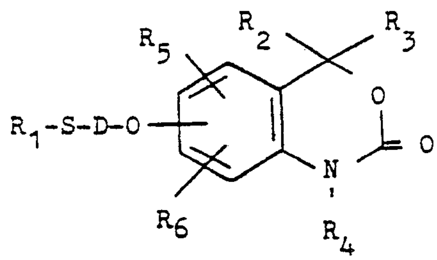

(X)

dans laquelle $R_1$ à $R_6$ et D sont définis comme au début, avec un halogénoamide de formule générale

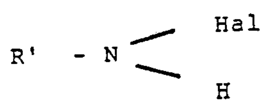

(XI)

dans laquelle

R′ possède les significations mentionnées au début pour R à l'exception de l'atome d'hydrogène et

Hal représente un atome de chlore ou de brome, ou avec son sel alcalin et éventuellement on hydrolyse ensuite un composé ainsi obtenu ou

h) pour la préparation d'un composé de formule générale I dans laquelle A représente le groupe H—N=SO—, on clive hydrolytiquement un radical acyle à partir d'un sulfoxyde de formule générale

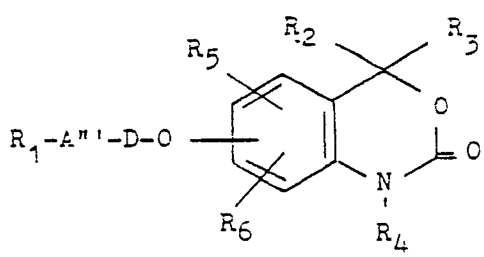

(XII)

dans laquelle

$R_1$ à $R_6$ et D sont définis comme au début et

A‴ représente un groupe H—N=S— ou H—N=SO— substitué par un radical acyle clivable hydrolytiquement ou

i) pour la préparation d'un composé de formule générale I dans laquelle R ne représente pas un

atome d'hydrogène, on fait réagir un composé de formule générale

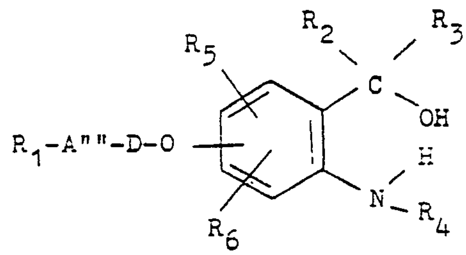

(XIII)

dans laquelle

D et $R_1$ à $R_6$ sont définis comme au début et

A'''' représente un atome de soufre, un groupe —SO—, —$SO_2$—, R'—N=S— ou R'—N=SO—, R' possédant les significations mentionnées au début pour R à l'exception d'un atome d'hydrogène, avec un dérivé d'acide carbonique de formule générale

X—CO—X (XIV)

dans laquelle X qui peuvent être identiques ou différents, représentent chacun un groupe partant nucléophile et, si on le désire, on transforme ensuite un composé obtenu selon l'invention, de formule générale I dans laquelle $R_4$ représente un atome d'hydrogène, au moyen d'une alcoylation, en un composé correspondant de formule générale I dans laquelle $R_4$ représente un groupe alcoyle avec 1 à 3 atomes de carbone.

**Revendications** (pour l'Etat contractant AT)

1. Procédé pour la préparation de nouvelles benzoxazine-2-ones de formule générale

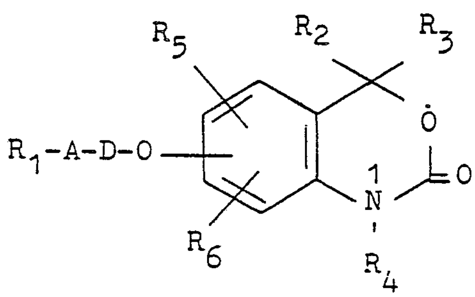

(I)

dans laquelle

A représente un atome de soufre, un groupe —SO—, —$SO_2$—, R—N=S— ou R—N=SO—, R représentant un atome d'hydrogène, un groupe alcanoyle rectiligne avec 1 à 9 atomes de carbone, un groupe pivaloyle, un groupe benzoyle éventuellement substitué par un atome de fluor, de chlore ou de brome, un groupe méthyle, éthyle, isopropyle, tert.butyle ou acétoxy, ou représentant un groupe benzoyle substitué par deux atomes de fluor, deux atomes de chlore, deux ou trois groupes méthyle, ou représentant un groupe phénylsulfonyle éventuellement substitué par un groupe méthyle, un atome de fluor, de chlore ou de brome, ou représentant un groupe naphtoyle, thénoyle, pyridinoyle, hydroxysulfonyle, méthanesulfonyle, éthanolsulfonyle, pentaméthylphénylsulfonyle, méthoxycarbonyle, éthoxycarbonyle, n-propoxycarbonyle, isopropoxycarbonyle, benzyloxycarbonyle, aminocarbonyle, méthylaminocarbonyle ou diméthylaminocarbonyle.

D représente un groupe alcoylène rectiligne ou ramifié avec 2 à 6 atomes de carbone.

$R_1$ représente un groupe alcoyle avec 1 à 3 atomes de carbone, éventuellement substitué par un groupe phényle ou représente un groupe phényle les noyaux phényle mentionnés plus haut pouvant dans chaque cas être substitués par un groupe alcoyle avec 1 à 4 atomes de carbone, un atome d'halogène, un groupe alcoxy avec 1 à 3 atomes de carbone, un groupe hydroxy, cyclohexyle ou phényle, un groupe amino ou un groupe alcanoylamino avec 1 à 3 atomes de carbone, ou représente un groupe alcoyle avec 4 à 8 atomes de carbone, un groupe cycloalcoyle avec 3 à 7 atomes de carbone, un groupe phényle di- ou trisubstitué ou hydroxyphényle ou aminophényle mono- ou disubstitué par des groupes alcoyle avec 1 à 4 atomes de carbone, des groupes alcoxy avec 1 à 3 atomes de carbone et/ou des atomes d'halogène, les substituants du noyau phényle pouvant dans chaque cas être identiques ou différents, ou représente un groupe pyridyle ou pyrimidinyle éventuellement substitué par un ou deux groupes méthyle, ou représente un groupe pyridyl-N-oxyde, éthylpyridyle, éthylpyrimidinyle, propylpyrimidinyle, diéthylpyrimidinyle, pyrazinyle, pyridazinyle, pyrazolyle, imidazolyle, triazolyle, indolyle, indazolyle, quinoléyle, isoquinoléyle,

quinazolinyle, benzimidazolyle ou benzthiazolyle,

$R_2$ et $R_3$ qui peuvent être identiques ou différents, représentent des atomes d'hydrogène, des groupes phényle, des groupes alcoyle avec 1 à 6 atomes de carbone ou des groupes cycloalcoyle avec 3 à 7 atomes de carbone,

$R_4$ représente un atome d'hydrogène ou un groupe alcoyle avec 1 à 3 atomes de carbone,

$R_5$ représente un atome d'hydrogène ou d'halogène, un groupe nitro ou alcoyle avec 1 à 3 atomes de carbone et

$R_6$ représente un atome d'hydrogène ou d'halogène ou un groupe alcoyle avec 1 à 3 atomes de carbone, caractérisé en ce que

a) on fait réagir un composé hydroxy de formule générale

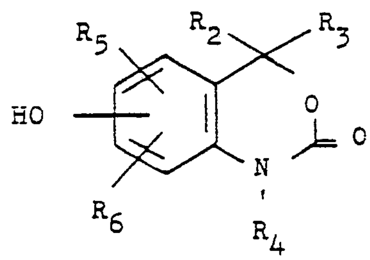

(II)

dans laquelle

$R_2$ à $R_6$ sont définis comme au début, ou son sel avec une base minérale ou organique tertiaire, avec un composé de formule générale

$$Z—D—A—R_1 \qquad (III)$$

dans laquelle

A, D et $R_1$ sont définis comme au début et

Z représente un groupe échangeable de façon nucléophile ou

b) pour la préparation d'un composé de formule générale I dans laquelle A représente un groupe —SO—, —$SO_2$— ou R—N=SO—, on oxyde un composé de formule générale

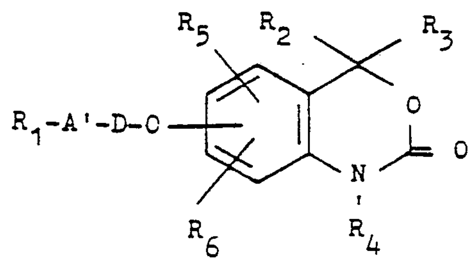

(IV)

dans laquelle

$R_1$ à $R_6$ et D sont définis comme au début et

A' représente un atome de soufre, un groupe —SO— ou R—N=S—, R étant défini comme au début ou

c) pour la préparation d'un composé de formule générale I dans laquelle A représente un atome de soufre ou un groupe —$SO_2$—, on fait réagir un composé de formule générale

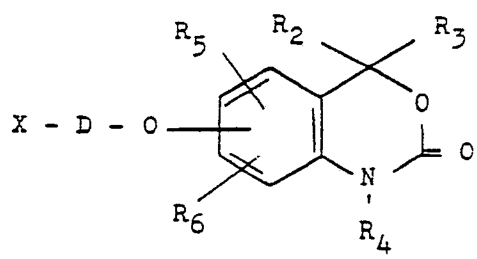

(V)

dans laquelle

$R_2$ à $R_6$ et D sont définis comme au début et

X représente un groupe échangeable nucléophile, avec un composé de formule générale

$$Y—R_1 \qquad (VI)$$

dans laquelle

$R_1$ est défini comme au début et

Y représente un groupe $MeSO_2$—, Me représentant un atome de métal alcalin ou alcalino-terreux/$_2$, ou représente le groupe mercapto ou

d) pour la préparation d'un composé de formule générale I dans laquelle A représente le groupe H—N=SO—, on fait réagir un sulfoxyde de formule générale

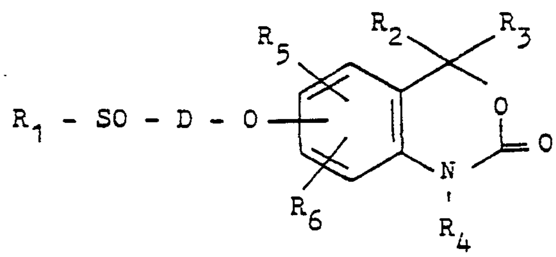

(VII)

dans laquelle $R_1$ à $R_6$ et D sont définis comme au début, avec de l'acide hydrazoïque éventuellement formé dans le mélange réactionnel ou

e) pour la préparation d'un composé de formule générale I dans laquelle A représente le groupe H—N=SO—, on fait réagir un sulfoxyde de formule générale

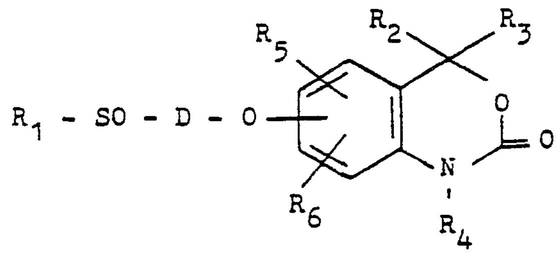

(VII)

dans laquelle $R_1$ à $R_6$ et D sont définis comme au début, avec un composé de formule générale

$$H_2N—O—X—R_7 \qquad (VIII)$$

dans laquelle

X représente un groupe carbonyle ou sulfonyle et

$R_7$ représente un groupe aryle disubstitué en position o ou

f) pour la préparation d'un composé de formule générale I dans laquelle R ne représente pas un atome d'hydrogène, on acyle un composé de formule générale

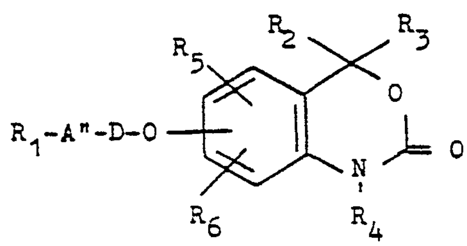

(IX)

dans laquelle

$R_1$ à $R_6$ et D sont définis comme au début et

A″ représente le groupe H—N=S— ou H—N=SO— ou

g) pour la préparation d'un composé de formule générale I dans laquelle A représente le groupe R—N=S—, on fait réagir un thioéther de formule générale

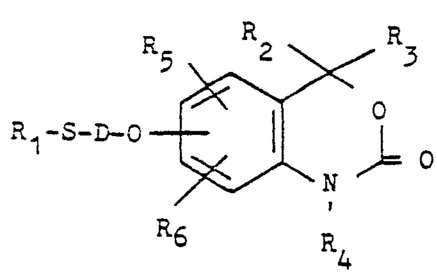

(X)

dans laquelle $R_1$ à $R_6$ et D sont définis comme au début, avec un halogénoamide de formule générale

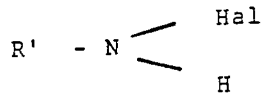

(XI)

dans laquelle

R′ possède les significations mentionnées au début pour R à l'exception de l'atome d'hydrogène et Hal représente un atome de chlore ou de brome, ou avec son sel alcalin et éventuellement on hydrolyse ensuite un composé ainsi obtenu ou

h) pour la préparation d'un composé de formule générale I dans laquelle A représente le groupe H—N=SO—, on clive hydrolytiquement un radical acyle à partir d'un sulfoxyde de formule générale

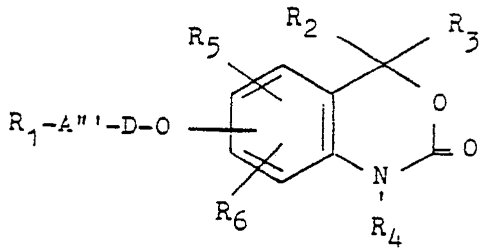

(XII)

dans laquelle

$R_1$ à $R_6$ et D sont définis comme au début et

A‴ représente un groupe H—N=S— ou H—N=SO— substitué par un radical acyle clivable hydrolytiquement ou

i) pour la préparation d'un composé de formule générale I dans laquelle R ne représente pas un atome d'hydrogène, on fait réagir un composé de formule générale

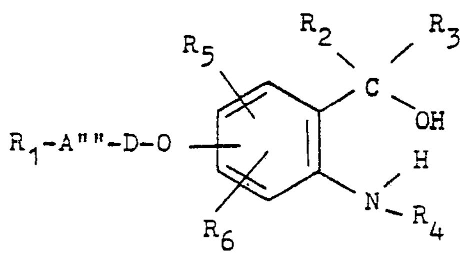

(XIII)

dans laquelle

D et $R_1$ à $R_6$ sont définis comme au début et

A⁗ représente un atome de soufre, un groupe —SO—, —$SO_2$—, R′—N=S— ou R′—N=SO—, R′ possédant les significations mentionnées au début pour R à l'exception d'un atome d'hydrogène, avec un dérivé d'acide carbonique de formule générale

X—CO—X (XIV)

dans laquelle X qui peuvent être identiques ou différents, représentent chacun un groupe partant nucléophile et si on le désire on transforme ensuite un composé obtenu selon l'invention, de formule générale I dans laquelle $R_4$ représente un atome d'hydrogène, au moyen d'une alcoylation, en un composé correspondant de formule générale I dans laquelle $R_4$ représente un groupe alcoyle avec 1 à 3 atomes de carbone.

2. Procédé selon la revendication 1 pour la préparation des nouvelles benzoxazine-2-ones de formule générale

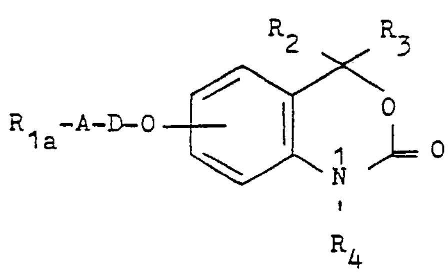

(Ia)

dans laquelle $R_2$ à $R_4$, A et D sont définis comme dans la revendication 1 et $R_{1a}$ représente un groupe alcoyle avec 1 à 3 atomes de carbone, éventuellement substitué par un groupe phényle ou représente un groupe phényle, les noyaux phényle mentionnés plus haut pouvant être substitués chacun par un groupe alcoyle avec 1 à 4 atomes de carbone, un atome d'halogène, un groupe alcoxy avec 1 à 3 atomes de carbone, un groupe hydroxy, cyclohexyle ou phényle ou un groupe alcanoylamino avec 1 à 3 atomes de carbone, ou représente un groupe alcoyle avec 4 à 8 atomes de carbone, un groupe cycloalcoyle avec 3 à 7 atomes de carbone, un groupe phényle di- ou trisubstitué ou hydroxy- ou aminophényle disubstitué par des groupes alcoyle avec 1 à 4 atomes de carbone, des groupes alcoxy avec 1 à 3 atomes de carbone et/ou des atomes d'halogène, les substituants du noyau phényle pouvant dans chaque cas être identiques ou différents, ou représente un groupe pyridyle, caractérisé en ce que

a) pour la préparation d'un composé de formule générale Ia dans laquelle A représente un atome de soufre, un groupe R—N=S—, —SO— ou —$SO_2$—, on fait réagir un composé hydroxy de formule générale

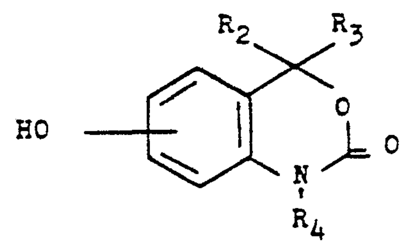

(IIa)

dans laquelle $R_2$ à $R_4$ sont définis comme dans la revendication 1, ou son sel avec une base minérale ou organique tertiaire, avec un composé de formule générale

$$Z—D—A_a—R_{1a} \qquad \text{(IIIa)}$$

dans laquelle

D et Z sont définis comme dans la revendication 1,

$R_{1a}$ possède les significations mentionnées au début et

$A_a$ représente un atome de soufre, un groupe R—N=S—, —SO— ou —$SO_2$—, R étant défini comme dans la revendication 1, ou

b) pour la préparation d'un composé de formule générale Ia dans laquelle A représente un groupe —SO—, —$SO_2$— ou R—N=SO—, on oxyde un composé de formule générale

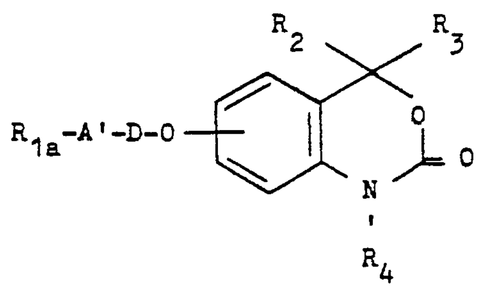

(IVa)

dans laquelle

$R_2$ à $R_4$, A' et D sont définis comme dans la revendication 1, et

$R_{1a}$ possède les significations mentionnées au début ou

c) pour la préparation d'un composé de formule générale Ia dans laquelle A représente un atome de soufre ou un groupe —$SO_2$—, on fait réagir un composé de formule générale

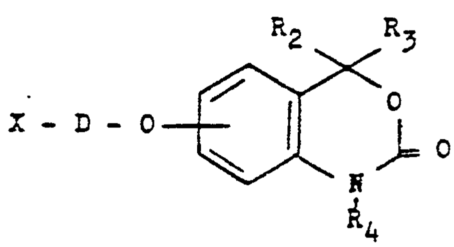

(Va)

dans laquelle $R_2$ à $R_4$, D et X sont définis comme dans la revendication 1, avec un composé de formule générale

$$Y—R_{1a} \qquad (VIa)$$

dans laquelle
Y est défini comme dans la revendication 1 et
$R_{1a}$ est défini comme au début ou
d) pour la préparation d'un composé de formule générale Ia dans laquelle A représente le groupe H—N=SO—, on fait réagir un sulfoxyde de formule générale

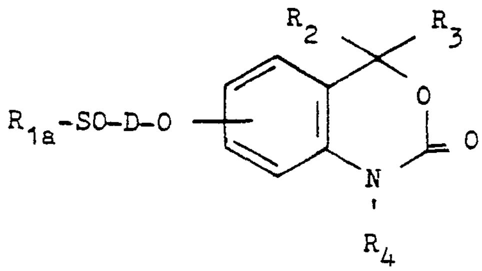
(VIIa)

dans laquelle
$R_2$ à $R_4$ et D sont définis comme dans la revendication 1, et
$R_{1a}$ possède les significations mentionnées au début, avec de l'acide hydrazoïque éventuellement formé dans le mélange réactionnel ou
e) pour la préparation d'un composé de formule générale Ia dans laquelle A représente le groupe H—N=SO—, on fait réagir un sulfoxyde de formule générale

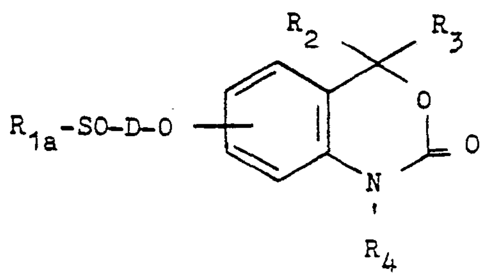
(VIIa)

dans laquelle
$R_2$ à $R_4$ et D sont définis comme dans la revendication 1, et
$R_{1a}$ possède les significations mentionnées au début, avec un composé de formule générale

$$H_2N—O—X—R_7 \qquad (VIII)$$

dans laquelle $R_7$ et X sont définis comme dans la revendication 1 ou
f) pour la préparation d'un composé de formule générale Ia dans laquelle R ne représente pas un atome d'hydrogène, on acyle un composé de formule générale

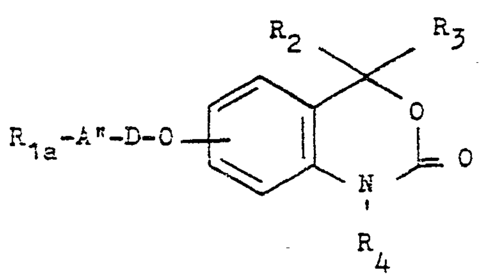
(IXa)

dans laquelle
$R_2$ à $R_4$, A" et D sont définis comme dans la revendication 1 et
$R_{1a}$ possède les significations mentionnées au début ou

g) pour la préparation d'un composé de formule générale Ia dans laquelle A représente le groupe R—N=S—, on fait réagir un thioéther de formule générale

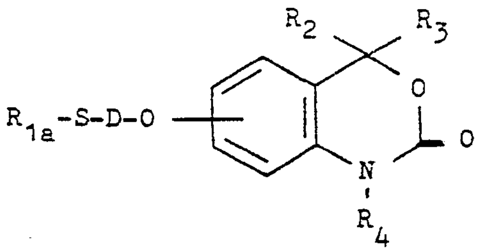

(Xa)

dans laquelle

$R_2$ à $R_4$ et D sont définis comme dans la revendication 1, et

$R_{1a}$ possède les significations mentionnées au début, avec un halogénoamide de formule générale

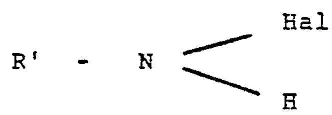

(XI)

dans laquelle R' et Hal sont définis comme dans la revendication 1, ou avec son sel alcalin et on hydrolyse éventuellement ensuite un composé ainsi obtenu, et, si on le désire, on transforme ensuite un composé, obtenu selon l'invention, de formule générale I dans laquelle $R_4$ représente un atome d'hydrogène, au moyen d'une alcoylation, en un composé correspondant de formule générale I dans laquelle $R_4$ représente un groupe alcoyle avec 1 à 3 atomes de carbone.

3. Procédé selon les revendications 1 et 2, caractérisé en ce que la réaction est effectuée dans un solvant.

4. Procédé selon les revendications 1a, 2a et 3, caractérisé en ce que la réaction est effectuée en présence d'une base alcaline ou d'un alcoolate.

5. Procédé selon les revendications 1b, 2b et 3, caractérisé en ce que, pour la préparation d'un composé de formule générale I dans laquelle A représente un groupe —SO— ou R—N=SO—, l'oxydation est effectuée au moyen d'un équivalent de l'agent d'oxydation mis en œuvre.

6. Procédé selon les revendications 1b, 2b et 3, caractérisé en ce que pour la préparation d'un composé de formule générale I dans laquelle A représente un groupe $—SO_2—$, l'oxydation à partir d'un thioéther de formule générale I est effectuée au moyen de deux équivalents ou à partir d'un composé sulfinylé de formule générale I au moyen d'un équivalent de l'agent d'oxydation utilisé.

7. Procédé selon les revendications 1c, 2c, 1g, 2g et 3, caractérisé en ce que la réaction est effectuée en présence d'une base alcaline.

8. Procédé selon les revendications 1e, 2e, 3 et 12, caractérisé en ce que l'on prépare un composé de formule générale VIII dans le mélange réactionnel.

9. Procédé selon les revendications 1f, 2f, et 3, caractérisé en ce que la réaction est effectuée en présence d'une base minérale ou organique tertiaire.

10. Procédé selon les revendications 1h et 3, caractérisé en ce que l'on utilise, en tant que radical acyle, un radical acyle d'un acide carboxylique ou d'un dérivé d'acide carbonique.